# EUROPEAN PATENT SPECIFICATION

(11) **EP 1 202 975 B1**
(45) Date of publication and mention of the grant of the patent: **06.04.2005**
(21) Application number: 00931916.1
(22) Date of filing: 18.05.2000
(51) Int. Cl.: C07D 241/20, C07D 417/12, C07D 401/12, C07D 403/12, C07D 413/12, C07D 409/12, A61K 31/495, A61K 31/497, A61P 7/02

(54) **SUBSTITUTED POLYCYCLIC ARYL AND HETEROARYL PYRAZINONES USEFUL FOR SELECTIVE INHIBITION OF THE COAGULATION CASCADE**
SUBSTITUIERTE POLYZYKLISCHE ARYL UND HETEROARYLPYRAZINONE ZUR SELEKTIVEN HEMMUNG VON DER BLUTGERINNUNGSKASKADE
PYRAZINONES D'HETEROARYLE ET D'ARYLE POLYCYCLIQUE SUBSTITUEES UTILES A L'INHIBITION SELECTIVE DE LA COAGULATION EN CASCADE

(30) Priority: 19.05.1999 US 134958 P
(43) Date of publication of application: 08.05.2002
(73) Proprietor: Pharmacia Corporation, Chicago, IL 60680 (US)
(72) Inventor: SOUTH, Michael, S., St. Louis, MO 63146 (US); PARLOW, John, J., Arnold, MO 63010 (US); JONES, Darin, E., Ballwin, MO 63021 (US); CASE, Brenda, St. Louis, MO 63141 (US); DICE, Tom, St. Charles, MO 63301 (US); LINDMARK, Richard, Korkwood, MO 63122 (US); HAYES, Michael, J., St. Louis, MO 63122 (US); RUEPPEL, Melvin, L., St. Louis, MO 63122 (US); FENTON, Rick, Collinsville, IL 62234 (US); FRANKLIN, Gary, W., Godfrey, IL 62035 (US); HUANG, Horng-Chih, Chesterfield, MO 63017 (US); HUANG, Wei, Wildwood, MO 63021 (US); KUSTURIN, Carrie, Edwardsville, IL 62025 (US); LONG, Scott, A., Ballwin, MO 63021 (US); NEUMANN, William, L., St. Louis, MO 63122 (US); REITZ, David B., Chesterfield, MO 63017 (US); TRUJILLO, John, I., Creve Coeur, MO 63141 (US); WANG, Ching-Cheng, Wildwood, MO 63011 (US); WOOD, Rhonda, Maryland Heights, MO 63043 (US); MAHONEY, Matthew, W., St.Paters, MO 63376 (US); Zeng, Qingping, Ballwin, MO 63021 (US)
(74) Representative: Albrecht, Thomas, Dr.
(86) International application number: PCT/US2000/008225
(87) International publication number: WO 2000/069834

(56) References cited:
- WO-A-97/01338
- WO-A-97/40024
- WO-A-98/42342
- WO-A-99/11267
- WO-A-99/59591
- WO-A-99/61442
- WO-A-99/64446

## Description

### FIELD OF THE INVENTION

This invention is in the field of anticoagulant therapy, and specifically relates to compounds and compositions for preventing and treating thrombotic conditions such as coronary artery and cerebrovascular disease. More particularly, the invention relates to substituted polycyclic aryl and heteroaryl pyrazinone compounds that inhibit serine proteases of the coagulation cascade.

### BACKGROUND OF THE INVENTION

Physiological systems control the fluidity of blood in mammals [Majerus, P. W. et al: Anticoagulant, Thrombolytic, and Antiplplatelet Drugs. In Hardman, J. G. and Limbird, L. E., editors: Goodman & Gilman's The Pharmacological Basis of Therapeutics. 9th edition. New York, McGraw-Hill Book Co., 1996, pp. 1341-1343]. Blood must remain fluid within the vascular systems and yet be able to undergo hemostasis, cessation of blood loss from a damaged vessel, quickly. Hemostasis or clotting begins when platelets first adhere to macromolecules in subendothelian regions of an injured and/or damaged vessels. These platelets aggregate to form the primary hemostatic plug and stimulate local activation of plasma coagulation factors leading to generation of a fibrin clot that reinforces the aggregated platelets.

Plasma coagulation factors include factors II, V, VII, VIII, IX, X, XI, and XII; these are also called protease zymogens. These coagulation factors or protease zymogens are activated by serine proteases leading to coagulation in a so called "coagulation cascade" or chain reaction [Handin, R. I.: Bleeding and Thrombosis. In Wilson, J., et al. editors: Harrison's Principles of Internal Medicine. 12th Edition, New York, McGraw-Hill Book Co., 1991,p.350]. Coagulation or clotting occurs in two ways through different pathways. An intrinsic or contact pathway leads from XII to XIIa to XIa to IXa and to the conversion of X to Xa. Xa with factor Va converts prothrombin (II) to thrombin (IIa) leading to conversion of fibrinogen to fibrin. Polymerization of fibrin leads to a fibrin clot. An extrinsic pathway is initiated by the conversion of coagulation factor VII to VIIa by Xa. The presence of Tissue Factor and VIIa accelerates formation of Xa in the presence of calcium ion and phospholipids. Formation of Xa leads to thrombin, fibrin. and a fibrin clot as described above. The presence of one or more of these many different coagulation factors and two distinct pathways of clotting could enable the efficacious, selective control and better understanding of parts of the coagulation or clotting process.

While clotting as a result of an injury to a blood vessel is a critical physiological process for mammals such as man, clotting can also lead to disease states. A pathological process called thrombosis results when platelet aggregation and/or a fibrin clot blocks (i.e., occludes) a blood vessel. Arterial thrombosis may result in ischemic necrosis of the tissue supplied by the artery. When the thrombosis occurs in a coronary artery, a myocardial infarction or heart attack can result. A thrombosis occurring in a vein may cause tissues drained by the vein to become edematous and inflamed. Thrombosis of a deep vein may be complicated by a pulmonary embolism. Preventing or treating clots in a blood vessel may be therapeutically useful by inhibiting formation of blood platelet aggregates, inhibiting formation of fibrin, inhibiting thrombus formation, inhibiting embolus formation, and for treating or preventing unstable angina, refractory angina, myocardial infarction, transient ischemic attacks, atrial fibrillation, thrombotic stroke, embolic stroke, deep vein thrombosis, disseminated intravascular coagulation, ocular build up of fibrin, and reocclusion or restenosis of recanalized vessels.

There have been several reports of non-peptidic and peptidic compounds that act as an inhibitor of a coagulation factor present in the coagulation cascade or clotting process. In PCT Patent Application WO 97/40024, Sanderson et al. describe alkyl, cycloalkyl, and trifluoromethyl substituted pyrazinones reported to inhibit thrombin activity. In PCT Patent Application WO 98/08840, Duggan et al. describe 2-heterocyclylacetyl derivatives of β-alanine esters reported to inhibit ανβ3 and ανβ5 receptors and possess utility in atheriosclerosis. In PCT Patent Application WO 98/09949, Suzuki et al. describe 2-heterocyclylacetamido derivatives of 1,2-diketones and report that they inhibit proteases, especially chymase inhibitors. In PCT Patent Application WO 98/42342, Isaacs et al. describe additional alkyl, cycloalkyl, and trifluoromethyl substituted pyrazinones reported to inhibit human thrombin. In PCT Patent Application WO 99/61442, Sanderson and Naylor-Olsen describe 1-(5-methylenecarboxamidomethyleneimidazo-[1,2-a]pyridinyl) pyrazinones without substitution in the imidazolyl portion and reported that the compounds inhibit thrombin activity. In PCT Patent Application WO 99/59591, Sanderson *et al*. describe 1-((N-substitutedaminopyridyl and N-substitutedphenyl)amidocarbonylmethylene)pyrazinones reported to inhibit thrombin. In PCT Patent Application WO 99/64446, Lu *et al*. describe 1-((N-amidinoaminooxyalkylene and N-amidinohydrazinoalkylene) amidocarbonylmethylene)pyrazinones reported to inhibit trypsin-like serine proteases and thrombin. In Japanese Patent Application 99/229491, Black *et al*. describe thrombin inhibiting halo and alkyl substituted pyrazinone acetamides in which the amide nitrogen is substituted by a group containing a benzimidazole or indole ring.

### SUMMARY OF THE INVENTION

It is an object of the present invention to provide compounds that are beneficial in anticoagulant therapy and that have a general structure: 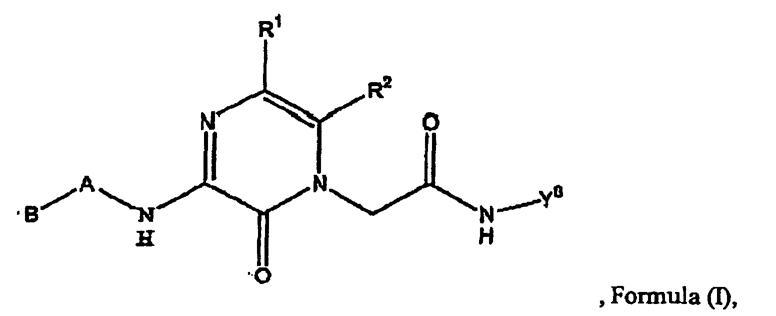 and are useful for
preventing and treating thrombotic conditions, such as coronary artery disease, cerebrovascular disease, and other coagulation related disorders. Such thrombotic conditions are prevented and treated by administering to a patient in need thereof an effective amount of compounds of Formula (I).

Various other objects and advantages of the present invention will become apparent from the following description of the invention.

### DESCRIPTION OF THE INVENTION

The present invention relates to a class of compounds comprising Substituted Polycyclic Aryl and Heteroaryl Pyrazinones, which are beneficial in anticoagulant therapy for the treatment and prevention of a variety of thrombotic conditions including coronary artery and cerebrovascular disease, as given in Formula (I): 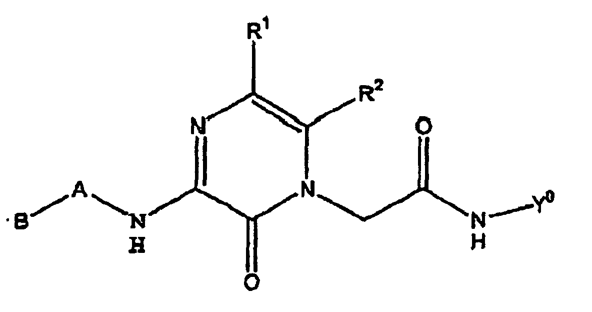 or a pharmaceutically acceptable salt thereof, wherein;
B is selected from the group consisting of:
(i) aryl and heteroaryl wherein a carbon adjacent to the carbon at the point of attachment is optionally substituted by R³², the other carbon adjacent to the carbon at the point of attachment is optionally substituted by R³⁶, a carbon adjacent to R³² and two atoms from the carbon at the point of attachment is optionally substituted by R³³, a carbon adjacent to R³⁶ and two atoms from the carbon at the point of attachment is optionally substituted by R³⁵, and any carbon adjacent to both R³³ and R³⁵ is optionally substituted by R³⁴;
(ii) hydrido, C₂-C₈ alkyl, C₃-C₈ alkenyl, C₃-C₈ alkynyl, and C₂-C₈ haloalkyl, wherein each member of group B is optionally substituted at any carbon up to and including 6 atoms from the point of attachment of B to A with one or more of the group consisting of R³², R³³, R³⁴, R³⁵, and R³⁶; and
(iii) C₃-C₇ cycloalkyl and C₄-C₆ saturated heterocyclyl, wherein each ring carbon is optionally substituted with R³³, a ring carbon other than the ring carbon at the point of attachment of B to A is optionally substituted with oxo provided that no more than one ring carbon is substituted by oxo at the same time, ring carbons and a nitrogen adjacent to the carbon atom at the point of attachment are optionally substituted with R⁹ or R¹³, a ring carbon or nitrogen adjacent to the R⁹ position and two atoms from the point of attachment is optionally substituted with R¹⁰, a ring carbon or nitrogen adjacent to the R¹³ position and two atoms from the point of attachment is optionally substituted with R¹², a ring carbon or nitrogen three atoms from the point of attachment and adjacent to the R¹⁰ position is optionally substituted with R¹¹, a ring carbon or nitrogen three atoms from the point of attachment and adjacent to the R¹² position is optionally substituted with R³³, and a ring carbon or nitrogen four atoms from the point of attachment and adjacent to the R¹¹ and R³³ positions is optionally substituted with R³⁴;
R³², R³³, R³⁴, R³⁵, and R³⁶ are independently selected from the group consisting of hydrido, acetamido, haloacetamido, amidino, guanidino, alkoxy, hydroxy, amino, alkoxyamino, lower alkylamino, alkylthio, amidosulfonyl, monoalkyl amidosulfonyl, dialkyl amidosulfonyl, alkyl, halo, haloalkyl, haloalkoxy, hydroxyalkyl, carboalkoxy, carboxy, carboxamido, and cyano;
A is selected from the group consisting of single covalent bond and (CH(R¹⁵))ₚₐ-(W⁷)ᵣᵣ wherein rr is an integer selected from 0 through 1, pa is an integer selected from 0 through 3, and W⁷ is N(R⁷);
R⁷ is selected from the group consisting of hydrido and alkyl;
R¹⁵ is selected from the group consisting of hydrido, halo, alkyl, and haloalkyl;
R¹ is selected from the group consisting of hydrido, cyano, haloalkyl, and halo;
R² is Z⁰-Q;
Z⁰ is a covalent single bond;
Q is phenyl wherein a carbon two atoms from the carbon at the point of attachment is optionally substituted by amino;
R⁹, R¹¹, and R¹³ are independently selected from the group consisting of hydrido, hydroxy, amino, amidino, guanidino, lower alkylamino, alkylthio, alkoxy, alkylsulfinyl, alkylsulfonyl, amidosulfonyl, monoalkylamidosulfonyl, alkyl, halo, haloalkyl, haloalkoxy, hydroxyalkyl, carboxy, carboxamido, and cyano;
R¹⁰ and R¹² are independently selected from the group consisting of hydrido, acetamido, haloacetamido, amidino, guanidino, alkyl, alkoxy, alkoxyamino, aminoalkyl, hydroxy, amino, lower alkylamino, alkylsulfonamido, amidosulfonyl, monoalkyl amidosulfonyl, dialkyl amidosulfonyl, hydroxyalkyl, aminoalkyl, halo, haloalkyl, carboalkoxy, carboxy, carboxyamido, carboxyalkyl, and cyano;
Y⁰ is formula (IV): 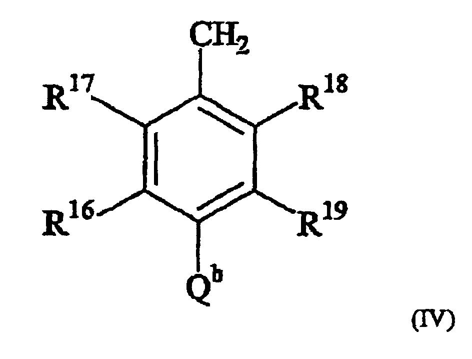 wherein
R¹⁶, R¹⁷, R¹⁸, and R¹⁹ are independently selected from the group consisting of hydrido, amidino, guanidino, carboxy, haloalkylthio, alkoxy, hydroxy, amino, lower alkylamino, alkylthio, alkylsulfinyl, alkylsulfonyl, alkanoyl, haloalkanoyl, alkyl, halo, haloalkyl, haloalkoxy, hydroxyalkyl, aminoalkyl, and cyano;
Q^{b} is C(NR²⁵)NR²³R²⁴;
R²³, R²⁴, and R²⁵ are hydrido;
and wherein
the term "alkyl", either alone or within other terms, unless indicated otherwise, means an acyclic alkyl radical containing from one to ten carbon atoms;
the term "alkoxy" means a linear or branched oxy-containing radical having an alkyl portion of one to ten carbon atoms;
the term "aryl" alone or in combination, means a carbocyclic aromatic system containing one, two or three rings wherein such rings may be attached together in a pendant manner or may be fused;
the term "heterocyclyl" means saturated and partially saturated heteroatom-containing ring-shaped radicals having from 4 through 15 ring members selected from carbon, nitrogen, sulfur and oxygen, wherein at least one ring atom is a heteroatom;
the term "beteroaryl" means fully unsaturated heteroatom-containing ring-shaped aromatic radicals having from 4 through 15 ring members selected from carbon, nitrogen, sulfur and oxygen, wherein at least one ring atom is a heteroatom.

In an embodiment of compounds of Formula I or a pharmaceutically acceptable salt thereof,
B is selected from the group consisting of phenyl, 2-thienyl, 3-thienyl, 2-furyl, 3-furyl, 2-pyrrolyl, 3-pyrrolyl, 2-imidazolyl, 4-imidazolyl, 3-pyrazolyl, 4-pyrazolyl, 2-thiazolyl, 3-isoxazolyl, and 5-isoxazolyl, wherein a carbon adjacent to the carbon at the point of attachment is optionally substituted by R³², the other carbon adjacent to the carbon at the point of attachment is optionally substituted by R³⁶, a carbon adjacent to R³² and two atoms from the carbon at the point of attachment is optionally substituted by R³³, a carbon adjacent to R³⁶ and two atoms from the carbon at the point of attachment is optionally substituted by R³⁵, and any carbon adjacent to both R³³ and R³⁵ is optionally substituted by R³⁴;
R³², R³³, R³⁴, R³⁵, and R³⁶ are independently selected from the group consisting of hydrido, amidino, guanidino, methyl, ethyl, methoxy, ethoxy, hydroxy, amino, N-methylamino, dimethylamino, methylthio, ethylthio, trifluoromethyl, pentafluoroethyl, 2,2,2-trifluoroethyl, fluoro, chloro, bromo, amidosulfonyl, N-methylamidosulfonyl, hydroxymethyl, amidocarbonyl, carboxy, and cyano;
A is selected from the group consisting of single covalent bond, NH, N(CH₃), CH₂, CH₃CH, and CH₂CH₂;
R¹ is selected from the group consisting of hydrido, trifluoromethyl, pentafluoroethyl, fluoro, and chloro;
R⁹, R¹¹, and R¹³ are independently selected from the group consisting of hydrido, methyl, ethyl, methoxy, ethoxy, hydroxy, amino, N-methylamino, N,N-dimethylamino, methylthio, trifluoromethyl, pentafluoroethyl, 2,2,2-trifluoroethyl, fluoro, chloro, bromo, amidosulfonyl, N-methylamidosulfonyl, N,N-dimethylamidosulfonyl, hydroxymethyl, 1-hydroxyethyl, amidocarbonyl, N-methylamidocarbonyl, carboxy, and cyano;
R¹⁰ and R¹² are independently selected from the group consisting of hydrido, amidino, amidocarbonyl, N-methylamidocarbonyl, guanidino, methyl, ethyl, methoxy, ethoxy, hydroxy, hydroxymethyl, 1-hydroxyethyl, 2-hydroxyethyl, carboxy, carboxymethyl, amino, acetamido, trifluoromethyl, pentafluoroethyl, 2,2,2-trifluoroethyl, trifluoroacetamido, aminomethyl, N-methylamino, dimethylamino, amidosulfonyl, N-methylamidosulfonyl, N,N-dimethylamidosulfonyl, methoxycarbonyl, fluoro, chloro, bromo, and cyano;
Y⁰ is 1-Q^{b}-4-Q^{s}-2-R¹⁶-3-R¹⁷-5-R¹⁸-6-R¹⁹benzene;
R¹⁶, R¹⁷, R¹⁸, and R¹⁹ are independently selected from the group consisting of hydrido, methyl, ethyl, amidino, guanidino, methoxy, hydroxy, amino, aminomethyl, 1-aminoethyl, 2-aminoethyl, N-methylamino, dimethylamino, methylthio, ethylthio, trifluoromethylthio, methylsulfinyl, methylsulfonyl, trifluoromethyl, pentafluoroethyl, 2,2,2-trifluoroethyl, trifluoromethoxy, fluoro, chloro, amidosulfonyl, N-methylamidosulfonyl, hydroxymethyl, carboxy, and cyano.

In another embodiment of compounds of Formula I or a pharmaceutically acceptable salt thereof,
B is selected from the group consisting of 2-aminophenyl, 3-aminophenyl, 3-amidinophenyl, 4-amidinophenyl, 3-carboxyphenyl, 3-carboxy-5-hydroxyphenyl, 3-chlorophenyl, 4-chlorophenyl, 3,4-dichlorophenyl, 2-fluorophenyl, 3-fluorophenyl, 3,4-difluorophenyl, 3-hydroxyphenyl, 4-hydroxyphenyl, 3-methoxyaminophenyl, 3-methoxyphenyl, 4-methoxyphenyl, 3-methylphenyl, 4-methylphenyl, phenyl, 3-trifluoromethylphenyl, 2-imidazoyl, 2-pyridyl, 3-pyridyl, 5-chloro-3-trifluoromethyl-2-pyridyl, 4-pyridyl, 2-thienyl, 3-thienyl, and 3-trifluoromethyl-2-pyridyl;
A is selected from the group consisting of CH₂, CH₃CH, CF₃CH, NHC(O), CH₂CH₂,and CH₂CH₂CH₂;
R¹ is selected from the group consisting of hydrido, trifluoromethyl, pentafluoroethyl, fluoro, and chloro;
Y⁰ is 1-Q^{b}-4-Q^{s}-2-R¹⁶-3-R¹⁷-5-R¹⁸-6-R¹⁹benzene;
R¹⁶ and R¹⁹ are independently selected from the group consisting of:
(i) hydrido, amidino, amino, aminomethyl, methoxy, methylamino, hydroxy, hydroxymethyl, fluoro, chloro, and cyano; and
(ii) Q^{b} with the proviso that no more than one of R¹⁶ and R¹⁹ is Q^{b} at the same time;
R¹⁷ and R¹⁸ are independently selected from the group consisting of hydrido, fluoro, chloro, hydroxy, hydroxymethyl, amino, carboxy, and cyano.

In a preferred embodiment of compounds of Formula I or a pharmaceutically acceptable salt thereof,
B is selected from the group consisting of 3-aminophenyl, 3-amidinophenyl, 4-amidinophenyl, 3-chlorophenyl, 4-chlorophenyl, 3,4-dichlorophenyl, 2-fluorophenyl, 4-methylphenyl, phenyl, 2-imidazoyl, 3-pyridyl, 4-pyridyl, and 3-trifluoromethyl-2-pyridyl;
A is selected from the group consisting of CH₂, NHC(O), CH₂CH₂,and CH₂CH₂CH₂;
R¹ is selected from the group consisting of hydrido and chloro;
Y⁰ is selected from the group consisting of 4-amidinobenzyl, 2-fluoro-4-amidinobenzyl, and 3-fluoro-4-amdinobenzyl.

In a more preferred embodiment of compounds of Formula I or a pharmaceutically acceptable salt thereof,
R² is 3-aminophenyl, B is 3-chlorophenyl, A is CH₂CH₂, Y⁰ is 4-amidinobenzyl, and R¹ is chloro;
R² is 3-aminophenyl, B is phenyl, A is CH₂, Y⁰ is 4-amidinobenzyl, and R¹ is chloro;
R² is 3-aminophenyl, B is 2-imidazoyl, A is CH₂CH₂CH₂, Y⁰ is 4-amidinobenzyl, and R¹ is chloro.

In another more preferred embodiment of compounds of Formula I or a pharmaceutically acceptable salt thereof,
B is selected from the group consisting of hydrido, ethyl, 2-propenyl, 2-propynyl, propyl, isopropyl, butyl, 2-butenyl, 2-butynyl, see-butyl, *tert*-butyl, isobutyl, 2-methylpropenyl, 1-pentyl, 2-pentenyl, 3-pentenyl, 2-pentynyl, 3-pentynyl, 2-pentyl, 3-pentyl, 2-methylbutyl, 2-methyl-2-butenyl, 3-methylbutyl, 3-methyl-2-butenyl, 1-hexyl, 2-hexenyl, 3-hexenyl, 4-hexenyl, 2-hexynyl, 3-hexynyl, 4-hexynyl, 2-hexyl, 1-methyl-2-pentenyl, 1-methyl-3-pentenyl, 1-methyl-2-pentynyl, 1-methyl-3-pentynyl, 3-hexyl, 1-ethyl-2-butenyl, 1-heptyl, 2-heptenyl, 3-heptenyl, 4-heptenyl, 5-heptenyl, 2-heptynyl, 3-heptynyl, 4-heptynyl, 5-heptynyl, 2-heptyl, 1-methyl-2-hexenyl, 1-methyl-3-hexenyl, 1-methyl-4-hexenyl, 1-methyl-2-hexynyl, 1-methyl-3-hexynyl, 1-methyl-4-hexynyl, 3-heptyl, 1-ethyl-2-pentenyl, 1-ethyl-3-pentenyl, 1-ethyl-2-pentynyl, 1-ethyl-3-pentynyl, 2,2,2-trifluoroethyl, 2,2-difluoropropyl, 4-trifluoromethyl-5,5,5-trifluoropentyl, 4-trifluoromethylpentyl, 5,5,6,6,6-pentafluorohexyl, and 3,3,3-trifluoropropyl, wherein each member of group B is optionally substituted at any carbon up to and including 5 atoms from the point of attachment of B to A with one or more of the group consisting of R³², R³³, R³⁴, R³⁵, and R³⁶;
R³², R³³, R³⁴, R³⁵, and R³⁶ are independently selected from the group consisting of hydrido, amidino, guanidino, methyl, ethyl, methoxy, ethoxy, hydroxy, amino, N-methylamino, dimethylamino, methylthio, ethylthio, trifluoromethyl, pentafluoroethyl, 2,2,2-trifluoroethyl, fluoro, chloro, bromo, amidosulfonyl, N-methylamidosulfonyl, hydroxymethyl, amidocarbonyl, carboxy, and cyano;
A is selected from the group consisting of:
(i) a single covalent bond, NH, N(CH₃), CH₂, CH₃CH, and CH₂CH₂; and
(ii) CH₂N(CH₃), CH₂N(CH₂CH₃), CH₂CH₂N(CH₃), and CH₂CH₂N(CH₂CH₃) with the proviso that B is hydrido;
R¹ is selected from the group consisting of hydrido, hifluoromethyl, pentafluoroethyl, fluoro, and chloro;
R⁹, R¹¹, and R¹³ are independently selected from the group consisting of hydrido, methyl, ethyl, methoxy, ethoxy, hydroxy, amino, N-methylamino, N,N-dimethylamino, methylthio, trifluoromethyl, pentafluoroethyl, 2,2,2-trifluoroethyl, fluoro, chloro, bromo, amidosulfonyl, N-methylamidosulfonyl, N,N-dimethylamidosulfonyl, hydroxymethyl, 1-hydroxyethyl, amidocarbonyl, N-methylamidocarbonyl, carboxy, and cyano;
R¹⁰ and R¹² are independently selected from the group consisting of hydrido, amidino, amidocarbonyl, N-methylamidocarbonyl, guanidino, methyl, ethyl, methoxy, ethoxy, hydroxy, hydroxymethyl, 1-hydroxyethyl, 2-hydroxyethyl, carboxy, carboxymethyl, amino, acetamido, trifluoromethyl, pentafluoroethyl, 2,2,2-trifluoroethyl, trifluoroacetamido, aminomethyl, N-methylamino, dimethylamino, amidosulfonyl, N-methylamidosulfonyl, N,N-dimethylamidosulfonyl, methoxycarbonyl, fluoro, chloro, bromo, and cyano;
Y⁰ is 1-Q^{b}-4-Q^{s}-2-R¹⁶-3-R¹⁷-5-R¹⁸-6-R¹⁹benzene;
R¹⁶, R¹⁷, R¹⁸, and R¹⁹ are independently selected from the group consisting of hydrido, methyl, ethyl, amidino, guanidino, methoxy, hydroxy, amino, aminomethyl, 1-aminoethyl, 2-aminoethyl, N-methylamino, dimethylamino, methylthio, ethylthio, trifluoromethylthio, methylsulfinyl, methylsulfonyl, trifluoromethyl, pentafluoroethyl, 2,2,2-trifluoroethyl, trifluoromethoxy, fluoro, chloro, amidosulfonyl, N-methylamidosulfonyl, hydroxymethyl, carboxy, and cyano.

In still another more preferred embodiment of compounds of Formula I or a pharmaceutically acceptable salt thereof,
B is selected from the group consisting of hydrido, ethyl, 2-propenyl, 2-propynyl, propyl, isopropyl, butyl, 2-butyl, (R)-2-butyl,(S)-2-butyl, *tert*-butyl, isobutyl, 1-pentyl, 3-pentyl, 2-methylbutyl, 2,2,2-trifluoroethyl, 6-amidocarbonylhexyl, 4-methyl-2-pentyl, 3-hydroxypropyl, 3-methoxy-2-propyl, 2-methoxyethyl, 2-methyl-2-butyl, 3-methyl-2-butyl, 2-dimethylaminopropyl, 2-cyanoethyl, 6-hydroxyhexyl, 2-hydroxyethyl, 2-amidinoethyl, 2-guanidinoethyl, 3-guanidinopropyl, 4-guanidinobutyl, 3-hydroxypropyl, 4-hydroxybutyl, 6-cyanohexyl, 2-dimethylaminoethyl, 3-methylbutyl, 2-methylbutyl, (S)-2-methylbutyl, 3-aminopropyl, 2-hexyl, and 4-aminobutyl;
A is selected from the group consisting of single covalent bond, CH₂, CH₃CH, and CH₂CH₂;
R¹ is selected from the group consisting of hydrido, trifluoromethyl, fluoro, and chloro;
Y⁰ is 1-Q^{b}-4-Q^{s}-2-R¹⁶-3-R¹⁷-5-R¹⁸-6-R¹⁹benzene;
R¹⁶ and R¹⁹ are independently selected from the group consisting of:
(i) hydrido, amidino, amino, aminomethyl, methoxy, methylamino, hydroxy, hydroxymethyl, fluoro, chloro, and cyano; and
(ii) Q^{b} with the proviso that no more than one of R¹⁶ and R¹⁹ is Q^{b} at the same time;
R¹⁷ and R¹⁸ are independently selected from the group consisting of hydrido, fluoro, chloro, hydroxy, hydroxymethyl, amino, carboxy, and cyano.

In a preferred embodiment of compounds of Formula I or a pharmaceutically acceptable salt thereof,
B is selected from the group consisting of hydride, ethyl, 2-propenyl, 2-propynyl, propyl, isopropyl, butyl, 2-butyl, (R)-2-butyl,(S)-2-butyl, *tert*-butyl, isobutyl, 1-pentyl, 3-pentyl, 2-methylbutyl, 2,2,2-trifluoroethyl, 6-amidocarbonylhexyl, 4-methyl-2-pentyl, 3-hydroxypropyl, 3-methoxy-2-propyl, 2-methoxyethyl, 2-methyl-2-butyl, 3-methyl-2-butyl, 2-dimethylaminopropyl, 2-cyanoethyl, 6-hydroxyhexyl, 2-hydroxyethyl, 2-amidinoethyl, 2-guanidinoethyl, 3-guanidinopropyl, 4-guanidinobutyl, 3-hydroxypropyl, 4-hydroxybutyl, 6-cyanohexyl, 2-dimethylaminoethyl, 3-methylbutyl, 2-methylbutyl, (S)-2-methylbutyl, 3-aminopropyl, 2-hexyl, and 4-aminobutyl;
A is selected from the group consisting of single covalent bond, CH₂, CH₃CH, and CH₂CH₂;
R¹ is selected from the group consisting of hydrido and chloro;
Y⁰ is selected from the group consisting of 4-amidinobenzyl, 2-fluoro-4-amidinobenzyl, and 3-fluoro-4-amidinobenzyl.

In another preferred embodiment of compounds of Formula I or a pharmaceutically acceptable salt thereof,
R² is 3-aminophenyl, B is 2,2,2-trifluoroethyl, A is single bond, Y⁰ is 4-amidinobenzyl, and R¹ is chloro;
R² is 3-aminophenyl, B is (S)-2-butyl, A is single bond, Y⁰ is 4-amidinobenzyl, and R¹ is chloro;
R² is 3-aminophenyl, B is ethyl, A is single bond, Y⁰ is 4-amidinobenzyl, and R¹ is chloro;
R² is 3-aminophenyl, B is ethyl, A is single bond, Y⁰ is 4-amidino-2-fluorobenzyl, and R¹ is chloro;
R² is 3-aminophenyl, B is 2-propenyl, A is single bond, Y⁰ is 4-amidinobenzyl, and R¹ is chloro;
R² is 3-aminophenyl, B is isopropyl, A is single bond, Y⁰ is 4-amidino-2-fluorobenzyl, and R¹ is chloro;
R² is 3-aminophenyl, B is isopropyl, A is single bond, Y⁰ is 4-amidinobenzyl, and R¹ is chloro;
R² is 3-aminophenyl, B is 2-butyl, A is single bond, Y⁰ is 4-amidinobenzyl, and R¹ is chloro;
R² is 3-aminophenyl, B is (R)-2-butyl, A is single bond, Y⁰ is 4-amidinobenzyl, and R¹ is chloro;
R² is 3-aminophenyl, B is 2-propynyl, A is single bond, Y⁰ is 4-amidinobenzyl, and R¹ is chloro;
R² is 3-aminophenyl, B is 3-pentyl, A is single bond, Y⁰ is 4-amidinobenzyl, and R¹ is hydrido;
R² is 3-aminophenyl, B is hydride, A is CH₂, Y⁰ is 4-amidinobenzyl, and R¹ is chloro;
R² is 3-aminophenyl, B is ethyl, A is CH₂, Y⁰ is 4-amidinobenzyl, and R¹ is chloro;
R² is 3-aminophenyl, B is 2-methylpropyl, A is single bond, Y⁰ is 4-amidinobenzyl, and R¹ is chloro;
R² is 3-aminophenyl, B is 2-propyl, A is CH₃CH, Y⁰ is 4-amidinobenzyl, and R¹ is chloro;
R² is 3-aminophenyl, B is propyl, A is single bond, Y⁰ is 4-amidino-2-fluorobenzyl, and R¹ is chloro;
R² is 3-aminophenyl, B is 6-amidocarbonylhexyl, A is single bond, Y⁰ is 4-amidinobenzyl, and R¹ is chloro;
R² is 3-aminophenyl, B is tert-butyl, A is single bond, Y⁰ is 4-amidinobenzyl, and R¹ is hydrido;
R² is 3-aminophenyl, B is tert-butyl, A is single bond, Y⁰ is 4-amidinobenzyl, and R¹ is chloro;
R² is 3-aminophenyl, B is 3-hydroxypropyl, A is single bond, Y⁰ is 4-amidinobenzyl, and R¹ is chloro;
R² is 3-aminophenyl, B is 2-methylpropyl, A is single bond, Y⁰ is 4-amidino-2-fluorobenzyl, and R¹ is chloro;
R² is 3-aminophenyl, B is butyl, A is single bond, Y⁰ is 4-amidinobenzyl, and R¹ is chloro;
R² is 3-aminophenyl, B is 3-methoxy-2-propyl, A is single bond, Y⁰ is 4-amidinobenzyl, and R¹ is chloro;
R² is 3-aminophenyl, B is 3-methoxy-2-propyl, A is single bond, Y⁰ is 4-amidinobenzyl, and R¹ is chloro;
R² is 3-aminophenyl, B is 2-methoxy-2-ethyl, A is single bond, Y⁰ is 4-amidinobenzyl, and R¹ is chloro;
R² is 3-aminophenyl, B is 2-propyl, A is single bond, Y⁰ is 4-amidino-3-fluorobenzyl, and R¹ is hydrido;
R² is 3-aminophenyl, B is 2-propyl, A is single bond, Y⁰ is 4-amidino-3-fluorobenzyl, and R¹ is chloro.

In still another preferred embodiment of compounds of Formula I or a pharmaceutically acceptable salt thereof,
B is selected from the group consisting of cyclopropyl, cyclobutyl, cyclopentyl, cyclohexyl, cycloheptyl, oxalan-2-yl, 2-(2R)-bicyclo[2.2.1]-heptyl, 1,1-dioxothiolan-3-yl, oxetan-3-yl, azetidin-1-yl, azetidin-2-yl, azetidin-3-yl, bicyclo[3.1.0]hexan-6-yl, 2-morpholinyl, 3-morpholinyl, 4-morpholinyl, 1-piperazinyl, 2-piperazinyl, 1-piperidinyl, 2-piperidinyl, 3-piperidinyl, 4-piperidinyl, 1-pyrrolidinyl, 2-pyrrolidinyl, 3-pyrrolidinyl, 2-dioxanyl, 2-tetrahydrofuranyl, 3-tetrahydrofuranyl, 2-tetrahydropyranyl, 3-tetrahydropyranyl, 4-tetrahydropyranyl, 2-tetrahydrothienyl, and 3-tetrahydrothienyl, wherein each ring carbon is optionally substituted with R³³, ring carbons and a nitrogen adjacent to the carbon atom at the point of attachment are optionally substituted with R⁹ or R¹³, a ring carbon or nitrogen adjacent to the R⁹ position and two atoms from the point of attachment are optionally substituted with R¹⁰, and a ring carbon or nitrogen atom adjacent to the R¹³ position and two atoms from the point of attachment is optionally substituted with R¹²;
R⁹, R¹¹, and R¹³ are independently selected from the group consisting of hydrido, methyl, ethyl, methoxy, ethoxy, hydroxy, amino, N-methylamino, N,N-dimethylamino, methylthio, trifluoromethyl, pentafluoroethyl, 2,2,2-trifluoroethyl, fluoro, chloro, bromo, amidosulfonyl, N-methylamidosulfonyl, N,N-dimethylamidosulfonyl, hydroxymethyl, 1-hydroxyethyl, amidocarbonyl, N-methylamidocarbonyl, carboxy, and cyano;
R¹⁰ and R¹² are independently selected from the group consisting of hydrido, amidino, amidocarbonyl, N-methylamidocarbonyl, guanidino, methyl, ethyl, methoxy, ethoxy, hydroxy, hydroxymethyl, 1-hydroxyethyl, 2-hydroxyethyl, carboxy, carboxymethyl, amino, acetamido, trifluoromethyl, pentafluoroethyl, 2,2,2-trifluoroethyl, trifluoroacetamido, aminomethyl, N-methylamino, dimethylamino, amidosulfonyl, N-methylamidosulfonyl, N,N-dimethylamidosulfonyl, methoxycarbonyl, fluoro, chloro, bromo, and cyano;
R³³ is selected from the group consisting of hydrido, amidino, guanidino, methyl, ethyl, methoxy, ethoxy, hydroxy, carboxy, amino, N-methylamino, dimethylamino, methylthio, ethylthio, trifluoromethyl, pentafluoroethyl, 2,2,2-trifluoroethyl, fluoro, chloro, bromo, amidosulfonyl, N-methylamidosulfonyl, hydroxymethyl, amidocarbonyl, and cyano;
A is selected from the group consisting of single covalent bond, NH, N(CH₃), CH₂, CH₃CH, CH₂CH₂, and CH₂CH₂CH₂;
R¹ is selected from the group consisting of hydrido, trifluoromethyl, pentafluoroethyl, fluoro, and chloro;
Y⁰ is 1-Q^{b}-4-Q^{s}-2-R¹⁶-3-R¹⁷-5-R¹⁸-6-R¹⁹benzene;
R¹⁶, R¹⁷, R¹⁸, and R¹⁹ are independently selected from the group consisting of hydrido, methyl, ethyl, amidino, guanidino, methoxy, hydroxy, amino, aminomethyl, 1-aminoethyl, 2-aminoethyl, N-methylamino, dimethylamino, methylthio, ethylthio, trifluoromethylthio, methylsulfinyl, methylsulfonyl, trifluoromethyl, pentafluoroethyl, 2,2,2-trifluoroethyl, trifluoromethoxy, fluoro, chloro, amidosulfonyl, N-methylamidosulfonyl, hydroxymethyl, carboxy, and cyano.

In a preferred specific embodiment of Formula I,
B is selected from the group consisting of cyclopropyl, cyclobutyl, cyclopentyl, cyclohexyl, oxalan-2-yl, 2-(2R)-bicyclo[2.2.1]-heptyl, 1,1-dioxothiolan-3-yl, oxetan-3-yl, azetidin-1-yl, azetidin-2-yl, azetidin-3-yl, 1-pyrrolidinyl and 1-piperidinyl;
A is selected from the group consisting of a single covalent bond, CH₂, NHC(O), CH₂CH₂ and CH₂CH₂CH₂;
R¹ is selected from the group consisting of hydrido, trifluoromethyl, fluoro, and chloro;
Y⁰ is 1-Q^{b}-4-Q^{s}-2-R¹⁶-3-R¹⁷-5-R¹⁸-6-R¹⁹benzene;
R¹⁶ and R¹⁹ are independently selected from the group consisting of:
(i) hydrido, amidino, amino, aminomethyl, methoxy, methylamino, hydroxy, hydroxymethyl, fluoro, chloro, and cyano;
(ii) Q^{b} with the proviso that no more than one of R¹⁶ is Q^{b} at the same time;
R¹⁷ and R¹⁸ are independently selected from the group consisting of hydrido, fluoro, chloro, hydroxy, hydroxymethyl, amino, carboxy, and cyano.

In a more preferred specific embodiment of Formula I,
B is selected from the group consisting of cyclopropyl, cyclobutyl, cyclopentyl, cyclohexyl, oxalan-2-yl, 2-(2R)-bicyclo[2.2.1]-heptyl, 1,1-dioxothiolan-3-yl, oxetan-3-yl, azetidin-1-yl, azetidin-2-yl, azetidin-3-yl, and1-piperidinyl;
A is selected from the group consisting of a single covalent bond, CH₂, CH₂CH₂ and CH₂CH₂CH₂;
R¹ is selected from the group consisting of hydrido and chloro;
Y⁰ is selected from the group consisting of 4-amidinobenzyl, 2-fluoro-4-amidinobenzyl, and 3-fluoro-4-amdinobenzyl.

In another more preferred specific embodiment of Formula I,
R² is 3-aminophenyl, B is cyclopropyl, A is single bond, Y⁰ is 4-amidinobenzyl, and R¹ is chloro;
R² is 3-aminophenyl, B is cyclobutyl, A is single bond, Y⁰ is 4-amidino-2-fluorobenzyl, and R¹ is chloro;
R² is 3-aminophenyl, B is cyclobutyl, A is single bond, Y⁰ is 4-amidinobenzyl, and R¹ is chloro;
R² is 3-aminophenyl, B is cyclopropyl, A is single bond, Y⁰ is 4-amidino-2-fluorobenzyl, and R¹ is chloro;
R² is 3-aminophenyl, B is cyclobutyl, A is single bond, Y⁰ is 4-amidinobenzyl, and R¹ is hydrido;
R² is 3-aminophenyl, B is cyclobutyl, A is single bond, Y⁰ is 4-amidino-3-fluorobenzyl, and R¹ is chloro;
R² is 3-aminophenyl, B is cyclopentyl, A is single bond, Y⁰ is 4-amidinobenzyl, and R¹ is chloro;
R² is 3-aminophenyl, B is cyclopropyl, A is CH₂, Y⁰ is 4-amidinobenzyl, and R¹ is chloro;
R² is 3-aminophenyl, B is 2-(2R)-bicyclo[2.2.1]-heptyl, A is single bond, Y⁰ is 4-amidinobenzyl, and R¹ is chloro;
R² is 3-aminophenyl, B is cyclopentyl, A is single bond, Y⁰ is 4-amidino-2-fluorobenzyl, and R¹ is chloro;
R² is 3-aminophenyl, B is cyclohexyl, A is CH₂CH₂, Y⁰ is 4-amidinobenzyl, and R¹ is hydrido;
R² is 3-aminophenyl, B is oxalan-2-yl, A is CH₂, Y⁰ is 4-amidinobenzyl, and R¹ is chloro;
R² is 3-aminophenyl, B is 1-piperidinyl, A is CH₂CH₂, Y⁰ is 4-amidinobenzyl, and R¹ is chloro;
R² is 3-aminophenyl, B is 1,1-dioxothiolan-3-yl, A is single bond, Y⁰ is 4-amidinobenzyl, and R¹ is chloro;
R² is 3-aminophenyl, B is 1-pyrrolidinyl, A is CH₂CH₂CH₂, Y⁰ is 4-amidinobenzyl, and R¹ is chloro.

The compounds of this invention can be used in anticoagulant therapy for the treatment and prevention of a variety of thrombotic conditions including coronary artery and cerebrovascular disease. The compounds of this invention can be used to inhibit serine protease associated with the coagulation cascade and factors II, VII, VIII, IX, X, XI, or XII. The compounds of the invention can inhibit the formation of blood platelet aggregates, inhibit the formation of fibrin, inhibit thrombus formation, and inhibiting embolus formation in a mammal, in blood, in blood products, and in mammalian organs. The compounds also can be used for treating or preventing unstable angina, refractory angina, myocardial infarction, transient ischemic attacks, atrial fibrillation, thrombotic stroke, embolic stroke, deep vein thrombosis, disseminated intravascular coagulation, ocular build up of fibrin, and reocclusion or restenosis of recanalized vessels in a mammal. The compounds can also be used in prophylactic treatment of subjects who are at risk of developing such disorders. The compounds can be used to lower the risk of atherosclerosis. The compounds of Formula (I) would also be useful in prevention of cerebral vascular accident (CVA) or stroke.

Besides being useful for human treatment, these compounds are also useful for veterinary treatment of companion animals, exotic animals and farm animals, including mammals, rodents, and the like. More preferred animals include horses, dogs, and cats.

In yet another embodiment of the present invention, the novel compounds are selected from the compounds set forth in Examples 1 through Example 109 and Tables I through Table 7.

The use of generic terms in the description of the compounds are herein defined for clarity.

Standard single letter elemental symbols are used to represent specific types of atoms unless otherwise defined. The symbol "C" represents a carbon atom.
The symbol "O" represents an oxygen atom. The symbol "N" represents a nitrogen atom. The symbol "P" represents a phosphorus atom. The symbol ''S" represents a sulfur atom. The symbol "H" represents a hydrido atom. Double letter elemental symbols are used as defined for the elements of the periodical table (i.e., Cl represents chlorine, Se represents selenium, etc.).

As utilized herein, the term "alkyl", either alone or within other terms such as "haloalkyl" and "alkylthio", means an acyclic alkyl radical containing from I to 10, preferably from 3 to 8 carbon atoms and more preferably 3 to 6 carbon atoms. Said alkyl radicals may be optionally substituted with groups as defined below. Examples of such radicals include methyl, ethyl, chloroethyl, hydroxyethyl, n-propyl, oxopropyl, isopropyl, n-butyl, cyanobutyl, isobutyl, sec-butyl, tert-butyl, pentyl, aminopentyl, iso-amyl, hexyl, octyl and the like.

The term "alkenyl" refers to an unsaturated, acyclic hydrocarbon radical in so much as it contains at least one double bond. Such alkenyl radicals contain from 2 to 10 carbon atoms, preferably from 3 to 8 carbon atoms and more preferably 3 to 6 carbon atoms. Said alkenyl radicals may be optionally substituted with groups as defined below. Examples of suitable alkenyl radicals include propenyl, 2-chloropropenyl, buten-1-yl, isobutenyl, penten-1-yl, 2-2-methylbuten-1-yl, 3-methylbuten-1-yl, hexen-1-yl, 3-hydroxyhexen-1-yl, hepten-1-yl, and octen-1-yl, and the like.

The term "alkynyl" refers to an unsaturated, acyclic hydrocarbon radical in so much as it contains one or more triple bonds, such radicals containing
2 to 10 carbon atoms, preferably having from 3 to 8 carbon atoms and more preferably having 3 to 6 carbon atoms. Said alkynyl radicals may be optionally substituted with groups as defined below. Examples of suitable alkynyl radicals include ethynyl, propynyl, hydroxypropynyl, butyn-1-yl, butyn-2-yl, pentyn-1-yl, pentyn-2-yl, 4-methoxypentyn-2-yl, 3-methylbutyn-1-yl, hexyn-1-yl, hexyn-2-yl, hexyn-3-yl, 3,3-dimethylbutyn-1-yl radicals and the like.

The term "bydrido" denotes a single hydrogen atom (H). This hydrido radical may be attached, for example, to an oxygen atom to form a "hydroxyl" radical, one hydrido radical may be attached to a carbon atom to form a "methine" radical -CH=, or two hydrido radicals may be attached to a carbon atom to form a "methylene" (-CH₂-) radical.

The term "carbon" radical denotes a carbon atom without any covalent bonds and capable of forming four covalent bonds.

The term "cyano" radical denotes a carbon radical having three of four covalent bonds shared by a nitrogen atom.

The term "hydroxyalkyl" embraces radicals wherein any one or more of the alkyl carbon atoms is substituted with a hydroxyl as defined above. Specifically embraced are monohydroxyalkyl, dihydroxyalkyl and polyhydroxyalkyl radicals.

The term "alkanoyl" embraces radicals wherein one or more of the terminal alkyl carbon atoms are substituted with one or more carbonyl radicals as defined below. Specifically embraced are monocarbonylalkyl and dicarbonylalkyl radicals. Examples of monocarbonylalkyl radicals include formyl, acetyl, and pentanoyl. Examples of dicarbonylalkyl radicals include oxalyl, malonyl, and succinyl.

The term "alkylene" radical denotes linear or branched radicals having from 1 to 10 carbon atoms and having attachment points for two or more covalent bonds. Examples of such radicals are methylene, ethylene, methylethylene, and isopropylidene.

The term "alkenylene" radical denotes linear or branched radicals having from 2 to 10 carbon atoms, at least one double bond, and having attachment points for two or more covalent bonds. Examples of such radicals are 1,1-vinylidene (CH₂=C), 1,2-vinylidene (-CH=CH-), and 1,4-butadienyl (-CH=CH-CH-CH-).

The term "halo" means halogens such as fluorine, chlorine, bromine or iodine atoms.

The term "haloalkyl" embraces radicals wherein any one or more of the alkyl carbon atoms is substituted with halo as defined above. Specifically embraced are monobaloalkyl, dihaloalkyl and polyhaloalkyl radicals. A monohaloalkyl radical, for one example, may have either a bromo, chloro or a fluoro atom within the radical. Dihalo radicals may have two or more of the same halo atoms or a combination of different halo radicals and polyhaloalkyl radicals may have more than two of the same halo atoms or a combination of different halo radicals. More preferred haloalkyl radicals are "lower haloalkyl" radicals having one to about six carbon atoms. Examples of such haloalkyl radicals include fluoromethyl, difluoromethyl, trifluoromethyl, chloromethyl, dichloromethyl, trichloromethyl, trifluoroethyl, pentafluoroethyl. heptafluoropropyl, difluorochloromethyl, dichlorofluoromethyl, difluoroethyl. difluoropropyl, dichloroethyl and dichloropropyl.

The term "hydroxyhaloalkyl" embraces radicals wherein any one or more of the haloalkyl carbon atoms is substituted with hydroxy as defined above. Examples of "hydroxyhaloalkyl" radicals include hexafluorohydroxypropyl.

The term "haloalkylene radical" denotes alkylene radicals wherein any one or more of the alkylene carbon atoms is substituted with halo as defined above. Dihalo alkylene radicals may have two or more of the same halo atoms or a combination of different halo radicals and polyhaloalkylene radicals may have more than two of the same halo atoms or a combination of different halo radicals. More preferred haloalkylene radicals are "lower haloalkylene" radicals having one to about six carbon atoms. Examples of "haloalkylene" radicals include difluoromethylene, tetrafluoroethylene, tetrachloroethylene, alkyl substituted monofluoromethylene, and aryl substituted trifluoromethylene.

The term "haloalkenyl" denotes linear or branched radicals having from 1 to 10 carbon atoms and having one or more double bonds wherein any one or more of the alkenyl carbon atoms is substituted with halo as defined above. Dihaloalkenyl radicals may have two or more of the same halo atoms or a combination of different halo radicals and polyhaloalkenyl radicals may have more than two of the same halo atoms or a combination of different halo radicals.

The terms "alkoxy" and "alkoxyalkyl" embrace linear or branched oxy-containing radicals each having alkyl portions of one to ten carbon atoms, such as methoxy radical. The term "alkoxyalkyl" also embraces alkyl radicals having one or more alkoxy radicals attached to the alkyl radical, that is, to form monoalkoxyalkyl and dialkoxyalkyl radicals. More preferred alkoxy radicals are "lower alkoxy" radicals having one to six carbon atoms. Examples of such radicals include methoxy, ethoxy, propoxy, butoxy, isopropoxy and *tert*-butoxy alkyls. The "alkoxy" radicals may be further substituted with one or more halo atoms, such as fluoro, chloro or bromo, to provide "haloalkoxy" and "haloalkoxyalkyl" radicals. Examples of such haloalkoxy radicals include fluoromethoxy, chloromethoxy, trifluoromethoxy, difluoromethoxy, trifluoroethoxy, fluoroethoxy, tetrafluoroethoxy, pentafluoroethoxy, and fluoropropoxy. Examples of such haloalkoxyalkyl radicals include fluoromethoxymethyl, chloromethoxyethyl, trifluoromethoxymethyl, difluoromethoxyethyl, and trifluoroethoxymethyl.

The terms "alkenyloxy" and "alkenyloxyalkyl" embrace linear or branched oxy-containing radicals each having alkenyl portions of two to ten carbon atoms, such as ethenyloxy or propenyloxy radical. The term "alkenyloxyalkyl" also embraces alkenyl radicals having one or more alkenyloxy radicals attached to the alkyl radical, that is, to form monoalkenyloxyalkyl and dialkenyloxyalkyl radicals. More preferred alkenyloxy radicals are "lower alkenyloxy" radicals having two to six carbon atoms. Examples of such radicals include ethenyloxy, propenyloxy. butenyloxy, and isopropenyloxy alkyls. The "alkenyloxy" radicals may be further substituted with one or more halo atoms, such as fluoro, chloro or bromo, to provide "haloalkenyloxy" radicals. Examples of such radicals include trifluoroethenyloxy, fluoroethenyloxy, difluoroethenyhloxy, and fluoropropenyloxy.

The term "haloalkoxyalkyl" also embraces alkyl radicals having one or more haloalkoxy radicals attached to the alkyl radical, that is, to form monohaloalkoxyalkyl and dihaloalkoxyalkyl radicals. The term "haloalkenyloxy" also embraces oxygen radicals having one or more haloalkenyloxy radicals attached to the oxygen radical, that is, to form monohaloalkenyloxy and dihaloalkenyloxy radicals. The term "haloalkenyloxyalkyl" also embraces alkyl radicals having one or more haloalkenyloxy radicals attached to the alkyl radical, that is, to form monohaloalkenyloxyalkyl and dihaloalkenyloxyalkyl radicals.

The term "alkylenedioxy" radicals denotes alkylene radicals having at least two oxygens bonded to a single alkylene group. Examples of "alkylenedioxy" radicals include methylenedioxy, ethylenedioxy, alkylsubstituted methylenedioxy, and arylsubstituted methylenedioxy. The term "haloalkylenedioxy" radicals denotes haloalkylene radicals having at least two oxy groups bonded to a single haloalkyl group. Examples of "haloalkylenedioxy" radicals include difluoromethylenedioxy, tetrafluoroethylenedioxy, tetrachloroethylenedioxy, alkylsubstituted monofluoromethylenedioxy, and arylsubstituted monofluoromethylenedioxy.

The term "aryl", alone or in combination, means a carbocyclic aromatic system containing one, two or three rings wherein such rings may be attached together in a pendant manner or may be fused. The term "fused" means that a second ring is present (ie, attached or formed) by having two adjacent atoms in common (ie, shared) with the first ring. The term "fused" is equivalent to the term "condensed". The term "aryl" embraces aromatic radicals such as phenyl, naphthyl, tetrahydronaphthyl, indane and biphenyl.

The term "perhaloaryl" embraces aromatic radicals such as phenyl, naphthyl, tetrahydronaphthyl, indane and biphenyl wherein the aryl radical is substituted with 3 or more halo radicals as defined below.

The term "heterocyclyl" embraces saturated and partially saturated heteroatom-containing ring-shaped radicals having from 4 through 15 ring members, herein referred to as "C4-C15 heterocyclyl", selected from carbon, nitrogen, sulfur and oxygen, wherein at least one ring atom is a heteroatom. Heterocyclyl radicals may contain one, two or three rings wherein such rings may be attached in a pendant manner or may be fused. Examples of saturated heterocyclic radicals include saturated 3 to 6-membered heteromonocylic group containing 1 to 4 nitrogen atoms[e.g. pyrrolidinyl, imidazolidinyl, piperidino, piperazinyl, etc.]; saturated 3 to 6-membered heteromonocyclic group containing 1 to 2 oxygen atoms and 1 to 3 nitrogen atoms [e.g. morpholinyl, etc.]; saturated 3 to 6-membered heteromonocyclic group containing I to 2 sulfur atoms and 1 to 3 nitrogen atoms [e.g., thiazolidinyl, etc.]. Examples of partially saturated heterocyclyl radicals include dihydrothiophene, dihydropyran, dihydrofuran and dihydrothiazole. Non-limiting examples of heterocyclic radicals include 2-pyrrolinyl, 3-pyrrolinyl, pyrrolindinyl, 1,3-dioxolanyl, 2H-pyranyl, 4H-pyranyl, piperidinyl, 1,4-dioxanyl, morpholinyl, 1,4-dithianyl, thiomorpholinyl, and the like.

The term "heteroaryl" embraces fully unsaturated heteroatom-containing ring-shaped aromatic radicals having from 4 through 15 ring members selected from carbon, nitrogen, sulfur and oxygen, wherein at least one ring atom is a heteroatom. Heteroaryl radicals may contain one, two or three rings wherein such rings may be attached in a pendant manner or may be fused. Examples of "heteroaryl" radicals, include unsaturated 5 to 6 membered heteromonocyclyl group containing 1 to 4 nitrogen atoms, for example, pyrrolyl, pyrrolinyl, imidazolyl, pyrazolyl, 2-pyridyl, 3-pyridyl, 4-pyridyl, pyrimidyl, pyrazinyl, pyridazinyl, triazolyl [e.g., 4H-1,2,4-triazolyl, 1H-1,2,3-triazolyl, 2H-1,2,3-triazolyl, etc.] tetrazolyl [e.g. 1H-tetrazolyl, 2H-tetrazolyl, etc.], etc.; unsaturated condensed heterocyclic group containing 1 to 5 nitrogen atoms, for example, indolyl, isoindolyl, indolizinyl, benzimidazolyl, quinolyl, isoquinolyl, indazolyl, benzotriazolyl, tetrazolopyridazinyl [e.g., tetrazolo [1,5-b]pyridazinyl, etc.], etc.; unsaturated 3 to 6-membered heteromonocyclic group containing an oxygen atom, for example, pyranyl, 2-furyl, 3-furyl, etc.; unsaturated 5 to 6-membered heteromonocyclic group containing a sulfur atom, for example, 2-thienyl, 3-thienyl, etc.; unsaturated 5- to 6-membered heteromonocyclic group containing 1 to 2 oxygen atoms and 1 to 3 nitrogen atoms, for example, oxazolyl, isoxazolyl, oxadiazolyl [e.g., 1,2,4-oxadiazolyl, 1,3,4-oxadiazolyl, 1,2,5-oxadiazolyl, etc.] etc.; unsaturated condensed heterocyclic group containing 1 to 2 oxygen atoms and 1 to 3 nitrogen atoms [e.g. benzoxazolyl, benzoxadiazolyl, etc.]; unsaturated 5 to 6-membered heteromonocyclic group containing 1 to 2 sulfur atoms and 1 to 3 nitrogen atoms, for example, thiazolyl, thiadiazolyl [e.g., 1,2,4- thiadiazolyl, 1,3,4-thiadiazolyl, 1,2,5-thiadiazolyl, etc.] etc.; unsaturated condensed heterocyclic group containing 1 to 2 sulfur atoms and 1 to 3 nitrogen atoms [e.g., benzothiazolyl, benzothiadiazolyl, etc.] and the like. The term also embraces radicals where heterocyclic radicals are fused with aryl radicals. Examples of such fused bicyclic radicals include benzofuran, benzothiophene, and the like. Said "heterocyclyl" group may have 1 to 3 substituents as defined below. Preferred heterocyclic radicals include five to twelve membered fused or unfused radicals. Non-limiting examples of heteroaryl radicals include pyrrolyl, pyridinyl, pyridyloxy, pyrazolyl, triazolyl, pyrimidinyl, pyridazinyl, oxazolyl, thiazolyl, imidazolyl, indolyl, thiophenyl, furanyl, tetrazolyl, 2-imidazolinyl, imidazolidinyl, 2-pyrazolinyl, pyrazolidinyl, isoxazolyl, isothiazolyl, 1,2,3-oxadiazolyl, 1,2,3-triazolyl, 1,3,4-thiadiazolyl, pyrazinyl, piperazinyl, 1,3,5-triazinyl, 1,3,5-trithianyl, benzo(b)thiophenyl, benzimidazoyl, quinolinyl, tetraazolyl, and the like.

The term "sulfonyl", whether used alone or linked to other terms such as alkylsulfonyl, denotes respectively divalent radicals -SO₂-. "Alkylsulfonyl", embraces alkyl radicals attached to a sulfonyl radical, where alkyl is defined as above. "Alkylsulfonylalkyl", embraces alkylsulfonyl radicals attached to an alkyl radical, where alkyl is defined as above. "Haloalkylsulfonyl", embraces haloalkyl radicals attached to a sulfonyl radical, where haloalkyl is defined as above. "Haloalkylsulfonylalkyl", embraces haloalkylsulfonyl radicals attached to an alkyl radical, where alkyl is defined as above. The term "aminosulfonyl" denotes an amino radical attached to a sulfonyl radical.

The term "sulfinyl", whether used alone or linked to other terms such as alkylsulfinyl, denotes respectively divalent radicals -S(O)-. "Alkylsulfinyl", embraces alkyl radicals attached to a sulfinyl radical, where alkyl is defined as above. "Alkylsulfinylalkyl", embraces alkylsulfinyl radicals attached to an alkyl radical, where alkyl is defined as above. "Haloalkylsulfinyl", embraces haloalkyl radicals attached to a sulfinyl radical, where haloalkyl is defined as above. "Haloalkylsulfinylalkyl", embraces haloalkylsulfinyl radicals attached to an alkyl radical, where alkyl is defined as above.

The term "aralkyl" embraces aryl-substituted alkyl radicals. Preferable aralkyl radicals are "lower aralkyl" radicals having aryl radicals attached to alkyl radicals having one to six carbon atoms. Examples of such radicals include benzyl, diphenylmethyl, triphenylmethyl, phenylethyl and diphenylethyl. The terms benzyl and phenylmethyl are interchangeable.

The term "heteroaralkyl" embraces heteroaryl-substituted alkyl radicals wherein the heteroaralkyl radical may be additionally substituted with three or more substituents as defined above for aralkyl radicals. The term "perhaloaralkyl" embraces aryl-substituted alkyl radicals wherein the aralkyl radical is substituted with three or more halo radicals as defined above.

The term "aralkylsulfinyl", embraces aralkyl radicals attached to a sulfinyl radical, where aralkyl is defined as above. "Aralkylsulfinylalkyl", embraces aralkylsulfinyl radicals attached to an alkyl radical, where alkyl is defined as above.

The term "aralkylsulfonyl", embraces aralkyl radicals attached to a sulfonyl radical, where aralkyl is defined as above. "Aralkylsulfonylalkyl", embraces aralkylsulfonyl radicals attached to an alkyl radical, where alkyl is defined as above.

The term "cycloalkyl" embraces radicals having three to 15 carbon atoms. More preferred cycloalkyl radicals are "lower cycloalkyl" radicals having three to seven carbon atoms. Examples include radicals such as cyclopropyl, cyclobutyl, cyclopentyl, cyclohexyl and cycloheptyl. The term cycloalkyl embraces radicals having seven to 15 carbon atoms and having two to four rings. Exmaples incude radicals such as norbornyl (i.e., bicyclo[2.2.1]heptyl) and adamantyl. The term "cycloalkylalkyl" embraces cycloalkyl-substituted alkyl radicals. Preferable cycloalkylalkyl radicals are "lower cycloalkylalkyl" radicals having cycloalkyl radicals attached to alkyl radicals having one to six carbon atoms. Examples of such radicals include cyclohexylhexyl. The term "cycloalkenyl" embraces radicals having three to ten carbon atoms and one or more carbon-carbon double bonds. Preferred cycloalkenyl radicals are "lower cycloalkenyl" radicals having three to seven carbon atoms. Examples include radicals such as cyclobutenyl. cyclopentenyl, cyclohexenyl and cycloheptenyl. The term "halocycloalkyl" embraces radicals wherein any one or more of the cycloalkyl carbon atoms is substituted with halo as defined above. Specifically embraced are monohalocycloalkyl, dihalocycloalkyl and polyhalocycloalkyl radicals. A monohalocycloalkyl radical, for one example, may have either a bromo, chloro or a fluoro atom within the radical. Dihalo radicals may have two or more of the same halo atoms or a combination of different halo radicals and polyhalocycloalkyl radicals may have more than two of the same halo atoms or a combination of different halo radicals. More preferred balocycloalkyl radicals are "lower halocycloalkyl" radicals having three to about eight carbon atoms. Examples of such halocycloalkyl radicals include fluorocyclopropyl, difluorocyclobutyl, trifluorocyclopentyl, tetrafluorocyclohexyl, and dichlorocyclopropyl. The term "halocycloalkenyl" embraces radicals wherein any one or more of the cycloalkenyl carbon atoms is substituted with halo as defined above. Specifically embraced are monohalocycloalkenyl, dihalocycloalkenyl and polyhalocycloalkenyl radicals.

The term "cycloalkoxy" embraces cycloalkyl radicals attached to an oxy radical. Examples of such radicals includes cyclohexoxy and cyclopentoxy. The term "cycloalkoxyalkyl" also embraces alkyl radicals having one or more cycloalkoxy radicals attached to the alkyl radical, that is, to form monocycloalkoxyalkyl and dicycloalkoxyalkyl radicals. Examples of such radicals include cyclohexoxyethyl. The "cycloalkoxy" radicals may be further substituted with one or more halo atoms, such as fluoro, chloro or bromo, to provide "halocycloalkoxy" and "halocycloalkoxyalkyl" radicals.

The term "cycloalkylalkoxy" embraces cycloalkyl radicals attached to an alkoxy radical. Examples of such radicals includes cyclohexylmethoxy and cyclopentylmethoxy.

The term "cycloalkenyloxy" embraces cycloalkenyl radicals attached to an oxy radical. Examples of such radicals includes cyclohexenyloxy and cyclopentenyloxy. The term "cycloalkenyloxyalkyl" also embraces alkyl radicals having one or more cycloalkenyloxy radicals attached to the alkyl radical, that is, to form monocycloallcenyloxyalkyl and dicycloalkenyloxyalkyl radicals. Examples of such radicals include cyclohexenyloxyethyl. The "cycloalkenyloxy" radicals may be further substituted with one or more halo atoms, such as fluoro, chloro or bromo, to provide "halocycloalkenyloxy" and "halocycloalkenyloxyalkyl" radicals.

The term "cycloalkylenedioxy" radicals denotes cycloalkylene radicals having at least two oxygens bonded to a single cycloalkylene group. Examples of "alkylenedioxy" radicals include 1,2-dioxycyclohexylene.

The term "cycloalkylsulfinyl", embraces cycloalkyl radicals attached to a sulfinyl radical, where cycloalkyl is defined as above. "Cycloalkylsulfinylalkyl", embraces cycloalkylsulfinyl radicals attached to an alkyl radical, where alkyl is defined as above. The term "Cycloalkylsulfonyl", embraces cycloalkyl radicals attached to a sulfonyl radical, where cycloalkyl is defined as above. "Cycloalkylsulfonylalkyl", embraces cycloalkylsulfonyl radicals attached to an alkyl radical, where alkyl is defined as above.

The term "cycloalkylalkanoyl" embraces radicals wherein one or more of the cycloalkyl carbon atoms are substituted with one or more carbonyl radicals as defined below. Specifically embraced are monocarbonylcycloalkyl and dicarbonylcycloalkyl radicals. Examples of monocarbonylcycloalkyl radicals include cyclohexylcarbonyl, cyclohexylacetyl, and cyclopentylcarbonyl. Examples of dicarbonylcycloalkyl radicals include 1,2-dicarbonylcyclohexane.

The term "alkylthio" embraces radicals containing a linear or branched alkyl radical, of one to ten carbon atoms, attached to a divalent sulfur atom. More preferred alkylthio radicals are "lower alkylthio" radicals having one to six carbon atoms. An example of "lower alkylthio" is methylthio (CH₃-S-). The "alkylthio" radicals may be further substituted with one or more halo atoms, such as fluoro, chloro or bromo, to provide "haloalkylthio" radicals. Examples of such radicals include fluoromethylthio, chloromethylthio, trifluoromethylthio, difluoromethylthio, trifluoroethylthio, fluoroethylthio, tetrafluoroethylthio, pentafluoroethylthio, and fluoropropylthio.

The term "alkyl aryl amino" embraces radicals containing a linear or branched alkyl radical, of one to ten carbon atoms, and one aryl radical both attached to an amino radical. Examples include N-methyl-4-methoxyaniline, N-ethyl-4-methoxyaniline, and N-methyl-4-trifluoromethoxyaniline.

The terms alkylamino denotes "monoalkylamino" and "dialkylamino" containing one or two alkyl radicals, respectively, attached to an amino radical.

The terms arylamino denotes "monoarylamino" and "diarylamino" containing one or two aryl radicals, respectively, attached to an amino radical. Examples of such radicals include N-phenylamino and N-naphthylamino.

The term "aralkylamino", embraces aralkyl radicals attached to an amino radical, where aralkyl is defined as above. The term aralkylamino denotes "monoaralkylamino" and "diaralkylamino" containing one or two aralkyl radicals, respectively, attached to an amino radical. The term aralkylamino further denotes "monoaralkyl monoalkylamino" containing one aralkyl radical and one alkyl radical attached to an amino radical.

The term "arylsulfinyl" embraces radicals containing an aryl radical, as defined above, attached to a divalent S(O) atom. The term "arylsulfinylalkyl" denotes arylsulfinyl radicals attached to a linear or branched alkyl radical, of one to ten carbon atoms.

The term "arylsulfonyl", embraces aryl radicals attached to a sulfonyl radical, where aryl is defined as above. "arylsulfonylalkyl", embraces arylsulfonyl radicals attached to an alkyl radical, where alkyl is defined as above. The term "heteroarylsulfinyl" embraces radicals containing an heteroaryl radical, as defined above, attached to a divalent S(O) atom. The term "heteroarylsulfinylalkyl" denotes heteroarylsulfinyl radicals attached to a linear or branched alkyl radical, of one to ten carbon atoms. The term "Heteroarylsulfonyl", embraces heteroaryl radicals attached to a sulfonyl radical, where heteroaryl is defined as above. "Heteroarylsulfonylalkyl", embraces heteroarylsulfonyl radicals attached to an alkyl radical, where alkyl is defined as above.

The term "aryloxy" embraces aryl radicals, as defined above, attached to an oxygen atom. Examples of such radicals include phenoxy, 4-chloro-3-ethylphenoxy, 4-chloro-3-methylphenoxy, 3-chloro-4-ethylphenoxy, 3,4-dichlorophenoxy, 4-methylphenoxy, 3-trifluoromethoxyphenoxy, 3-trifluoromethylphenoxy, 4-fluorophenoxy, 3,4-dimethylphenoxy, 5-bromo-2-fluorophenoxy, 4-bromo-3-fluorophenoxy, 4-fluoro-3-methylphenoxy, 5,6,7,8-tetrahydronaphthyloxy, 3-isopropylphenoxy, 3-cyclopropylphenoxy, 3-ethylphenoxy, 3-pentafluoroethylphenoxy, 3-(1,1,2,2-tetrafluoroethoxy)-phenoxy, and 4-*tert*-butylphenoxy.

The term "aroyl" embraces aryl radicals, as defined above, attached to an carbonyl radical as defined above. Examples of such radicals include benzoyl and toluoyl.

The term "aralkanoyl" embraces aralkyl radicals, as defined herein, attached to an carbonyl radical as defined above. Examples of such radicals include, for example, phenylacetyl.

The term "aralkoxy" embraces oxy-containing aralkyl radicals attached through an oxygen atom to other radicals. More preferred aralkoxy radicals are "lower aralkoxy" radicals having phenyl radicals attached to lower alkoxy radical as described above. Examples of such radicals include benzyloxy, 1-phenylethoxy, 3-trifluoromethoxybenzyloxy, 3-trifluoromethylbenzyloxy, 3,5-difluorobenyloxy, 3-bromobenzyloxy, 4-propylbenzyloxy, 2-fluoro-3-trifluoromethylbenzyloxy, and 2-phenylethoxy.

The term "aryloxyalkyl" embraces aryloxy radicals, as defined above, attached to an alkyl group. Examples of such radicals include phenoxymethyl.

The term "haloaryloxyalkyl" embraces aryloxyalkyl radicals, as defined above, wherein one to five halo radicals are attached to an aryloxy group.

The term "heteroaroyl" embraces heteroaryl radicals, as defined above, attached to an carbonyl radical as defined above. Examples of such radicals include furoyl and nicotinyl.

The term "heteroaralkanoyl" embraces heteroaralkyl radicals, as defined herein, attached to an carbonyl radical as defined above. Examples of such radicals include, for example, pyridylacetyl and furylbutyryl.

The term "heteroaralkoxy" embraces oxy-containing heteroaralkyl radicals attached through an oxygen atom to other radicals. More preferred heteroaralkoxy radicals are "lower heteroaralkoxy" radicals having heteroaryl radicals attached to lower alkoxy radical as described above.

The term "haloheteroaryloxyalkyl" embraces heteroaryloxyalkyl radicals, as defined above, wherein one to four halo radicals are attached to an heteroaryloxy group.

The term "heteroarylamino" embraces heterocyclyl radicals, as defined above, attached to an amino group. Examples of such radicals include pyridylamino.

The term "heteroarylaminoalkyl" embraces heteroarylamino radicals, as defined above, attached to an alkyl group. Examples of such radicals include pyridylmethylamino.

The term "heteroaryloxy" embraces heterocyclyl radicals, as defined above, attached to an oxy group. Examples of such radicals include 2-thiophenyloxy, 2-pyrimidyloxy, 2-pyridyloxy, 3-pyridyloxy, and 4-pyridyloxy.

The term "heteroaryloxyalkyl" embraces heteroaryloxy radicals, as defined above, attached to an alkyl group. Examples of such radicals include 2-pyridyloxymethyl, 3-pyridyloxyethyl, and 4-pyridyloxymethyl.

The term "arylthio" embraces aryl radicals, as defined above, attached to an sulfur atom. Examples of such radicals include phenylthio.

The term "arylthioalkyl" embraces arylthio radicals, as defined above, attached to an alkyl group. Examples of such radicals include phenylthiomethyl.

The term "alkylthioalkyl" embraces alkylthio radicals, as defined above, attached to an alkyl group. Examples of such radicals include methylthiomethyl. The term "alkoxyalkyl" embraces alkoxy radicals, as defined above, attached to an alkyl group. Examples of such radicals include methoxymethyl.

The term "carbonyl" denotes a carbon radical having two of the four covalent bonds shared with an oxygen atom. The term "carboxy" embraces a hydroxyl radical, as defined above, attached to one of two unshared bonds in a carbonyl group. The term "carboxamide" embraces amino, monoalkylamino, dialkylamino, monocycloalkylamino, alkylcycloalkylamino, and dicycloalkylamino radicals, attached to one of two unshared bonds in a carbonyl group. The term "carboxamidoalkyl" embraces carboxamide radicals, as defined above, attached to an alkyl group. The term "carboxyalkyl" embraces a carboxy radical, as defined above, attached to an alkyl group. The term "carboalkoxy" embraces alkoxy radicals, as defined above, attached to one of two unshared bonds in a carbonyl group. The term "carboaralkoxy" embraces aralkoxy radicals, as defined above, attached to one of two unshared bonds in a carbonyl group. The term "monocarboalkoxyalkyl" embraces one carboalkoxy radical, as defined above, attached to an alkyl group. The term "dicarboalkoxyalkyl" embraces two carboalkoxy radicals, as defined above, attached to an alkylene group. The term "monocyanoalkyl" embraces one cyano radical, as defined above, attached to an alkyl group. The term "dicyanoalkylene" embraces two cyano radicals, as defined above, attached to an alkyl group. The term "carboalkoxycyanoalkyl" embraces one cyano radical, as defined above, attached to an carboalkoxyalkyl group.

The term "acyl", alone or in combination, means a carbonyl or thionocarbonyl group bonded to a radical selected from, for example, hydrido, alkyl, alkenyl, alkynyl, haloalkyl, alkoxy, alkoxyalkyl, haloalkoxy, aryl, heterocyclyl, heteroaryl, alkylsulfinylalkyl, alkylsulfonylalkyl, aralkyl, cycloalkyl, cycloalkylalkyl, cycloalkenyl, alkylthio, arylthio, amino. alkylamino, dialkylamino, aralkoxy, arylthio, and alkylthioalkyl. Examples of "acyl" are formyl, acetyl, benzoyl, trifluoroacetyl, phthaloyl, malonyl, nicotinyl, and the like. The term "haloalkanoyl" embraces one or more halo radicals, as defined herein, attached to an alkanoyl radical as defined above. Examples of such radicals include, for example, chloroacetyl, trifluoroacetyl, bromopropanoyl, and heptafluorobutanoyl.

The term "phosphono" embraces a pentavalent phosphorus attached with two covalent bonds to an oxygen radical. The term "dialkoxyphosphono" denotes two alkoxy radicals, as defined above, attached to a phosphono radical with two covalent bonds. The term "diaralkoxyphosphono" denotes two aralkoxy radicals, as defined above, attached to a phosphono radical with two covalent bonds. The term "dialkoxyphosphonoalkyl" denotes dialkoxyphosphono radicals, as defined above, attached to an alkyl radical. The term "diaralkoxyphosphonoalkyl" denotes diaralkoxyphosphono radicals, as defined above, attached to an alkyl radical.

The term "amino" denotes a nitrogen atom containing two substituents such as hydrido, hydroxy or alkyl and having one covalent bond available for bonding to a single atom such as carbon. Examples of such amino radicals include, for example, -NH₂, -NHCH₃, -NHOH, and -NHOCH₃. The term "imino" denotes a nitrogen atom containing one substituent such as hydrido, hydroxy or alkyl and having two covalent bonds available for bonding to a single atom such as carbon. Examples of such imino radicals include, for example, =NH, =NCH₃, =NOH, and =NOCH₃. The term "imino carbonyl" denotes a carbon radical having two of the four covalent bond sites shared with an imino group. Examples of such imino carbonyl radicals include, for example, C=NH, C=NCH₃, C=NOH, and C=NOCH₃. The term "amidino" embraces a substituted or unsubstituted amino group bonded to one of two available bonds of an iminocarbonyl radical. Examples of such amidino radicals include, for example, NH₂-C=NH, NH₂-C=NCH₃, NH₂-C=NOCH₃ and CH₃NH-C=NOH. The term "guanidino" denotes an amidino group bonded to an amino group as defined above where said amino group can be bonded to a third group. Examples of such guanidino radicals include, for example, NH₂-C(NH)-NH-, NH₂-C(NCH₃)-NH-, NH₂-C(NOCH₃)-NH-, and CH₃NH-C(NOH)-NH-.

The term "sulfonium" denotes a positively charged trivalent sulfur atom where said sulfur is substituted with three carbon based groups such as alkyl, alkenyl, aralkyl, or aryl. The term "dialkyl sulfonium" denotes a sulfonium group where said sulfur is substituted with two alkyl groups. Examples of such dialkylsulfonium radicals include, for example, (CH₃)₂S⁺-. The term "dialkyl sulfonium alkyl" denotes a dialkyl sulfonium group where said group is bonded to one bond of an alkylene group as defined above. Examples of such dialkylsulfoniumalkyl radicals include (CH₃)₂S⁺-CH₂CH₂-.

The term "phosphonium" denotes a positively charged tetravalent phosphorus atom where said phosphorus is substituted with four carbon based groups such as alkyl, alkenyl, aralkyl, or aryl. The term "trialkyl phosphonium" denotes a phosphonium group where said phosphorus is substituted with three alkyl groups. Examples of such trialkylphosphonium radicals include, for example, (CH₃)₃P⁺-.

Said "alkyl", "alkenyl", "alkynyl", "alkanoyl", "alkylene", "alkenylene", "hydroxyalkyl", "haloalkyl", "haloalkylene", "haloalkenyl", "alkoxy", "alkenyloxy", "alkenyloxyalkyl", "alkoxyalkyl", "aryl", "perhaloaryl", "haloalkoxy", "haloalkoxyalkyl", "haloalkenyloxy", "haloalkenyloxyalkyl", "alkylenedioxy", "haloalkylenedioxy", "heterocyclyl", "heteroaryl", "hydroxyhaloalkyl", "alkylsulfonyl", "haloalkylsulfonyl", "alkylsulfonylalkyl", "haloalkylsulfonylalkyl", "alkylsulfinyl", "alkylsulfinylalkyl", "haloalkylsulfinylalkyl", "aralkyl", "heteroaralkyl", "perhaloaralkyl", "aralkylsulfonyl", "aralkylsulfonylalkyl", "aralkylsulfinyl", "aralkylsulfinylalkyl", "cycloalkyl", "cycloalkylalkanoyl", "cycloalkylalkyl", "cycloalkenyl", "halocycloalkyl", "halocycloalkenyl", "cycloalkylsulfinyl", "cycloalkylsulfinylalkyl", "cycloalkylsulfonyl", "cycloalkylsulfonylalkyl", "cycloalkoxy", "cycloalkoxyalkyl", "cycloalkylalkoxy", "cycloalkenyloxy", "cycloalkenyloxyalkyl", "cycloalkylenedioxy", "halocycloalkoxy", "halocycloalkoxyalkyl", "halocycloalkenyloxy", "halocycloalkenyloxyalkyl", "alkylthio", "haloalkylthio", "alkylsulfinyl", "amino", "oxy", "thio", "alkylamino", "arylamino", "aralkylamino", "arylsulfinyl", "arylsulfinylalkyl", "arylsulfonyl", "arylsulfonylalkyl", "heteroarylsulfinyl", "heteroarylsulfinylalkyl", "heteroarylsulfonyl", "heteroarylsulfonylalkyl", "heteroarylamino", "heteroarylaminoalkyl", "heteroaryloxy", "heteroaryloxylalkyl", "aryloxy", "aroyl", "aralkanoyl", "aralkoxy", "aryloxyalkyl", "haloaryloxyalkyl", "heteroaroyl", "heteroaralkanoyl", "heteroaralkoxy", "heteroaralkoxyalkyl", "arylthio", "arylthioalkyl", "alkoxyalkyl", "acyl", "amidino", "guanidino", "dialkylsulfonium", "trialkylphosphonium", and "dialkylsulfoniumalkyl" groups defined above may optionally have 1 or more non-hydrido substituents such as amidino, guanidino, dialkylsulfonium, trialkylphosphonium, dialkylsulfoniumalkyl, perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroarylaminoalkyl, heteroaryloxy, heteroaryloxylalkyl, haloalkylthio, alkanoyloxy, alkoxy, alkoxyalkyl, haloalkoxylalkyl, heteroaralkoxy, cycloalkoxy, cycloalkenyloxy, cycloalkoxyalkyl, cycloalkylalkoxy, cycloalkenyloxyalkyl, cycloalkylenedioxy, halocycloalkoxy, halocycloalkoxyalkyl, halocycloalkenyloxy, halocycloalkenyloxyalkyl, hydroxy, amino, thio, nitro, lower alkylamino, alkylthio, alkylthioalkyl, arylamino, aralkylamino, arylthio, arylthioalkyl, heteroaralkoxyalkyl, alkylsulfinyl, alkylsulfinylalkyl, arylsulfinylalkyl, arylsulfonylalkyl, heteroarylsulfinylalkyl, heteroarylsulfonylalkyl, alkylsulfonyl, alkylsulfonylalkyl, haloalkylsulfinylalkyl, haloalkylsulfonylalkyl, alkylsulfonamido, alkylaminosulfonyl, amidosulfonyl, monoalkyl amidosulfonyl, dialkyl amidosulfonyl, monoarylamidosulfonyl, arylsulfonamido, diarylamidosulfonyl, monoalkyl monoaryl amidosulfonyl, arylsulfinyl, arylsulfonyl, heteroarylthio, heteroarylsulfinyl, heteroarylsulfonyl, alkanoyl, alkenoyl, aroyl, heteroaroyl, aralkanoyl, heteroaralkanoyl, haloalkanoyl, alkyl, alkenyl, alkynyl, alkenyloxy, alkenyloxyalky, alkylenedioxy, haloalkylenedioxy, cycloalkyl, cycloalkylalkanoyl, cycloalkenyl, lower cycloalkylalkyl, lower cycloalkenylalkyl, halo, haloalkyl, haloalkenyl, haloalkoxy, hydroxyhaloalkyl, hydroxyaralkyl, hydroxyalkyl, aminoalkyl, hydoxyheteroaralkyl, haloalkoxyalkyl, aryl, aralkyl, aryloxy, aralkoxy, aryloxyalkyl, saturated heterocyclyl, partially saturated heterocyclyl, heteroaryl, heteroaryloxy, heteroaryloxyalkyl, arylalkyl, heteroarylalkyl, arylalkenyl, heteroarylalkenyl, carboxyalkyl, carboalkoxy, alkoxycarbonyl, carboaralkoxy, carboxamido, carboxamidoalkyl, cyano, carbohaloalkoxy, phosphono, phosphonoalkyl, diaralkoxyphosphono, and diaralkoxyphosphonoalkyl.

The term "spacer" can include a covalent bond and a linear moiety having a backbone of 1 to 7 contiguous atoms. The spacer may have 1 to 7 atoms of a univalent or multi-valent chain. Univalent chains may be constituted by a radical selected from =C(H)-, =C(R^{2a})-, -O-, -S-, -S(O)-, -S(O)₂-, -NH-, -N(R^{2a})-, -N=, -CH(OH)-, =C(OH)-, -CH(OR^{2a})-, =C(OR^{2a})-, and -C(O)- wherein R^{2a} is selected from alkyl, alkenyl, alkynyl, aryl, heteroaryl, aralkyl, aryloxyalkyl, alkoxyalkyl, alkylthioalkyl, arylthioalkyl, cycloalkyl, cycloalkylalkyl, haloalkyl, haloalkenyl, haloalkoxyalkyl, perhaloaralkyl, heteroarylalkyl, heteroaryloxyalkyl, heteroarylthioalkyl, and heteroarylalkenyl. Multi-valent chains may consist of a straight chain of 1 or 2 or 3 or 4 or 5 or 6 or 7 atoms or a straight chain of 1 or 2 or 3 or 4 or 5 or 6 atoms with a side chain. The chain may be constituted of one or more radicals selected from: lower alkylene, lower alkenyl, -O-, -O-CH₂-, -S-CH₂-, -CH₂CH₂-, ethenyl, -CH=CH(OH)-, -OCH₂O-, -O(CH₂)₂O-, -NHCH₂-, -OCH(R^{2a})O-, -O(CH₂CHR^{2a})O-, -OCF₂O-, -O(CF₂)₂O-, -S-, -S(O)-, -S(O)₂-, -N(H)-, -N(H)O-, -N(R^{2a})O-, -N(R^{2a})-, -C(O)-, -C(O)NH-, -C(O)NR^{2a} -, -N=, -OCH₂-, -SCH₂-, S(O)CH₂-, -CH₂C(O)-, -CH(OH)-, =C(OH)-, -CH(OR^{2a})-, =C(OR^{2a})-, S(O)₂CH₂-, and -NR^{2a}CH₂- and many other radicals defined above or generally known or ascertained by one of skill-in-the art. Side chains may include substituents such as 1 or more non-hydrido substituents such as amidino, guanidino, dialkylsulfonium, trialkylphosphonium, dialkylsulfoniumalkyl, perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroarylaminoalkyl, heteroaryloxy, heteroaryloxylalkyl, haloalkylthio, alkanoyloxy, alkoxy, alkoxyalkyl, haloalkoxylalkyl, heteroaralkoxy, cycloalkoxy, cycloalkenyloxy, cycloalkoxyalkyl, cycloalkylalkoxy, cycloalkenyloxyalkyl, cycloalkylenedioxy. halocycloalkoxy, halocycloalkoxyalkyl, halocycloalkenyloxy, halocycloalkenyloxyalkyl, hydroxy, amino. thio, nitro, lower alkylamino, alkylthio, alkylthioalkyl, arylamino, aralkylamino, arylthio, arylthioalkyl, heteroaralkoxyalkyl, alkylsulfinyl, alkylsulfinylalkyl, arytsulfinylalkyl, arylsulfonylalkyl, heteroarylsulfinylalkyl, heteroarylsulfonylalkyl, alkylsulfonyl. alkylsulfonylalkyl, haloalkylsulfinylalkyl, baloalkylsulfonylalkyl, alkylsulfonamido, alkylaminosulfonyl, amidosulfonyl, monoalkyl amidosulfonyl, dialkyl amidosulfonyl, monoarylamidosulfonyl, arylsulfonamido, diarylamidosulfonyl, monoalkyl monoaryl amidosulfonyl, arylsulfinyl, arylsulfonyl, heteroarylthio, heteroarylsulfinyl, heteroarylsulfonyl, alkanoyl, alkenoyl, aroyl, heteroaroyl, aralkanoyl, beteroaralkanoyl, haloalkanoyl, alkyl, alkenyl, alkynyl, alkenyloxy, alkenyloxyalky, alkylenedioxy, haloalkylenedioxy, cycloalkyl, cycloalkenyl, lower cycloalkylalkyl, lower cycloalkenylalkyl, halo, haloalkyl, haloalkenyl, haloalkoxy, hydroxyhaloalkyl, hydroxyaralkyl, hydroxyalkyl, aminoalkyl, hydoxyheteroaralkyl, halaalkoxyalkyl, aryl, aralkyl, aryloxy, aralkoxy, aryloxyalkyl, saturated heterocyclyl, partially saturated heterocyclyl, heteroaryl, heteroaryloxy, heteroaryloxyalkyl, arylalkyl. heteroarylalkyl, arylalkenyl, heteroarylalkenyl, carboxyalkyl, carboalkoxy, carboaralkoxy, carboxamido, carboxamidoalkyl, cyano, carbohaloalkoxy, phosphono, phosphonoalkyl, diaralkoxyphosphono, and diaralkoxyphosphonoalkyl.

Compounds of the present invention can exist in tautomeric, geometric or stereoisomeric forms. The present invention contemplates all such compounds, including cis- and trans-geometric isomers, E- and Z-geometric isomers, R- and S-enantiomers, diastereomers, d-isomers, 1-isomers, the racemic mixtures thereof and other mixtures thereof, as falling within the scope of the invention.
Pharmaceutically acceptable sales of such tautomeric, geometric or stereoisomeric forms are also included within the invention.

The terms "cis" and "trans" denote a form of geometric isomerism in which two carbon atoms connected by a double bond will each have a hydrogen atom on the same side of the double bond ("cis") or on opposite sides of the double bond ("trans").

Some of the compounds described contain alkenyl groups, and are meant to include both cis and trans or "E" and "Z" geometric forms.

Some of the compounds described contain one or more stereocenters and are meant to include R, S, and mixtures of R and S forms for each stereocenter present.

Some of the compounds described herein may contain one or more ketonic or aldehydic carbonyl groups or combinations thereof alone or as part of a heterocyclic ring system. Such carbonyl groups may exist in part or principally in the "keto" form and in part or principally as one or more "enol" forms of each aldehyde and ketone group present. Compounds of the present invention having aldehydic or ketonic carbonyl groups are meant to include both "keto" and "enol" tautomeric forms.

Some of the compounds described herein may contain one or more amide carbonyl groups or combinations thereof alone or as part of a heterocyclic ring system. Such carbonyl groups may exist in part or principally in the "keto" form and in part or principally as one or more "enol" forms of each amide group present. Compounds of the present invention having amidic carbonyl groups are meant to include both "keto" and "enol" tautomeric forms. Said amide carbonyl groups may be both oxo (C=O) and thiono (C=S) in type.

Some of the compounds described herein may contain one or more imine or enamine groups or combinations thereof. Such groups may exist in part or principally in the "imine" form and in part or principally as one or more "enamine" forms of each group present. Compounds of the present invention having said imine or enamine groups are meant to include both "imine" and "enamine" tautomeric forms.

The present invention also comprises the use of a therapeutically-effective amount of a compound of Formula (I) or a pharmaceutically-acceptable salt thereof for a treatment and prophylaxis in anticoagulant therapy for the treatment and prevention of a variety of thrombotic conditions including coronary artery and cerebrovascular disease in a subject.

As a further embodiment, compounds of the present invention of Formula (I) or a pharmaceutically-acceptable salt thereof as defined above. comprise a treatment and prophylaxis of coronary artery disease, cerebrovascular disease and other coagulation cascade related disorders in a subject, comprising administering to the subject having such disorder a therapeutically-effective amount of compounds of formula (I) of the present invention or a pharmaceutically-acceptable salt thereof.

Compounds of the present invention of Formula (I) or a pharmaceutically-acceptable salt thereof can also be used whenever inhibition of blood coagulation is required such as to prevent coagulation of stored whole blood and to prevent coagulation in other biological samples for testing or storage. Thus coagulation inhibitors of the present inhibition can be added to or contacted with stored whole blood and any medium containing or suspected of containing plasma coagulation factors and in which it is desired that blood coagulation be inhibited, e.g. when contacting the mammal's blood with material selected from the group consisting of vascular grafts, stents, orthopedic prothesis, cardiac prosthesis, and extracorporeal circulation systems.

Compounds of Formula (1) are capable of inhibiting activity of serine proteases related to the coagulation cascade, and thus could be used in the manufacture of a medicament, a method for the prophylactic or therapeutic treatment of diseases mediated by coagulation cascade serine proteases, such as inhibiting the formation of blood platelet aggregates, inhibiting the formation of fibrin, inhibiting thrombus formation, and inhibiting embolus formation in a mammal, in blood, in blood products, and in mammalian organs. The compounds also can be used for treating or preventing unstable angina, refractory angina, myocardial infarction, transient ischemic attacks, atrial fibrillation, thrombotic stroke, embolic stroke, deep vein thrombosis, disseminated intravascular coagulation, ocular build up of fibrin, and reocclusion or restenosis of recanalized vessels in a mammal. The compounds also can be used to study the mechanism of action of coagulation cascade serine proteases to enable the design of better inhibitors and development of better assay methods. The compounds of Formula (I) would be also useful in prevention of cerebral vascular accident (CVA) or stroke.

Also included in the family of compounds of Formula (I) are the pharmaceutically-acceptable salts thereof. The term "pharmaceutically-acceptable salt" embraces salts commonly used to form alkali metal salts and to form addition salts of free acids or free bases. The nature of the salt is not critical, provided that it is pharmaceutically acceptable. Suitable pharmaceutically-acceptable acid addition salts of compounds of Formula (I) may be prepared from inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, araliphatic, heterocyclic, carboxylic and sulfonic classes of organic acids, examples of which are formic, acetic, propionic, succinic, glycolic, gluconic, lactic, malic, tartaric, citric, ascorbic, glucoronic, maleic, fumaric, pyruvic, aspartic, glutamic, benzoic, anthranilic, mesylic, salicylic, p-hydroxybenzoic, phenylacetic, mandelic, embonic (pamoic), methanesulfonic, ethylsulfonic, benzenesulfonic, sulfanilic, stearic, cyclohexylaminosulfonic, algenic, galacturonic acid. Suitable pharmaceutically-acceptable base addition salts of compounds of Formula (I) include metallic salts made from aluminum, calcium, lithium, magnesium, potassium, sodium and zinc or organic salts made from N,N'-dibenzylethyleneldiamine, choline, chloroprocaine, diethanolamine, ethylenediamine, meglumine (N-methylglucamine) and procain. All of these salts may be prepared by conventional means from the corresponding compound of Formula (I) by reacting, for example, the appropriate acid or base with the compound of Formula (I).

The present invention also comprises a pharmaceutical composition comprising a therapeutically-effective amount of a compound of Formulas (I) in association with at least one pharmaceutically-acceptable carrier, adjuvant or diluent. Pharmaceutical compositions of the present invention can comprise the active compounds of Formula (I) in association with one or more non-toxic, pharmaceutically-acceptable carriers and/or diluents and/or adjuvants (collectively referred to herein as "carrier" materials) and, if desired, other active ingredients. The active compounds of the present invention may be administered by any suitable route, preferably in the form of a pharmaceutical composition adapted to such a route, and in a dose effective for the treatment intended.

The active compounds and composition may, for example, be administered orally, intravascularly, intraperitoneally, subcutaneously, intramuscularly, oculary, or topically. For treating ocular build up of fibrin, the compounds may be administered intraocularly or topically as well as orally or parenterally.

The compounds can be administered in the form of a depot injection or implant preparation which may be formulated in such a manner as to permit a sustained release of the active ingredient. The active ingredient can be compressed into pellets or small cylinders and implanted subcutaneously or intramusculary as depot injections or implants. Implants may employ inert materials such as biodegradable polymers or synthetic silicones, for example, Silastic, silicone rubber or other silicon containing polymers.

The compounds can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The compounds may also be delivered by the use of monoclonal antibodies as individual carriers to which the compound molecules are coupled. The compounds may also be coupled with soluble polymers as targetable drug carriers. Such polymers can include polyvinylpyrrolidone, pyran copolymer, polyhydroxy-propyl-methacrylamide-phenol, polyhydroxyethyl-aspartamide-phenol, or ployethyleneoxide-polylysine substituted with palmitoyl residues. Furthermore, the compounds may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross linked or amphitpathic block copolymers of hydrogels.

For oral administration, the pharmaceutical composition may be in the form of, for example, tablets, capsules (each of which includes sustained release or timed release formulations), pills, powders, granules, elixers, tinctures, suspensions, liquids including syrups, and emulsions. The pharmaceutical composition is preferably made in the form of a dosage unit containing a particular amount of the active ingredient. Examples of such dosage units are tablets or capsules. The active ingredient may also be administered by injection as a composition wherein, for example, saline, dextrose or water may be used as a suitable carrier.

The amount of therapeutically active compounds which are administered and the dosage regimen for treating a disease condition with the compounds and/or compositions of this invention depends on a variety of factors, including the age, weight, sex and medical condition of the subject, the severity of the disease, the route and frequency of administration, and the particular compound employed, and thus may vary widely.

The pharmaceutical compositions may contain active ingredients in the range of about 0.1 to 2000 mg, and preferably in the range of about 0.5 to 500 mg. A daily dose of about 0.01 to 100 mg/kg body weight, and preferably between about 0.5 and about 20 mg/kg body weight, may be appropriate. The daily dose can be administered in one to four doses per day.

The compounds may be formulated in topical ointment or cream, or as a suppository, containing the active ingredients in a total amount of, for example, 0.075 to 30% w/w, preferably 0.2 to 20% w/w and most preferably 0.4 to 15% w/w. When formulated in an ointment, the active ingredients may be employed with either paraffinic or a water-miscible ointment base.

Alternatively, the active ingredients may be formulated in a cream with an oil-in-water cream base. If desired, the aqueous phase of the cream base may include, for example at least 30% w/w of a polyhydric alcohol such as propylene glycol, butane-1,3-diol, mannitol, sorbitol, glycerol, polyethylene glycol and mixtures thereof. The topical formulation may desirably include a compound which enhances absorption or penetration of the active ingredient through the skin or other affected areas. Examples of such dermal penetration enhancers include dimethylsulfoxide and related analogs. The compounds of this invention can also be administered by a transdermal device. Preferably topical administration will be accomplished using a patch either of the reservoir and porous membrane type or of a solid matrix variety. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

The oily phase of the emulsions of this invention may be constituted from known ingredients in a known manner. While the phase may comprise merely an emulsifier, it may comprise a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make-up the so-called emulsifying wax, and the wax together with the oil and fat make up the so-called emulsifying ointment base which forms the oily dispersed phase of the cream formulations. Emulsifiers and emulsion stabilizers suitable for use in the formulation of the present invention include Tween 60, Span 80, cetostearyl alcohol, myristyl alcohol, glyceryl monostearate, and sodium lauryl sulfate, among others.

The choice of suitable oils or fats for the formulation is based on achieving the desired cosmetic properties, since the solubility of the active compound in most oils likely to be used in pharmaceutical emulsion formulations is very low. Thus, the cream should preferably be a non-greasy, non-staining and washable product with suitable consistency to avoid leakage from tubes or other containers. Straight or branched chain, mono- or dibasic alkyl esters such as diisoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters may be used. These may be used alone or in combination depending on the properties required. Alternatively, high melting point lipids such as white soft paraffin and/or liquid paraffin or other mineral oils can be used.

For therapeutic purposes, the active compounds of the present invention are ordinarily combined with one or more adjuvants appropriate to the indicated route of administration. If administered *per os,* the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

In practicing the methods of the present invention for the treatment and prevention of a variety of thrombotic conditions including coronary artery and cerebrovascular disease, the compounds and pharmaceutical compositions of the present invention are administered alone or in combination with one another, or in combination with other therapeutics or in vivo diagnostic agents. The coagulation cascade inhibitors of the present invention can also be co-administered with suitable anti-platelet agreggation agents, including, but not limited to ticlopidine or clopidrogel, fibrinogen receptor antagonists (e.g. to treat or prevent unstable angina or to prevent reocculsion after angioplasty and restenosis), anti-coagulants such as aspirin, warfarin or heparins, thrombolytic agents such as plasminogen activators or streptokinase to achieve synergistic effects in the treatment of various pathologies, lipid lowering agents including antihypercholesterolemics (e.g. HMG CoA reductase inhibitors such as mevastatin, lovastatin, simvastatin, pravastatin, and fluvastatin, HMG CoA synthatase inhibitors, etc.), anti-diabetic drugs, or other cardiovascular agents (loop diuretics, thiazide type diuretics, nitrates, aldosterone antagonistics (i.e., spironolactone and epoxymexlerenone), angiotensin converting enzyme (e.g. ACE) inhibitors, angiotensin II receptor antagonists, beta-blockers, antiarrythmics, anti-hypertension agents, and calcium channel blockers) to treat or prevent atheriosclerosis. For example, patients suffering from coronary artery disease, and patients subjected to angioplasty procedures, would benefit from coadministration of fibrinogen receptor antagonists and coagulation cascade inhibitors of the present invention. Also, coagulation cascade inhibitors could enhance the efficiency of tissue plasminogen activator-mediated thrombolytic reperfusion.

Typical doses of coagulation cascade inhibitors of the present invention with other suitable anti-platelet agents, anticoagulation agents, cardiovascular therapeutic agents, or thrombolytic agents may be the same as those doses of coagulation cascade inhibitors administered without coadministration of additional anti-platelet agents, anticoagulation agents, cardiovascular therapeutic agents, or thrombolytic agents, or may be substantially less than those doses of coagulation cascade inhibitors administered without coadministration of additional anti-platelet agents, anticoagulation agents, cardiovascular therapeutic agents, or thrombolytic agents, depending on a patient's therapeutic needs.

All mentioned references are incorporated by reference as if here written.

Although this invention has been described with respect to specific embodiments, the details of these embodiments are not to be construed as limitations. The following examples are provided to illustrate the present invention and are not intended to limit the scope thereof. Without further elaboration, it is believed that one skilled in the art can, using the preceding descriptions, utilize the present invention to its fullest extent. Therefore the following preferred specific embodiments are to be construed as merely illustrative and not limitative of the remainder of the disclosure in any way whatsoever. Compounds containing multiple variations of the structural modifications illustrated in the schemes or the following Examples are also contemplated. Those skilled in the art will readily understand that known variations of the conditions and processes of the following preparative procedures can be used to prepare these compounds.

One skilled in the art may use these generic methods to prepare the following specific examples, which have been or may be properly characterized by ¹H NMR, mass spectrometry, elemental composition, and similar procedures. These compounds also may be formed in vivo. The following examples contain detailed descriptions of the methods of preparation of compounds of Formula (I). These detailed descriptions fall within the scope and are presented for illustrative purposes only and are not intended as a restriction on the scope of the invention. All parts are by weight and temperatures are Degrees centigrade unless otherwise indicated.

The following general synthetic sequences are useful in making the present invention. Abbreviations used in the schemes and tables include: "AA" represents amino acids, "AcCN" represents acetonitrile, "AcOH" represents acetic acid, "BINAP" represents 2,2'-bis(diphenylphosphino)-1,1'-binaphthyl, "BnOH" represents benzyl alcohol, "BnCHO" represents 2-phenylethanal," BnSO₂Cl" represents benzylsulfonyl chloride, "Boc" represents *tert*-butyloxycarbonyl, "BOP" represents benzotriazol-1-yl-oxy-tris-(dimethylamino), "bu" represents butyl, "dba" represents dibenzylideneacetone, "DCC" represents 1,3-dicyclohexylcarbodiimide, "DCM" represents dichloromethane or methylene chloride, "DIBAH" or "DIBAL" represents diisobutylaluminum hydride, "DMF" represents dimethylformamide, "DMSO" represents dimethylsulfoxide, "DPPA" represents diphenylphosphoryl azide", "EDC" represents 1-[3-(dimethylamino)propyl]-3-ethylcarbodiimide hydrochloride, "Ex. No." represents Example Number, "Fmoc" represents 9-fluorenylmethoxycarbonyl, "HOBt" represents hydroxybenzoltriazole", "LDA" represents lithium diisopropylamide, "MW" represents molecular weight, "NMM" represents N-methylmorpholine, "Ph" represents phenyl or aryl, "PHTH" represents a phthaloyl group, "pnZ" represents 4-nitrobenzyloxycarbonyl, "PTC" represents a phase transfer catalyst, "py" represents pyridine, " RNH₂" represents a primary organic amine, "p-TsOH" represents paratoluenesulfonic acid, "TBAF" represents tetrabutylammonium fluoride, "TBTU" represents 2-(1H-benzotriozole-1-yl)-1,1,3,3-tetramethyl uronium tetrafluoroborate, "TEA" represents triethylamine, "TFA" represents trifluoroacetic acid, "THF" represents tetrahydrofuran, "TMS" represents trimethylsilyl, "TMSCN" represents trimethylsilyl cyanide, and "Cbz" or "Z" represents benzyloxycarbonyl.

### GENERAL SYNTHETIC PROCEDURES AND SPECIFIC EXAMPLES

The compounds of the present invention can be synthesized, for example, according to the following procedures and Schemes given below.

The general synthetic approach to substituted pyrazinones is shown in **Schemes 1 and 2** below. Treatment of benzyl glycine under Strecker reaction conditions followed by cyclocondensation with oxalyl chloride provides the pyrazinone heterocyclic core with an acetic acid ester at N-1. Heating a solution of the pyrazinone in ethyl acetate in the presence of excess amine results in the nucleophilic displacement of the C-3 chlorine atom by the amine. Stirring the substituted pyrazinone in the presence of lithium hydroxide results in the unmasking of the acid functional group. Alternatively, treatment of the pyrazinone with potassium hydroxide and catalytic palladium on carbon under an atmosphere of hydrogen results in the reductive dechlorination of the C-5 chlorine atom as well as the unmasking of the acid functional group. These acids are then coupled under standard peptide coupling conditions with various amines. These amines are typically multi-functional, and are introduced in a protected form. Removal of these protecting groups in any of several ways provides the compounds for screening. These synthetic schemes are exemplified in specific examples disclosed herein.

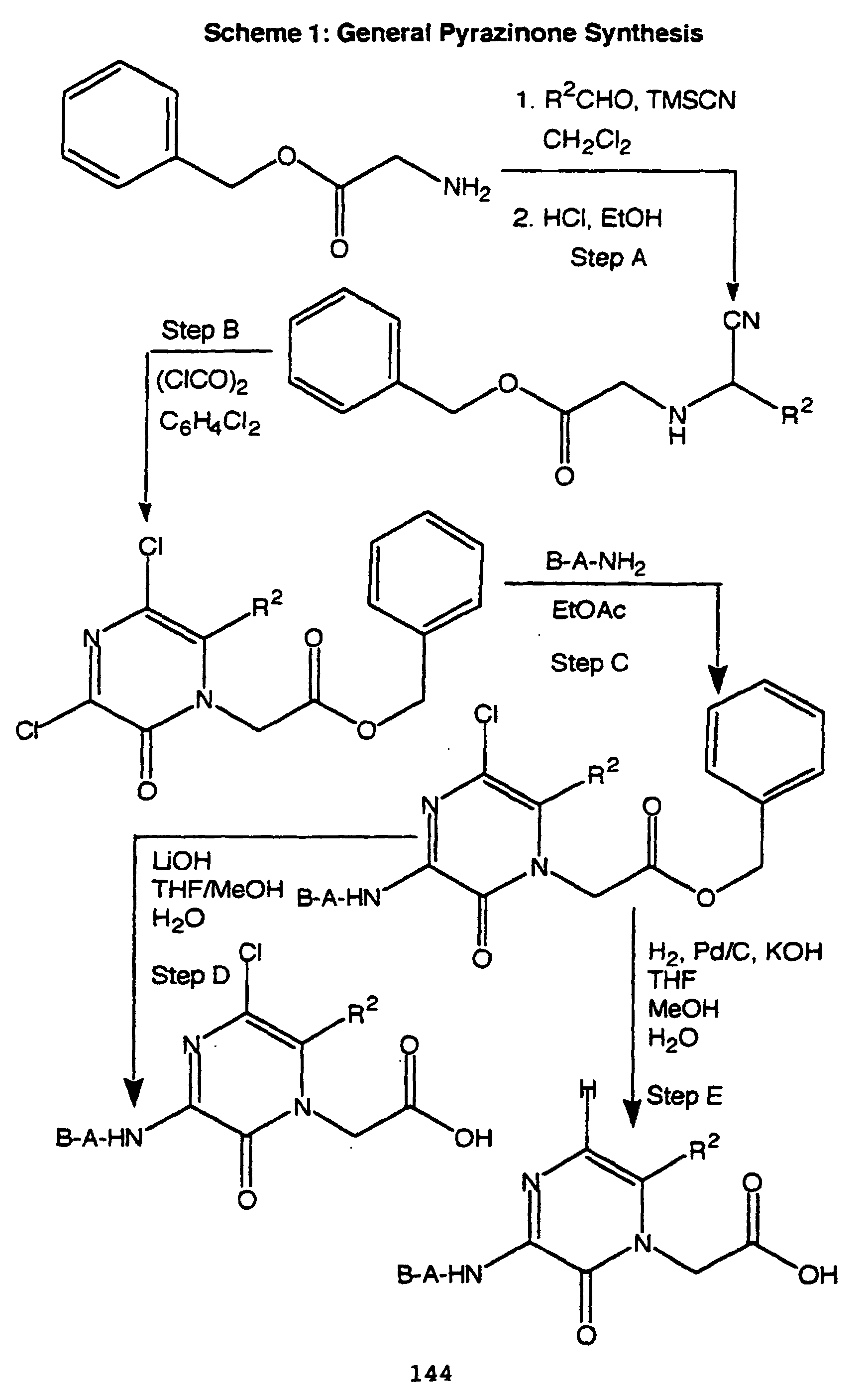

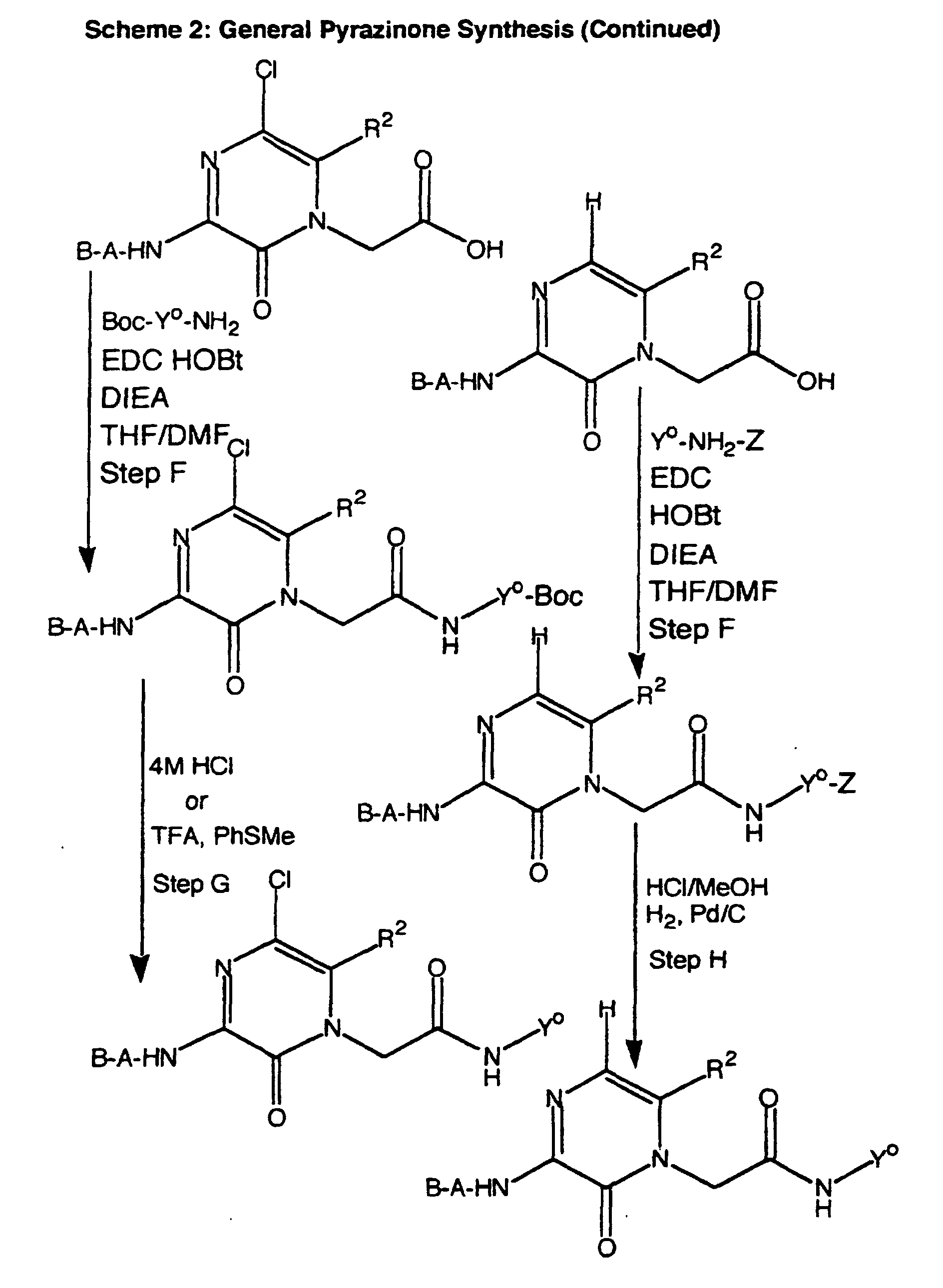

### Example 1

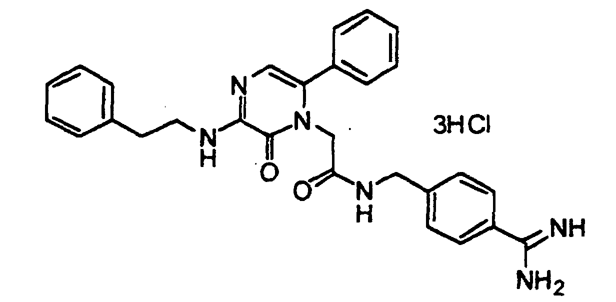

A solution of benzyl glycine hydrochloride (78.00 g, 386.8 mmol) in 1.2 L ethyl acetate was washed with brine and saturated Na₂CO₃ (1:1, 3 x 1L). The organic solution was dried (MgSO₄), filtered, and concentrated. The resulting light yellow oil was placed on the high vacuum for approximately 15 minutes to remove residual solvent. The yellow oil was then diluted with 137.0 mL dichloromethane (2.82 M) and added benzaldehyde (39.30 mL, 386.6 mmol) slowly by syringe at room temperature. The reaction becomes slightly exothermic and turbid. The mixture was then added trimethylsilyl nitrile (51.60 mL, 386.9 mmol) drop wise via syringe over a 10 minute period, upon which a slight exotherm occurs and the reaction becomes clear and golden brown in color. The reaction was stirred for 4 hours at room temperature. The reaction mixture was then concentrated under reduced pressure . The resulting brown oil was diluted with ethyl acetate (500.0 mL), washed with brine (3 x 150 mL), dried (MgSO₄), and concentrated to leave a yellow oil. The oil was diluted ethyl acetate (80 mL) and added 9.9 M HCl (406.4 mmol) in ethanol (prepared by addition of 28.90 mL acetyl chloride to 41.0 mL cold ethanol). Upon which a white precipitate forms exothermically. The precipitate was collected by filtration, washed with ethyl ether, and dried which gave pure benzyl-N-(1-cyanobenzyl)glycine hydrochloride **(EX-1A)** in 35% yield: ¹H NMR (300 MHz, DMSO) δ 9.13-9.00 (br s, 1H) 7.68-7.60 (m, 2H), 7.55-732 (m, 8H) 5.70 (s, 1H), 5.19 (s, 2H), 3.81 (d, *J* = 5.4 Hz, 1H); ¹³C NMR (75 MHz, DMSO) d 168.6, 136.1, 130.7, 129.78, 129.49, 129.17, 128.99, 128.92, 127.10, 67.3, 51.7, 47.1; HRMS (ES) calcd for C₁₇H₁₇N₂O₂ 281.1290, found 281.1311.

A suspension of benzyl-N-(1-cyanobenzyl)glycine hydrochloride (**EX-1A**) (42.90 g, 135.4 mmol) in 135.0 mL dry 1,2-dichlorobenzene (1.0 M) was added to oxalyl chloride (47.50 mL, 544.5 mmol) with stirring at room temperature. The resulting light brown suspension was heated to 100°C for approximately 18 hours. Upon heating to mixture 100°C, the mixture became homogeneous and dark brown in color with gaseous HCl being evolved. The reaction was allowed to cool to room temperature and the volatiles were removed under reduced pressure. The remaining solution was passed through a silica gel column (1 L hexane flush, followed by 2 L 50% ethyl acetate/hexanes). Concentration of the solution gave a dark brown solid. The crude product was purified by MPLC (2 L hexane flush to 25% ethyl acetate/hexanes) to gave pure 1-Benzyloxycarbonylmethyl-3,5-dichloro-6-phenylpyraziaone (**EX-1B**) in 60% yield as a yellow solid: ¹H NMR (300 MHz, CDCl₃) δ 7.58-7.37 (m, 6H), 7.31-7.26 (m, 4H), 5.18 (s, 2H), 4.53 (s, 2H); ¹³C NMR (75 MHz, CDCl₃) δ 166.4, 152.4, 146.0, 138.3. 134.9, 131.1, 130.0, 129.8, 129.1, 129.0, 128.8, 124.3 , 68.2, 49.5; HRMS (ES) calcd for C₁₉H₁₅Cl₂N₂O₃ 389.0460, found 389.0475.

A solution of 1-benzyloxycarbonylmethyl-3,5-dichloro-6-phenylpyrazinone (**EX-1B**) (10.19 g, 26.19 mmol) in 103.0 mL ethyl acetate (0.255M) was added 9.90 mL phenethyl amine in one portion at room temperature. The resulting solution was heated to reflux for 18 hours. The solution was allowed to cool to room temperature which resulted in a thick precipitate forming. The reaction mixture was diluted with ethyl acetate (750.0 mL) and was washed with 0.5 N HCl (1 x 250 mL), saturated NaHCO₃ (1 x 250 mL) and brine (1 x 250 mL). The organic solution was dried (MgSO₄), filtered and concentrated to give the crude product. Recrystallization from ethyl acetate and hexanes afforded pure 3-(2-phenylethylamino)-5-chloro-6-phenyl-1-benzyloxycarbonylmethylpyrazinone (**EX-1C**) as light yellow crystals in 96% yield: ¹H NMR (300 MHz, CDCl₃) δ 7.46-7.28 (m, 15H), 639 (br s, 1H), 5.25 (s, 2H), 4.54 (s, 2H), 3.81-3.79 (m, 2H), 3.04-3.00 (m, 2H); ¹³C NMR (75 MHz, CDCl₃) δ 167.2, 151.3, 149.2, 138.9, 135.2, 131.9, 130.7, 129.9, 129.3, 129.0, 128.95, 128.86, 128.83, 128.7, 126.9, 123.3, 67.7, 47.9, 42.5, 35.4; HRMS (EI) calcd for C₂₇H₂₅ClN₃O₃ 474.1584, found 474.1591.

A suspension of 3-(2-phenylethylamino)-5-chloro-6-phenyl-1-benzyloxycarbonylmethylpyrazinone (**EX-1C**) (1.26 g, 2.66 mmol) in 27.0 mL tetrahydrofuran and ethanol (1:1, 0.12 M) was added potassium hydroxide (463.1 mg, 8.25 mmol) in 4.0 mL water. The resulting solution was degassed (via high vacuum) three times. The solution was then added 421.1 mg 5 % Pd/C in one portion. The resulting mixture was then stirred under an atmosphere of hydrogen overnight. The reaction mixture was filtered through a pad of Celite 545 then concentrated under reduced pressure to half of the original volume. The solution was then diluted with brine and acidified with 20% (w/w) KHSO₄ to a pH of 1. The resulting turbid solution was extracted with ethyl acetate (4 x 25 mL). The combined organic solutions were washed with brine (1 x 25 mL), dried (MgSO₄), filtered, concentrated to give pure 3-(2-phenethylamino)-6-phenyl-1-methylenecarboxypyrazinone (**EX-1D**) in 97 % yield as a white solid: ¹H NMR (300 MHz, DMSO) δ 7.49-7.48 (m, 3H), 7.40-7.23 (m, 7H), 6.77 (s, 1H), 4.52 (s, 1H), 4.40 (s, 2H), 3.64-3.57 (m, 2H), 2.93 (t, *J* = 7.4 Hz, 2H); ¹³C NMR (75 MHz, DMSO) δ 169.6, 151.8, 150.6, 150.5, 143.2, 140.3, 133.2, 130.2. 129.6, 129.4, 1293, 129.1, 128.8, 128.7, 1273, 127.1, 126.8, 122.3, 63.6, 47.5,42.5,35.2; HRMS (EI) calcd for C₂₀H₂₀N₃O₃ 350.1505, found 350.1502. p-Cyanobenzaldehyde (38.13 mmoles, 5g) was stirred in 50 mL of tetrahydrofuran at 0°C under nitrogen while lithium bis(trimethylsilyl)amide (83.89 mmoles, 84 mL of a 1.0M solution in tetrahydrofuran) was added dropwise over 10 min. After addition the mixture was allowed to warm to room temperature and stirred for 3 hr. Water (50 mL) was then added and stirring continued for 30 min. Then 2.5N sodium hydroxide (763 mmoles, 305 mL) and di-tert-butyl dicarbonate (83.89 mmoles, 18309g) were added along with tetrahydrofuran (100 mL) and the mixture was allowed to stir for 3 hr. The layers were then separated. The tetrahydrofuran layer was diluted with ethyl acetate and washed with brine. The water layer was extracted twice with ethyl acetate. The combined organic layers were dried over magnesium sulfate, filtered, the solvent removed in vacuo. The residue was chromatographed medium pressure liquid chromatography with 30% ethyl acetate/hexanes to give 4.03g of desired 4-(t-butoxycarbonylamidino)-benzaldehyde in 43% yield. ¹H NMR (300 MHz, CDCl₃) δ10.03 (s, 1H), 7.97 (d, 2H) 7.89 (d, 2H), 1.53 (s, 9H).

The 4-(t-butoxycarbonylamidino)benzaldehyde (4.03 mmoles, 1.0g) was stirred in tetrahydrofuran (20 mL) at room temperature under nitrogen while allylamine (6.05 mmoles, 453 uL) was added dropwise. After addition the mixture was allowed to stir for 6 hr. The mixture was diluted with methanol (20 mL) and cooled to 0°C. Then sodium borohydride (6.04 mmoles, 22.8 mg) added in small amounts and allowed to warm to room temperature. After 2 hr the reaction was quenched with water and extracted with dichloromethane. The organic layer was dried over magnesium sulfate, filtered, and the solvent removed in vacuo. The oily residue solidified on standing. The N-allyl-4-(t-butoxycarbonylamidino)-benzylamine product was used without further purification. ¹H NMR (300 MHz, CDCl₃) δ 7.79 (d, 2H), 7.37 (d, 2H), 5.90-6.10 (m, 1H), 5.19 (dd, 2H), 3.81 (s, 2H), 3.24 (d, 2H), 1.53 (s, 9H)

The N-allyl-4-(t-butoxycarbonylamidino)benzylamine (3.97 mmoles, 1.15g) and chlorotris(triphenylphosphine)-rhodium(I) (0.21 mmoles, 195 mg) was stirred in acetonitrile/water (84:16,92 mL) under nitrogen. The mixture was refluxed for 3 hr and allowed to cool to room temperature. Then the mixture was filtered through a pad of celite and the solvent removed in vacuo. The residue was dried on a high vacuum pump to yield an orange glassy product. 4-(t-Butoxycarbonylamidino)benzylamine product was verified by HPLC/MS and used without further purification.

A solution of 3-(2-phenethylamino)-6-phenyl-1-methylenecarboxypyrazinone **(EX-1D)** (521.1 mg, 1.491 mmol) in 15.0 mL tetrahydrofuran and dimethylformamide (1:1, 0.1 M) was added N,N-diisopropylethylamine (130 mL, 7.463 mmol), N-hydroxybenzotriazole (610.5 mg, 4.518 mmol), and 1-[3-(dimethylamino)propyl]-3-ethylcarbodiimide hydrochloride (8553 mg, 4.461 mmol). The resulting mixture was allowed to stir for 30 minutes. The reaction mixture was then added 4-(t-butoxycarbonylamidino)benzylamine (763.1 mg, 3.061 mmol) prepared above in one portion. The resulting mixture was allowed to stir over night. The reaction mixture was diluted with ethyl acetate (50 mL) and washed with 5% citric acid (1 x 25 mL), saturated NaHCO₃ (1 x 25 mL), and brine (1 x 25 mL). The organic solution was dried (MaSO₄), filtered and concentrated. The crude reaction was purified by MPLC (75% ethyl acetate/hexanes) to give the product (**EX-1E**): ¹H NMR (300 MHz, DMSO) δ 9.06 (br s, 1 H), 8.65 (t, *J* = 5.6 Hz, 1 H), 7.94 (d, *J* = 8.1 Hz, 2H), 7.47-7.40 (m, 6H),7.35-7.21 (m, 8H), 6.75 (s, 1H), 4.41 (s, 2H), 4.36-4.34 (m, 2H), 3.63-3.57 (m, 2H), 2.93 (t, *J* = 7.3 Hz, 2H), 1.48 (s, 9H); ¹³C NMR (75 MHz, DMSO) δ 167.3, 151.9, 150.7, 143.7, 140.4, 133.8, 133.4, 130.3, 129.4, 129.34, 129.29, 129.15, 129.10, 128.3, 127.6, 126.8, 122.2, 78.5, 48.7, 42.6, 35.2, 28.7; HRMS (EI) calcd for C₃₃H₃₇N₆O₄ 581.2876, found 581.2871.

A flask of protected pyrazinone (260.7 mg, 0.449 mmol) was added 5.0 mL of 4 M HCl in dioxane. The resulting solution was allowed to stir overnight (approximately 18 hours). The solution was concentrated and the crude product was triturated from ethyl ether. The resulting white solid was collected by filtration, washed with ethyl ether and dried to give pure product: ¹H NMR (300 MHz, DMSO) δ 9.57 (br s, 2H), 9.38 (br s, 2H), 9.06 (br s, 1H), 7.88 (d, *J* = 7.9 Hz, 2H), 7.55-7.52 (m, 3H), 7.42-7.24 (m, 9H), 6.66 (s, 1H), 4.43 (s, 2H), 4.38-4.37 (m, 2H), 3.83 (br s, 2H), 3.03-2.98 (m, 2H); HRMS (EI) calcd for C₂₈H₂₉N₆O₂ 481.2352, found 481.2348.

### Example 2

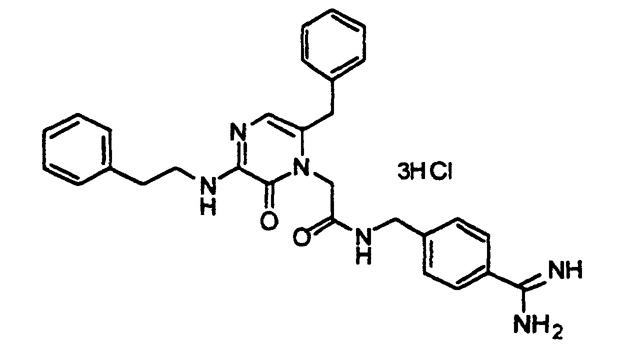

By following the method of **Example 1** and substituting phenylacetaldehyde for benzaldehyde, the compound was prepared: ¹H NMR (400 MHz, DMSO) δ 9.43 (s, 2H), 9.25 (s, 2H), 8.84 (br s, 1H), 7.79 (d, *J* = 8.1 Hz, 2H), 7.40-7.16 (m, 12H), 6.61 (s, 1H), 4.47 (s, 2H), 4.27 (s, 2H), 3.86 (s, 2H), 3.75 (br s, 2H), 2.94-2.90 (m, 2H); HRMS (EI) calcd for C₂₉H₃₀N₆O₂ 494.2430, found 494.2438.

### Example 3

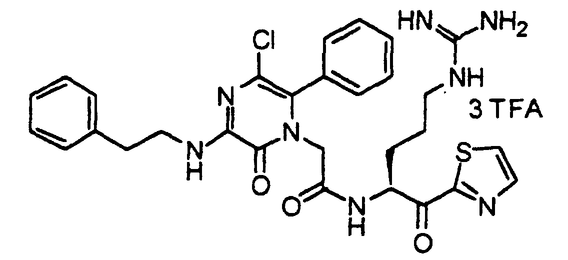

A suspension of 3-(2-phenylethylamino)-5-chloro-6-phenyl-1-benzyloxycarbonylmethylpyrazinone (1.35 g, 2.85 mmol) in 28.0 mL tetrahydrofuran, methanol and water (3:3:1, 0.10 M) was added potassium hydroxide (0.50 g, 8.93 mmol). The mixture was then stirred 3 hours. The reaction mixture was concentrated under reduced pressure to half of the original volume. The solution was then diluted with brine and acidified with 20% (w/w) KHSO₄ to a pH of 1. The resulting turbid solution was extracted with ethyl acetate (4 x 25 mL). The combined organic solutions were washed with brine (1 x 25 mL), dried (MgSO₄), filtered, concentrated to give pure **EX-3A** (3-(2-phenethylamino)-5-chloro-6-phenyl-1-methylenecarboxypyrazinone) in 88% yield as a white solid: ¹H NMR (300 MHz, DMSO) δ 13.15 (br s, 1H), 7.84 (br s, 1H), 7.51-7.50 (br m, 3H), 7.33-7.24 (m, 7H), 4.24 (s, 2H), 3.58 (br s, 2H), 2.94 (br s, 2H); ¹³C NMR (75 MHz, DMSO) δ 169.2, 151.0, 149.6, 140.1, 132.4, 131.1, 130.2, 129.6, 129.3, 129.1, 126.9, 125.4, 123.4, 48.1, 42.8, 34.8; HRMS (EI) calcd for C₂₀H₁₉ClN₃O₃ 384.1115, found 384.1118.

A solution of (S)-N-[[[4-amino-5-oxo-5-(thiazolyl)pentyl]amino] iminomethyl]-4-methoxy-2,3,6-trimethylbenzenesulfonamide dihydrochloride (1.664 g, 3.161 mmol) in 29.0 mL tetrahydrofuran and dimethylformamide (1:1, 0.10 M) was added N,N-diisopropylethylamine (5.00 mL, 28.70 mmol). The resulting mixture was allowed to stir for 10 minutes at room temperature. The solution was then added 3-(2-Phenylethylamino)-5-chloro-6-phenyl-1-methylenecarboxypyrazinone (1.104 g, 2.877 mmol), N-hydroxybenzotriazole (466.8 mg, 3.454 mmol), and 1-[3-(dimethylamino)propyl]-3-ethylcarbodiimide hydrochloride (673.1 mg, 3.511 mmol). After the addition was complete the solution was allowed to stir over night. The reaction mixture was diluted with ethyl acetate (50 mL). The organic solution was washed with 5% citric acid (1 x 25 mL), saturated NaHCO₃ (1 x 25 mL), and brine (1 x 25 mL). The organic solution was dried (MgSO₄), filtered and concentrated. The crude reaction mixture was purified by MPLC (75% ethyl acetate/hexanes) to give pure product **EX-3B**: ¹H NMR (300 MHz, CDCl₃) δ 8.04 (d, *J* = 3.0 Hz, 1H), 7.75 (d, *J* = 2.8 Hz, 1H), 7.57 (br s, 1H), 7.42-7.21 (m, 11H), 6.54 (s, 2H), 6.31 (s. 2H), 5.63 (br s, 1H), 4.60 (d, *J* = 16.2 Hz, 1H), 4.21 (d, J = 16.5 Hz, 1H), 3.84 (s, 3H), 3.77-3.66 (m, 2H), 3.17 (br s, 1H), 2.96 (d, *J* = 7.2 Hz, 2H), 2.68 (s, 3H), 2.60 (s, 3H), 2.14 (s, 3H), 1.79-1.66 (m, 3H); HRMS (EI) calcd for C₃₉H₄₄ClN₈O₆S₂ 819.2514, found 819.2512.

A solution of material **EX-3B** (928.1 mg, 1.133 mmol) in 113 mL trifluoroacetic acid (0.1 M) was added to thioanisole (0.400 mL, 3.407 mmol) at room temperature with stirring. The resulting mixture was allowed to stir 6 hours. The reaction mixture was concentrated under reduced pressure. The crude product was purified by trituration from ethyl ether. A yellow powder was collected by filtration, washed with ethyl ether to give the pure product: ¹H NMR (300 MHz, DMF) δ 8.76 (d, *J* = 7.2 Hz, 1H), 8.51-8.50 (m, 1H), 8.42-8.41 (m, 1H), 8.17 (br s, 1H), 7.92-7.45 (m, 10 H), 5.74 (br s, 1H), 4.67-4.65 (m, 2H), 3.89-3.87 (m, 2H), 3.52 (br s, 2H), 3.23-3.20 (m, 2H), 2.73 (s, 2H), 2.19 (br s, 1H), 1.88 (br s, 3H); HRMS (EI) calcd for C₂₉H₃₂ClN₈O₃S 607.2007, found 607.2000.

### Example 4

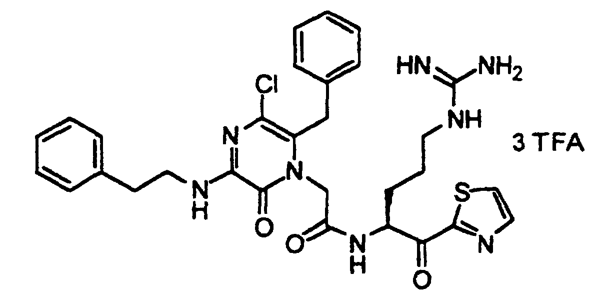

By following the method of **Example 3** and substituting 3-(2-phenethylamino)-5-chloro-6-benzyl-1-methylenecarboxypyrazinone for 3-(2-phenethylamino)-5-chloro-6-phenyl-1-methylenecarboxypyrazinone, the title compound was prepared: HRMS (EI) calcd for C₃₀H₃₄ClN₈O₃S 621.2163, found 621.2171.

### Example 5

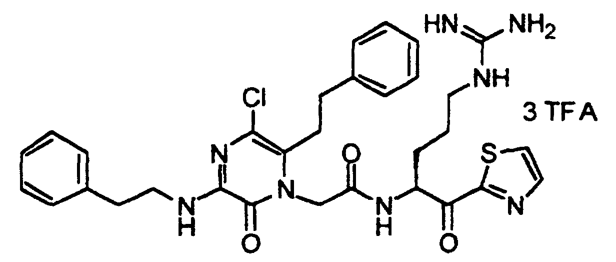

By following the method of **Example 3** and substituting 3-(2-phenethylamino)-5-chloro-6-(2-phenylethyl)-1-methylenecarboxypyrazinone for 3-(2-phenethylamino)-5-chloro-6-phenyl-1-methylenecarboxypyrazinone, the title compound was prepared: HRMS (EI) calcd for C₃₁H₃₆ClN₈O₃S 635.2320, found 635.2330.

### Example 6

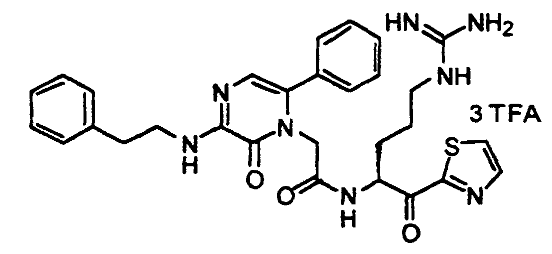

By following the method of **Example 3** and substituting 3-(2-phenethylamino)-6-phenyl-1-methylenecarboxypyrazinone for 3-(2-phenethylamino)-5-chloro-6-phenyl-1-methylenecarboxypyrazinone, the title compound was prepared: HRMS (EI) calcd for C₂₉H₃₃N₆O₃S 573.2396, found 573.2399.

### Example 7

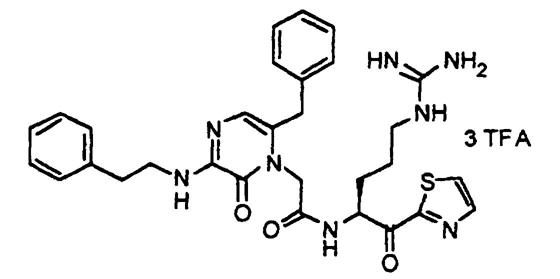

By following the method of **Example 3** and substituting 3-(2-phenethylamino)-6-benzyl-1-methylenecarboxypyrazinone for 3-(2-phenethylamino)-5-chloro-6-phenyl-1-methylenecarboxypyrazinone, the tide compound was prepared: HRMS (EI) calcd for C₃₀H₃₅N₈O₃S 587.2553, found 587.2564.

### Example 8

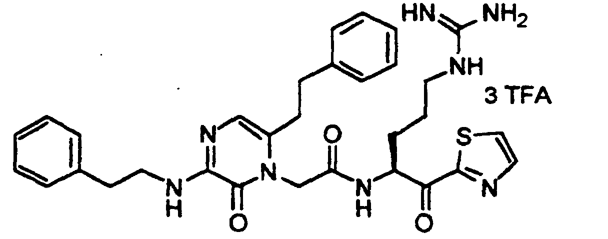

By following the method of **Example 3** and substituting 3-(2-phenethylamino)-6-(2-phenylethyl)-1-methylenecarboxypyrazinone for 3-(2-phenethylamino)-5-chloro-6-phenyl-1-methylenecarboxypyrazinone, the tide compound was prepared: HRMS (El) calcd for C₃₁H₃₇N₈O₃S 601.2709, found 601.2714.

### Example 9

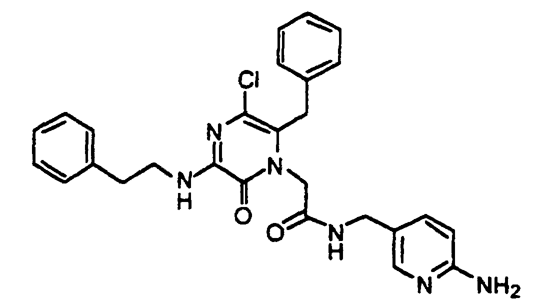

A solution of 2-amino-5-aminomethylpyridine in 1.60 mL tetrahydrofuran (0.13 M) was added to N,N-diisopropylethylamine (0.145 mL, 0.832 mmol). The resulting mixture was allowed to stir for 10 minutes at room temperature. The solution was then added to 3-(2-Phenylethylamino)-6-benzyl-5-chloro-1-methylenecarboxypyrazinone (81.6 mg, 0.2051 mmol), N-hydroxybenzotriazole (38.1 mg, 0.2819 mmol), and 1-[3-(dimethylamino)propyl]-3-ethylcarbodiimide hydrochloride (49.6 mg, 3.511 mmol). The reaction mixture was then allowed to stir over night. The reaction mixture was diluted with ethyl acetate (50 mL). The organic solution was washed with 5% citric acid (1 x 25 mL), saturated NaHCO₃ (1 x 25 mL), and brine (1 x 25 mL). The organic solution was dried (MgSO₄), filtered and concentrated. The crude reaction mixture was purified by MPLC (ethyl acetate) to give pure product: ¹H NMR (300 MHz, DMSO) δ 8.50 (br s, 1H), 7.83 (s, 1H), 7.68 (br s, 1H), 7.34-7.19 (m, 13H), 6.46-6.43 (m, 1H), 5.90 (br s, 1H), 4.42 (s, 2H), 4.09 (br s, 2H), 3.98 (br s, 2H), 3.56 (br s, 3H), 2.94 (br s, 3H); HRMS (EI) calcd for C₂₇H₂₈ClN₆O₂ 503.1962, found 503.1968.

### Example 10

### 3-(2-Phenylethylamino)-5-chloro-6-phenethyl-1-(2-amino-5-methylcarboxamidomethylpyridinyl)pyrazinone

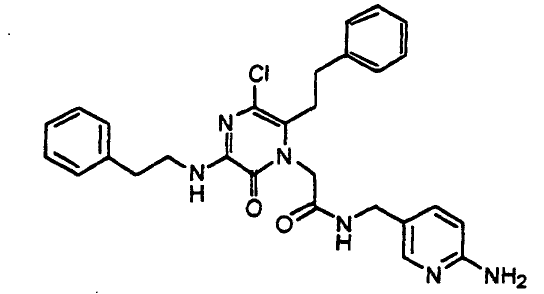

By following the method of **Example 9** and substituting 3-(2-phenethylamino)-5-chloro-6-(2-phenylethyl)-1-methylenecarboxypyrazinone for 3-(2-phenethylamino)-5-chloro-6-benzyl-1-methylenecarboxypyrazinone, the title compound was prepared: HRMS (EI) calcd for C₂₈H₃₀N₆O₂ 517.2119, found 517.2127.

### Example 11

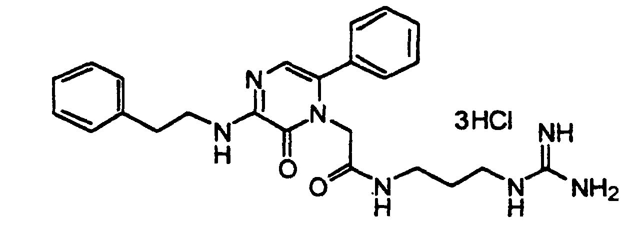

A solution of 3-(2-phenethylamino)-6-phenyl-1-methylenecarboxypyrazinone (217.6 mg, 0.6228 mmol) in 63 mL tetrahydrofuran and dimethylformamide (1:1, 0.1 M) was added N,N-diisopropylethylamine (1.00 mL, 5.741 mmol), N-hydroxybenzotriazole (171.1 me, 1.266 mmol). and 1-[3-(dimethylamino)propyl)-3-ethylcarbodiimide hydrochloride (240.0 mg, 1.252 mmol). The resulting mixture was allowed to stir for 30 minutes. The reaction mixture was then added to the 3-(di-Boc-guanidino)propanamine (1.30 mg, 3.684 mmol) in one portion. The resulting mixture was allowed to stir over night. The reaction mixture was diluted with ethyl acetate (50 mL) and washed with 5% citric acid (1 x 25 mL), saturated NaHCO₃ (1 x 25 mL), and brine (1 x 25 mL). The organic solution was dried (MgSO₄), filtered and concentrated. The crude reaction was purified by MPLC (75% ethyl acetate/hexanes) to give the product **EX-11A**: ¹H NMR (300 MHz, DMSO) δ 11.42 (s, 1H), 8.46 (t, *J* = 6.3 Hz, 1H), 7.92 (t, J = 6.0 Hz, 1H), 7.46-7.41 (m, 5H), 7.37-7.23 (m, 5H), 6.86 (s, 1H), 6.24 (br s, 1H), 4.49 (s, 2H), 3.79-3.72 (m, 2H), 3.48-3.42 (m, 2H), 3.31-3.25 (m. 2H), 3.00 (t, *J* = 7.1 Hz, 2 H), 1.53 (s, 9H), 1.40 (s, 9H); HRMS (EI) calcd for C₃₄H₄₅N₇O₆ 648.3510, found 648.3498.

A flask of protected guanidine **EX-11A** (260.7 mg, 0.449 mmol) was added to 5.0 mL of 4 M HCl in dioxane. The resulting solution was allowed to stir for 4 hours. The solution was concentrated and the crude product was triturated from ethyl ether. The resulting white solid was collected by filtration, washed with ethyl ether and dried to give pure product: ¹H NMR (300 MHz, DMSO) δ 9.65 (br s, 1H), 8.48 (t, *J* = 5.1 Hz, 1H), 7.96 (t, *J* = 5.4 Hz, 1H), 7.60-7.22 (m, 13H), 6.66 (s, 1H), 4.32 (s, 2H), 3.82 (br s, 2H), 3.13-3.09 (m, 2H), 3.03-2.98 (m, 2H), 1.59-1.54 (m, 2H); HRMS (EI) calcd for C₂₄H₂₉N₇O₂ 448.2461, found 448.2425.

### Example 12

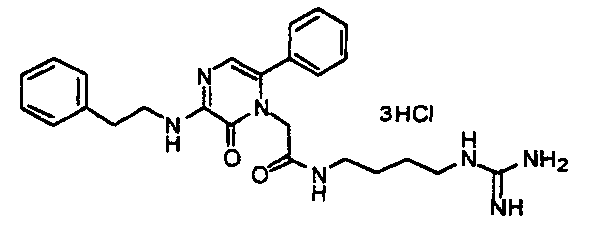

By following the method of **Example 11** and using the appropriate butanamine, the title compound was prepared: HRMS (EI) calcd for C₂₅H₃₁N₇O₂ 462.2617, found 462.2575.

### Example 13

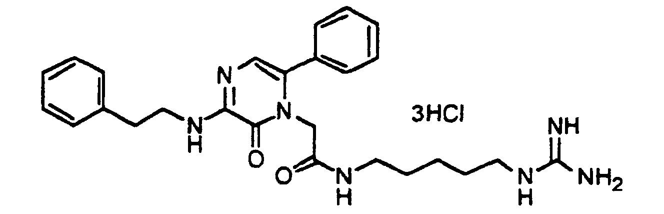

By following the method of **Example 11** and using the appropriate pentanamine, the title compound was prepared: HRMS (EI) calcd for C₂₆H₃₃N₇O₂ 476.2774, found 476.2783.

### Example 14

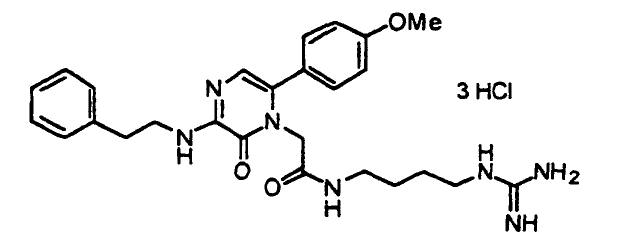

By following the method of **Example 11** and using the appropriate butanamine with 3-(2-phenethylamino)-6-(4-methoxyphenyl)-1-methylenecarboxypyrazinone, title compound was prepared: HRMS (EI) calcd for C₂₆H₃₄N₇O₃ 492.2723, found 492.2693.

### Example 15

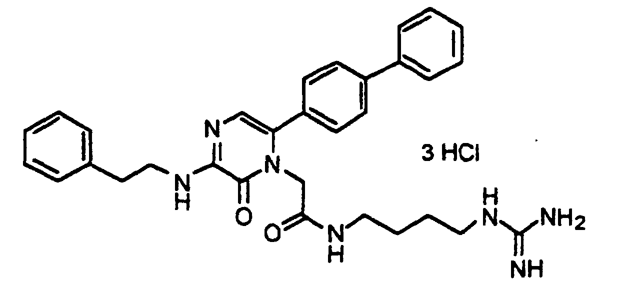

By following the method of **Example 11** and using the appropriate butanamine with 3-(2-phenethylamino)-6-(4-biphenyl)-1-methylenecarboxypyrazinone, the title compound was prepared: HRMS (EI) calcd for C₃₁H₃₆N₇O₂ 538.2930, found 538.2918.

### Example 16

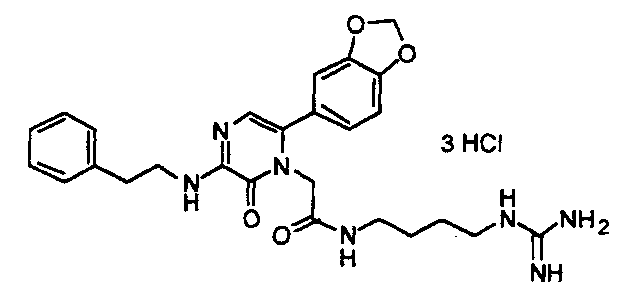

By following the method of **Example 11** and using the appropriate butanamine with 3-(2-phenethylamino)-6-(3,4-methylenedioxyphenyl)-1-methylenecarboxypyrazinone, the title compound was prepared: HRMS (EI) calcd for C₂₆H₃₂N₇O₄ 506.2516. found 506.2506.

### Example 17

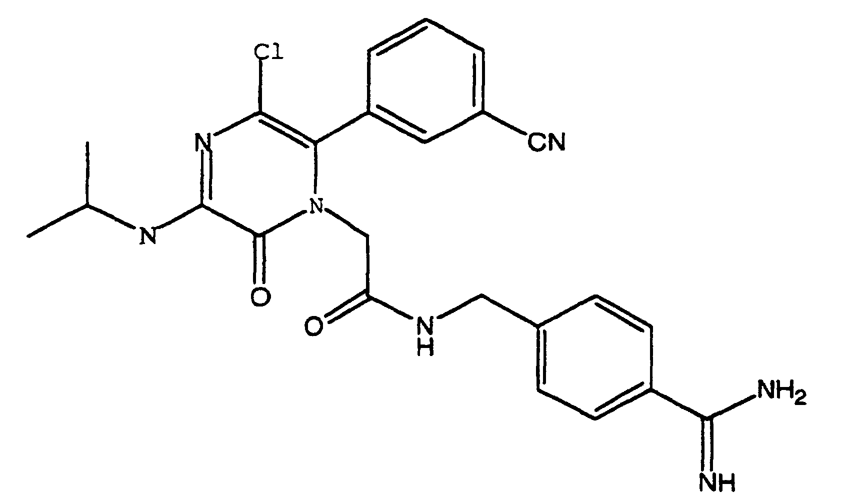

Using the procedures of Schemes 1 and 2 and **Example 1,** 2-{5-chloro-6-(3-bromophenyl)-3-[(methylethyl)amino]-2-oxohydropyrazinyl}acetic acid was prepared.

A solution of 2-{5-chloro-6-(3-bromophenyl)-3-[(methylethyl)amino]-2-oxohydropyrazinyl}acetic acid (7.4 g, 18.47 mmol) and copper (I) cyanide (1.75 g, 19.55 mmol) in 75.0 mL dimethylsulfoxide was heated at 150°C for 20 hours. The flask was then cooled and the contents were poured into a solution of 500 mL water and 100 mL 1M HCl. The mixture was then extracted with ethyl acetate (2 x 1 L). The ethyl acetate layers were separated, combined, dried over magnesium sulfate, filtered, and stripped of solvent under reduced pressure. Purification by HPLC (25% ethyl acetate in hexanes) provided 2-{5-chloro-6-(3-cyanophenyl)-3-[(methylethyl)amino]-2-oxohydropyrazinyl}acetic acid (**EX-17A**) of adequate purity: ¹H NMR (400 MHz, CDCl₃) d 7.8-7.4 (br, 4H), 4.4 (br, 2H), 2.2 (br, 1H), 1.3 (br d, 6H); MS (ES) calcd for C₁₅H₁₅ClN₄O₃ 346, found 347 (M+H).

2-{5-chloro-6-(3-cyanophenyl)-3-[(methylethyl)amino]-2-oxohydropyrazinyl}acetic acid (**EX-17A**) was converted to the product as described in **Example 1**. Mass spectral analysis gave an m/z+1 of 478.

### Example 18

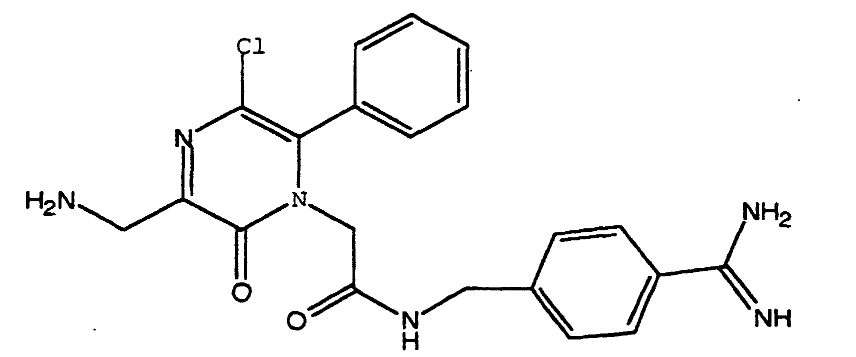

A solution of 3.90 g (10 mmol) of **EX-1B** in 50 mL of CH₃NO₂ was treated with 4.5 mL of 1,8-diazabicyclo[5.4.0]undec-7-ene (DBU). After 30 minutes, the reaction was poured into 300 mL of ethyl acetate and washed with 2 x 25 mL 1 N HCl (aq). The excess organic solvent was removed under reduced pressure. The resulting oil was treated with 25 mL H₂O and 25 mL CH₃OH. The solution was treated with 2.8 g of KOH. After 60 minutes, the reaction was diluted with 300 mL of acetonitrile. The resulting solid was washed with 100 mL of acetonitrile. The solid was dissolved in 50 mL 1N HCl (aq) and extracted with 2 x 100 mL CH₂Cl₂. The organic layer was dried with MgSO₄, and the excess solvent removed under reduced pressure. The resulting solid was washed with diethyl ether and dried in ambient conditions to give 2.1 g (6.4 mmol; 64% yield) of desired product (**EX-18A**). LC/MS showed a single peak at 254 nm and a M+Na at 346. ¹H-NMR (dmso-d₆): 4.4 ppm (2H, s); 5.6 ppm (2H, s); 7.3 - 7.5 ppm (5H, m)

The product was obtained using standard coupling conditions and deprotection methods under reducing conditions of **Example 1** to give the desired product after purification by HPLC. LC/MS showed a single peak at 254 nm and a M+H at 425. ¹H-NMR (dmso-d₆): 4.2 ppm (2H, s); 4.4 ppm (2H, m); 4.6 ppm (2H, m); 7.4 ppm (5H, m) 7.6 - 7.8 ppm (4H, m); 8.4 ppm (2H, bs); 8.7 (1H,m); 9.1 ppm (2H, bs); 9.3 ppm (2H,bs).

Using the procedures of **Scheme 1**, **Scheme 2**, and **Example 1** through **Example 18** with suitable reagents, starting materials, intermediates, and additional pyrazinones of the present invention were prepared by one skilled in the art using similar methods and these pryazinones summarized in Table 1.

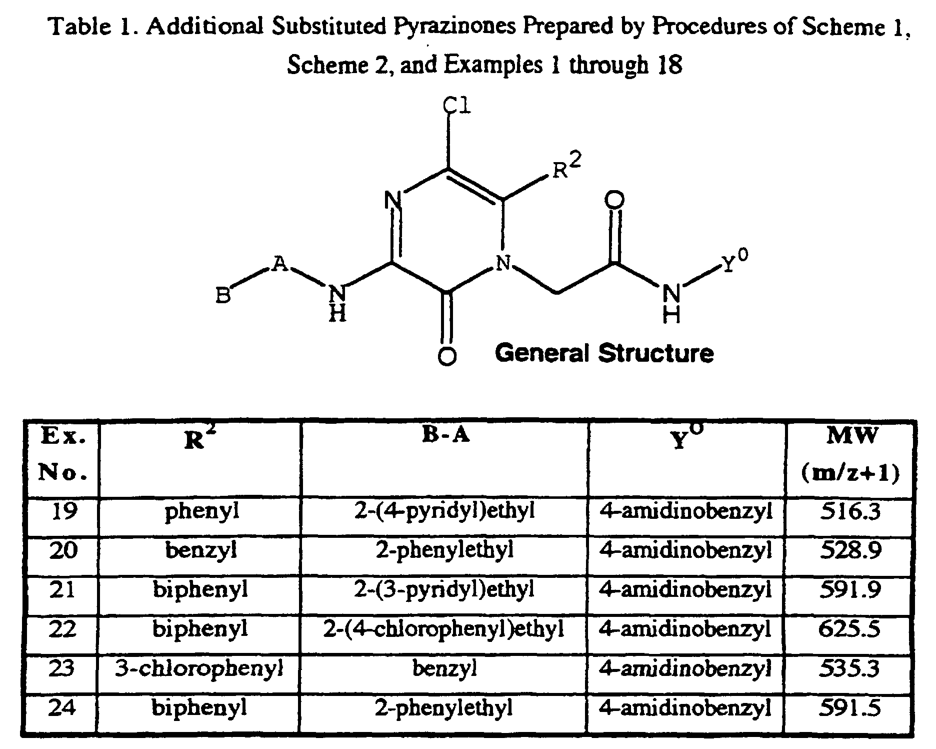

### Example 25

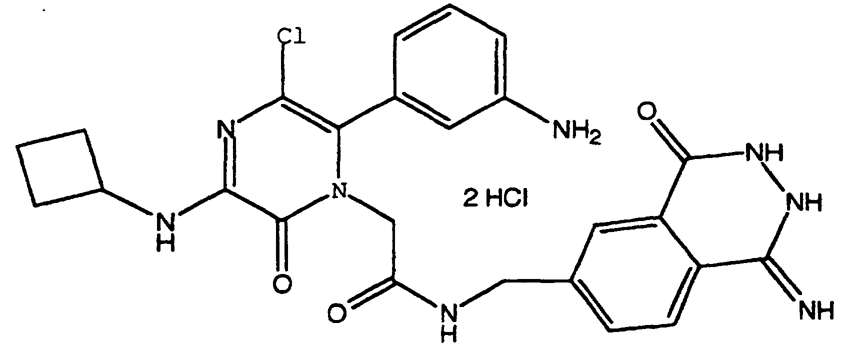

To a solution of 2-iodo-5-methyl benzoic acid (10.0g, 0.038 mol) in toluene (200 mL) was added trimethylorthoacetate (25 mL) at room temperature. The reaction mixture was refluxed for 12 hours. The reaction mixture was cooled to room temperature and diluted with saturated sodium bicarbonate and ethyl acetate. The layers were separated and the organic layer washed with brine. The organic layer was dried (MaSO₄) and solvent removed to give 10.35 g of methyl 2-iodo-5-methylbenzoate (**EX-25A**) as a yellow oil with an m/z +1 = 277.

A degasssed mixture of ester **EX-25A** (10.35 g, 0.037 mol)), Pd(dba)₃ (0.017 g, 0.018 mmol)), dppf (0.025 g, 0.045 mmol)) and Zn(CN)₂ (2.6 g, 0.02 mol) in DMF (100 mL) was heated to 120 °C for 2 hours. The reaction mixture was poured into water and ethyl acetate. The organic layer was washed with water (2x) and brine (1x). The organic layer was collected, dried (MgSO₄) and the solvent removed *in vacuo* to give 5.28 g methyl 2-cyano-5-methylbenzoate (**EX-25B**) as a brownish oil with m/z +1 = 176

To a solution of **EX-25B** (5.28 g, 0.03 mol) in CCl₄ (100 mL) was added NBS (5.37 g, 0.03 mol) and benzoyl peroxide (0.36 g, 0.0015 mol) at room temperature. The reaction mixture was heated to reflux for 15 hours. The reaction was cooled and the precipitate filtered away. The organic filtrate was diluted with ether and washed with saturated sodium bicarbonate. The organic layer was dried (MgSO₄) and the solvent removed *in vacuo* to give an oil, which after chromatography (silica, hexanes to 30% ether/hexanes) gave 157 g methyl 2-cyano-5-bromomethylbenzoate (**EX-25C**) as a tan solid with m/z +1 = 255.

To a solution of di-*tert*-butyl iminodicarboxylate (1.48 g, 7.5 mmol) in THF at 0°C was added NaH (031 g, 7.8 mmol). After stirring at room temperature for 30 minutes, **EX-25C** (1.57 g, 6.0 mmol) was added as a solution in THF via canula. The reaction was complete after 2.5 hours at room temperature. The reaction was quenched by addition of water and ether. The layers were separated and the organic layer washed with brine (2x), dried (MgSO₄) and the solvent removed *in vacuo* to give 236 g methyl 2-cyano-5-(N,N-bis-Bocaminomethylbenzoate (**EX-25D**) as a yellowish solidwith m/z +1 = 391.

To a solution of **EX-25D** (0.20 g, 0.5 mmol) in anhydrous methanol (10 mL) was added anhydrous hydrazine (1 mL, 32 mmol)) at room temperature. The reaction was heated to 70°C overnight. The solvent was removed *in vacuo* to give a solid, which was suspended in ether and filtered to give 0.11 g of the product **EX-25E** as a white solid with an m/z +1 = 291.

To a solution of **EX-25E** (0.22 g, 0.78 mmol) in dichloromethane (5 mL) at room temperature was added trifluoroacetic acid (5 mL). After 30 min, the solvent was removed *in vacuo* to give a clear residue, which upon drying on high vacuum became a white solid **EX-25F** (0.39 g) with m/z +1 = 191.

To a solution of 2-{5-chloro-6-(3-aminophenyl)-3-cyclobutylamino-2-oxohydropyrazinyl}acetic acid (0.93 g, 2.6 mmol) in DMF (20mL) was added EDC (0.64 g, 33 mmol) and HOBt (0.44 g, 3.2 mmol) at room temperature. After 30 min, the amine **EX-25F** in a solution of DMF and triethylamine (1.76 mL, 0.01 mol) was added to the acid. The reaction mixture was stirred for 1 hour and then poured into NaHCO_{3 (aq)} and ethyl acetate. The layers were separated and the aqueous layer extracted with ethyl acetate (2x) The organic layer was washed with brine (1x), dried (MaSO₄), and the solvent removed *in vacuo* to give a brown oil, which after chromatography (silica, dicholoromethane to 10% methanol/dichloromethane) gave the product **EX-25G** (0.29 g) with m/z + 1 = 521.

To a suspension of **EX-25G** (0.29 g, 5.6 mmol) in ether (5 mL) was added 25 mL of 2.0 M HCl in ether. The reaction was stirred for 30 min to give a fine precipitate which was filtered and dried to give the product (037 g) with m/z + 1 = 521. Analysis C₂₅H₂₅Cl₂N₈O₃+1.8 HCl+ 2.15 H₂O gave C, 47.78%; H, 5.22%; N, 16.29%; O, 12.59%; Cl, 15.43%.

### Example 26

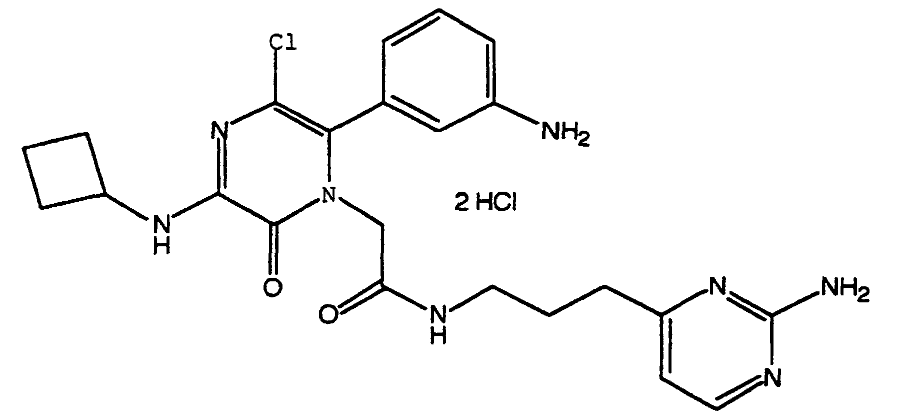

To a solution of 2-Amino-4-methylpyrimidine (1.0 g, 9.0 mmol) in THF (100 mL) at 0 °C was added TMEDA (4.15 mL, 27.0 mmol) and n-butyl lithium (17.2 mL, 27.0 mmol). After stirring at 0 °C for 30 min, (2-bromoethoxy)-tert-butyldimethylsilane (2.16 mL, 10 mmol) was added in a solution of THF (20 mL) dropwise via canula. The reaction was allowed to warm to room temperature overnight. The reaction was diluted with water and ether. The aqueous layer was extracted (2x) with ether. The organic layer was washed with brine, dried (MgSO₄), and solvent removed *in vacuo* to give a brown oil, which after chromatography (silica, dichloromethane to 10% methanol/dicholoromethane) gave **EX-26A** (1.26 g) with m/z + 1 = 268.

To a solution of **EX-26A** (436 g, 16.0 mmol) in dichloromethane (100 mL) was added triethylamine (3.4 mL, 24 mmol), di-tert-butyl dicarbonate (4.53 g, 24 mmol) and DMAP (0.2 g, .16 mmol). After stirring at room temperature for 24 hours the reaction was poured into aqueous sodium bicarbonate and ether. The layers were separated and the aqueous layer extracted (2x) with ether. The organic layer was washed with brine, and the solvent removed in vacuo to give a red oil (4.4 g). The oil was purified by chromatography (silica, 60% ethyl acetate/hexanes) to give a yellow oil **EX-26B** (2.77 g) with m/z +1 = 468.

To a solution of **EX-26B** (2.77 g, 6.0 mmol) in THF (100 mL) was added TBAF (7.1 mL, 1 M in THF) dropwise. After 4 hours at room temperature the reaction was complete. The reaction mixture was poured into ethyl acetate and brine. The aqueous layer was extracted 2x with ethyl acetate. The organic layer was dried (MgSO₄) and the solvent removed to give a yellow oil, which after chromatography (silica, 70% ethyl acetate/hexanes to 100% ethyl acetate) gave 1.51 g of the alcohol 2-(bis-Boc-amino)-4-(3-hydroxypropyl)pyrimidine (**EX-26C**) as a yellow oil with an m/z + 1 = 354.

To a solution of **EX-26C** (1.38 g, 4.0 mmol) in toluene (20 mL) was added triethylamine (0.54 mL, 4.0 mmol) and methanesulfonyl chloride (030 mL, 4.0 mmol). After 10 min, no starting material was observed by TLC. The reaction mixture was poured into dichloromethane and water. The layers were separated and the organic layer was washed with brine and dried (Na₂SO₄). The solvent was removed to give a yellow oil **EX-26D** which was used without further purification. To the crude mesylate **EX-26D** (1.73 g, 4.0 mmol) in DMF (10 mL) was added NaN₃ (2.6 g, 40 mmol) and water (1 mL). The reaction mixture was stirred at room temperature for 18 hours. The reaction was diluted with ether and water. The layers were separated and the organic layer washed with brine and dried (Na₂SO₄). The solvent was removed to give an oil, which after chromatography (silica, 60% ethyl acetate/hexanes) gave the azide **EX-26E** (0.91 g) with a m/z + 1 = 379.

To a solution of 2-(bis-Boc-amino)-4-(3-azidopropyl)pyrimidine (**EX-26E**) (039 g, 1.0 mmol) in ethanol at room temperature was added 10% Pd/C and a hydrogen balloon. After stirring at room temperature for 3 hours the reaction was complete by TLC. The reaction mixture was filtered through a pad of celite and washed with ethanol. The solvent was removed *in vacuo* to give an oil (035 g) which was a mixture of Boc derivatives **EX-26F**. To a solution of **EX-26F** (032 g) in dichloromethane (7 mL) was added trifluoroacetic acid (3 mL) dropwise. After 30 min, the solvent was removed *in vacuo* to give 034 g of the free amine 2-amino-4-(3-aminopropyl)pyrimidine **(EX-26G)** as an oil with an m/z + 1 = 353.

To a solution of anilino-acid 2-{5-chloro-6-(3-aminophenyl)-3-cyclobutylamino-2-oxohydropyrazinyl}acetic acid (1.46 g, 4.2 mmol) in DMF (30 mL) was added HOBt (0.91 g, 6.7 mmol) and EDAC (1.29 g, 6.7 mmol) at room temperature. After stirring for 30 min, **EX-26G** (1.59 g, 4.2 mmol) in DMF (8 mL) and triethylamine (3.5 mL, 25.2 mmol) was added. After 30 min , the reaction was diluted with aqueous sodium bicarbonate and ethyl acetate. The layers were separated and aqueous layer extracted (2x) with ethyl acetate. The organic layer was washed with brine and dried (MgSO₄). The solvent was removed *in vacuo* to give an oil, which after chromatography (dichloromethane to 15% methanol dichloromethane) gave the product **EX-26H** (1.20 g) as a yellow foam with an mlz + 1 = 483.

To a solution of **EX-26H** (032 g, 0.67 mmol) in 5 mL of ether was added 20 mL of 3.0 M HCl in ether at room temperature. The reaction mixture was stirred at room temperature for 20 minutes to give a precipitate which was filtered to give a yellow solid (034 g) of the di-hydrochloride salt. The solid was purified by RP-HPLC to give (0.22 g) with an m/z + 1 = 483.

### Example 27

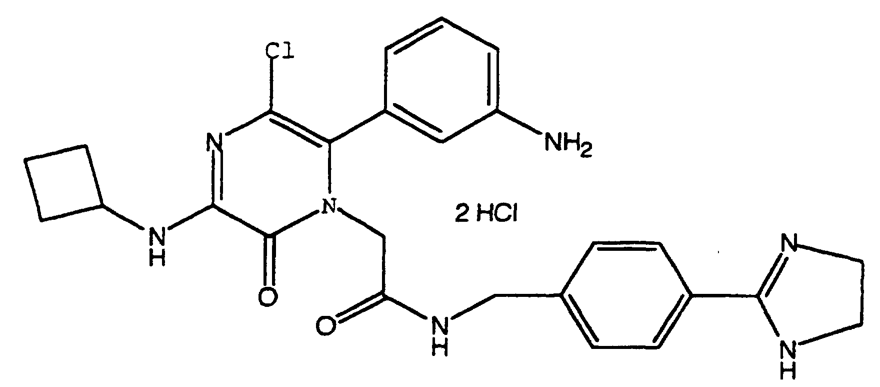

To a solution of the mixture N-Boc and N,N-bis-Boc 4-(N'-Z-amidino)benzylamines (3.0 g, 6.2 mmol) in 50 mL of EtOH and 20 mL THF was added 300 mg of 5% Pd(C). The solution was hydrogenated at 40psi H_{2.} in a Parr shaker for 18 hrs. The catalyst was filtered off, and the filtrate concentrated in-vacuo to afford the mixture **EX-27A** (2.1 g, 6.0 mmol) of N-Boc and N,N-bis-Boc 4-amidinobenzylamines as a brownish oil with M+H of 250 (monoBoc) and M+H of 350 (diBoc).

A solution of **EX-27A** (2.1g, 6.0 mmol) in MeOH was treated with ethylenediamine (1.13g, 18.9 mmol). The mixture was heated to reflux for 18 hrs, cooled to room temperature and concentrated *in vacuo*. 50 mL of H₂O was added and extracted 3x with CH₂Cl₂. The organic extracts were dried over MgSO₄, filtered and condensed *in vacuo* to afford the mixture **EX-27B** (2.2 g, 5.9 mmol) as a tan solid with M+H of 276 (monoBoc) and M+H of 376 (diBoc).

A solution of the mixture **EX-27B** (2.2 g, 5.9 mmol) in 20 mL methylene chloride and 5 mL pyridine was treated with benzyl chloroformate (13 g, 7.7 mmol). The mixture was stirred for 1.5 hrs and then was added 100 mL methylene chloride and 100 mL 0.5 N HCl. The layers were seperated, and the aqueous extracted 2x with methylene chloride The organics were combined, washed 1x with brine, dried over MgSO₄ , filtered and condensed *in vacuo*. Purification by column chromatography (silica gel 200-400 mesh) using 50% ethyl acetate as elutant afforded the mixture **EX-27C** (1.1 g , 2.2 mmol) as a tan oil with M+H 410 (monoBoc) and M+H 510 (diBoc).

A solution of the mixture **EX-27C** (800 mg, 1.6mmol) in 10 mL of methylene chloride was treated with 5 mL of 4N HCl in dioxane. The mixture was stirred for 1.5 hrs. and then diethyl ether was added to precipitate the product. The precipitate was filtered off and washed extensively with diethyl ether to afford the HCl salt **EX-27D** (520mg, 1.7 mmol) as a tan soild with an M+H of 310.

A solution of 2-{5-chloro-6-(3-nitrophenyl)-3-cyclobutylamino-2-oxohydropyrazinyl}acetic acid (329 mg, 0.86 mmol) in 10 mL of methylene chloride was treated with HOBt (127 mg, 0.94 mmol) for 20 min. Then was added EDC (180 mg, 0.94 mmol), DIEA (335 mg, 2.6 mmol), and **EX-27D** (300 mg, 0.86 mmol), and the reaction was allowed to stir for 1 hr. Water was then added, and the reaction mixture extracted 3x with methylene chloride. The organics were then washed 1x with brine, dried over MaSO₄, filtered and condensed *in vacuo*. Purification by column chromatography (silica gel 200-400 mesh) eluting with 90% ethyl acetate/hexane and then 100% ethyl acetate afforded **EX-27E** (325 mg, 0.48 mmol) as a yellow solid which gave an M+H of 670.

A solution of **EX-27E** (325 mg, 0.48 mmol) in 10 mL of MeOH was treated with 0.7 mL of 3N HCl in MeOH and 5% Pd(C) (50 mg). The mixture was hydrogenated at 45 psi on a Parr shaker apparatus for 2hrs. The catalyst was then filtered off and washed extensively with MeOH. The filtrate was concentrated *in vacuo*. The residue was dissolved in EtOH and triturated with diethyl ether.
The solid formed was filtered and extensively washed with diethyl ether to afford the HCl salt product (220 mg , 0.43 mmol) as an off-white solid which gave M+H's of 506 (100%) and 508 (60%).

### Example 28

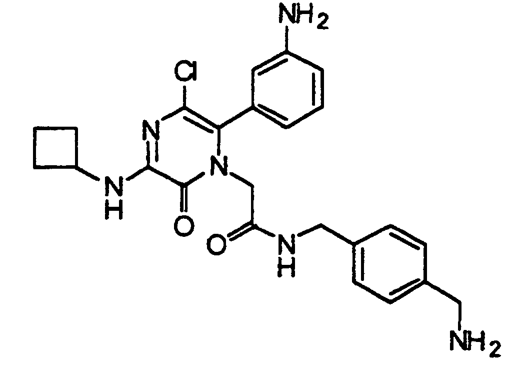

Using the procedures of **Scheme 1**, **Scheme 2**, and **Example 1** through **Example 18** with suitable reagents, starting materials, 2-{5-chloro-6-(3-nitrophenyl)-3-cyclobutylamino-2-oxohydropyrazinyl}acetic acid and 4-(N-Boc-aminomethyl)benzylamine prepared according to the literature reference (Callahan. J. F., Ashton-Shue, D., et al., *J*. *Med. Chem.* **1989**, 32, 391-396), the product was obtained and gave an m/z(M+H)⁺ of 467.

### Example 29

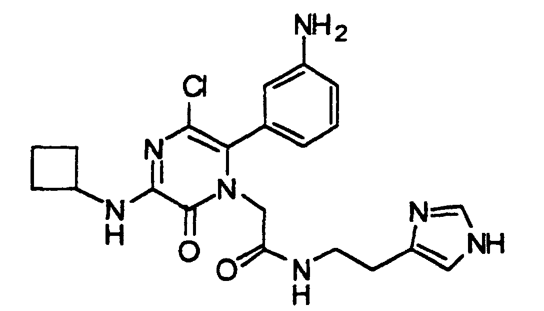

Using the procedures of **Scheme 1**, **Scheme 2**, and **Example 1** through **Example 18** with suitable reagents, starting materials, 2-{5-chloro-6-(3-nitrophenyl)-3-cyclobutylamino-2-oxohydropyrazinyl}acetic acid, and 2-(4-imidazoyl)ethanamine commercially available form Fluka, the product was obtained and gave an m/z(M+H)⁺ of 442.

### Example 30

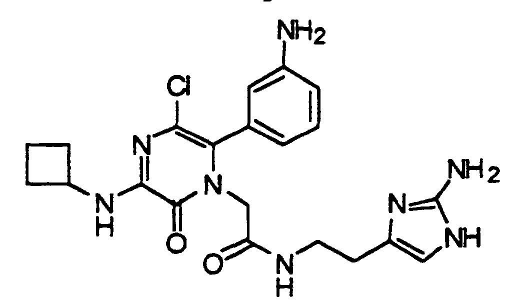

Using the procedures of **Scheme 1**, **Scheme 2**, and **Example 1** through **Example 18** with suitable reagents, starting materials, 2-{5-chloro-6-(3-nitrophenyl)-3-cyclobutylamino-2-oxohydropyrazinyl}acetic acid and 2-(4-(2-aminoimidazoyl))ethanamine prepared according to the literature reference (Nagai, W. Kirk, K. L., Cohen, L. A., *J. Org. Chem.* **1973**, 33, 1971-1974), the product was obtained and gave an m/z(M+H)⁺ of 457.

### Example 31

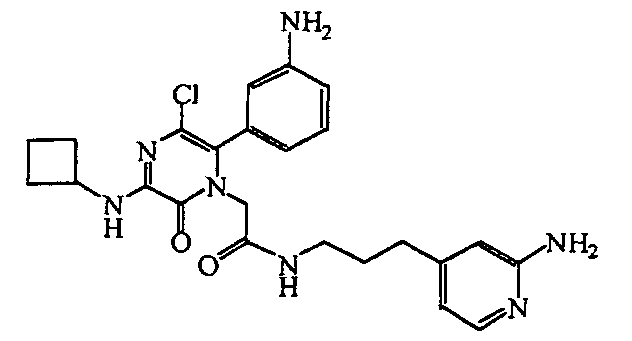

2-Amino-4-picoline (5.00 g, 46.2 mmol) and 11.20 g of di-tert-butyl dicarbonate (50.8 mmol) were stirred in 100 mL of tert-butanol at 30 °C overnigh The reaction mixture was concentrated in vacuo and chromatographed on silica ge with 25% EtOAc/Hexane to give 8.20 g (85% yield) of the product **EX-31 A**.

To a solution of 3.00 g of N-Boc-2-amino-4-picoiine (**EX-31A** , 14.4 mmol) in 150 mL of THF at -78 °C was added 14.4 mL of 2.5 M n-BuLi/Hexane solution. The reaction mixture was allowed to warm up to room temperature and stirred for 40 min. The reaction mixture was cooled down to -78 °C and I-bromo-2-chloroethane was added. The mixture was stirred at -78 °C for overnight. The reaction mixture was quenched with HOAc at -78 °C and concentrated in vacuo. The crude was dissolved in EtOAc and washed with brine. The EtOAc layer was dried over MgSO₄ and concentrated in vacuo. The crude product was purified by silica gel chromatography with 20% EtOAc/Hexane to give 2.00 g (50%) of the product **EX-31B**.

To a solution of 2.00 g of the chloride **EX-31B** (6.91 mmol) and 0.50 g of sodium azide (7.69 mmol) in 80 mL of DMF was added 10 mL of water and 0-52 g of sodium iodide. The reaction mixture was stirred at 55 °C overnight. The mixture was washed with brine and extracted with EtOAc. The EtOAc layer was dried over MgSO₄ and concentrated *in vacuo.* The crude product was chromatographed on silica gel with 20% EtOAc/Hexane to give 1.80 g (94%) of the product **EX-31C**.

To a solution of 1.74 g of the azide **EX-31C** (6.27 mmol) in 30 mL of THF was added 1.64 g of triphenylphosphine (6.27 mmol) and 1 mL of water. The reaction mixture was stirred at room temperature overnight. The reaction mixture was concentrated in vacuo and chromatographed on silica get with 10% CH₃OH/CH₂Cl₂ to give 1.26 g (80%) of the amine product **EX-31D**.

To a solution of 0.77 g of 2-{5-chloro-6-(3-aminophenyl)-3-cyclobutylamino-2-oxohydropyrazinyl}acetic acid (2.21 mmol) in 50 mL of DMF was added 0.47 g of EDC.HCI and 033 g of HOBt. The mixture was stirred at room temperature for 30 min. After the addition of 0.61 of the amine **EX-31D** (2.43 mmol) and 0.50 g of triethylamine the reaction mixture was stirred at room temperature overnight. The mixture was washed with water and extracted with EtOAc. The EtOAc layer was washed with brine and concentrated in vacuo. The crude product was chromatographed on silica gel with 3% CH₃OH/CH₂Cl₂ to afford 1.10 g (86%) of the Boc protected product **EX-31E**.

The Boc protected product **EX-31E** (0.50 g) was treated with 2.0 M of HCl/ether solution for overnight. The mixture was concentrated in vacuo and chromatographed by DeltaPrep with 10% CH₃CN/H₂O to give 0.32 g of the product (78%) as a TFA salt. The TFA salt was converted to the HCl salt by ion exchange chromatography with BioRad AG 2-X8 resin and 10% CH₃CN/H₂O and analyzed by mass sprectrometry to give an (M+H) of 482.16.

### Example 32

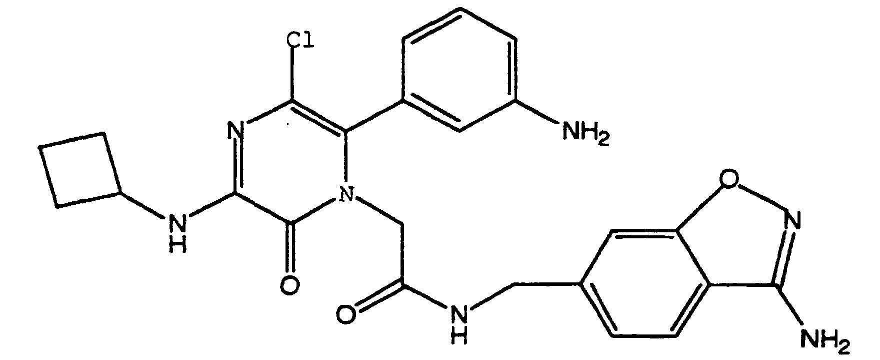

A mixture of 26.5mmol of 4-bromo-3-fluorotoluene, 29mmol of copper cyanide and 25ml of dry DMF is refluxed for 12 hr, then 150ml water was added and the reaction mixture filtered. The precipitate was triturated with 100ml of concentrated ammonium hydroxide, extracted twice with 50ml of dichloromethane. The organic layer was washed with ammonium hydroxide (100ml) and water (100) and then concentrated and recrystallized (hexane) to yield 2g solid 4-cyano-3-fluorotoluene (**EX-32A**). NMR and MS confirmed the structure of **EX-32A**.

A mixture of 2-fluoro-4-methylbenzonitrile (**EX-32A**) (2g, 14.8mmol), NBS (2.6g, 14.8mmol) and benzoyl peroxide (178mg, 0.74mmol) in CCl₄ (30ml) was refluxed for 16 hr, then cooled and filtered. The mixture was then concentrated and purified with silica-gel column to yield 1.5g oil **EX-32B**. NMR and MS confirmed the structure of **EX-32B**.

N,N-(Boc)₂NH (1.1g, 5.17mmol) in THF (20ml) was cooled to 0°C and NaH (60%, 0.25g, 6.1 1mmol) was added. The mixture was kept stirring for 30 min., then benzylbromide **EX-32B** (1g, 4.7mmol) in THF (2ml) was added. The mixture was stirred for 3hr. Then water was added and extracted with EtOAc (3X15ml). The combined EtOAc was then concentrated and recrystalized in hexane to yield 0.6g white solid **EX-32C**. NMR and MS all confirmed the structure of **EX-32C**.

To the compound **EX-32C** (200 mg) in CH₂Cl₂ (3ml) was added TFA (1.5ml). The reaction mixture was stirred at RT for 3h and concentrated to afford oil **EX-32D** which was directly used for next amide coupling reaction.

To 2-{5-chloro-6-(3-nitrophenyl)-3-cyclobutylamino-2-oxohydropyrazinyl}acetic acid (227mg, 0.6mmol) was added HOBt (106.1mg, 0.7mmol) and EDC (126.7mg, 0.7mmol) in DMF (3ml). The mixture was stirred at RT for 30min. Then the amine TFA salt **EX-32D** in DMF (1ml) and triethyl amine (0.2 ml) was added to the mixture which was stirred overnight. The mixture was concentrated, purified to yield 200mg solid **EX-32E**, confirmed by NMR and MS.

**EX-32E**(0.2g) in THF (5ml) was added with Pd/C (10%, 20mg). The mixture was stirred at RT under N₂, and then H₂ gas balloon was connected to the flask. The reaction was stirred for 24hr to complete reaction. The mixture was filtered, washed with ethanol, and then dried to yield 0.16g white solid **EX-32F** which was directly used for next cyclization reaction.

To acetohydroxamic acid (37mg, 0.5mmol) in DMF (2ml) was added potassium t-butoxide (1M, 0.5ml, 0.5mmol) at room temperature. After stirring for 30min, benzonitrile **EX-32F** (160mg, 0.33mmol) in DMF (2ml) was added. The reaction mixture was stirred overnight, and then poured into a mixture of brine and ethyl acetate. The aqueous layer was extracted with EtOAc (3X2ml), and the combined EtOAc was washed with brine, dried, concentrated and purified on reverse-phase HPLC to yield 60mg of the HCl salt. NMR and MS both confirmed the structure of product.

### Example 33

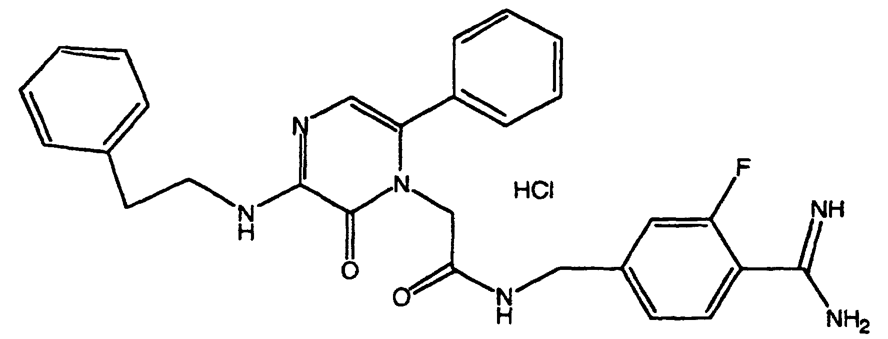

By substituting 2-fluoro-4-methylbenzonitrile for 4-methylbenzonitrile, 2-fluoro-4-methylbenzonitrile (**EX-32A**) was converted to the protected amidine, 4-(N-benzyloxycarbonylamidino)-3-fluorobenzylamine hydrogen chloride salt (**EX-33A**), using the procedure outlined in Synthetic Communications, 28(23), 4419-4429 (1998) for preparing 4-(N-benzyloxycarbonylamidino)benzylamine hydrogen chloride salt. **EX-33A** was characterized by: MS (LR-ESI) m/z 302 (M+H)⁺; ¹HNMR (DMSO, 300MHz) δ 8.75 (bs, 3H, CH₂NH₃), δ 7.79-7.02 (m, 8H, aromatic CH), δ 5.31-5.07 (m, 2H, C₆H₅CH₂), δ 4.10 (s, 2H, CH₂NH₃).

Using the procedure of **Example 44** by substituting 2-[3-(N-{2-phenylethyl}amino)-2-oxo-6-phenylhydropyrazinyl]acetic acid (**EX-1D**) for 2-[3-({2-[(tert-butoxy)carbonylamino]ethyl}amino)-5-chloro-2-oxo-6-phenylhydropyrazinyl]acetic acid, **EX-33A** was converted to the product which gave an m/z+1 of 499.

### Example 34

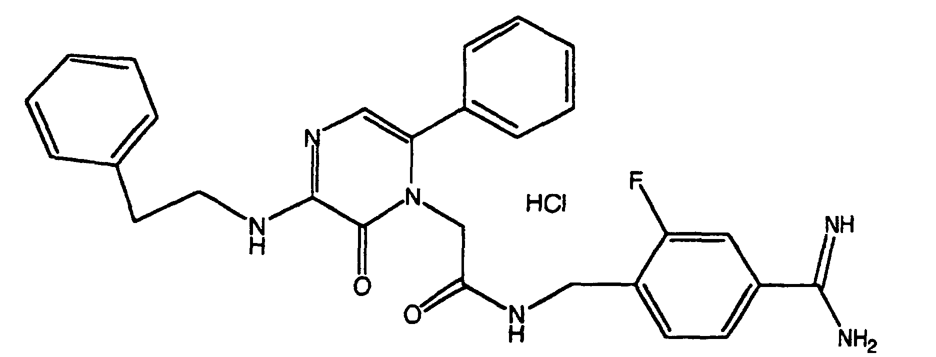

By substituting 3-fluoro-4-methylbenzonitrile for 4-methylbenzonitrile, 3-fluoro-4-methylbenzonitrile was converted to the protected amidine, 4-(N-benzyloxycarbonylamidino)-2-fluorobenzylamine hydrogen chloride salt (**EX-34A**), using the procedure outlined in Synthetic Communications, 28(23), 4419-4429 (1998) for preparing 4-(N-benzyloxycarbonylamidino)benzylamine hydrogen chloride salt. **EX-34A** was characterized by: MS (LR-ESI) m/z 302 (M+H)⁺; ¹HNMR (DMSO, 300MHz) δ 8.82 (bs, 3H, CH₂NH₃), δ 7.92-7.26 (m, 8H, aromatic CH), δ 5.32 (s, 2H, C₆H₅CH₂), δ 4.10 (s, 2H, CH₂NH₃).

Using the procedure of **Example 44** by substituting 2-[3-(N-{2-phenylethyl}amino)-2-oxo-6-phenylhydropyrazinyl]acetic acid (**EX-1D**) for 2-[3-({2-[(tert-butoxy)carbonylamino]ethyl}amino)-5-chloro-2-oxo-6-phenylhydropyrazinyl]acetic acid, **EX-34A** was converted to the product which gave an m/z+1 of 499.

### Example 35

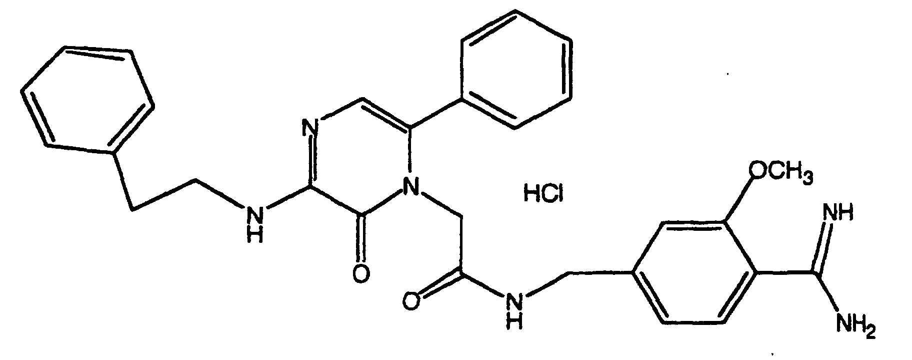

By substituting 2-methoxy-4-methylbenzonitrile for 4-methylbenzonitrile, 2-methoxy-4-methylbenzonitrile was converted to the protected amidine, 4-(N-benzyloxycarbonylamidino)-3-methoxybenzylamine hydrogen chloride salt (**EX-35A**), using the procedure outlined in Synthetic Communications, 28(23), 4419-4429 (1998) for preparing 4-(N-benzyloxycarbonylamidino)benzylamine hydrogen chloride salt. **EX-35A** was characterized by: MS (LR-ESI) m/z 314 (M+H)⁺; ¹HNMR (DMSO, 300MHz) δ 7.77-6.95 (m, 8H, aromatic CH), δ 4.74 (bs, 2H, C₆H₅CH₂), δ 4.10-3.95 (m, 2H, CH₂NH₃), δ 3.80 (s, 3H, OCH₃).

Using the procedure of **Example 44** by substituting 2-[3-(N-{2-phenylethyl}amino)-2-oxo-6-phenylhydropyrazinyl]acetic acid (**EX-1D**) for 2-[3-({2-[(tert-butoxy)carbonylamino]ethyl}amino)-5-chloro-2-oxo-6-phenylhydropyrazinyl]acetic acid, **EX-35A** was converted to the product which gave an m/z+1 of 511.

Using the procedures of **Scheme 1**, **Scheme 2**, and the **Examples** herein with suitable reagents, starting materials, and intermediates, additional pyrazinones of the present invention were prepared and these pryazinones are summarized in Table 2.

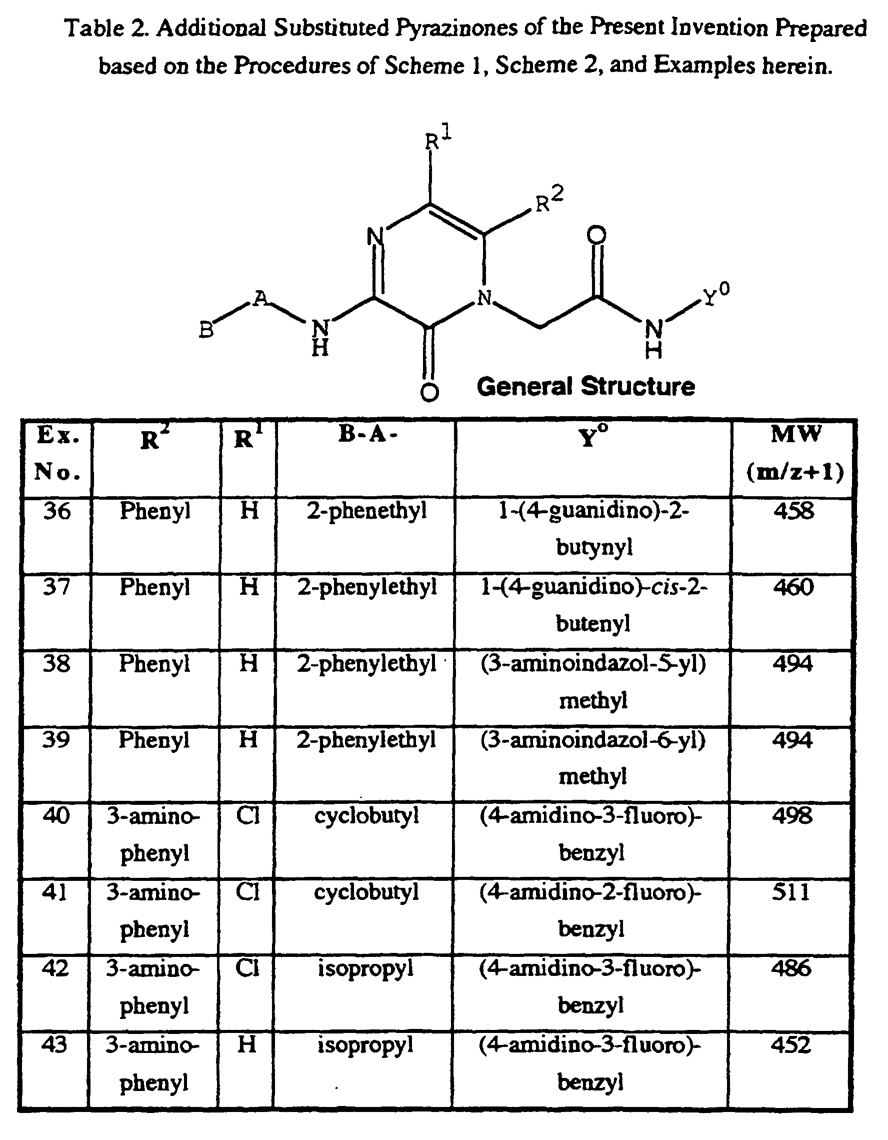

### Example 44

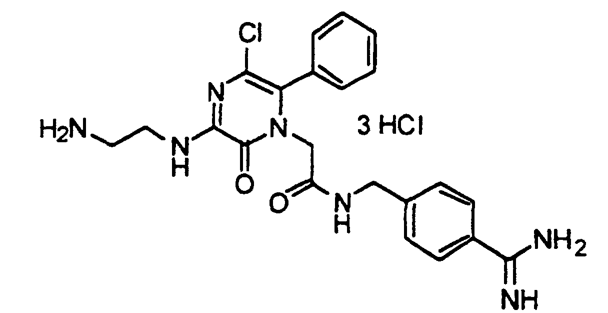

To a solution of 2-[3-({2-[(tert-butoxy)carbonylamino]ethyl}amino)-5-chloro-2-oxo-6-phenylhydropyrazinyl]acetic acid (6.50 g, 15.38 mmol) prepared as described in **EX-1C** using 2-(tert-butoxycarbonylamino)ethylamine in place of 2-phenylethylamine in 100.0 mL dimethylformamide was added N,N-diisopropylethylamine (21.0 mL, 120.56 mmol), N-hydroxybenzotriazole (2.73g, 20.21 mmol), and 1-[3-(dimethylamino)propyl]-3-ethylcarbodiimide hydrochloride (3.84 g, 20.04 mmol). The resulting mixture was stirred for 30 minutes. To the reaction mixture was added in one portion (5.9723 g, 18.68 mmol) of the protected amidine, 4-(N-benzyloxycarbonylamidino)benzylamine hydrogen chloride salt, prepared using the procedure outlined Synthetic Communications, 28(23), 4419-4429 (1998). The resulting mixture was stirred over night. The reaction mixture was diluted with ethyl acetate (250 mL) and washed with 5% citric acid (1 x 50 mL), saturated NaHCO₃ (1 x 50 mL), and brine (1 x 50 mL). The organic solution was dried (MgSO₄), filtered and concentrated. The crude reaction was purified by MPLC (80% ethyl acetate/hexanes) to give pure product **EX-44A**: ¹H NMR (300 MHz, DMSO) δ 8.59-8.53 (1H), 7.99-7.96 (m, 2H), 7.81-7.75 (m, 1H), 7.51-7.25 (m, 12H), 6.99 (br m, 1H), 5.14 (s, 2H), 432-4.27 (m, 4H), 3.42-3.35 (m, 4H), 3.24-3.20 (m, 2H), 1.41 (s, 9H); ¹³C NMR (75 MHz, CDCl₃) δ 166.6, 163.0, 156.5, 1513, 149.9, 143.9, 137.8, 133.5, 132.6, 131.3, 130.1, 129.5, 129.2, 129.1, 128.9, 128.7, 128.4, 127.7, 124.0, 78.5, 66.8, 49.3, 42.6, 36.5, 31.5,29.0; HRMS (EI) calcd for C₃₅H₃₈ClN₇O₆ 688.2650, found 688.2614.

A solution of pyrazinone **EX-44A** (334.4 mg, 0.4593 mmol) in 5.0 mL ethanol/4 M HCl in dioxane (3:1, 0.1 M) was flushed with hydrogen gas. To the solution was then added 113.1 mg 10% Pd/C (wet), and the resulting suspension was stirred at room temperature under an atmosphere of hydrogen (balloon pressure) for approximately 18 hours. The reaction mixture was filtered through a pad of Celite 545 and rinsed with ethanol. The solvent was removed under reduced pressure. The resulting oil was triturated with ethyl ether to provide pure product as a white solid: ¹H NMR (400 MHz, DMSO) d 9.50 (s, 2H), 9.29 (s, 2H), 8.8/0 (s, 1H), 8.22 (s, 3H), 7.85-7.80 (m, 3H), 7.44 (br s, 3H), 7.27-7.24 (m, 4H), 4.23 (s, 4H), 3.58-3.54 (m, 4H); HRMS (ES) calcd for C₂₂H₂₅ClN₇O₂ 454.1758, found 454.1741.

### Example 45

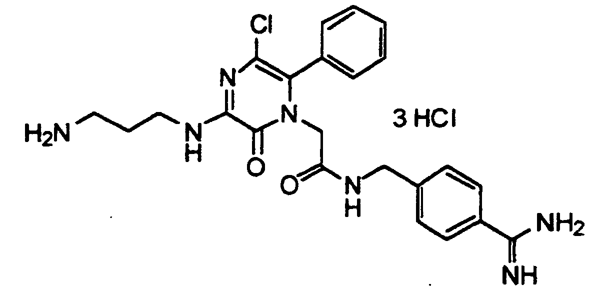

By following the method of **Example 45** and substituting 2-[3-({3-[(tert-butoxy)carbonylamino]propyl}amino)-5-chloro-2-oxo-6-phenylhydropyrazinyl] acetic acid for 2-[3-({2-[(tert-butoxy)carbonylamino]ethyl}amino)-5-chloro-2-oxo-6-phenylhydropyrazinyl]acetic acid, the product was prepared: ¹H NMR (400 MHz, DMSO) d 9.51 (br s, 2H), 8.28 (br s, 2H), 8.77 (s, 1H), 8.15 (3, 3H), 7.86-7.79 (m, 3H), 7.42 (s, 3H), 7.26-7.24 (m, 4H), 5.37 (br s, 2H), 4.21 (s 4H), 3.39-3.29 (m, 2H), 2.81-2.76 (br s, 2H), 1.86 (br s, 2H); ¹³C NMR (100 MHz, DMSO) d 166.7, 166.0, 151.2, 149.7, 146.0, 132.5, 131.1, 129.5, 128.8, 127.9, 126.8, 125.1, 123.9, 65.6, 56.6, 49.2, 42.4, 38.1, 37.3, 34.6, 26.7, 19.2, 15.8; HRMS (EI) calcd for C₂₃H₂₆ClN₃O₆ 469.1755, found 469.1725.

### Example 46

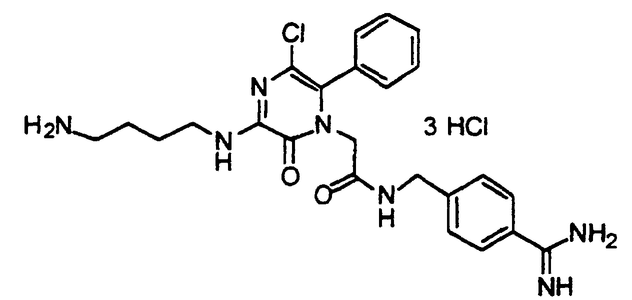

By following the method of **Example 44** and substituting 2-[3-({4-[(tert-butoxy)carbonylamino]butyl}amino)-5-chloro-2-oxo-6-phenylhydropyrazinyl] acetic acid for 2-[3-({2-[(tert-butoxy)carbonylamino]ethyl}amino)-5-chloro-2-oxo-6-phenylhydropyrazinyl]acetic acid, the product was prepared: ¹H NMR (400 MHz, DMSO) d 9.49 (br s, 2H), 9.28 (s, 2H), 8.75 (s, 1H), 8.08 (s, 3H), 7.89-7.76 (m, 3H), 7.42 (s, 3H), 7.26-7.24 (m, 4H), 4.70 (br s, 4H), 4.23-4.21 (m, 3H), 2.73 (br s, 2H), 1.57 (br s, 3H), 1.03-0.96 (m, 2H); HRMS (EI) calcd for C₂₄H₂₉ClN₇O₂ 482.2071, found 482.2040.

### Example 47

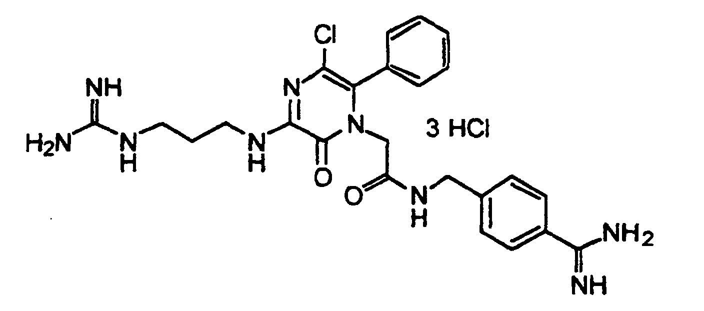

A solution of 1-(N-{4-[N-benzyloxycarbonylamidino]benzylamido} carbonylmethyl)-3-({3-[(tert-butoxy)carbonylamino]propyl}amino)- 5-chloro-6-phenylpyrazinone hydrochloride (2.0075 g, 2.859 mmol), prepared as an intermediate in **Example 45**, in 28.0 mL ethanol/4 M HCl in dioxane (1:1, 0.1 M) was allowed to stir at room temperature for approximately 4 hours. The solvent was removed under reduced pressure. Purification by trituration with ethyl ether gave pure product **EX-47A** as a yellow solid: ¹H NMR (400 MHz, DMSO) d 11.67 (br s, 1H), 10.53 (br s, 1H), 8.90-8.87 (m, 1H), 8.30-8.25 (m, 3H), 7.89-7.83 (m, 1H), 7.75-7.73 (m, 2H), 7.46-7.23 (m, 13H), 5.532 (s, 2H), 4.26-4.23 (m, 3H), 3.50 (s, 2H), 3.36-3.35 (m, 2H), 2.75 (br m, 2H); HRMS (EI) calcd for C₃₁H₃₂ClN₇O₄ 602.2283, found 602.2253.

To a solution of amino pyrazinone **EX-47A** (1.9093 g, 2.684 mmol) in 10.0 mL dimethyl formamide (0.25 M) was added triethylamine (1.90 mL, 13.63 mmol) To the resulting mixture was then added N,N'-di-BOC-N'-triflylguanidine (1.4021 g, 3.583 mmol, prepared according to Feichtinger, K., Zapf, C., Sings, H.L., and Goodman, M., *J. Org. Chem*., 63, 3804-3805 (1998)) in one portion at room temperature. The resulting suspension was allowed to stir over night. The reaction mixture was diluted ethyl acetate (250 mL) and washed with saturated NaHCO₃ (2 x 100 mL) and brine (2 x 100 mL). The organic solution was dried (MaSO₄), filtered and concentrated. Purification by MPLC (75% ethyl acetate/hexanes) afforded **EX-47B**: ¹H NMR (400 MHz, CDCl₃) d 11.53 (s, 1H), 8.55-8.48 (m, 2H), 7.97-7.93 (m, 4H), 7.49-7.24 (m, 13H), 5.13 (s, 2H), 4.30-4.25 (m, 4H), 3.90-3.33 (m, 4H), 1.84-1.79 (m, 2H), 1.49 (s, 9H), 1.41 (s, 9H); HRMS (EI) calcd for C₄₂H₅₁ClN₉O₈ 844.3549, found 844.3521.

A solution of pyrazinone **EX-47B** (1.5450 g, 1.8298 mmol) in 18.0 mL ethanol/4 M HCl in dioxane (3:1, 0.1 M) was flushed with hydrogen gas. To the solution was then added 157.2 mg 10% Pd/C (wet), and the resulting suspension was stirred at room temperature under an atmosphere of hydrogen (balloon pressure) for approximately 18 hours. The reaction mixture was filtered through a pad of Celite 545 and rinsed with ethanol. The solvent was removed under reduced pressure. The resulting oil was triturated with ethyl ether to provide the pure product in 63% yield: ¹H NMR (400 MHz, DMSO) d 9.50 (s, 2H), 9.28 (s, 2H), 8.77 (s, 1H), 7.91 (s, 1H), 7.81-7.79 (m, 3H), 7.42 (br s, 4H), 7.26-7.24 (m, 5H), 6.28 (br s, 2H), 4.23-4.21 (m, 4H), 3.36-3.27 (m, 2H), 3.14-3.13 (br m, 2H), 1.77-1.74 (m, 2H); HRMS (ES) calcd for C₂₄H₂₉ClN₉O₂ 510.2133, found 510.2080.

### Example 48

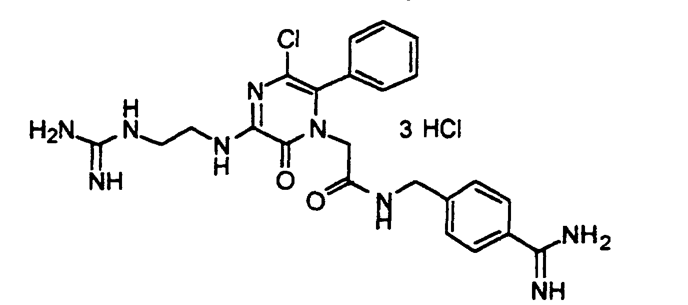

Using the method of **Example 47** and substituting 1-(N-{4-[N-benzyloxycarbonylamidino]benzylamido}carbonylmethyl)-3-({2-[(tert-butoxy) carbonylamino]ethyl}amino)-5-chloro-6-phenylpyrazinone hydrochloride, prepared as an intermediate in **Example 44**, for the propyl analog used in **Example 47,** the product was prepared: ¹H NMR (400 MHz, DMSO) d 9.51 (s, 2H), 9.29 (s, 2H), 8.80 (s, 1H), 7.90-7.78 (m, 5H), 7.43-737 (m, 5H), 7.35-7.23 (m, 5H), 4.23 (s, 4H), 4.03 (s, 2H), 3.40-334 (m, 4H); HRMS (EI) calcd for C₂₃H₂₇ClN₉O₂ 496.1976, found 496.1952.

### Example 49

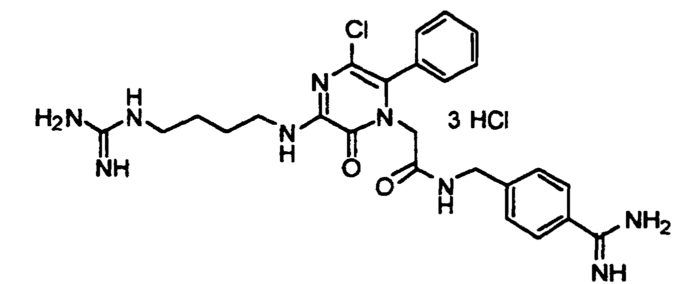

By following the method of **Example 47** and substituting 1-(N-{4-[N-benzyloxycarbonylamidino]benzylamido}carbonylmethyl)-3-({4-[(tert-butoxy)carbonylamino]butyl}amino)-5-chloro-6-phenylpyrazinone hydrochloride (2.0075 g, 2.859 mmol), prepared as an intermediate in **Example 46**, for the propyl analog used in **Example 47**, the product was prepared: ¹H NMR (400 MHz, DMSO) d 9.47 (s, 2H), 9.28 (s, 2H), 8.74-8.72 (m, 1H), 7.88 (br s, 1H), 7.80-7.73 (m, 3H), 7.43-7.31 (m, 4H), 7.27-7.20 (m, 5H), 5.36-5.32 (m, 3H), 4.25-4.21 (m, 4H), 3.28-3.27 (m, 2H), 3.10-3.08 (m, 2H), 1.58-1.53 (m, 2H), 1.48-1.43 (m, 2H); HRMS (EI) calcd for C₂₅H₃₁ClN₉O₂ 524.2289, found 524.2292.

### Example 50

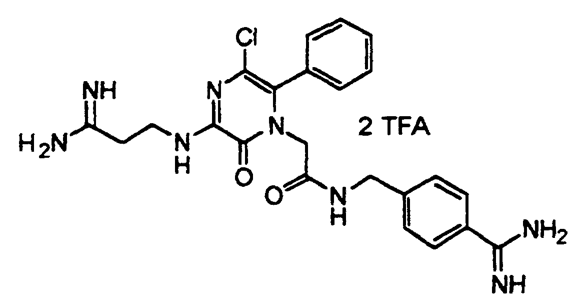

To a solution of 2-{5-chloro-3-[(2-cyanoethyl)amino]-2-oxo-6-phenylhydropyrazinyl}acetic acid (2.09 g, 6.28 mmol) in 31.0 mL dimethylformamide/tetrahydrofuran (1:1) was added N,N-diisopropylethylamine (5.50 mL, 31.57 mmol), N-hydroxybenzotriazole (1.02 g, 7.6 mmol), and 1-[3-(dimethylamino)propyl]-3-ethylcarbodiimide hydrochloride (1.45 g, 7.51 mmol). The resulting mixture was stirred for 30 minutes. To the reaction mixture was then added 4-cyanobenzylamine (1.28 g, 7.57 mmol) in one portion. The resulting mixture was allowed to stir over night. The reaction mixture was diluted with ethyl acetate (250 mL) and washed with 5% citric acid (1 x 50 mL), saturated NaHCO₃ (1 x 50 mL), and brine (1 x 50 mL). The organic solution was dried (MgSO₄), filtered and concentrated. The crude reaction was purified trituration with ethyl ether to give **EX-50A**: ¹H NMR (300 MHz, DMSO) δ 8.59 (t, *J =* 5.6 Hz, 1H), 8.10 (t, *J* = 5.6 Hz, 1H), 7.82 (d, *J* = 8.1 Hz, 2H), 7.53-7.46 (m, 3H), 7.35-7.32 (m, 4H) 4.33-4.29 (m, 4H), 3.63-3.57 (m, 2H), 2.89 (t, J= 6.3 Hz, 2H); ¹³C NMR (75 MHz, DMSO) δ 166.7, 151.1, 149.6, 145.6, 132.93, 132.45, 131.16, 130.21, 129.55, 128.63, 124.97, 124.72. 120.0, 119.6, 110.4, 49.3, 42.6, 37.3, 17.3; HRMS (EI) calcd for C₂₃H₂₀ClN₆O₂ 447.1336, found 447.1330.

To a suspension of bis-nitrile pyrazinone **EX-50A** (2.26, 58.07 mmol) in 50 mL ethanol/H₂O (2.6:1, 0.1 M) was added hydroxyl amine hydrochloride (2.61 g, 37.6 mmol) followed by potassium carbonate (3.08 g, 223 mmol). The resulting white suspension was stirred and heated to 60 °C over night. The reaction mixture was cooled to room temperature and diluted with water (75.0 mL). The mixture was placed in an ice bath, and the pH was adjusted to approximately 7 using dilute acid. The precipitate that formed was collected by filtration, washed with cold water and dried under vacuum to afford pure **EX-50B**: ¹H NMR (300 MHz, DMSO) δ 9.63 (s, 1H), 8.95 (s, 1H), 8.47 (br s, 1H), 7.66-7.61 (m, 2H), 7.53-7.47 (m, 4H), 7.32 (d, *J* = 5.2 Hz), 7.16-7.13 (m, 2H), 5.83 (s, 2H), 5.47 (s, 2H), 4.25 (s, 4H), 3.61-3.53 (m, 2H), 2.39-2.35 (m, 2H); ¹³C NMR (75 MHz, DMSO) δ 166.5, 151.68, 151.35, 151.23, 149.6, 140.3, 132.70, 132.63, 131.3, 130.1, 129.5, 127.6, 126.0, 125.3, 49.2, 42.6, 38.4, 30.6; HRMS (EI) calcd for C₂₃H₂₆ClN₈O₄ 513.1766, found 513.1735.

To a solution of Bis-hydroxyamidine **EX-50B** (2.40 g, 4.67 mmol) in 19.0 mL acetic acid (0.25 M) was added acetic anhydride (1.80 mL, 19.1 mmol). The resulting mixture was stirred for 10 minutes and flushed with hydrogen gas. To the solution was then added Pd/C (wet) and the resulting mixture was allowed to stir under an atmosphere of hydrogen (balloon pressure) at room temperature, over night. The reaction mixture was filtered through a pad of Celite 545 and concentrated under vacuum. Purification by HPLC (1% acetonitrile to 60% acetonitrile/H₂O/0.1 % trifluoroacetic acid) afford pure product: ¹H NMR (400 MHz, DMSO) δ 9.51 (s, 2H), 9.30 (s, 2H), 9.03 (s, 2H), 8.93 (s, 2H), 8.62-8.59 (m, 1H), 7.89-7.86 (m, 2H), 7.74 (d, *J* = 8.3 Hz, 2H), 7.47-7.40 (m, 3H), 7.28-7.23 (m, 4H), 4.27-4.24 (m, 4H), 3.63-3.59 (m, 2H), 2.70-2.68 (m, 2H); HRMS (EI) calcd for C₂₃H₂₆ClN₈O₂ 481.1867, found 481.1836.

### Example 51

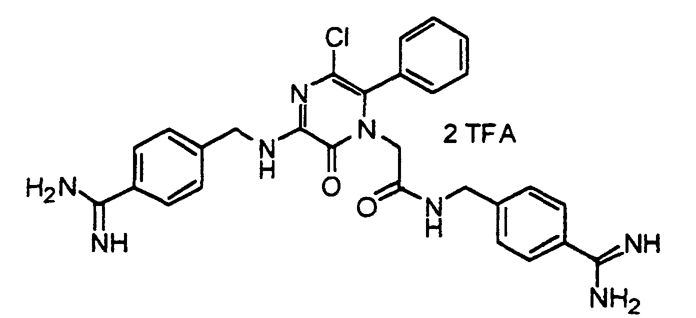

By following the method of **Example 50** and substituting 2-{5-chloro-3-[(4-cyanobenzyl)amino]-2-oxo-6-phenylhydropyrazinyl}acetic acid for the 2-cyanoethylamino analog, the product was prepared: ¹H NMR (400 MHz, DMSO) d 9.44 (d, *J* = 16.9 Hz, 3H), 9.26 (d, *J* = 17.2 Hz, 8.61 (br s, 1H), 8.47-8.44 (m, 1H), 7.74 (d, J = 7.0 Hz, 4H), 7.52 (d, *J* = Hz, 2H), 7.43-7.42 (m, 3H), 7.28-7.22 (m, 4H), 4.56-4.55 (m, 2H), 4.25 (s, 4H); HRMS (EI) calcd for C₂₈H₂₈ClN₈O₂ 543.2024, found 543.1986.

### Example 52

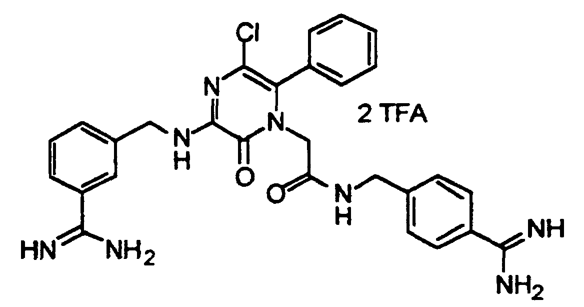

By following the method of **Example 50** andsubstituting 2-{5-chloro-3-[(3-cyanobenzyl)amino]-2-oxo-6-phenylhydropyrazinyl}acetic acid for the 2-cyanoethylamino analog, the product was prepared: ¹H NMR (400 MHz, DMSO) d 9.41 (s, 4H), 9.28 (d, *J* = 11.0 Hz, 4H), 8.61-8.58 (m, 1H), 835-8.32 (m, 1H), 7.77-7.72 (m, 3H), 7.66-7.64 (m, 2H), 7.55-7.52 (m, 1H), 7.45-7.39 (m, 3H), 7.29-7.23 (m, 4H), 4.57-4.55 (m, 2H), 4.26-4.21 (m, 4H); ¹³C NMR (100 MHz, DMSO) d 166.7, 166.1, 159.8, 159.4, 151.2, 149.6, 145.9, 140.6, 133.3, 132.4, 131.1, 130.1, 129.6, 129.5, 129.1, 128.7, 127.93, 127.86, 1273, 124.9, 124.5, 49.1, 44.0, 42.4; HRMS (EI) calcd for C₂₈H₂₈ClN₈O₂ 543.2024, found 543.2032.

### Example 53

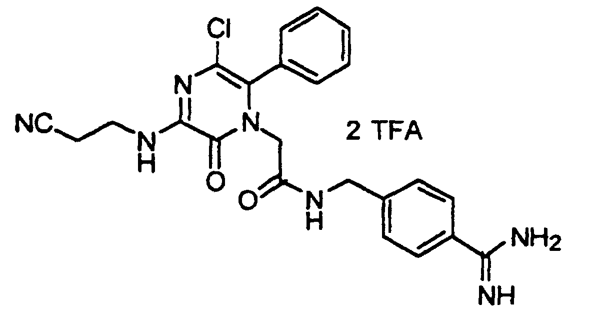

To a solution of 2-{5-chloro-3-[(2-cyanoethyl)amino]-2-oxo-6-phenylhydropyrazinyl}acetic acid (1.45 g, 3.25 mmol) in 17.0 mL dimethylformamide was added N,N-diisopropylethylamine (3.00 mL, 17.2 mmol), N-hydroxybenzotriazole (0.536 mg, 3.96 mmol), and 1-[3-(dimethylamino)propyl]-3-ethylcarbodiimide hydrochloride (0.752 mg, 3.923mmol). The resulting mixture was allowed to stir for 30 minutes. The reaction mixture was then added the Cbz protected amidine (1.2631g, 3.950 mmol) prepared and used in **Example 44** in one portion. The resulting mixture was allowed to stir over night. The reaction mixture was diluted with ethyl acetate (250 mL) and washed with 5% citric acid (1 x 50 mL), saturated NaHCO₃ (1 x 50 mL), and brine (1 x 50 mL). The organic solution was dried (MgSO₄), filtered and concentrated. The crude reaction was purified by MPLC (100% ethyl acetate) to give pure **EX·53A** in 82 % yield: ¹H NMR (400 MHz, DMSO) δ 9.06 (br s, 1H), 8.50-8.47 (m, 1H), 8.05-8.02 (m, 1H), 7.89 (d, *J* = 8.2 Hz, 2H) 7.46-7.26 (m, 11H), 7.18 (d, *J* = 8.2 Hz, 2H), 5.07 (s, 2H), 4.24-4.21 (m, 4H), 3.55-3.51 (m, 2H), 2.83-2.80 (m, 2H); HRMS (EI) calcd for C₃₁H₂₈ClN₇O₄ 598.1970, found 598.1970.

To a solution of pyrazinone **EX-53A** (1.497 g, 2.50 mmol) in 25.0 mL ethanol/4 M HCl in dioxane (3:1, 0.1 M) was flushed with hydrogen gas. To the solution was then added 10% Pd/C (wet) and the resulting suspension was allowed to stir at room temperature under an atmosphere of hydrogen (balloon pressure) for approximately 18 hours. The reaction mixture was filtered through a pad of Celite 545 and rinsed with ethanol. The solvent was removed under reduced pressure. Purification by HPLC (5% acetonitrile to 95 % acetonitrile/H₂O/0.1% trifluoroacetic acid) provided pure product: ¹H NMR (400 MHz, DMSO) d 9.51 (s, 2H), 9.29 (s, 2H), 8.63-8.60 (m, 1H), 8.03-8.00 (m, 1H), 7.74 (d, *J* = 8.3 Hz, 2H), 7.44-7.40 (m, 3H), 7.29-7.27 (m, 4H), 4.27-4.24 (m, 4H), 3.55-3.51 (m, 3H), 2.83-2.80 (m, 3H); HRMS (ES) calcd for C₂₃H₂₃ClN₇O₂ 464.1602, found 464.1624.

### Example 54

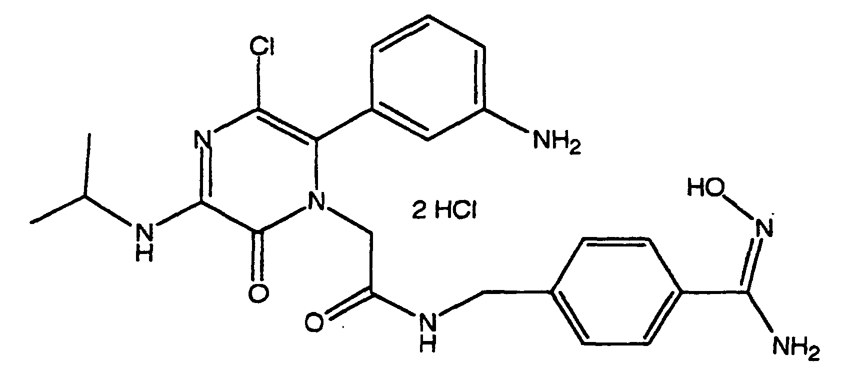

A solution of *p*-bromophenethylamine (40 g, 199.92 mmol) and phthalic anhydride (29.6 g, 199.84 mmol) in 250 mL of dioxane and 25 mL of dimethylformamide was heated at 120°C for 24 hours. The flask was then cooled, and the resulting white precipitate was filtered and washed with methanol (200 mL) to give **EX-54A** in exceptional yield and purity: ¹H NMR (400 MHz, CDCl₃) d 7.8 (m, 2H), 7.7 (m, 2H), 7.4 (d, 2H), 7.1 (d, 2H), 3.8 (t, 2H), 2.95 (t, 2H); MS (ES) calcd for C₁₆H₁₂BrNO₂ 330, found 331 (M+H).

A nitrogen purged solution of **EX-54A** (40 g, 121.15 mmol) and copper (I) cyanide (16.28 g, 181.72 mmol) in 500 mL of dimethylformamide was heated at 170°C for 24 hours. The solvent was removed under vacuum, and the resulting material was taken up in ethyl acetate. The ethyl acetate suspension was flashed through celite and concentrated under vacuum. The resulting white precipitate **EX-54B** was of exceptional yield and purity: ¹H NMR (300 MHz, CDCl₃) d 7.82 (m, 2H), 7.75 (m, 2H), 7.6 (d, 2H), 7.35 (d, 2H), 3.95 (t, 2H), 3.05 (t, 2H); MS (ES) calcd for C₁₇H₁₂N₂O₂ 276, found 277 (M+H).

A solution of *p*-cyanophenethylamine **EX-54B** (25 g, 90.48 mmol) and hydroxylamine hydrochloride (8 g, 115.12 mmol) in 1 L of ethanol and 20 mL ( 114.82 mmol) of diisopropylethylamine was heated at reflux for 16 hours. The flask was then cooled, and the resulting white precipitate was filtered and air dried to give **EX-54C** in an adequate yield and purity: ¹H NMR (300 MHz, DMSO) d 7.75 (d, 2H), 7.55 (d, 2H), 7.25 (d, 2H), 7.2 (d, 2H), 3.8 (m, 2H), 2.95 (m, 2H); MS (ES) calcd for C₁₇H₁₅N₃O₃ 309, found 310 (M+H).

A solution of *p*-(N-hydroxy)amidinophenethyl phthalimide (**EX-54C**) (4.53 g, 14.64 mmol) in 200 mL of chloroform was treated with hydrazine monohydrate (1 mL, 20.62 mmol). The reaction was stirred vigorously at 50°C for 24 hours. The flask was then cooled and the resulting white precipitate was filtered and washed with chloroform (200 mL). A 50:50 mixture of product **EX-54D** and phthalhydrazide was obtained and used as is: ¹H NMR (300 MHz, DMSO) d 7.6 (d, 2H), 7.2 (d, 2H), 2.8 (t, 2H), 2.7 (m, 2H); MS (ES) calcd for C₁₇H₁₅N₃O₃ 179, found 180 (M+H).

Reacting **EX-54D** containing phthalhydrazide with 2-{5-chloro-6-(3-nitrophenyl)-3-[N-(1-methylethyl)amino]-2-oxohydropyrazinyl}acetic acid in place of 2-{5-chloro-6-(3-nitrophenyl)-3-cyclobutylamino-2-oxohydropyrazinyl}acetic acid and **EX-27D** and then hydrogenating the resulting intermediate according to the final two procedures described in **Example 27** gave the product with an m/z+1 of 484.

### Example 55

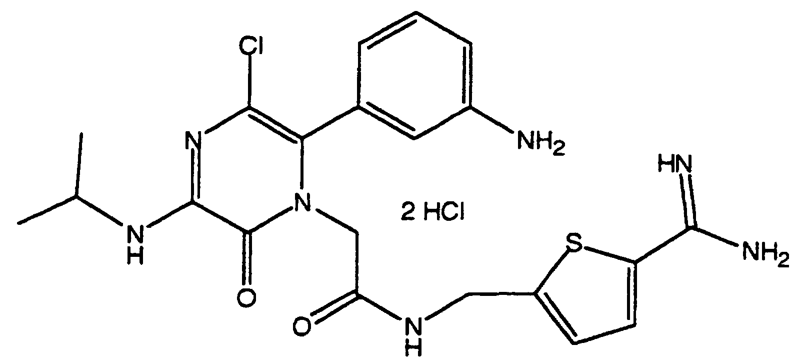

A solution of diisopropylamine (35.3ml, 0.251 moles) in tetrahydrofuran (500ml) was cooled to -78°C under a nitrogen blanket. To this was added 1.6M n-butyllithium in hexanes (157ml, 0.251moles) and allowed to stir for 5 min. Then slowly added thiopene-2-carbonitrile (21.33ml, 0.229moles) in tetrahydrofuran (115 ml) and allowed to stir. After 45min. was added *NN*-dimethylformamide (88.66ml, 1.145moles) at -78°C. Citric acid (40g) was added after 2h. followed by water (240ml) and stirred for 18 h. The reaction was concentrated *in vacuo*, transferred to a separatory funnel, diluted with brine, and extracted twice with ether. The combined ether layers were washed with brine, dried over magnesium sulfate, filtered, and the solvent removed *in vacuo*. Chromatography yielded 15.8g (50%) of 2-cyano-5-formylthiophene (**EX-55A**) as a brown solid: ¹H NMR (300MHZ, CDCl₃) d 10.02 (s, 1H), 7.79 (m, 1H), 7.30 (m, 1H).

2-Cyano-5-formylthiophene (**EX-55A**) (15.8g, 0.229moles) was stirred in ethanol (375ml), and sodium borohydride (4.36g, 0.115moles) added in small portions. After 15min., the solvent was removed in vacuo, and residue taken up in ethyl acetate. After the ethyl acetate was washed with 1N potassium hydrogen sulfate and brine, the organic layer was dried over magnesium sulfate, filtered, and solvent removed *in vacuo*. The residue was dried on vacuum pump to yield 9.57g (59%) of the alcohol **EX-55B** as a brown-orange oil: ¹H NMR (300MHz, CDCl₃) d 7.53 (m, 1H), 7.00 (m, 1H), 4.88 (s, 2H), 2.84 (br s, 1H).

To a stirring solution of **EX-55B** (9.57g, 0.069moles) in tetrahydrofuran (80ml)was added triphenylphosphine (19.86g, 0.075moles) and carbon tetrabromide (25.11g, 0.075moles). After 18h. the reaction was concentrated *in* *vacuo,* and the crude material chromatographed to yield **EX-55C** as a brown oil: ¹H NMR (300MHz, CDCl₃) d 7.52 (m, 1H), 7.14 (m, 1H), 4.69 (s,2H).

2-Aminomethyl-5-carbobenzyloxyamidinothiophene dihydrogen chloride salt **(EX-55D)** was prepared by the method outlined in Synthetic Communications, 28(23), 4419-4429 (1998) by substituting 5-bromomethyl-2-cyanothiophene (**EX-55C**) for 4-cyanobenzyl bromide to give after trituration with acetonitrile **EX-55D:** ¹H NMR (300MHz, DMSO) d 9.98 (br s, 1H), 8.83 (br s, 2H), 8.10 (s, 1H), 7.40-7.48 (m, 7H), 5.26 (s, 2H), 4.31 (s. 2H); HRMS calcd for C₁₄H₁₆N₃O₂S 290.0963, found 290.0949.

Reacting **EX-55D** with 2-{-5-chloro-6-(3-nitrophenyl)-3-[N-(1-methylethyl)amino]-2-oxohydropyrazinyl}acetic acid in place of 2-{5-chloro-6-(3-nitrophenyl)-3-cyclobutylamino-2-oxohydropyrazinyl}acetic acid and **EX-27D** and then hydrogenating the resulting intermediate according to the final two procedures described in **Example 27** gave the product with an m/z+1 of 474.

### Example 56

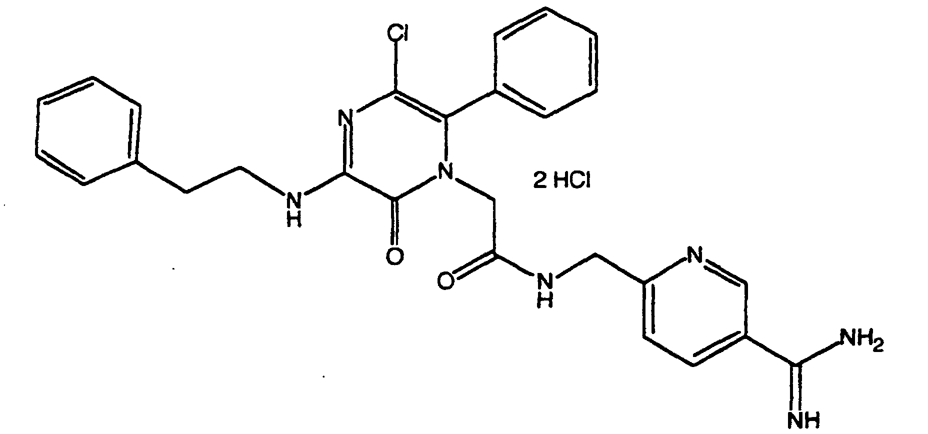

To a stirring solution of 3-cyano-6-methylpyridine (20a, 0.169moles) in carbon tetrachloride (850ml) was added N-bromosuccinimde (30a, 0.169moles) and benzoyl peroxide (4.1g, 0.0169moles), and the solution was heated to reflux. After 18h, the heat was discontinued, diluted with carbon tetrachloride (1L) and washed twice with water (1L). The solvent was removed *in vacou* and the crude material chromatographed to yield 12.05g (36%) of dark brown solid **EX-56A**: ¹H NMR (300MHz, CDCl₃) d 8.86 (d, 1H), 7.00 (m, 1H), 7.62 (m, 1H), 4.60 (s, 2H); ¹³C NMR (300MHz, CDCl₃) d 156.38, 147.70, 135.82, 118.98, 111.75, 104.66, 27.82; HRMS (EI) calcd for C₇H₆BrN₂ 196.9714, found 196.9661.

2-Aminomethyl-5-carbobenzyloxyamidinopyridine dihydrogen chloride salt (**EX-56B**) was prepared by the method outlined in Synthetic Communications, 28(23), 4419-4429 (1998) by substituting 5-bromomethyl-2-cyanopyridine (**EX-55A**) for 4-cyanobenzyl bromide to give **EX-56B:** HPLC/LRMS; 98%, (M+H)⁺ 285.

Reacting **EX-56B** with 2-{5-chloro-6-phenyl-3-[N-(2-phenylethyl)amino]-2-oxohydropyrazinyl}acetic acid in place of 2-{5-chloro-6-(3-nitrophenyl)-3-cyclobutylamino-2-oxohydropyrazinyl}acetic acid and **EX-27D** and then hydrogenating the resulting intermediate according to the final two procedures described in **Example 27** gave the product with an m/z+1 of 482.

### Example 57

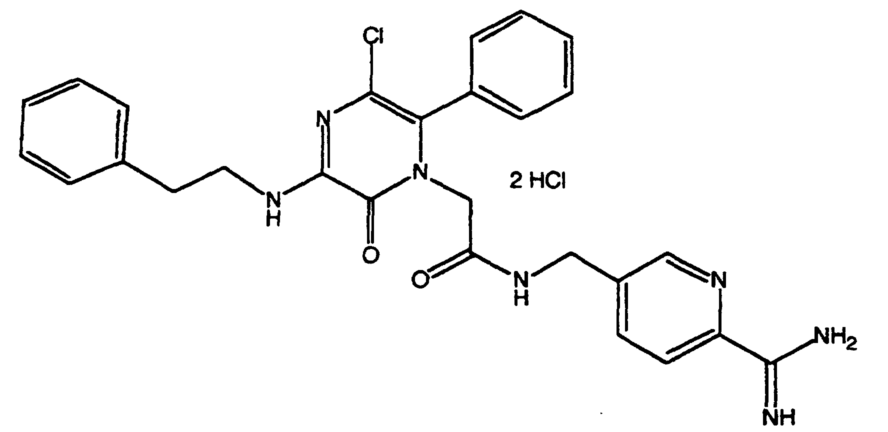

2-Cyano-5-methylpyridine (**EX-57A**) was prepared following the procedure outlined in Synthetic Communications, 19(13&14),2371-2374 (1989): HRMS (EI) calcd for C₇H₇N₂ 119.0609, found 119.0587.

By following the procedure of **Example 56** and substituting 2-cyano-5-methylpyridine for 3-cyano-6-methylpyridine, the intermediate 5-bromomethyl-2-cyanopyridine (**EX-57B**) was prepared.

2-Aminomethyl-5-carbobenzyloxyamidinopyridne dihydrogen chloride salt (**EX-57C**) was prepared by the method outlined in **Example 56** substituting 5-bromomethyl-2-cyanopyridine for 6-bromomethyl-3-cyanopyridine: HPLC/LRMS; 95%, (M+H)⁺ 285.

Reacting 2-Aminomethyl-5-casbobenzyloxyamidinopyridne dihydrogen chloride salt **(EX-57C)** with 2-{5-chloro-6-phenyl-3-[N-(2-phenylethyl)amino]-2-oxohydropyrazinyl}acetic acid as described in **Example 56** gave the product with an m/z+1 of 482.

### Example 58

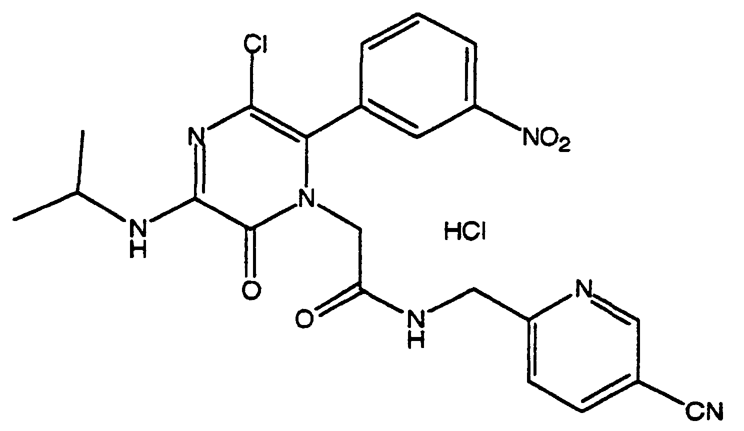

2-Aminomethyl-5-cyanopyridine hydrochloride (**EX-58A**) was prepared by the deprotection with 4N HCl Dioxane of the intermediate 2-{N,N-bis-(tert-butoxycarbonyl)aminomethyl}-5-cyanopyridine used to prepare 2-aminomethyl-5-carbobenzyloxyamidinopyridine dihydrogen chloride salt in **Example 56**: ¹H NMR (400MHz, DMSO) d 9.04 (s, 1H), 8.64 (br s, 2H), 8.34 (m, 1H), 7.69 (m, 1H), 4.25 (s, 2H); HRMS (EI) calcd for C₇H₈N₃ 134.0718, found 134.0699.

Using the procedure of **Example 44** by substituting 2-[5-chloro-3-(N-{1-methylethyl}amino)-2-oxo-6-phenylhydropyrazinyl]acetic acid (**EX-1D**) for 2-[3-({2-[(tert-butoxy)carbonylamino]ethyl}amino)-5-chloro-2-oxo-6-phenylhydropyrazinyl]acetic acid, **EX-58A** was converted to the product which gave an m/z+1 of 482.

### Example 59

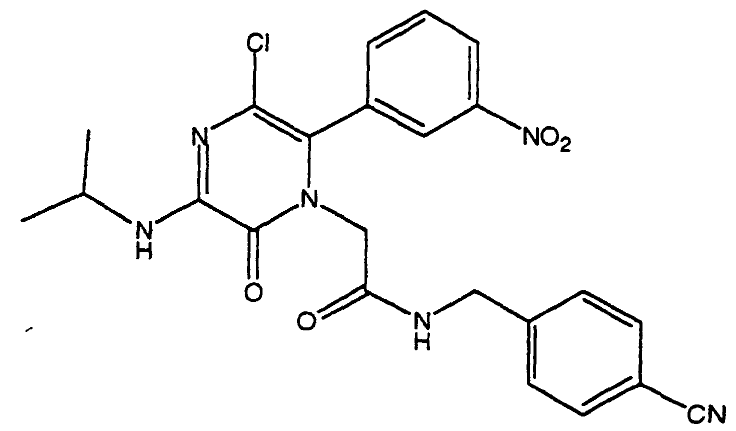

4-cyanobenzylamine hydrochloride (**EX-59A**) was prepared from 10g (0.030moles) of 4-{N,N-bis-(tert-butoxycarbonyl)aminomethyl}benzonitrile, prepared following Synthetic Communication, 28(23), 4419-4429 (1998), by stirring it in 4N HCl Dioxane (75ml). After 3h, the solution was concentraed *in vacuo* and triturated with ether. The solid was collected by filtration and vacuum dried to yield 5g (98%) of **EX-59A** as a white solid: ¹H NMR (DMSO) d 8.68 (br s, 2H), 7.84 (m, 2H), 7.67 (m, 2H), 4.06 (s, 2H); HRMS (EI) calcd for C₈H₈N₂ 133.0766, found 133.0807

Using the procedure of **Example 58, EX-59A** was converted to the product which gave an m/z+1 of 481.

Using the procedures of **Scheme 1**, **Scheme 2**, and the **Examples** herein with suitable reagents, starting materials, and intermediates, additional pyrazinones of the present invention were prepared and these pryazinones are summarized in Table 3.

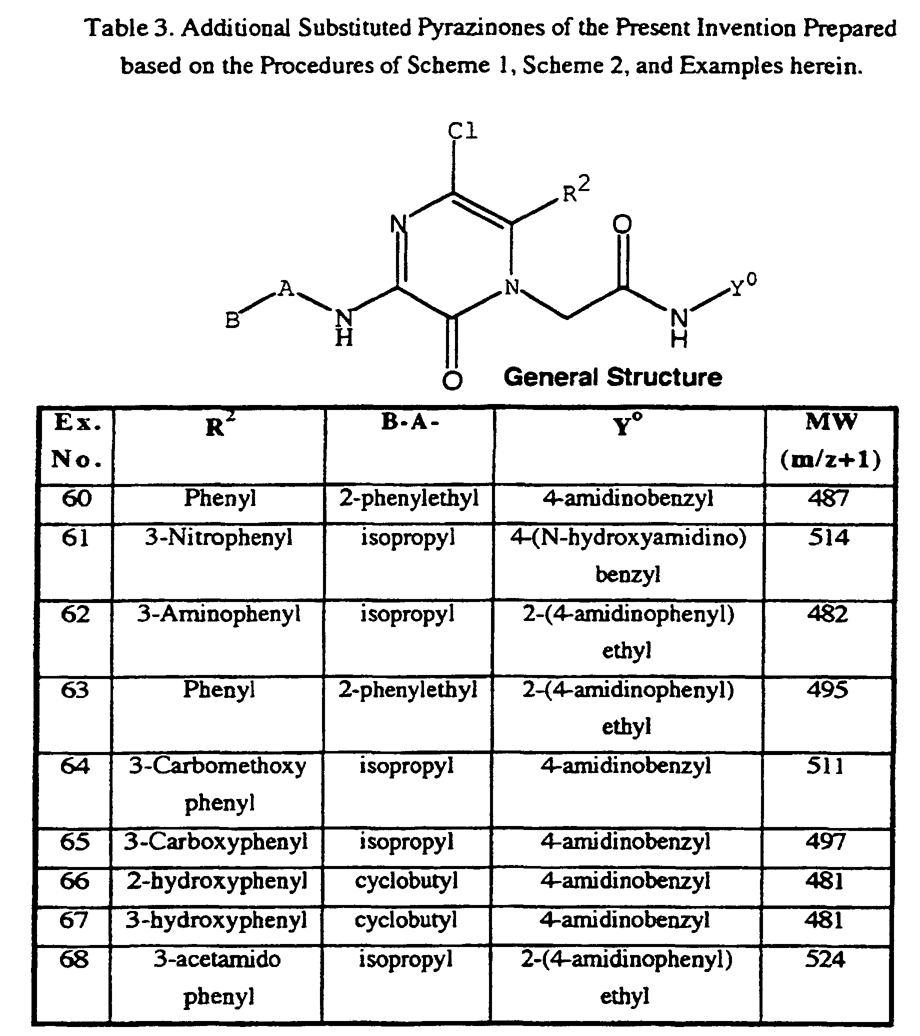

### Example 69

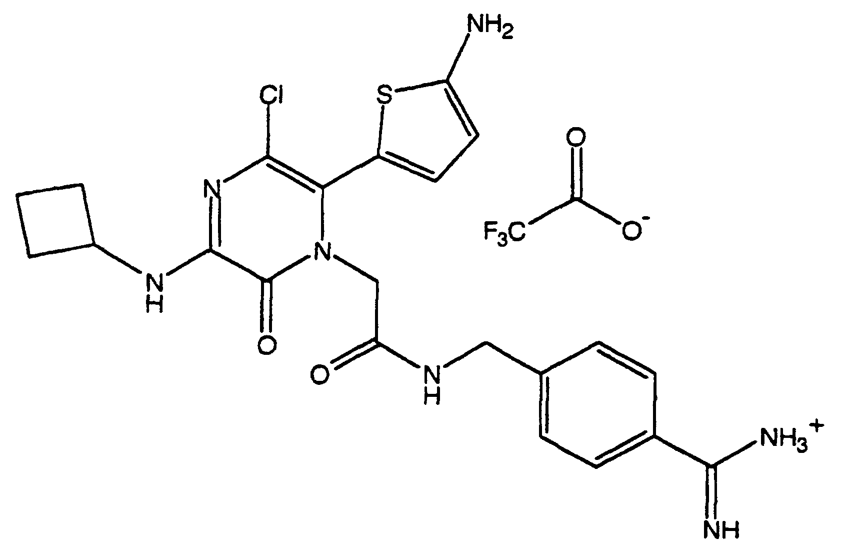

1-Benzyloxycarbonylmethyl-6-(5-bromothiophen-2-yl)-3,5-dichloro pyrazinone (**EX-69A**) was synthesized as described in the general schemes of the patent and as described, for example, specifically for **EX-1B** substituting 5-bromothiophenecarbaldehyde for benzaldehyde. **EX-69A** is a yellow crystalline solid: HPLC-MS (5 to 95% AcCN / 6 min @ 1.0 mL / Min @ 254 nm @ 50 °C): retention time 438 min, M+Na⁺ = 494.9 for formula C₁₇H₁₁BrCl₂N₂O₃SNa; ¹H NMR (400 MHz, CDCl₃): d 4.62 (s, 2H), 5.19 (s, 2H), 6.79 (d, J = 4.0 Hz, 1H), 7.00 (d, J = 4.0 Hz, 1H) 732 (m, 2H), 7.37 (m, 3H); ¹³C NMR (101 MHz, CDCl₃): d 49.0, 68.1, 117.8, 126.5, 128.6, 128.7, 128.8, 130.1, 130.7, 132.0, 134.5, 147.8, 151.9, 166.2.

**EX-69A** (12.15 g. 25.75 mmol) was treated with cyclobutylamine (3.80 g, 53.52 mmol) in 250 ml toluene at room temperature for 4 hours. The toluene solution was washed with saturated ammonium chloride solution and dried over anhydrous MaSO₄. After removing the toluene, the pure product **EX-69B** was obtained as a yellow solid (13.05 g, 99%): HPLC-MS (5 to 95% AcCN / 6 min @ 1.0 mL / Min @ 254 nm @ 50 °C): retention time 4.90 min, M+H⁺ = 508.0 for formula C₂₁H₂₀BrClN₃O₃S.

Potassium phthalimide (4.56 g, 24.6 mmol) and Cul (18.0 g, 94.7 mmol) were mixed in 200 ml dimethylacetamide. The mixture was stirred at room temperature for 10 minutes. To this mixture was added compound **EX-69B** (12.0 g. 23.7 mmol). The resulting mixture was heated to 160°C and stirred for 5 hours at an open air atmosphere. The reaction solution was filtered to remove all the insoluble solid and was concentrated via high vacuum distillation at a rotavapor. Aqueous work-up and silica gel flush chromatography yielded the pure product **EX-69C** as light yellow solid (6.8 g, 50%) with the des-bromo side product formation the reason for the low yield: HPLC-MS (5 to 95% AcCN / 6 min @ 1.0 mL / Min @ 254 nm @ 50 °C): retention time 3.82 min, M+H⁺ = 575.5 for formula C₂₉H₂₄ClN₄O₅S; ¹H NMR (400 MHz, CDCl₃): d 1.69 (m, 2H), 1.92 (m, 2H), 2.36 (m, 2H), 4.45 (m, 1H), 4.49 (s, 2H), 5.07 (s, 2H), 6.54 (d, J = 8.0 Hz, 1H), 6.75 (d, J = 3.6 Hz, 1H), 7.17-7.25 (m, 7H), 7.50 (d, J = 4.0 Hz, 1H), 7.71 (dd, J = 2.8, 5.2 Hz, 2H), 7.85 (dd, J = 2.8, 5.2 Hz, 2H); ¹³C NMR (101 MHz, CDCl₃): d 15.2, 30.9, 45.8, 47.4, 67.4, 114.7, 118.2, 123.9, 126.5, 128.28, 128.33, 128.36, 128.39, 128.42, 128.45, 129.5, 129.9, 131.1. 134.8, 134.9, 135.7, 148.5, 150.8, 165.2, 166.9.

**EX-69C** (0.55 g, 0.96 mmol) was treated with 1 ml hydrazine in 10 ml methanol and 5 ml dichloromethane for 4 hours. The reaction solution was acidified with 1N HCl and filtered to remove the solid by-product. Aqueous work-up yield the crude (90% pure) product **EX-69D** (0.49 g): HPLC-MS (5 to 95% AcCN / 6 min @ 1.0 mL / Min @ 254 nm @ 50 °C): retention time 3.29 min, M+H⁺ = 445.3 for formula C₂₁H₂₂ClN₄O₃S.

**EX-69D** (0.48 g, 1.08 mmol) was mixed with Boc anhydride (0.28 g, 130 mmol), triethylamine (0.22 g, 2.16 mmol) and DMAP (12 mg, 0.1 mmol). The reaction mixture was stirred for 4 hours at room temperature. After an aqueous work-up, the crude product in 2 ml CH₃CN and 2 ml THF was treated with 2 ml 1M LiOH for 3 hours. Aqueous work-up yield the crude carboxylic acid **EX-69E**: HPLC-MS (5 to 95% AcCN / 6 min @ 1.0 mL / Min @ 254 nm @ 50°C): retention time 3.18 min, M+H⁺ = 455.4 for formula C₁₉H₂₄ClN₄O₅S.

**EX-69E** was coupled with the protected amidine, 4-(N-benzyloxycarbonylamidino)benzylamine hydrogen chloride salt, prepared using the procedure outlined Synthetic Communications, 28(23), 4419-4429 (1998) in the same way as described before using EDC, HOBt and DIEA in DMF to give the protected product **EX-69F**. **EX-69F** was purified by reverse phase HPLC using C18 column to give an off-white amorphous solid: HPLC-MS (5 to 95% AcCN / 6 min @ 1.0 mL / Min @ 254 nm @ 50 °C): retention time 3.28 min, M+H⁺ = 720.9 for formula C₃₅H₃₈ClN₇O₆S; ¹H NMR (400 MHz, CDCl₃): d 1.49 (s, 9H), 1.80 (m, 2H), 2.03 (m, 2H), 2.45 (m, 2H), 4.3 (b, 2H), 4.49 (b, 3H), 5.07 (s, 2H), 6.66-6.78 (m, 2H), 7.0-7.18 (m, 2H), 7.33-7.47 (m, 5H).

**EX-69F** was converted to the product by hydrogenation as described before. After the hydrogenation, it was treated with HCl saturated methanol solution to remove the Boc group. The product was purified by reverse phase HPLC with a C18 column with amobile phase was 0.1% TFA in water and acetonitrile to give the product as a TFA salt and an off-white amorphous solid: HPLC-MS (5 to 95% AcCN / 6 min @ 1.0 mL / Min @ 254 nm @ 50 °C): retention time 1.94 min, M+H⁺ = 486.4 for formula C₂₂H₂₅ClN₇O₂S; ¹H NMR (400 MHz, methanol-d₄): d 1.79 (m, 2H), 2.06 (m, 2H), 2.39 (m, 2H), 4.45 (s, 2H), 4.46 (m, 1H), 4.57 (s, 1H), 4.58 (s, 1H), 6.01 (d, J = 4 Hz, 1H), 6.52 (m, 1H), 7.49 (d, J = 8.4 Hz, 2H), 7.75 (m, 2H).

Sulfonyl analogs of pyrazinones wherein a sulfonyl is present as a replacement for the carbonyl of the acetamide at the N-1 position of the pyrazinone can be prepared using **Scheme 3: Sulfonyl Pyrazinone** detailed below along with the specific **Example 70**.

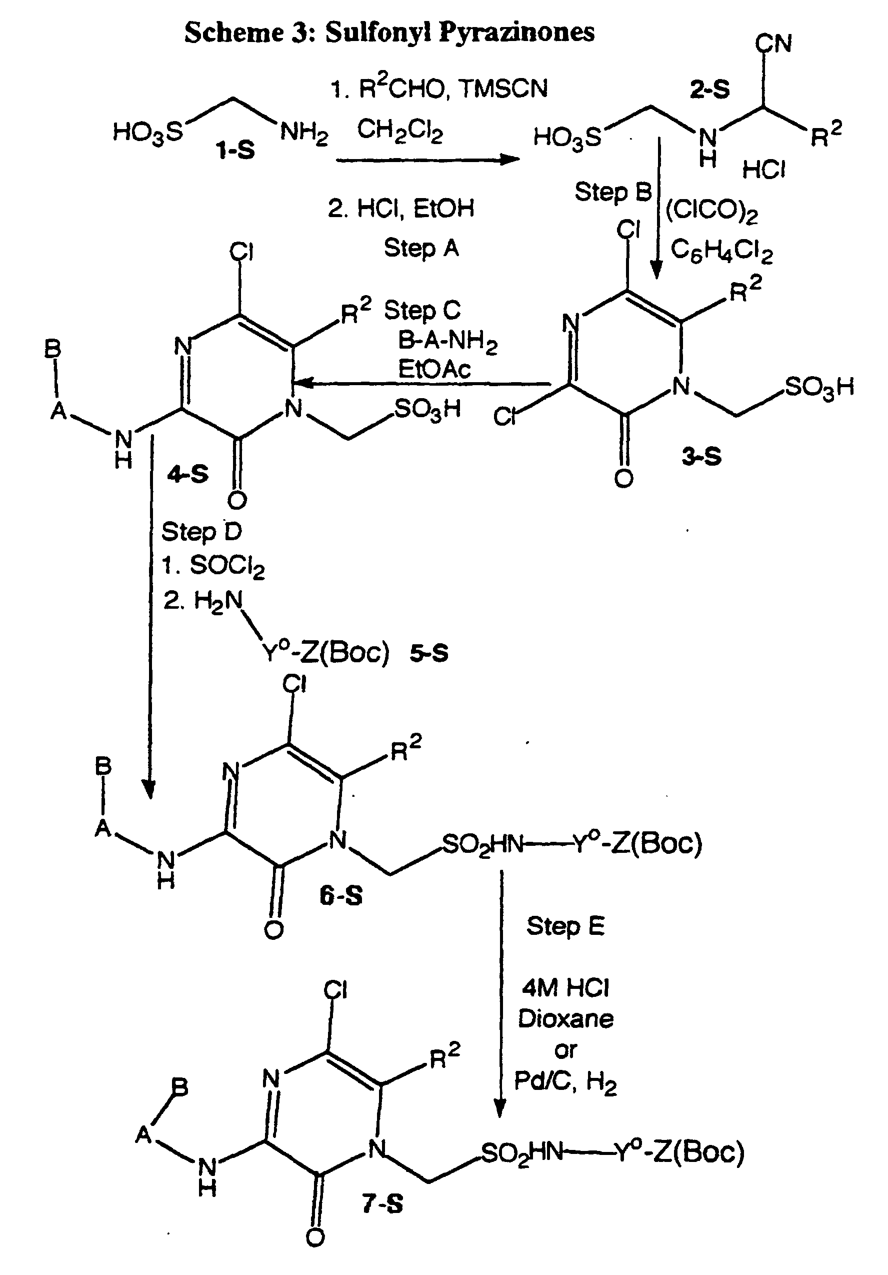

### Example 70

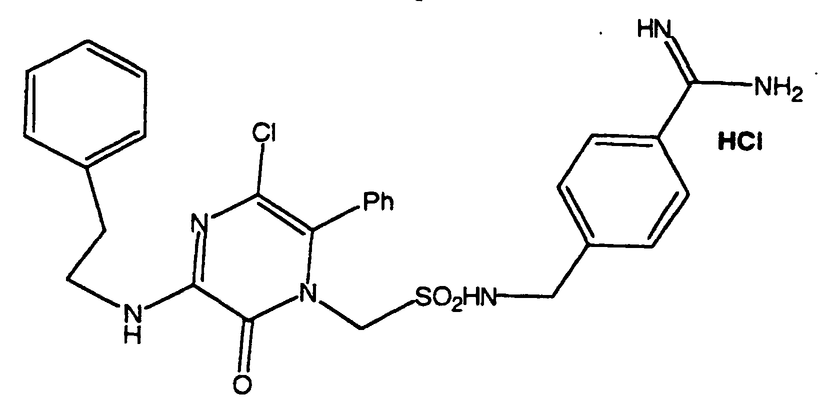

Benzaldehyde (1 eq.) is added slowly by syringe to a solution of aminomethanesulfonic acid (1 eq.) in dichloromethane at room temperature. Trimethylsilyl cyanide (1 eq.) is added dropwise via syringe over a 10 minute period. The reaction is stirred for 4 hours at room temperature and then concentrated under reduced pressure. The residue is diluted with ethyl acetate, washed with brine, dried (MaSO₄), and concentrated. The residue is diluted with ethyl acetate (80 mL) and 9.9 M HCl (1.05 eq.) in ethanol is added (prepared by addition of 28.90 mL acetyl chloride to 41.0 mL cold ethanol), resulting in precipitation of the intermediate product **EX-70A.** The precipitate is collected by filtration, washed with ethyl ether, and dried to give pure product **EX-70A.**

To a suspension of 1 eq. of **EX-70A** in dry 1,2-dichlorobenzene (1.0 M) is added oxalyl chloride (4 eq.) with stirring at room temperature. The resulting suspension is heated at 100°C for approximately 18 hours. The reaction is allowed to cool to room temperature and the volatiles are removed under reduced pressure. The remaining solution is passed through a silica gel column (hexane flush, followed by 50% ethyl acetate/hexanes). Concentration of the solution gives crude product **EX-70B**, which is purified by column chromatography.

Phenethylamine (3 eq.) is added to a solution of **EX-70B** (1 eq.) in ethyl acetate at room temperature. The resulting solution is heated at reflux for 18 hours. The solution is allowed to cool to room temperature, resulting in formation of a thick precipitate. The reaction mixture is diluted with ethyl acetate, washed with 0.5 N HCl, saturated NaHCO₃ and brine. The organic solution is dried (MgSO₄), filtered and concentrated to give the crude product. Recrystallization from ethyl acetate and hexanes affords pure product **EX-70C**.

A solution of 1 eq. of **EX-70C** in dichloromethane with several drops of dimethylformamide added is cooled to 0°C. Thionyl chloride (1.1 eq.) is added dropwise and the solution is slowly warmed to room temperature. After completion of the reaction, the volatile components are removed under reduced pressure and the product **EX-70D** is immediately used in the next step.

To the sulfonyl chloride **EX-70D** (1 eq.) in dichloromethane is added the amine, 4-(N-tert-butoxycarbonylamidino)benzylamine hydrochloride, in DMF with 5 eq. of N-methylmorpholine. After completion of the reaction, polyaldehyde and/or polyamine resin (10 eq.) are added to remove any unreacted starting materials. The resins are filtered, rinsed with DMF/DCM (1:1) and the solvents are removed under reduced pressure to give pure product **EX-70E**.

To 1 eq. of **EX-70E** is added 40 eq. of 4 M HCl/dioxane. The resulting solution is stirred at room temperature overnight. The solution is concentrated and the crude product is triturated from solvent to afford pure product.

Methylene analogs of pyrazinones wherein a methylene is present as a replacement for the carbonyl of the acetamide at the N-1 position of the pyrazinone can be prepared using **Scheme 4: Methylene Pyrazinone** detailed below along with the specific **Example 71**.

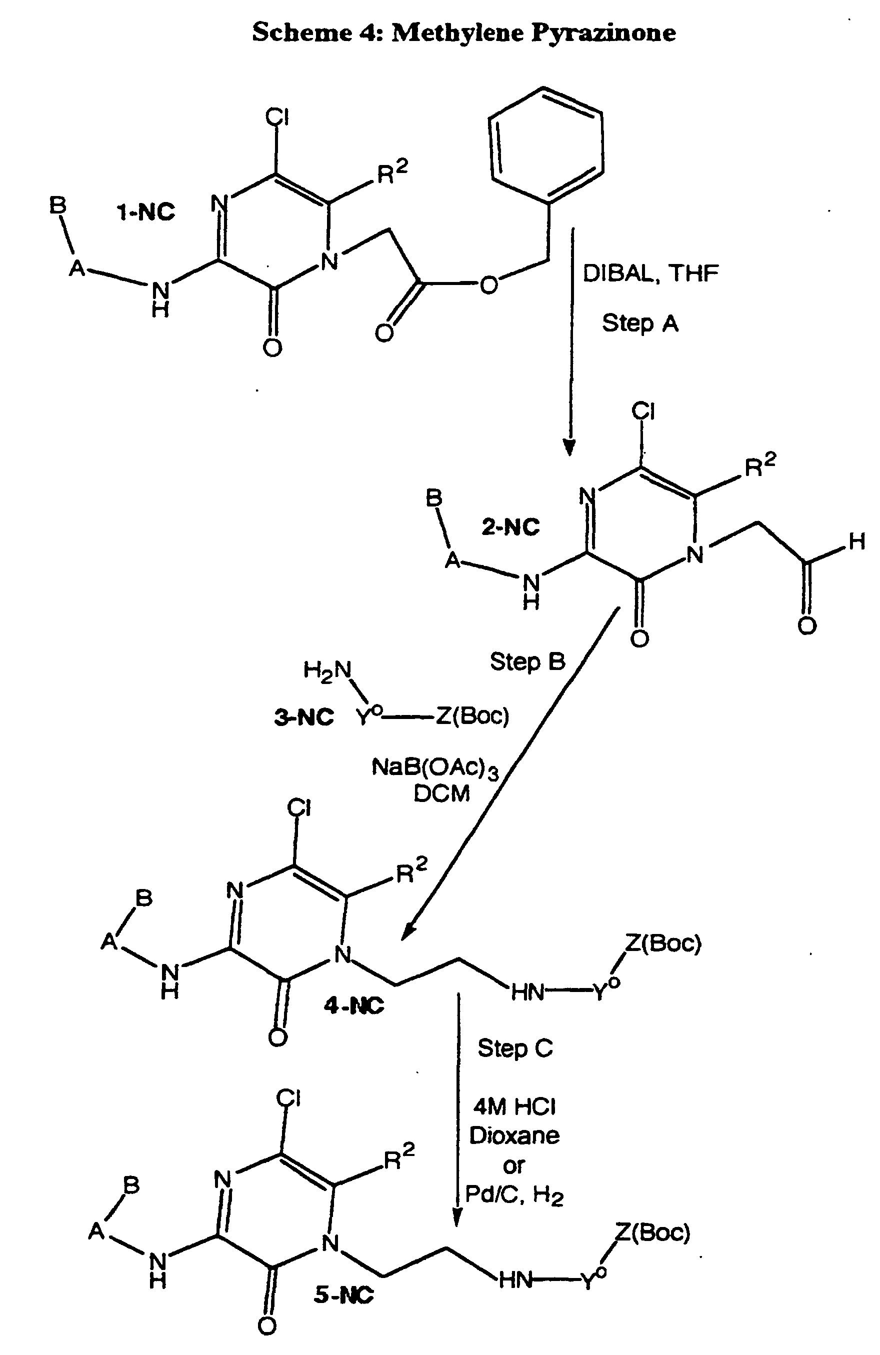

### Example 71

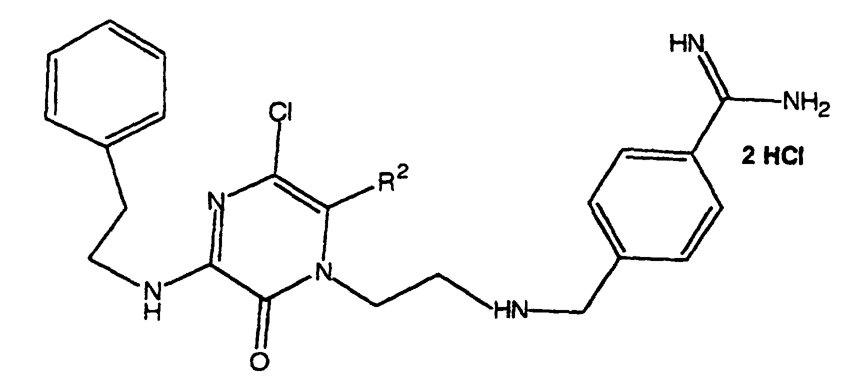

Diisobutylaluminum hydride (1.05 equiv.) is added over a period of 15 min to a cooled solution (-78 °C) of I eq. of 1-benzyloxycarbonylmethyl-5-chloro-6-phenyl-3-(2-phenylethylamino)pyrazinone in tetrahydrofuran . After stirring for I h at -78 °C the reaction is slowly quenched at -78 °C with cold methanol. The mixture is slowly poured into ice-cold 1N HCl and the aqueous mixture is extracted with ethyl acetate. The combined organic layers are washed with brine, dried with MgSO₄, filtered, and the solvents are removed under reduced pressure. The crude product is purified by column chromatography to afford purified product **EX-71A**.

Sodium triacetoxyborohydride (1.2 eq.) and a catalytic amount of acetic acid are added to a suspension of 1.0 eq. of **EX-71A** and 1.0 eq. of the amine, 4-(N-tert-butoxycarbonylamidino)benzylamine hydrochloride, in dichloromethane. The suspension quickly clears and becomes homogeneous. The reaction is stirred for several hours. The solution is cooled in an ice bath and basified with 1.0 N NaOH. The reaction mixture is diluted with dichloromethane and washed with brine. The organic solution is dried (MgSO₄), filtered and concentrated to give the crude product. The crude product is purified by silica gel chromatagraphy to afford purified product **EX-71B**.

To 1 eq. of **EX-71B** is added 40 eq. of 4 M HCl/dioxane. The resulting solution is stirred at room temperature overnight. The solution is concentrated and the crude product is triturated from ethyl ether to afford pure product.

### General Robotics and Experimental Procedure for the Robotic Parallel Synthesis of a Series of Amides E-i and Z-i from A-i.

**Scheme 5** specifically illustrates the derivatization of the scaffold **A-i** to afford the desired product **D-i** in a parallel array synthesis format. In a parallel array synthesis reaction block, individual reaction products were prepared in each of multiple reaction block vessels in a spatially addressed format. A solution of the desired scaffold **A-i** (limiting amount) in acetonitrile (ACN) was added to the reaction vessels followed by a three-fold stoichiometric excess solution of the primary amine **B-i** in acetonitrile. Excess primary amine was used as a base and to effect complete conversion of scaffold **A-i** to product **C-i**. The reaction mixtures were incubated at 70°C for 16-20 h. After cooling to ambient temperature, each reaction vessel was charged with one mL of methanol and an excess (3-4 fold stoichiometric excess) of aqueous potassium hydroxide. The reaction block was shaken vertically for 14-20 h on an orbital shaker at ambient temperature. The contents of each reaction vessel were then acidified with aqueous HCl. Each reaction vessel was then opened, and the solutions were evaporated to dryness under N₂ and/or a Savant apparatus. Polyamine resin **R-1** (10-15 fold stoichiometric excess) was added to the solid carboxylic acid followed by dichloromethane and water (10:1). The mixture was shaken laterally for 14-20 h on an orbital shaker at ambient temperature (rotating the vials at least once so each side of the vial was agitated for a minimum of 2 h). The desired product **D-i** was sequestered away from the reaction by-products and excess reactants as the insoluble adduct **D-x**. Simple filtration of the insoluble resin-adduct **D-x** and rinsing of the resin cake with DMF, DCM, MeOH, and DCM afforded the desired resin-bound product. After drying the resin under vacuum for 2 h, an excess of HCl/dioxane (7-8 fold stoichiometric excess based on the loading of amine functionality) along with dichloromethane was added to each reaction vessel to cleave the desired product **D-i** from the resin. The reaction block was shaken laterally for 2-20 h on an orbital shaker at ambient temperature. Simple filtration of the solution, rinsing of the resin cake with dimethylformamide/dichloromethane, and evaporation of the solvents afforded the desired product **D-i** in purified form.

**Scheme 6** and **Scheme 7** illustrate the conversion of the carboxylic acid-containing scaffold **D-i** to the desired amide product **E-i** in a parallel synthesis format. A unique scaffold **D-i** was added as a solution in dichloromethane/ dimethylformamide to each reaction vessel. A solution of hydroxybenzotriazole **B-2** in dichloromethane/ dimethylformamide was added to each reaction vessel, followed by the polymer-bound carbodiimide reagent **R-2** (1.5 fold stoichiometric excess). The parallel reaction block was agitated vertically on an orbital shaker for 30 min to 1 h. A limiting amount of the same amine **B-3** (0.8 equivalents) in DMF, along with a 3 fold stoichiometric excess of NMM if the amine **B-3** was a salt, was added to the unique contents of each vessel. The parallel reaction block was then agitated vertically on an orbital shaker for 2-3 h at ambient temperature. An excess of the amine-functionalized resin **R-1** and aldehyde resin **R-3**, along with dichloromethane solvent were added to each reaction vessel. The resin-charged reaction block was shaken vertically for 2 h on an orbital shaker at ambient temperature. The amine-containing resin **R-1** sequestered **B-2** and any remaining **D-i** as their resin-bound adducts, **B-4** and **D-2**, respectively. The aldehyde-containing resin **R-3** sequestered any unreacted **B-3** as its resin-bound adduct **R-5**. Filtration of the insoluble resins and resin adducts **R-1**, **R-2**, **R-3**, **R-4**, **R-5**, **B-4**, and **D-2** and subsequent rinsing of the vessel resin-bed with dichloromethane/dimethylformamide afforded filtrates containing the purified products **E-i**. Concentration of the filtrates afforded the purified products **E-i**, which were weighed and analyzed by LC/MS.

For those amines **B-3** which contain a protecting group, a final deprotection step was required after the coupling reaction (**Scheme 8**). The residues **E-i** were dissolved in methanol, Pd/C was added, and the reaction mixtures were stirred under 10 psi of H₂ for 16-20 h. The mixtures were filtered through Celite, rinsed with methanol and concentrated to afford pure products **Z-i**, which were weighed and analyzed by LC/MS. If necessary, the products were purified by reverse-phase HPLC. Conversely, the deprotection step was done, as needed, in the presence of ammonium formate (5 fold stoichiometric excess) in place of the 10 psi of H₂.

A third method of deprotection uses TMSI generated in situ. The residues **E-i** were dissolved in acetonitrile. Sodium iodide and TMSCl (5 fold stoichiometric excess of each) were added, and the reaction mixtures were agitated vertically at 55°C for 14-20 h. Methanol and (N,N-dimethyl)aminomethylpolystyrene resin were added to each vessel, and the mixtures were agitated for another 3 h. The mixtures were filtered through Celite, rinsed with acetonitrile and concentrated to afford products **Z-i**, which were weighed and analyzed by LC/MS. If necessary, the products were purified by reverse-phase HPLC.

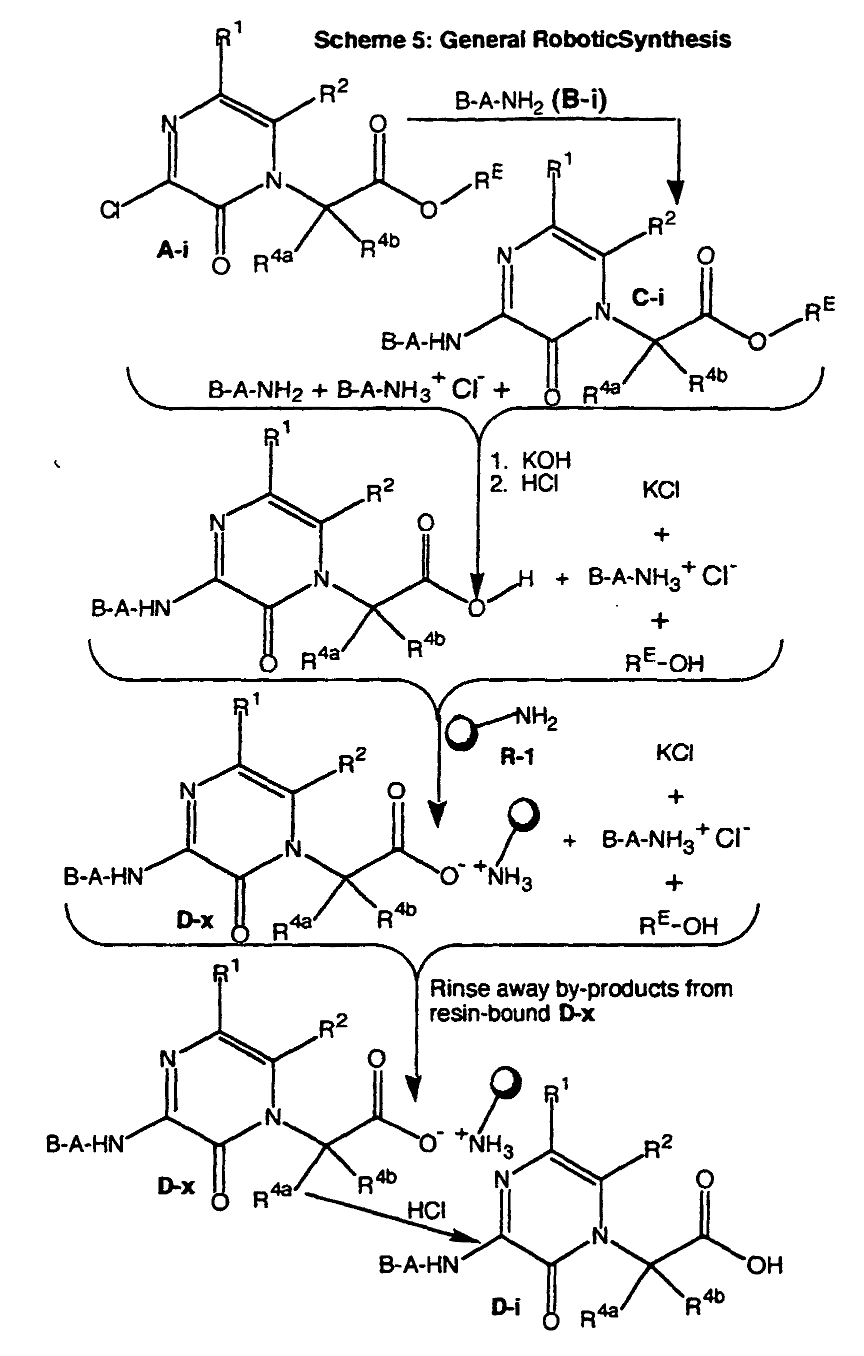

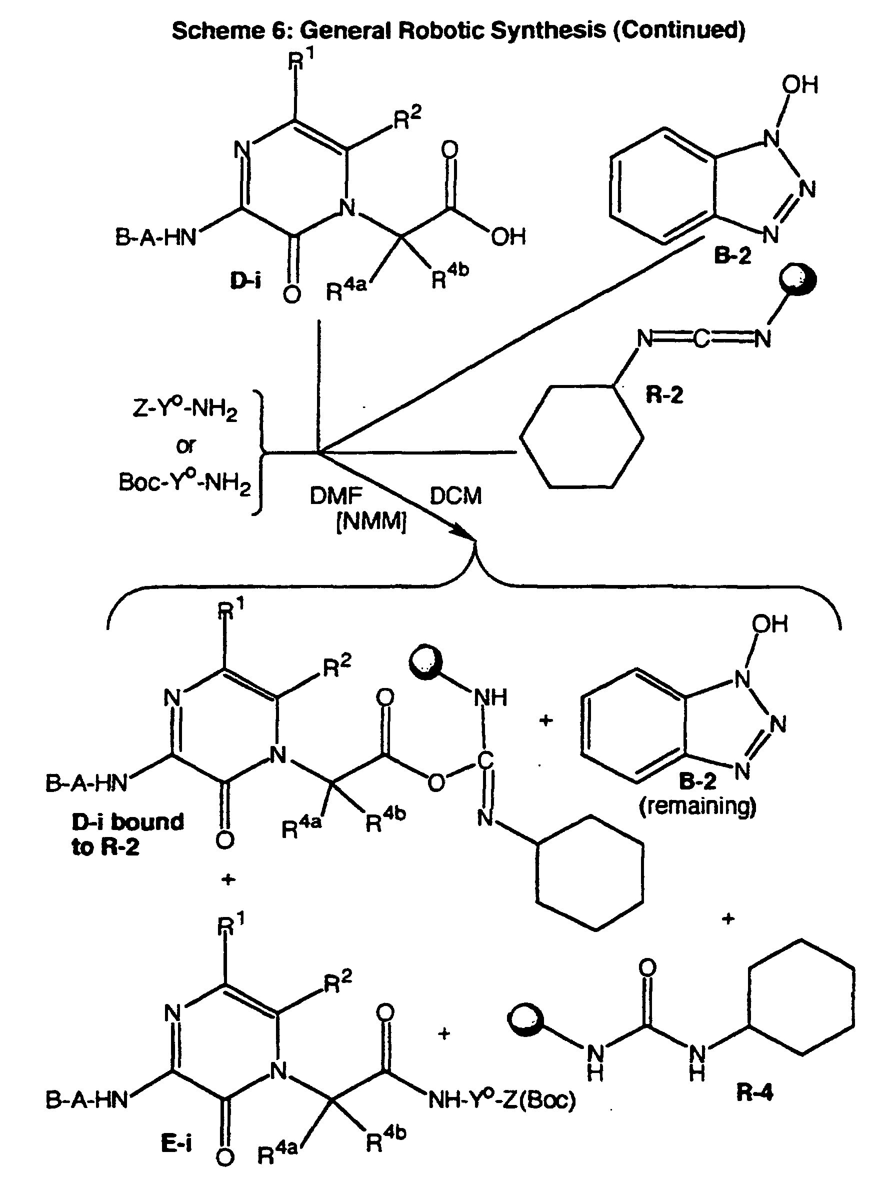

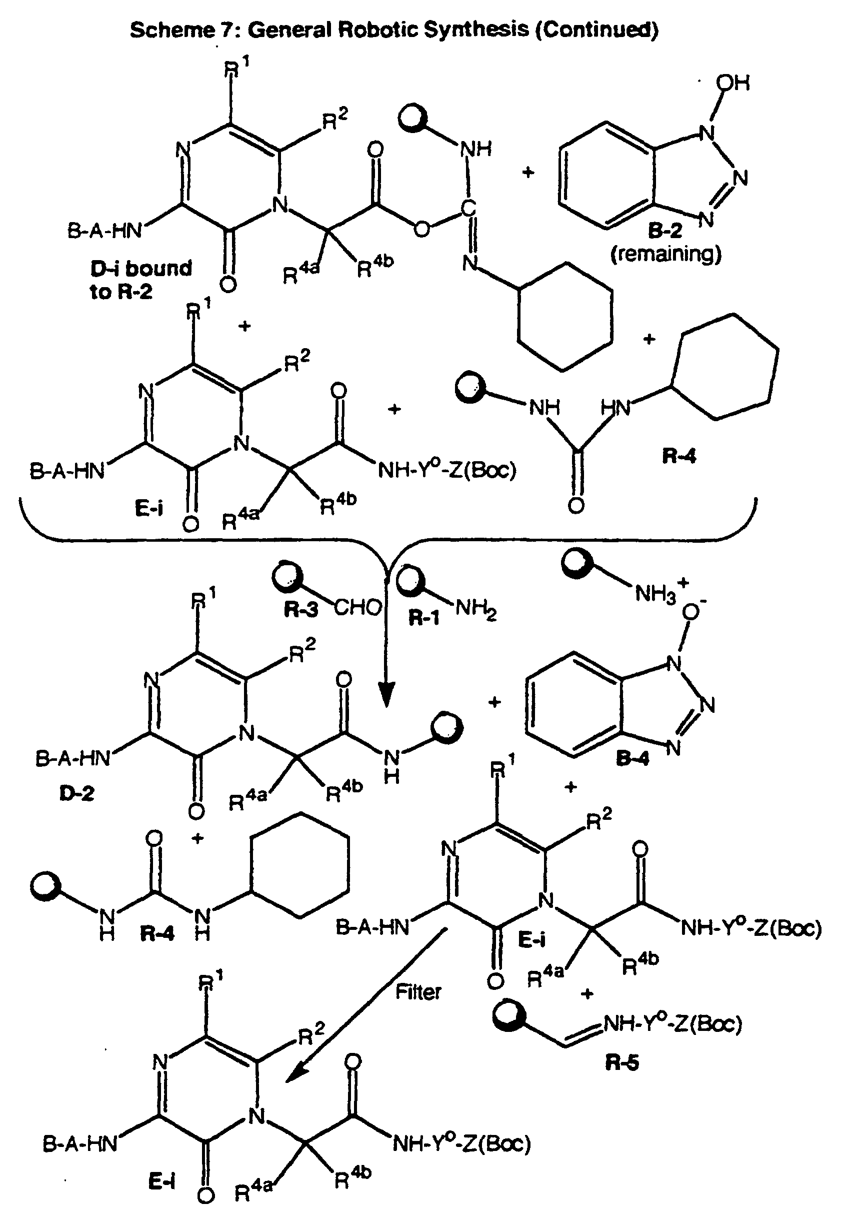

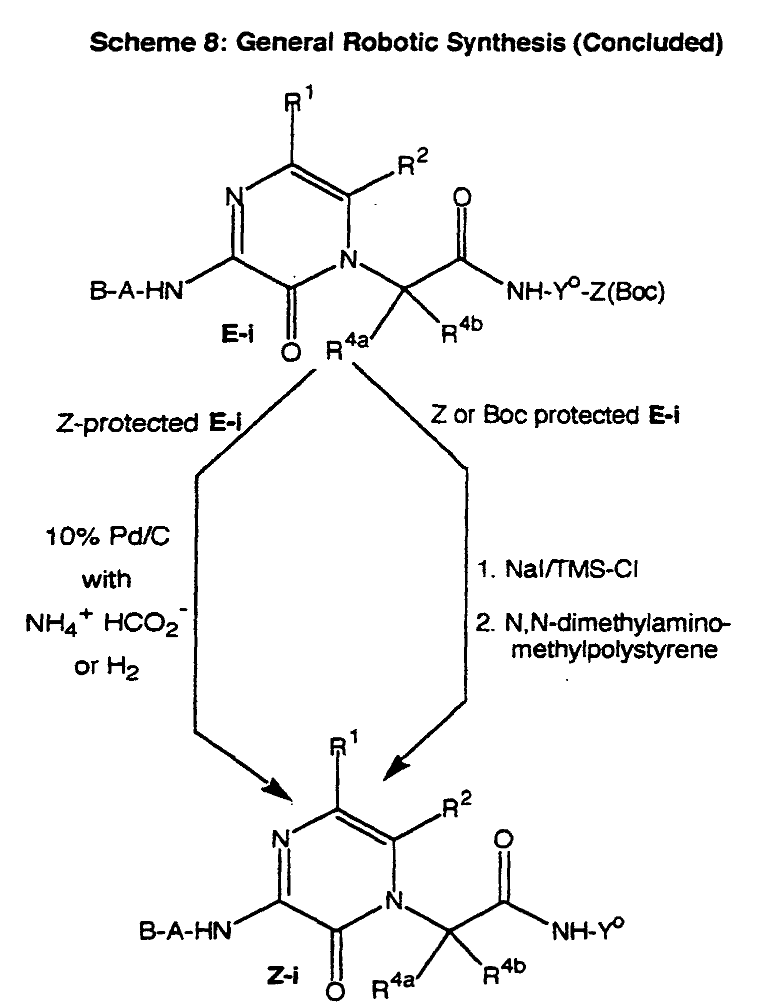

Although **Schemes 5, 6, 7,** and **8** describe the use of parallel array chemical library technology to prepare compounds of general formulae **D-i, E-i** and **Z-i,** it is noted that one with ordinary skill in the art of classical synthetic organic chemistry would be able to prepare **D-i, E-i,** and **Z-i** by conventional means (one compound prepared at a time in conventional glassware and purified by conventional means such as chromatography and/or crystallization).

The various functionalized resins utilized to prepare and purify parallel reaction mixtures, their source commercially or in the scientific literature, and the three representations (ie, the R number, an abbreviated functional structure, and the actual structural unit bound to the resin for each) are summarized below as follows:

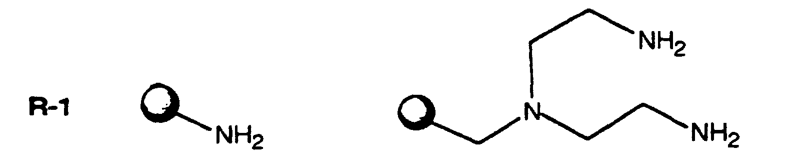

### R-1 Reference: Prepared as reported in J. J. Parlow, D. A. Mischke, and S. S. Woodard, J. Organic Chemistry, 62, 5908-5919 (1997)

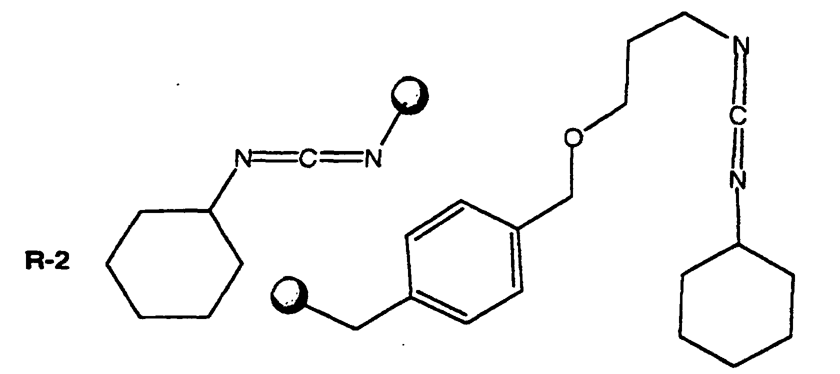

### R-2 Reference: Polystyrene bound N-cyclohexylcarbodiimide (Argonaut Catalog Number 800371

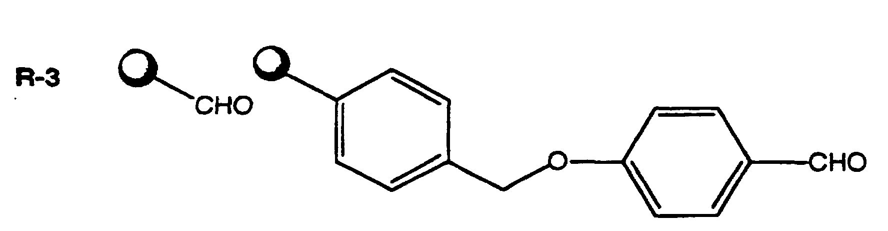

### R-3 Reference: Polystyrene bound benzaldehyde Novabiochem Catalog Number 01-640182

The specific compounds prepared, by using the **General Robotics and Experimental Procedure, Schemes 5 through 8**, and general synthetic methods and processes disclosed herein, are listed below in **Tables 4** through **Table 7. Tables 4** through **Table 7** further summarize the mass spectral characterization data that confirmed the indicated structure for each compound of the present invention disclosed in these tables.

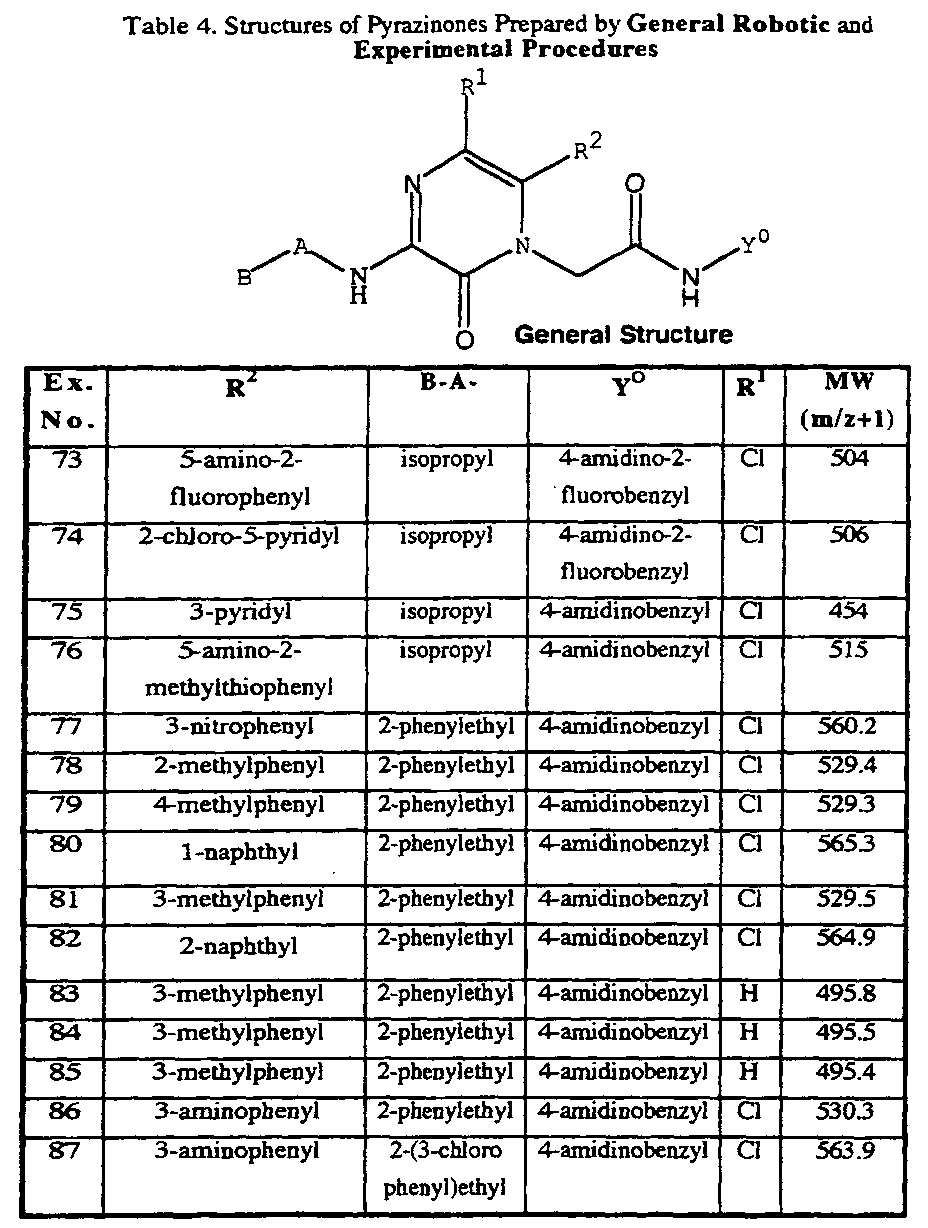

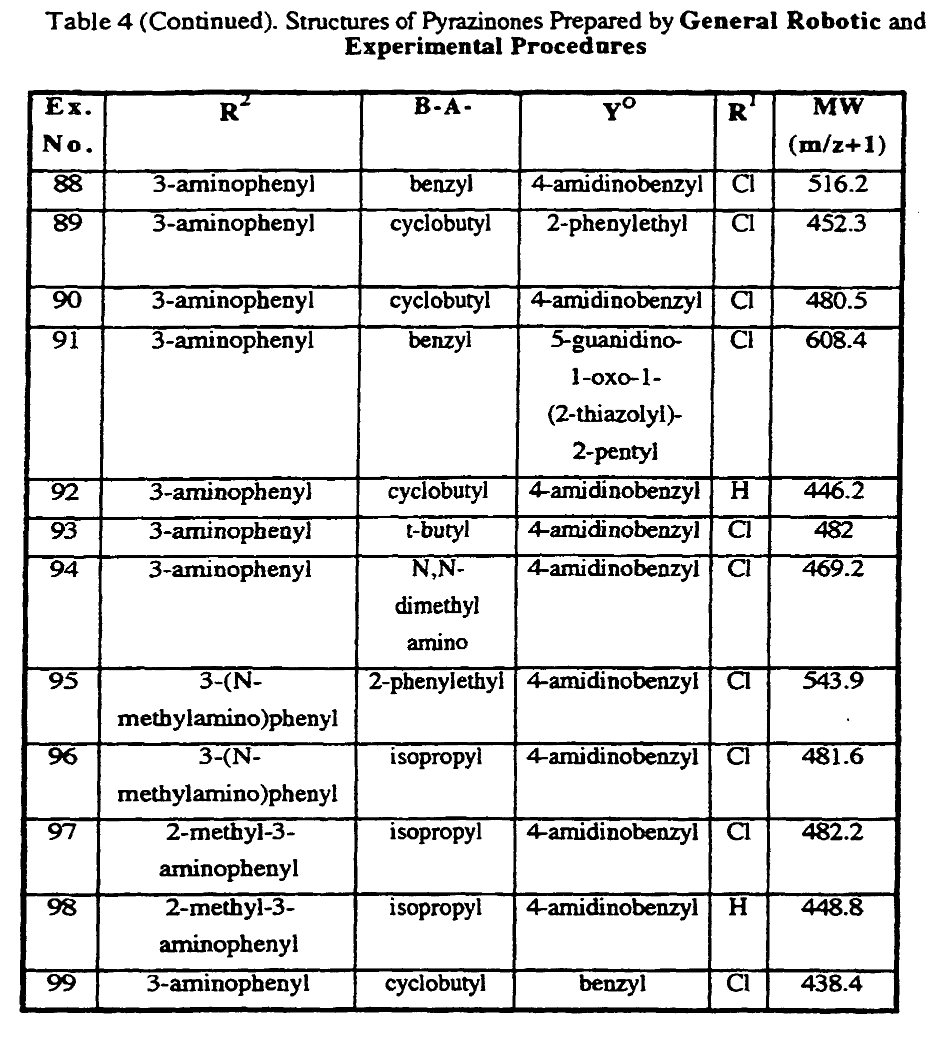

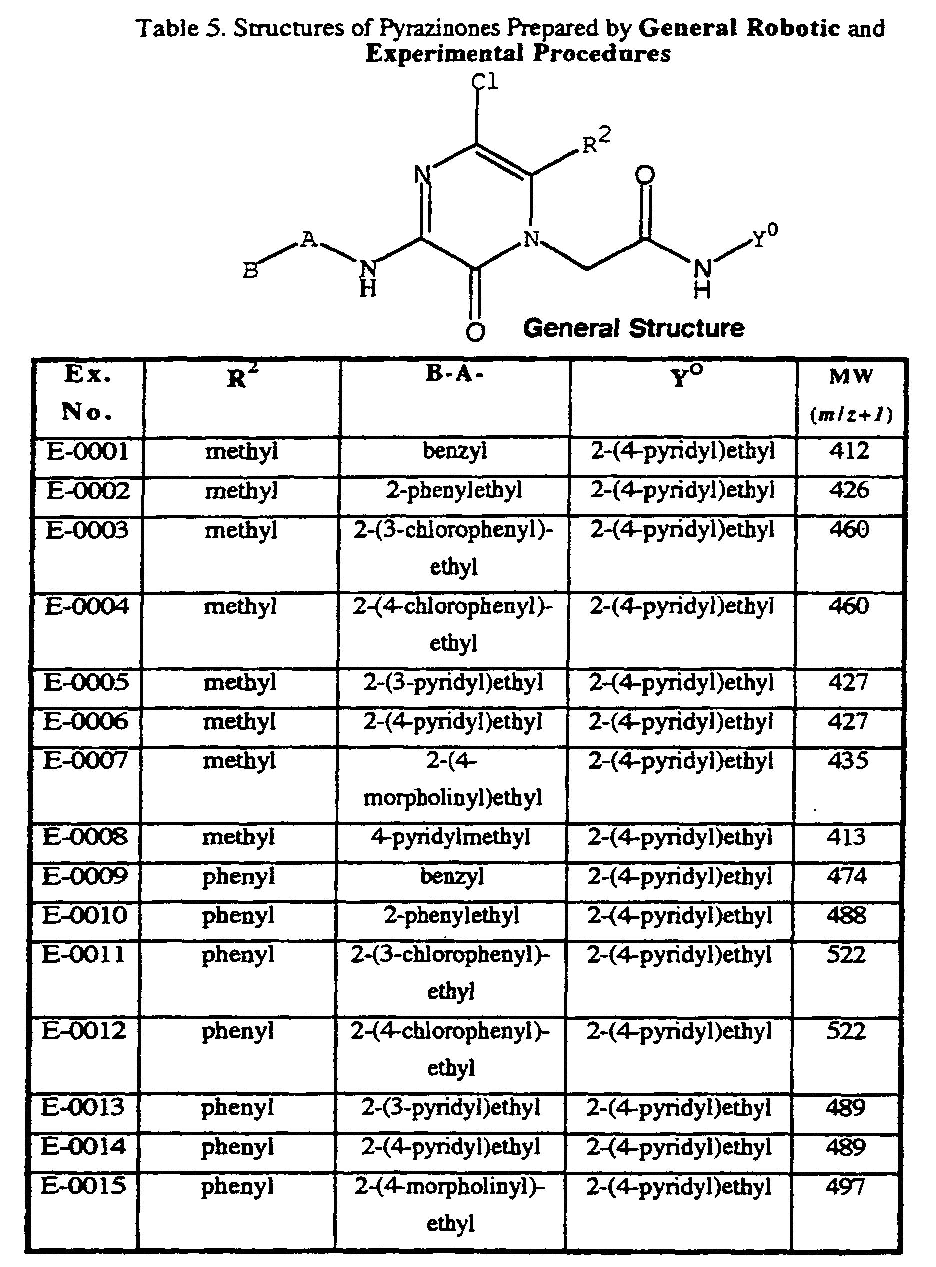

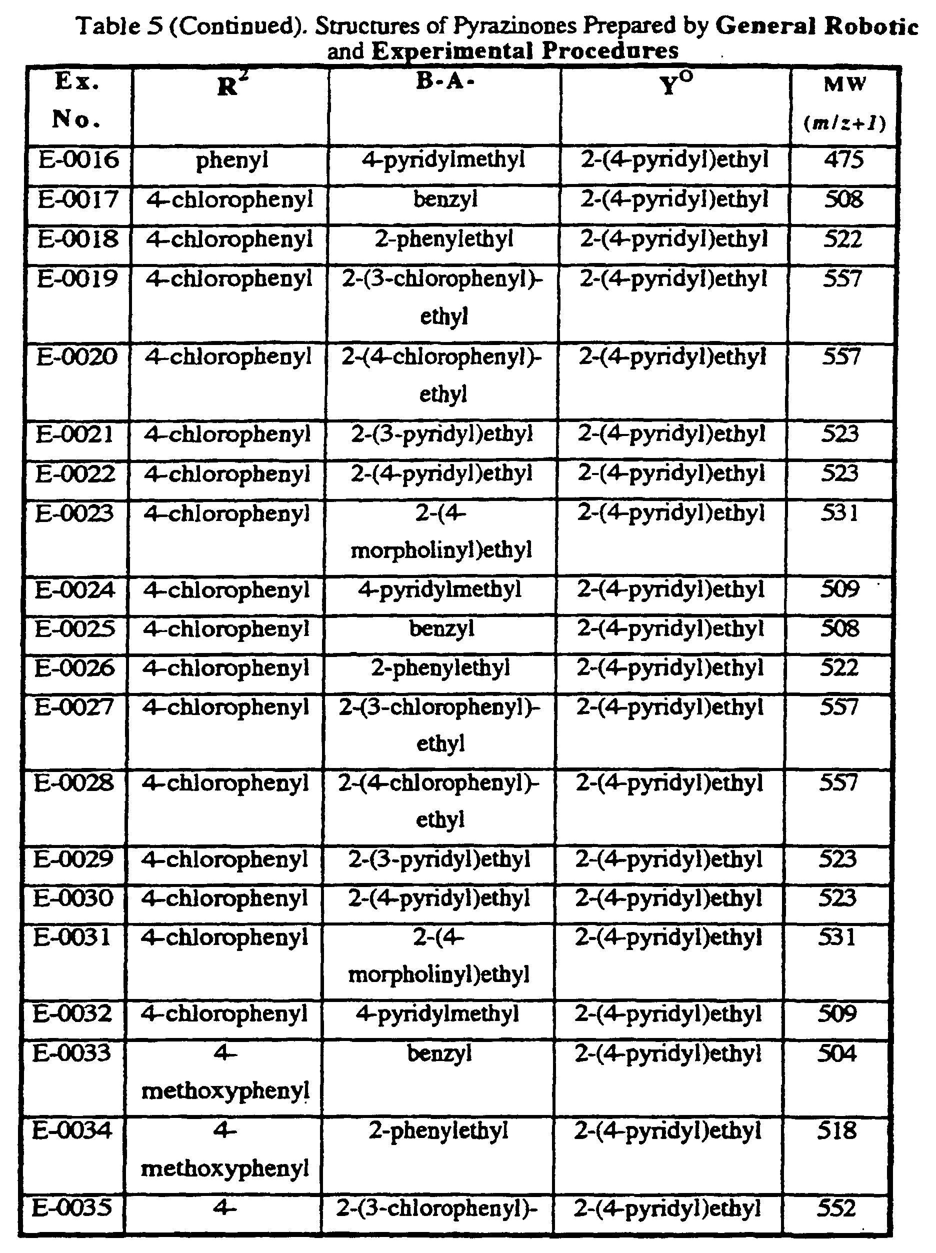

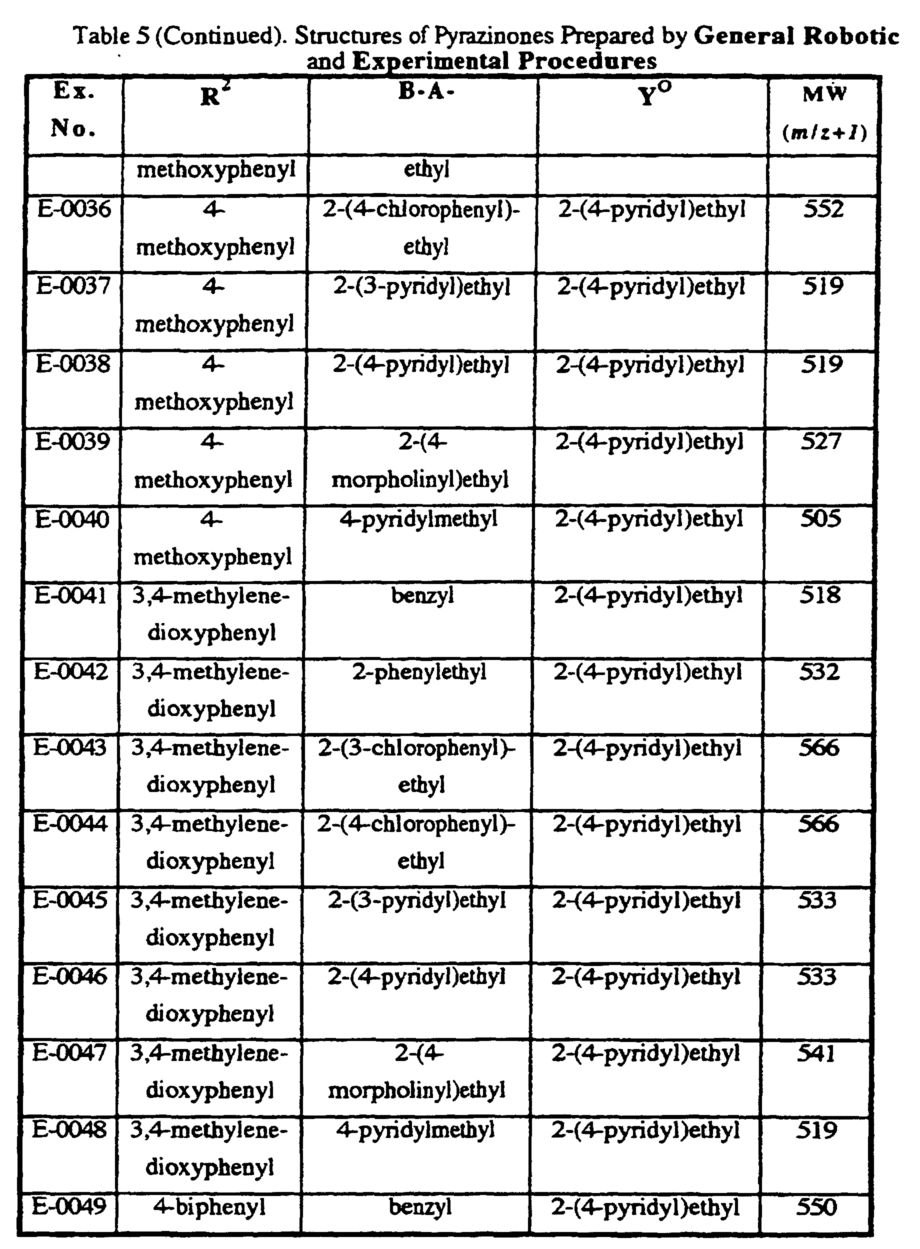

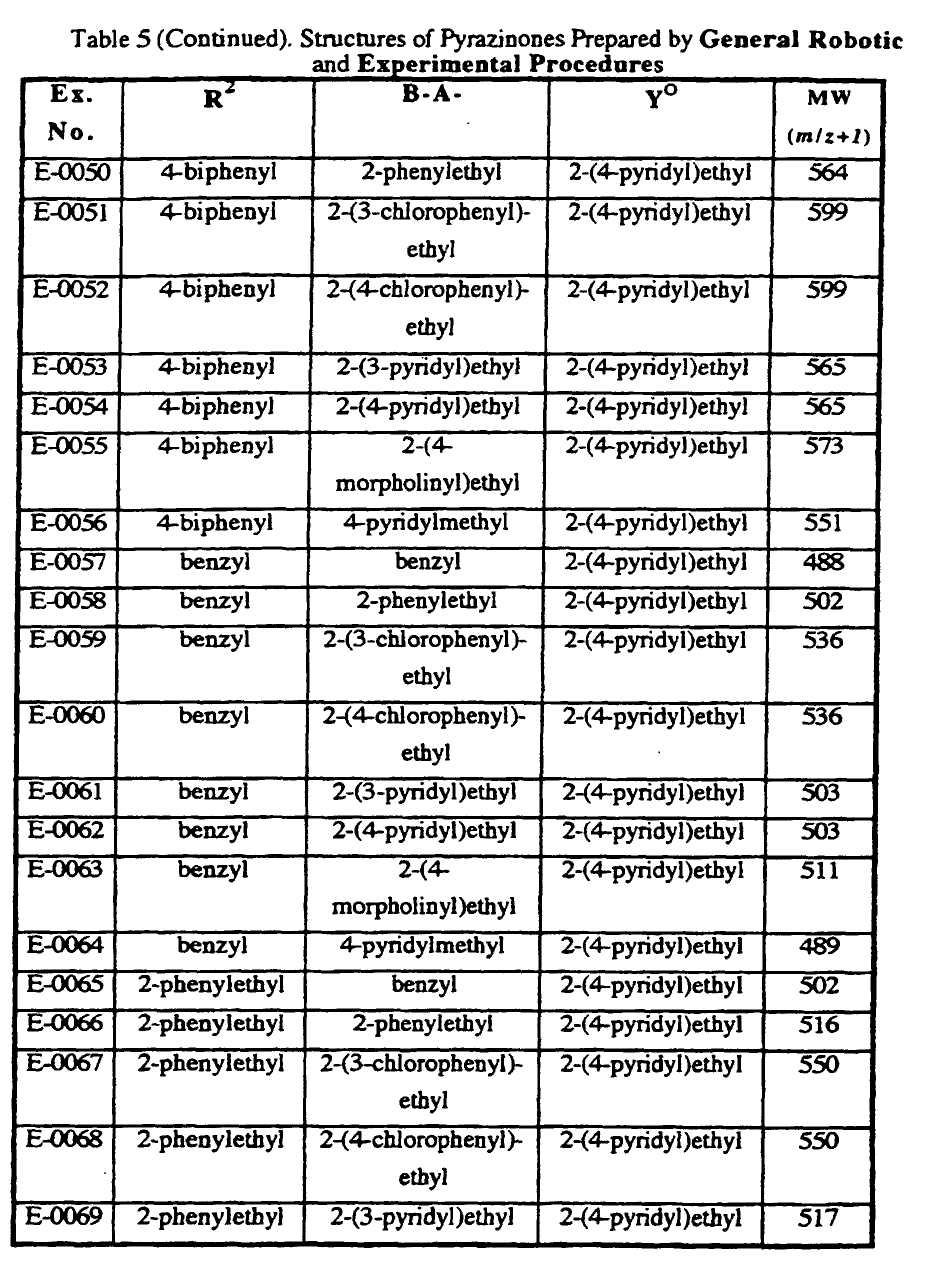

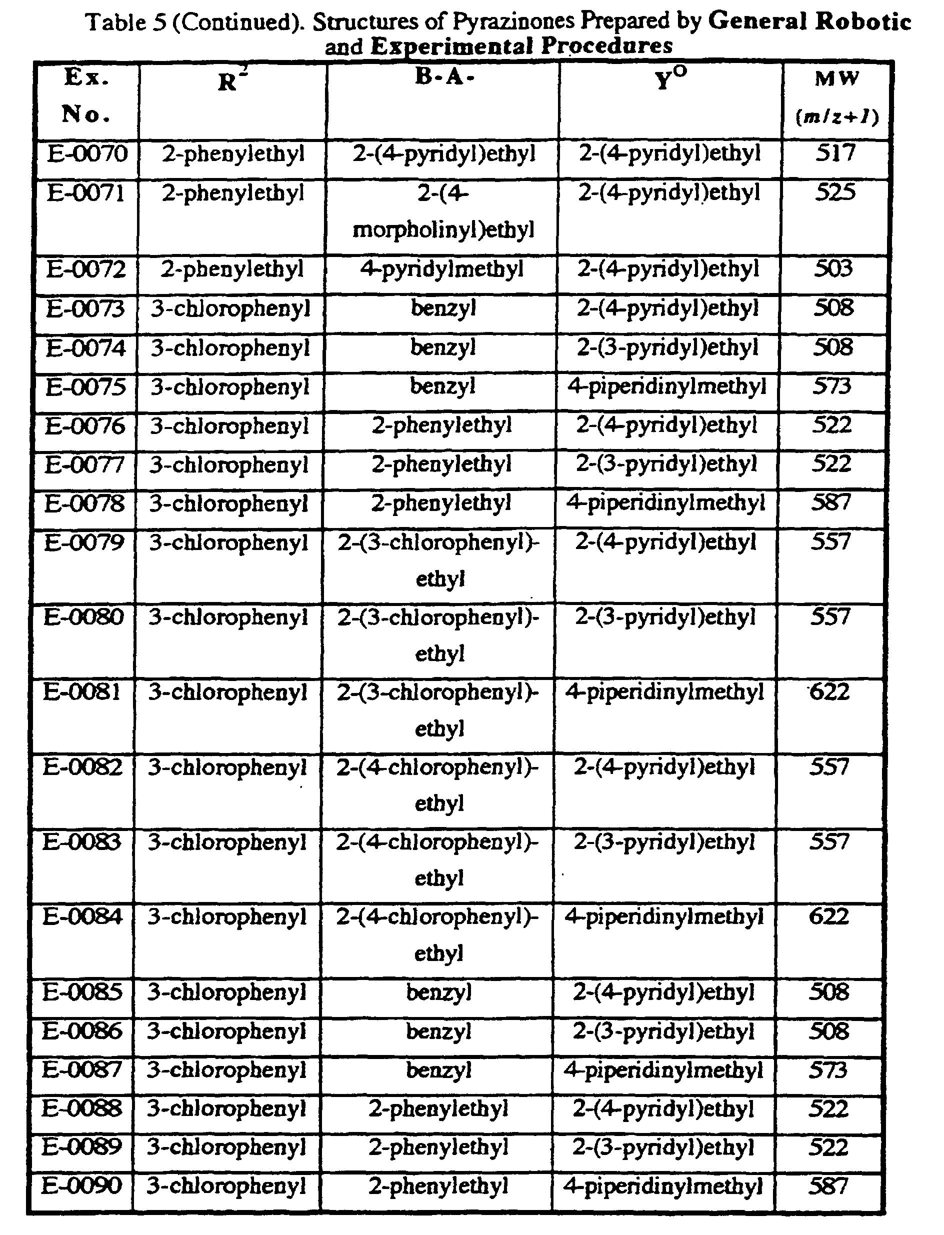

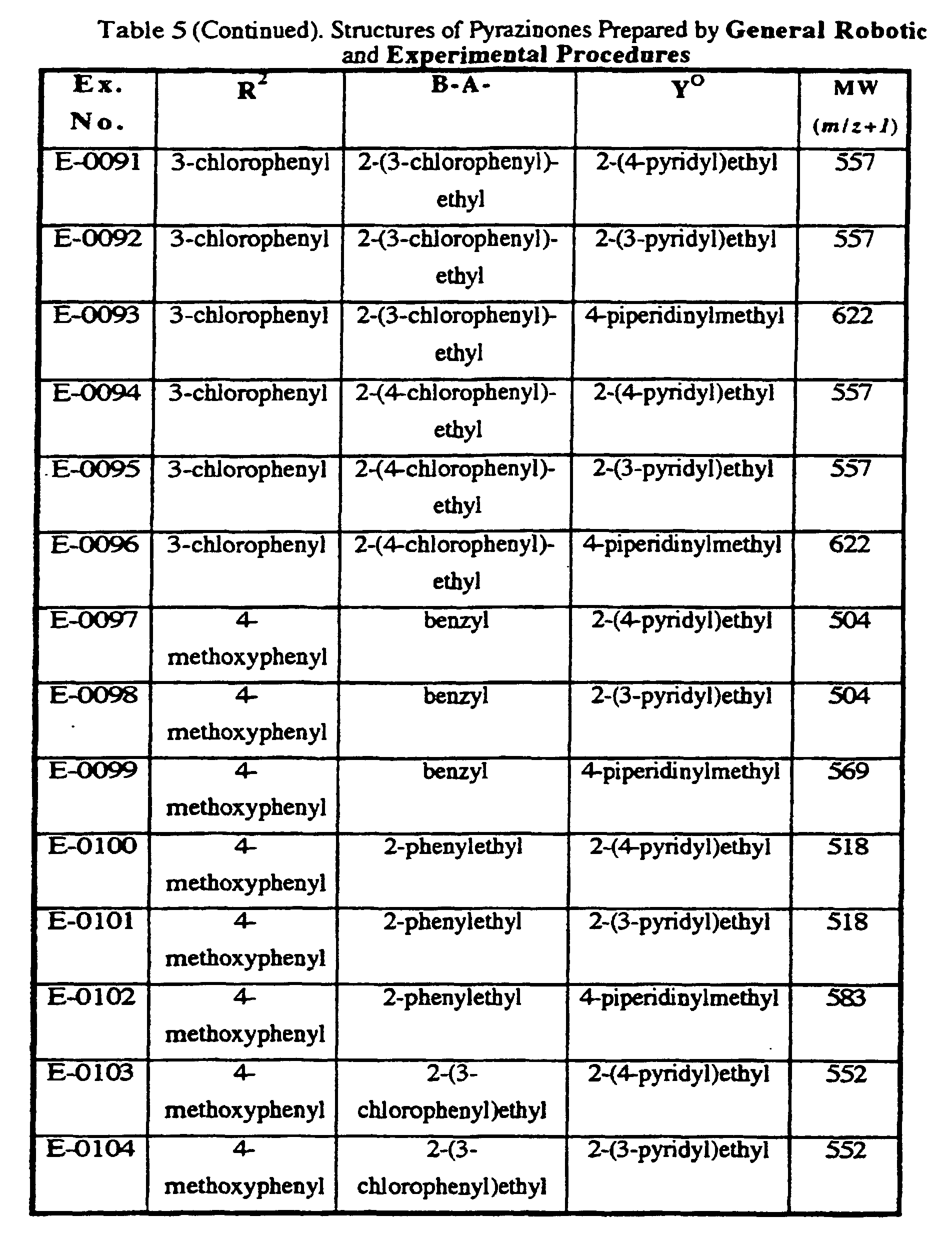

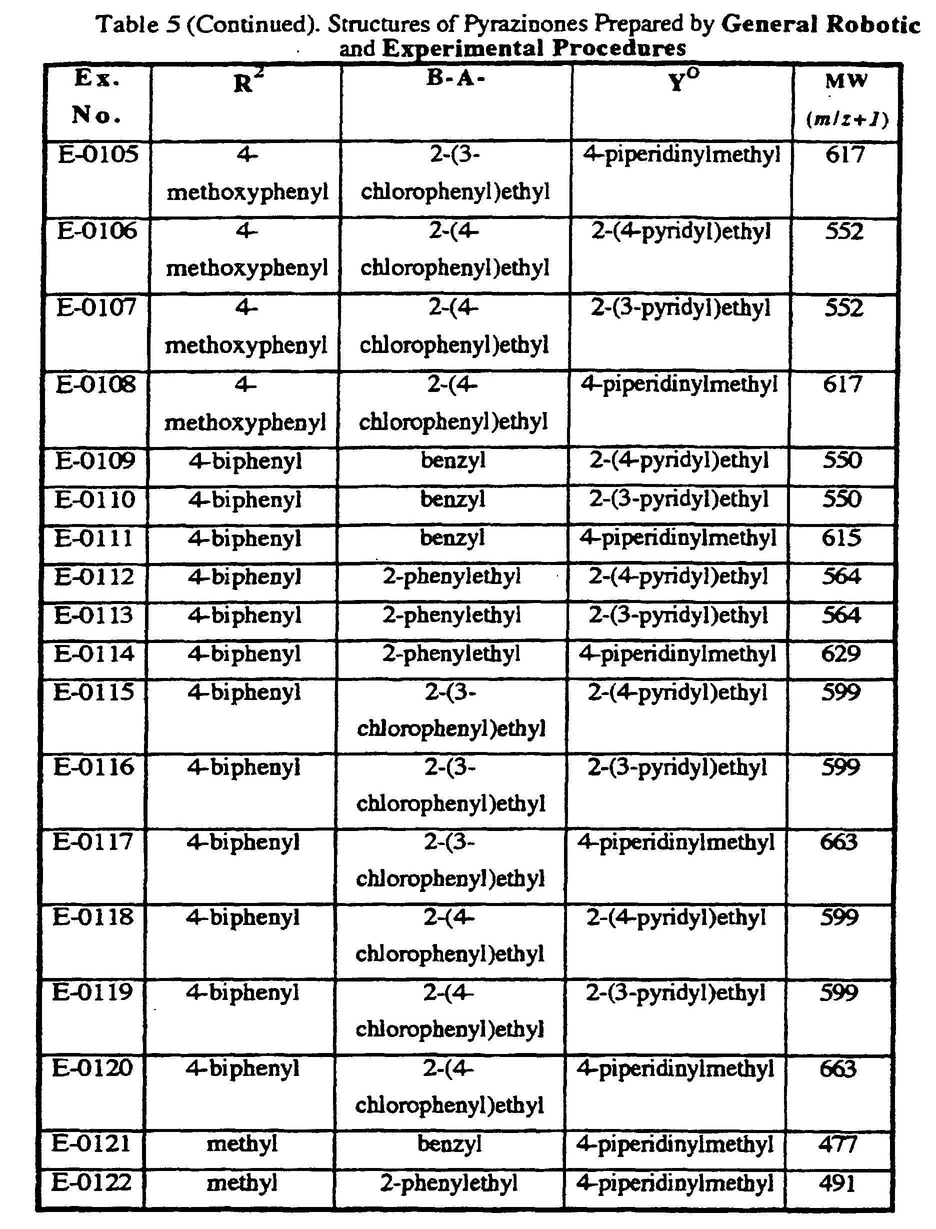

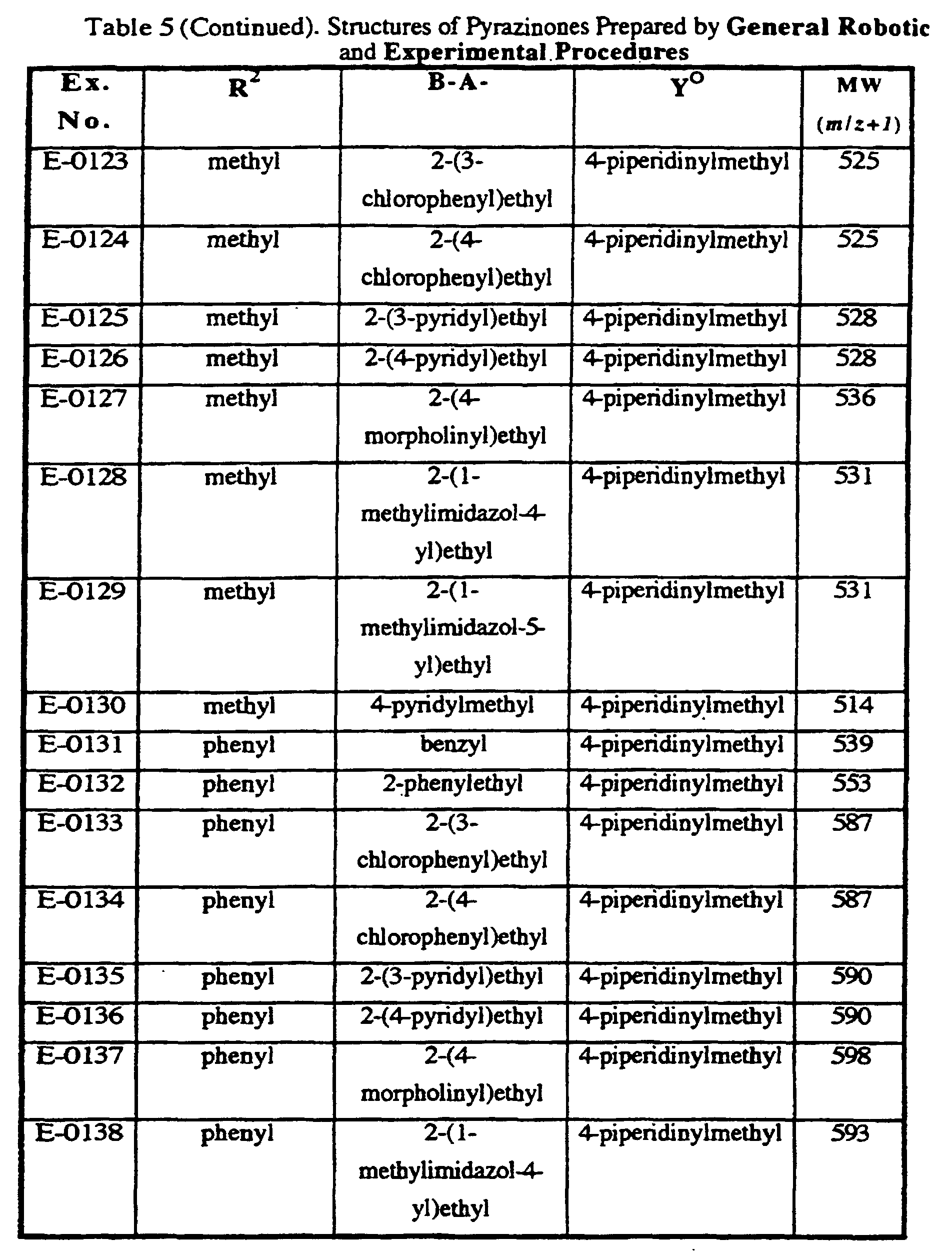

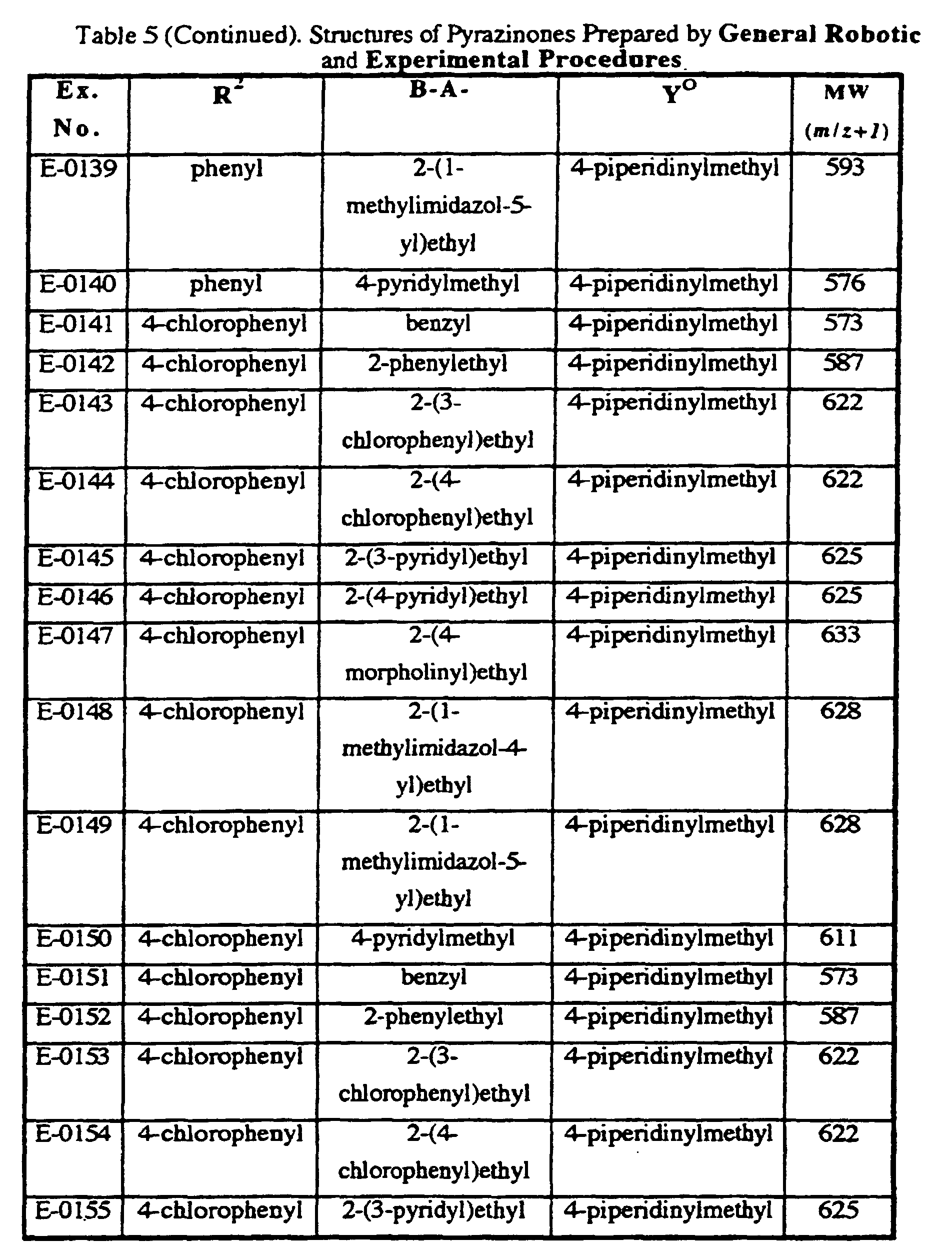

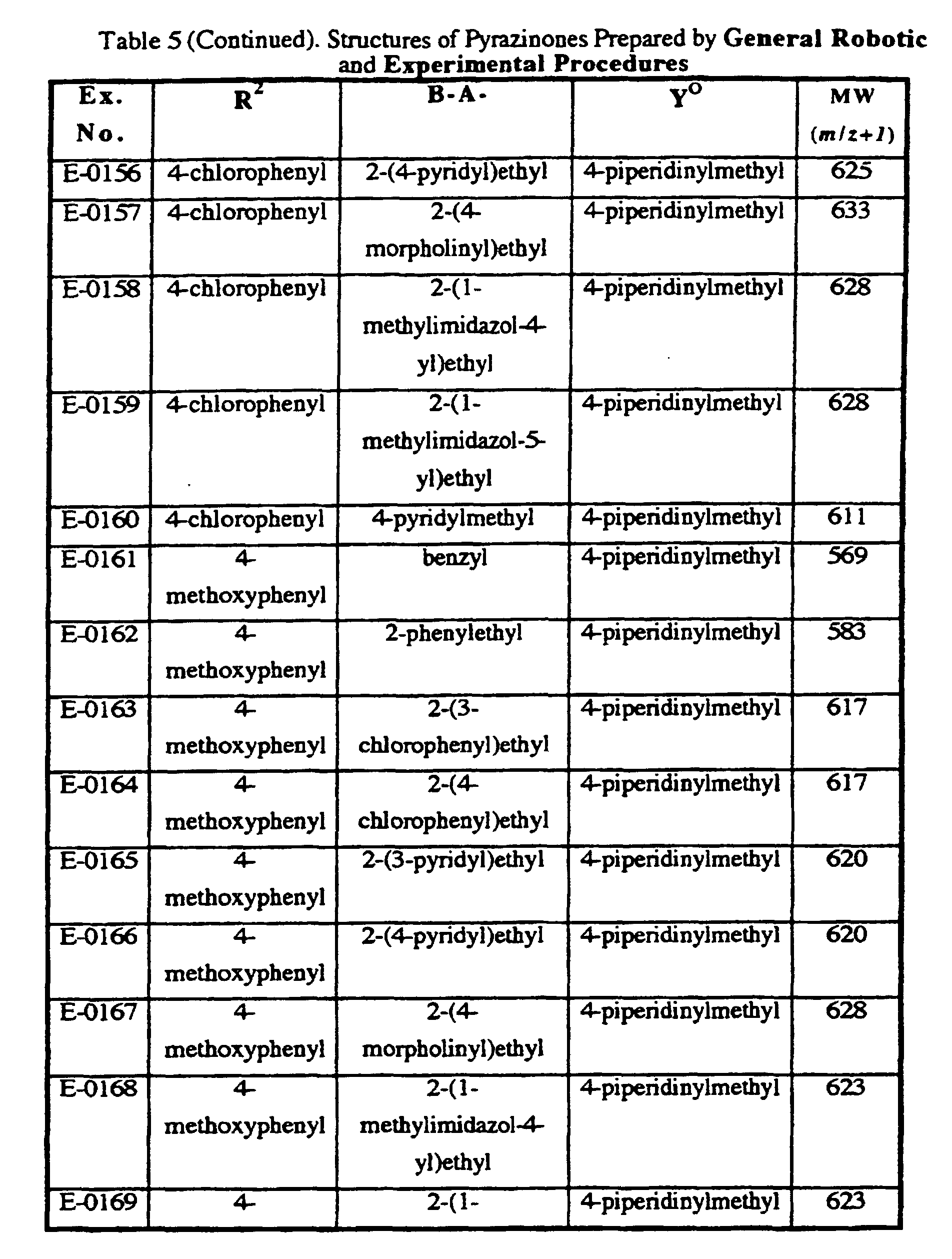

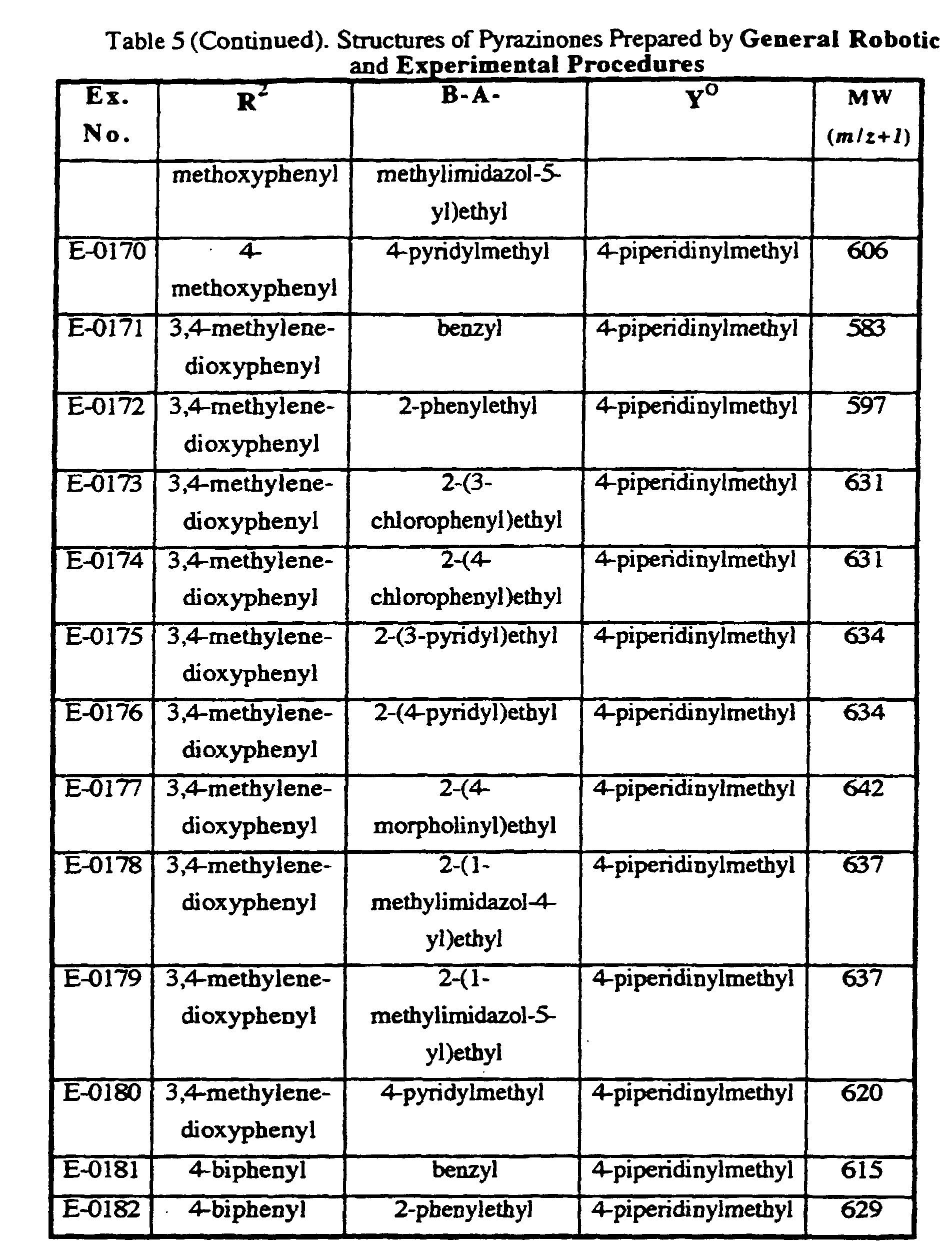

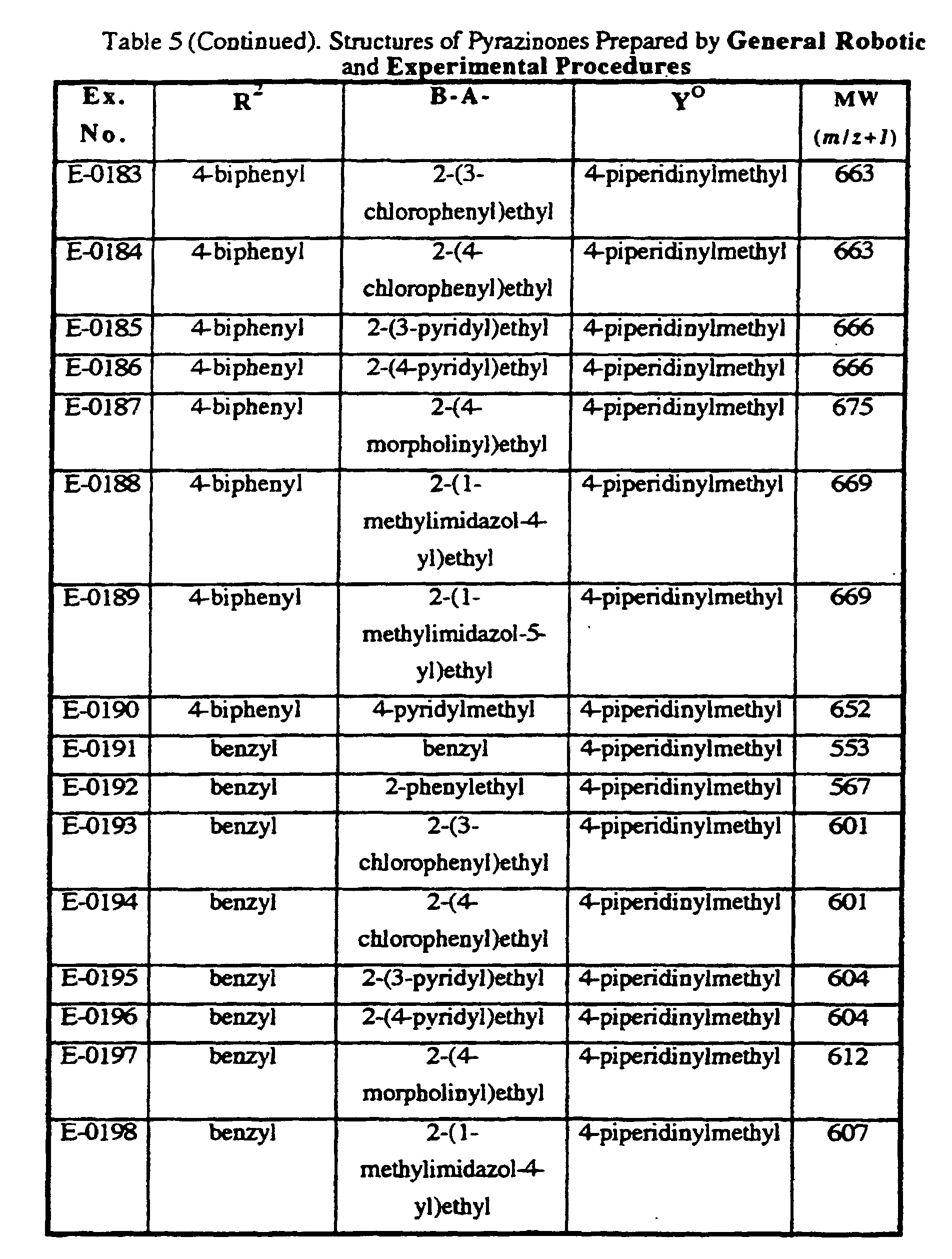

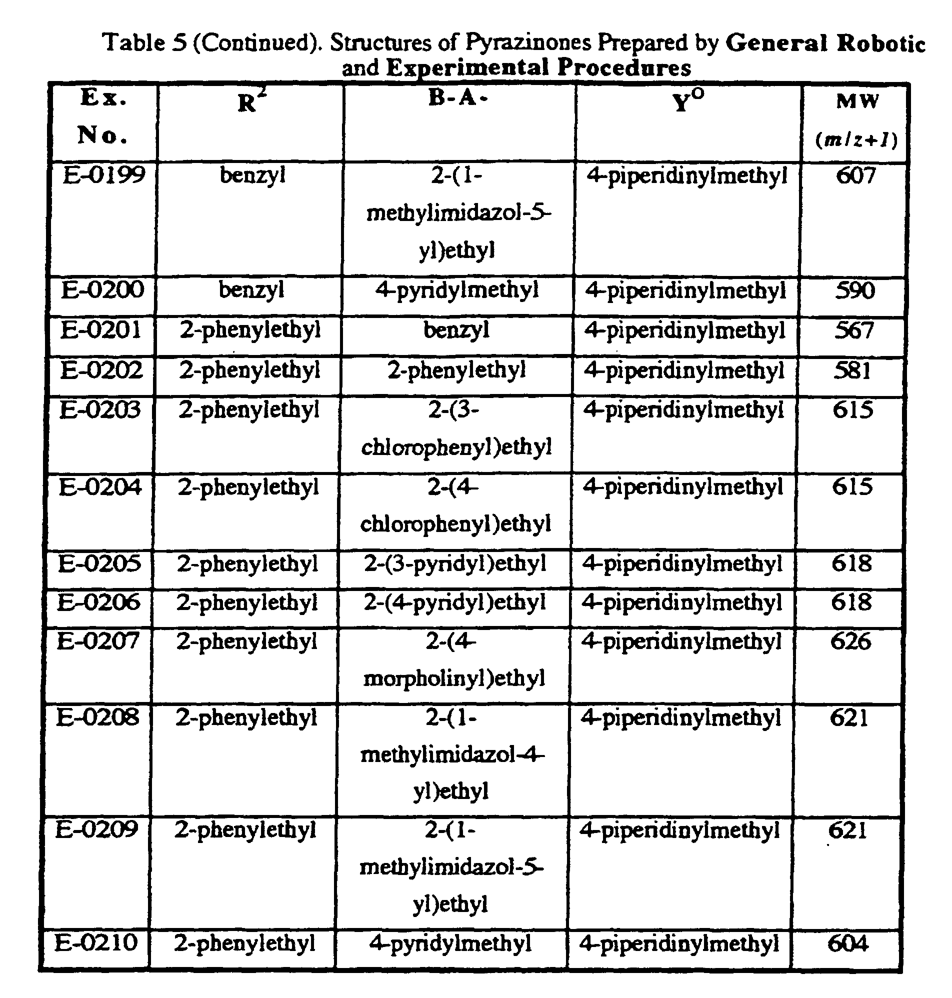

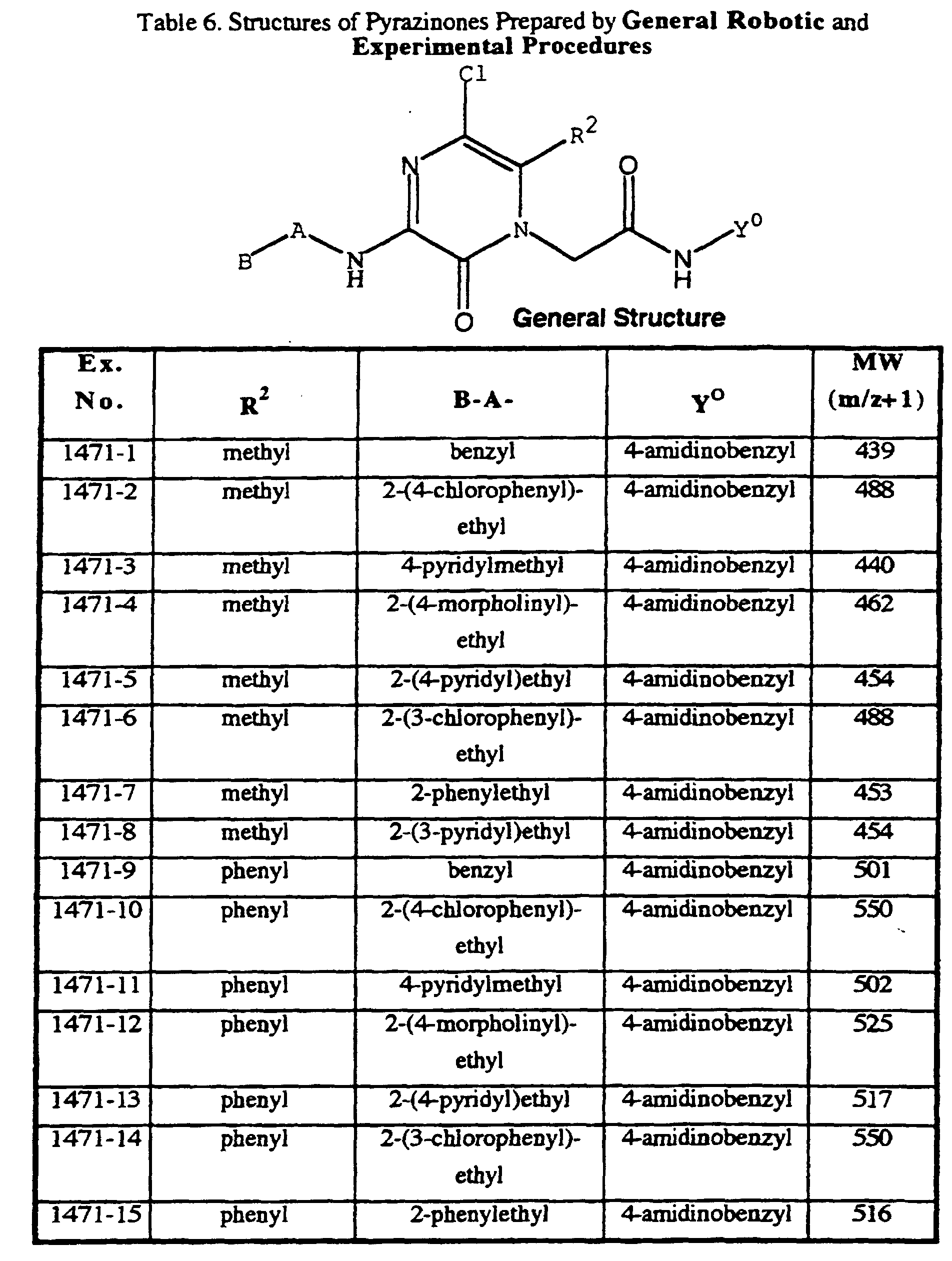

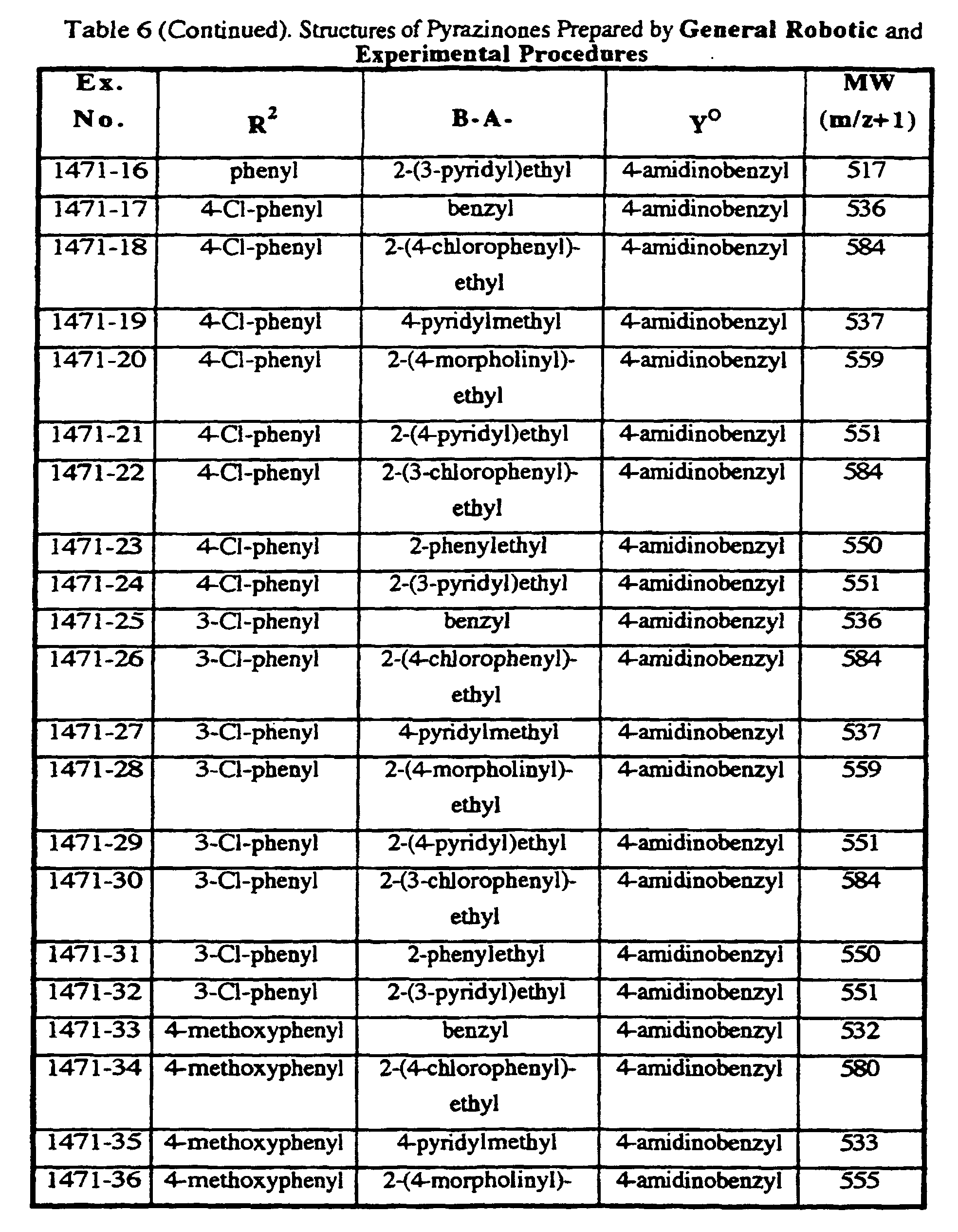

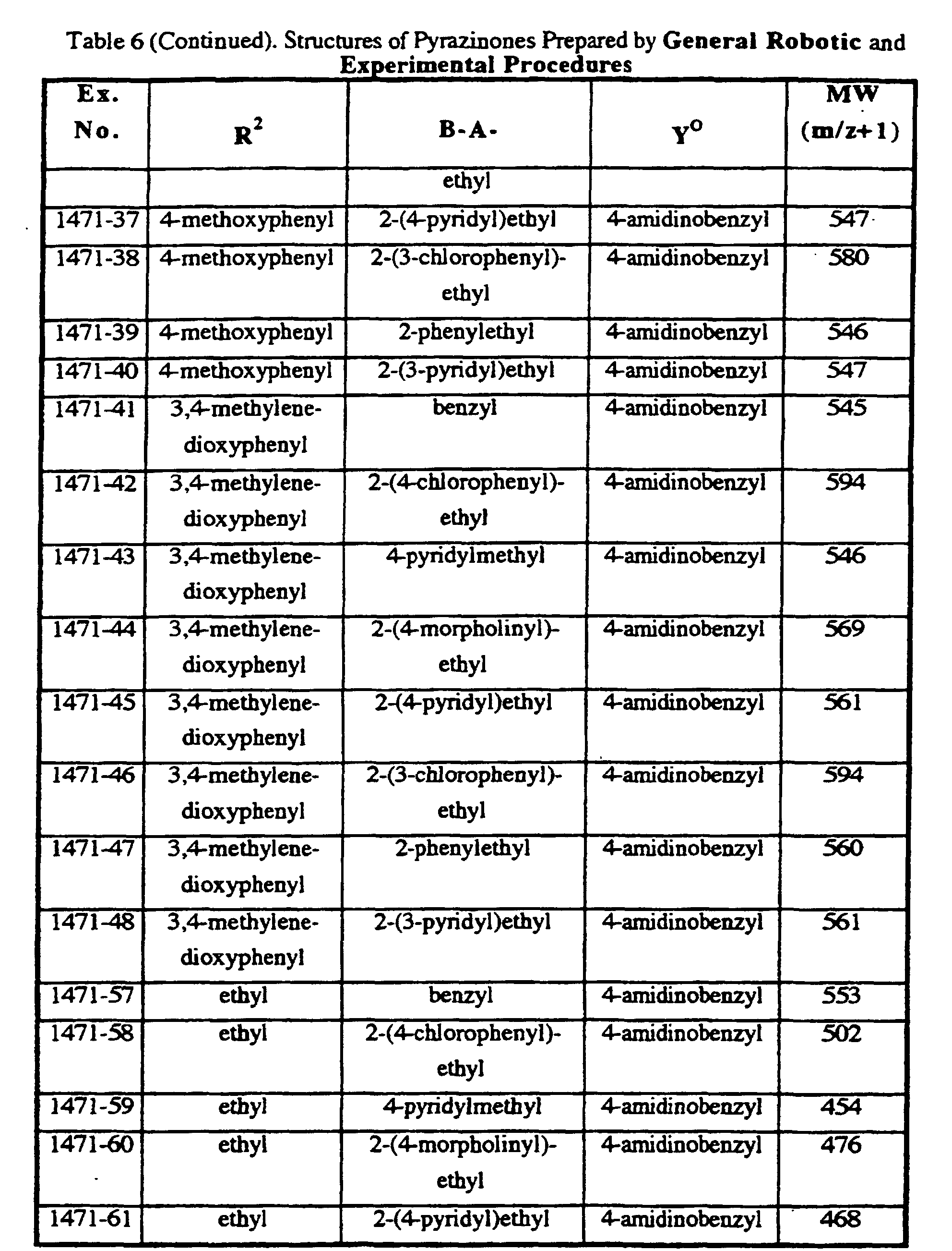

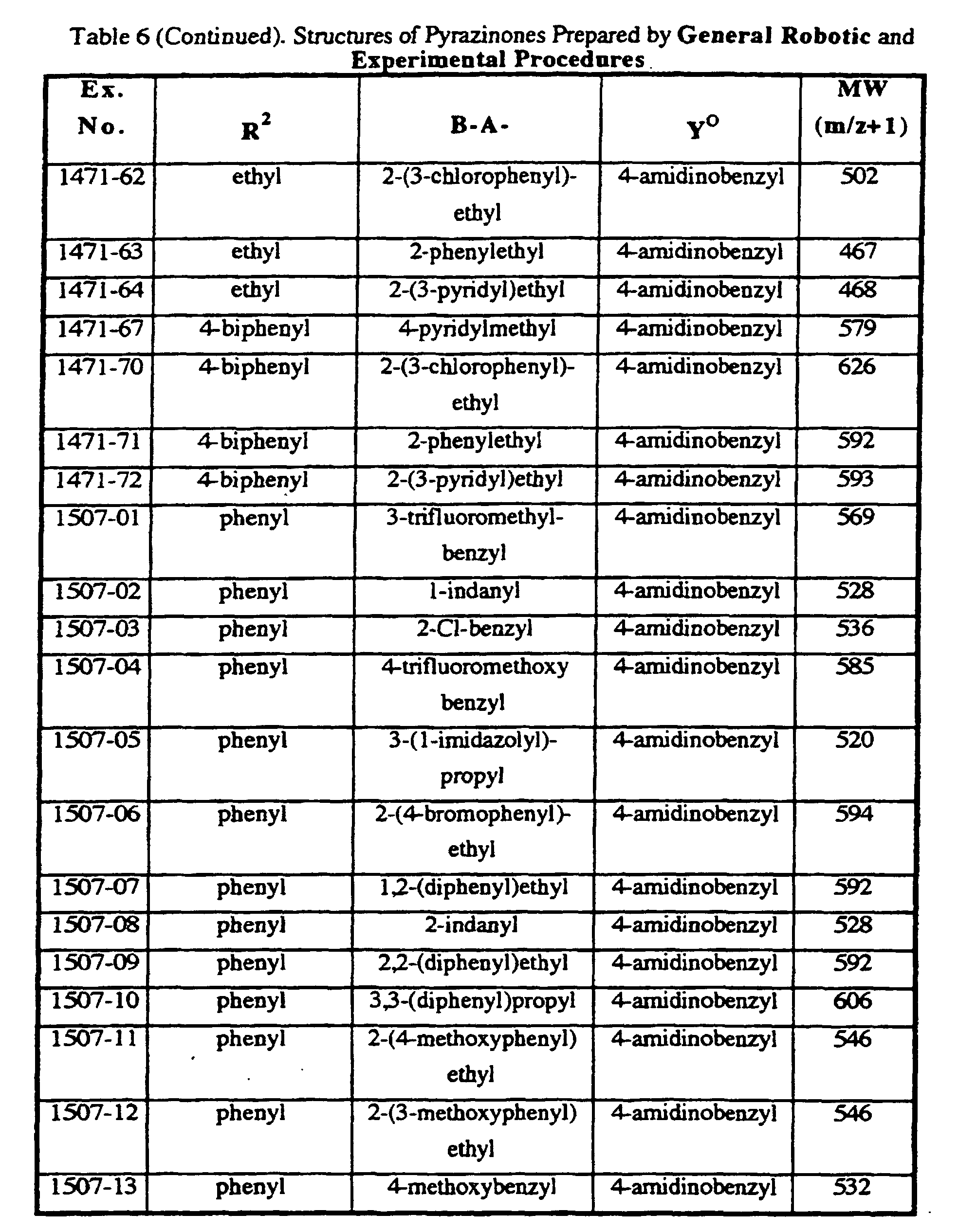

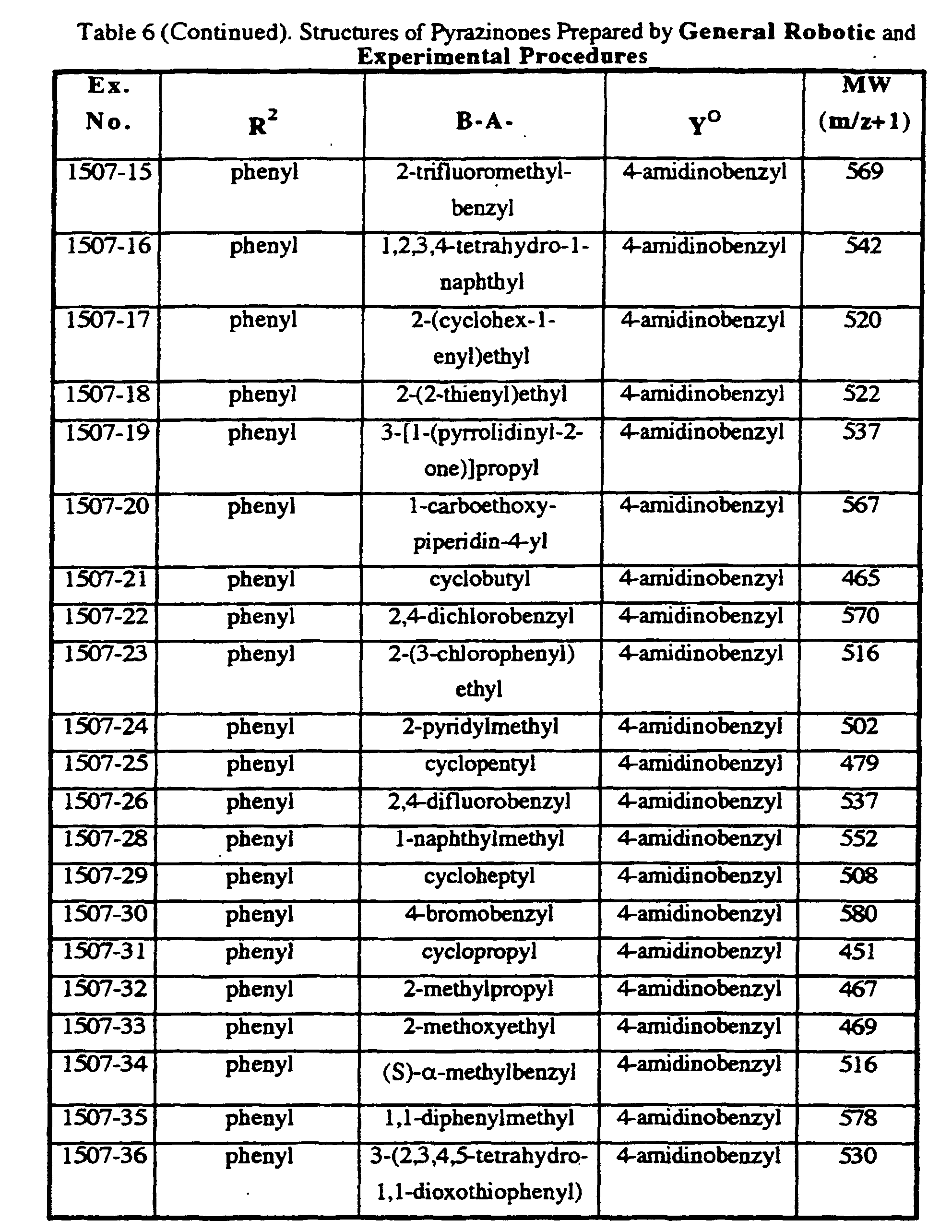

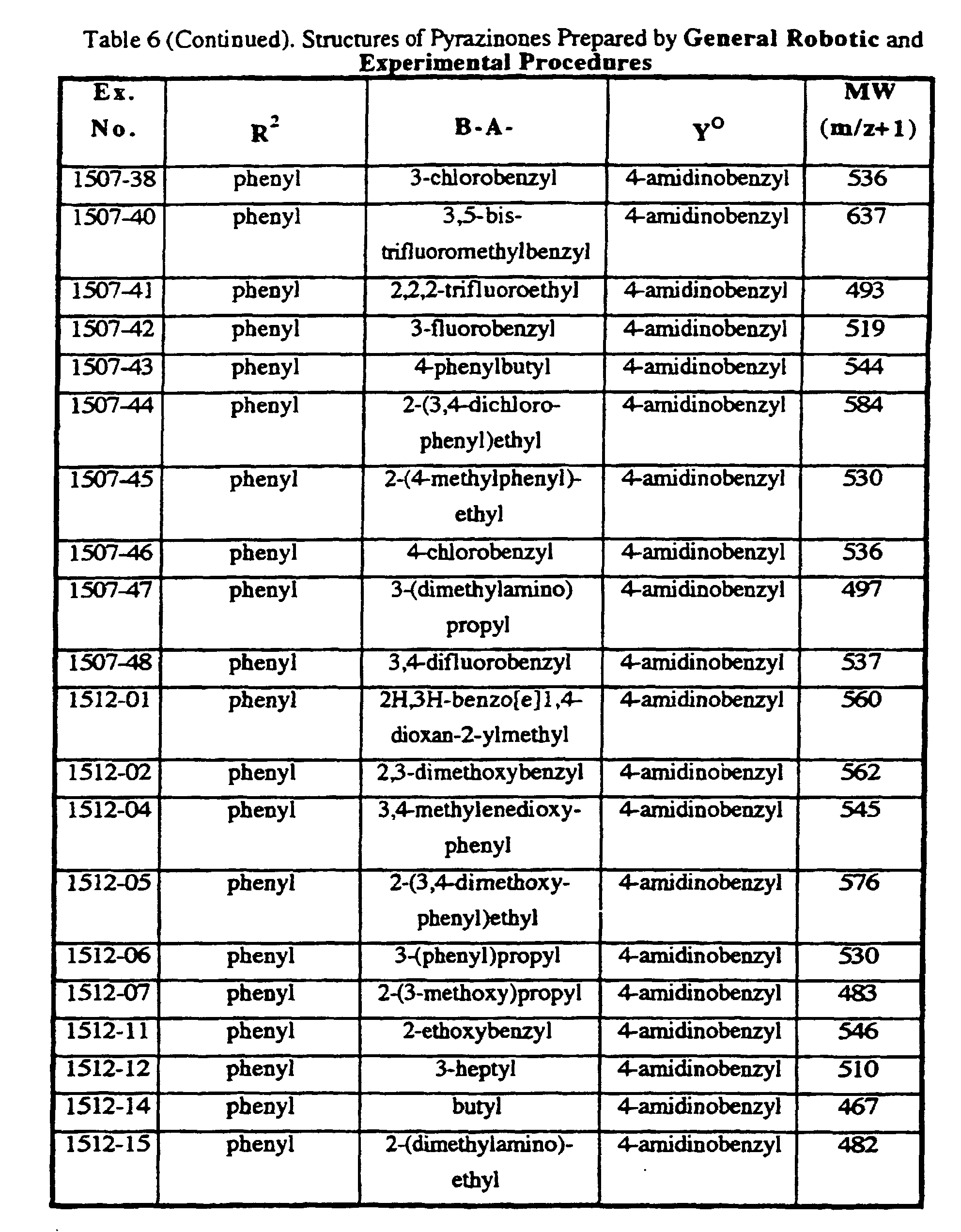

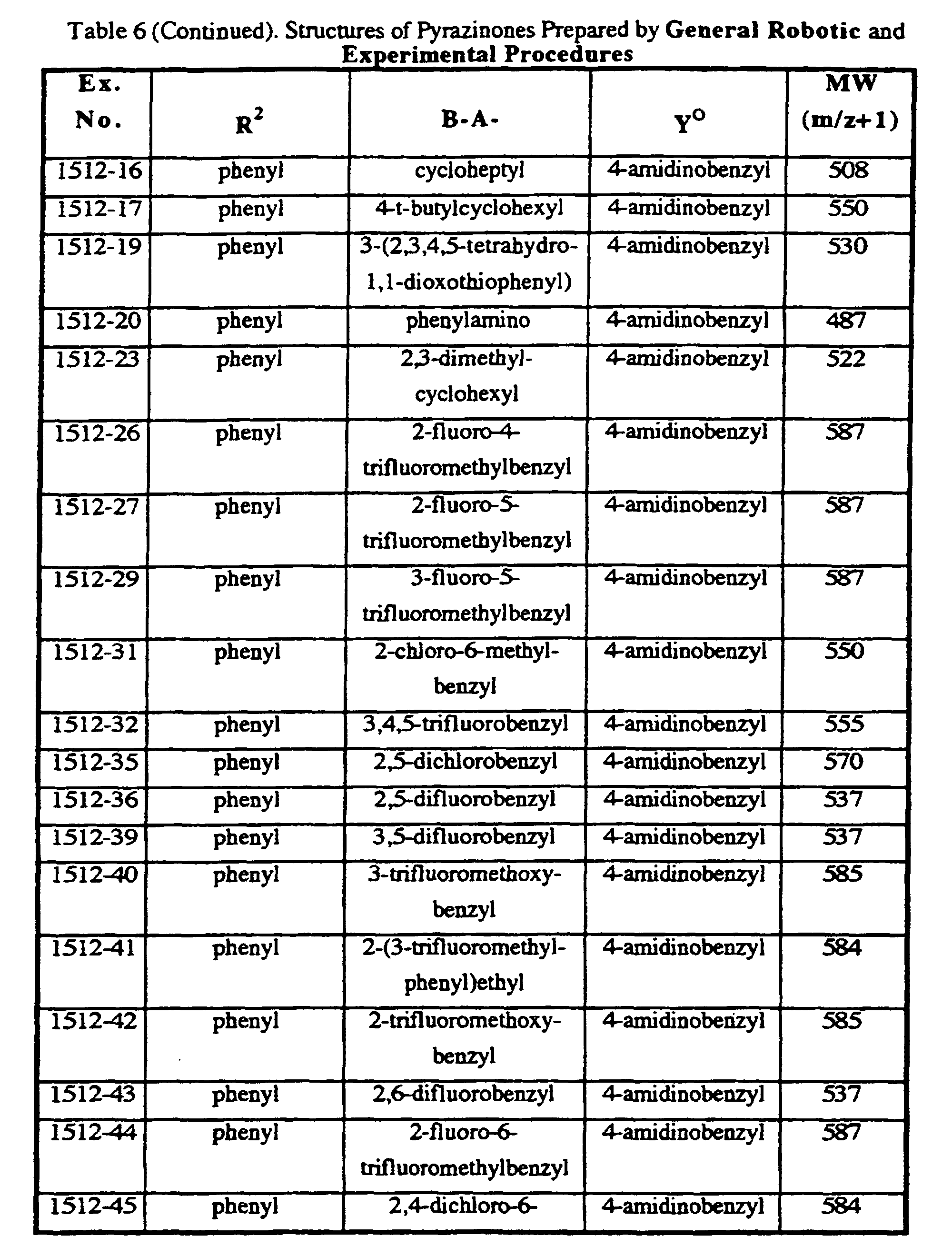

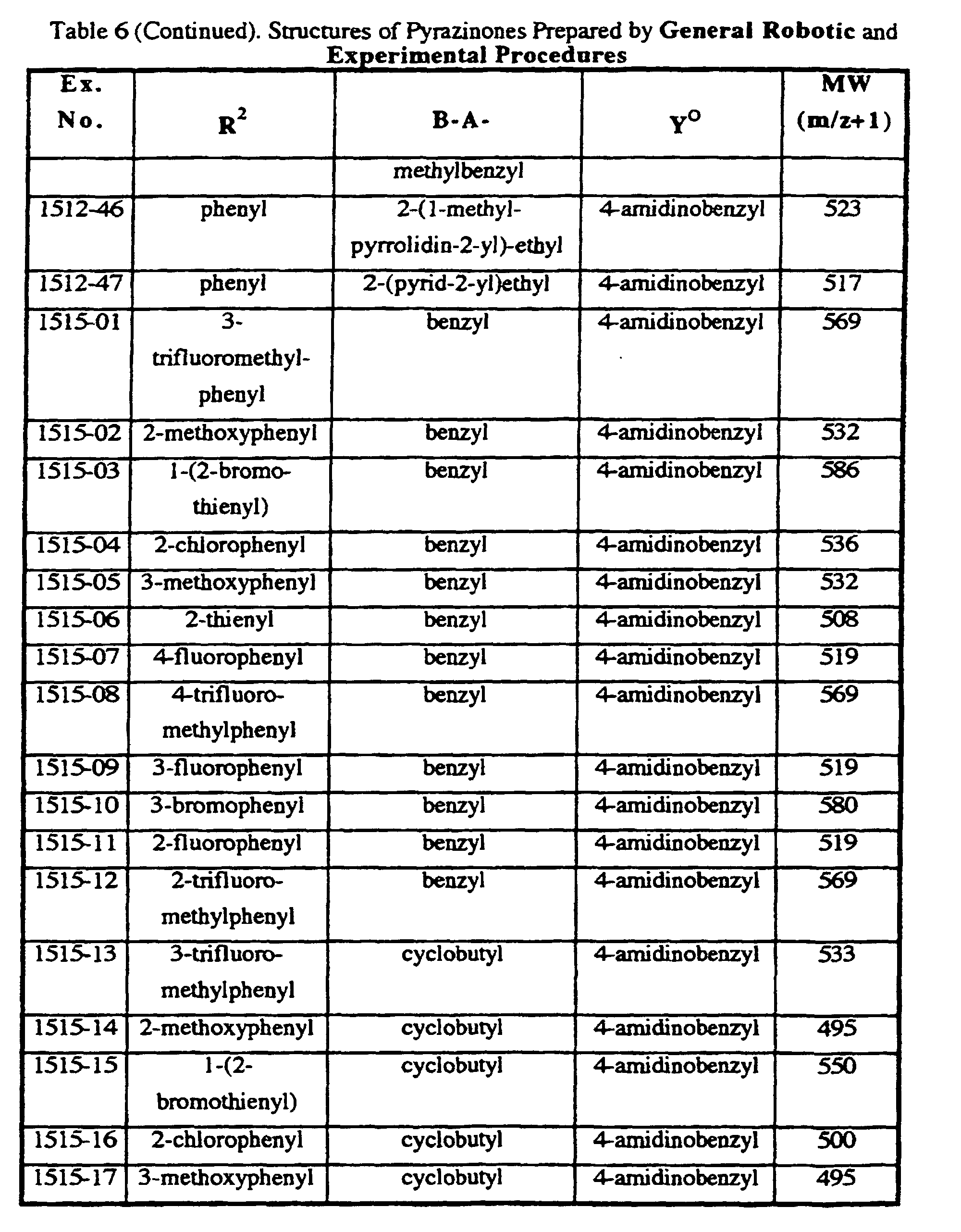

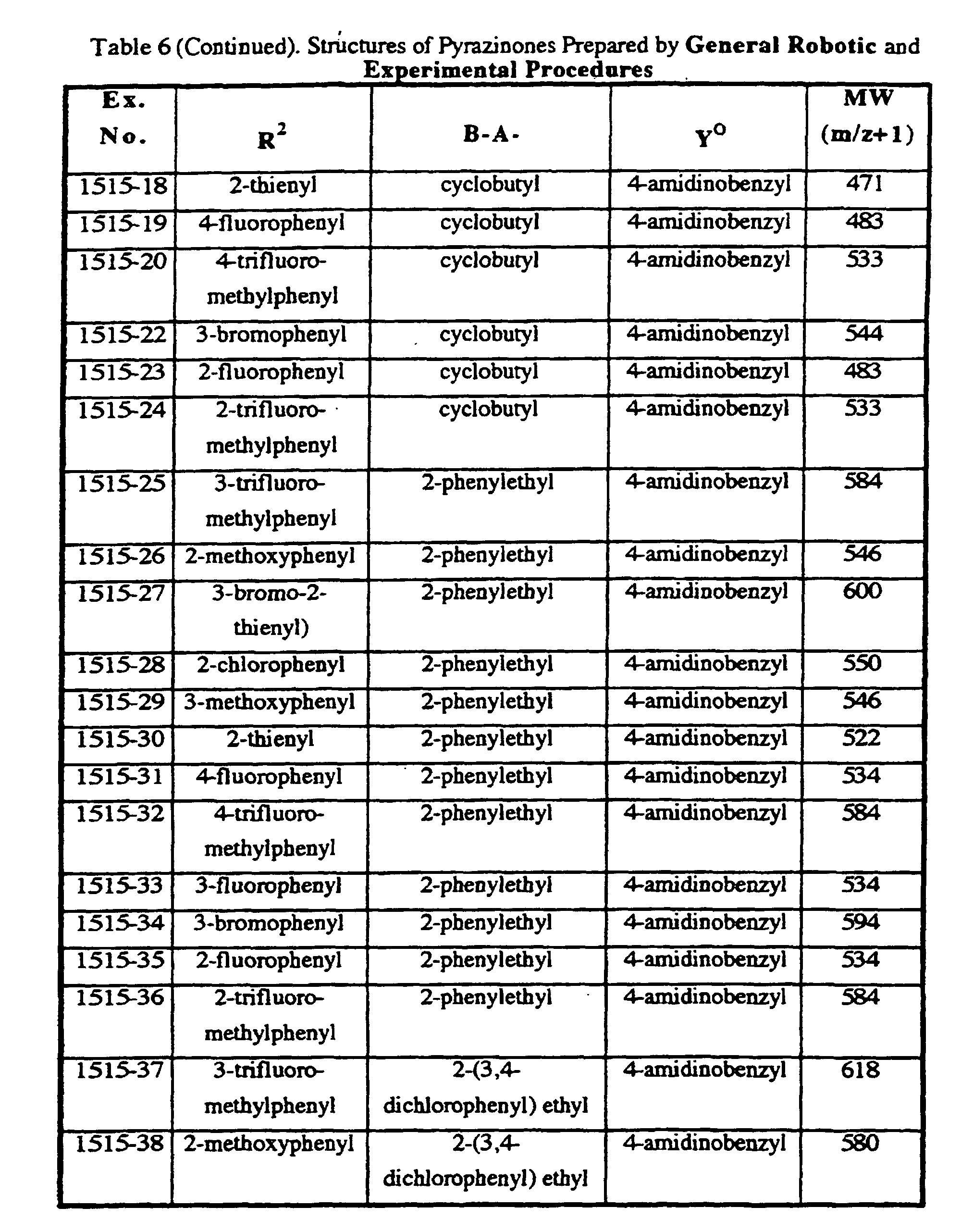

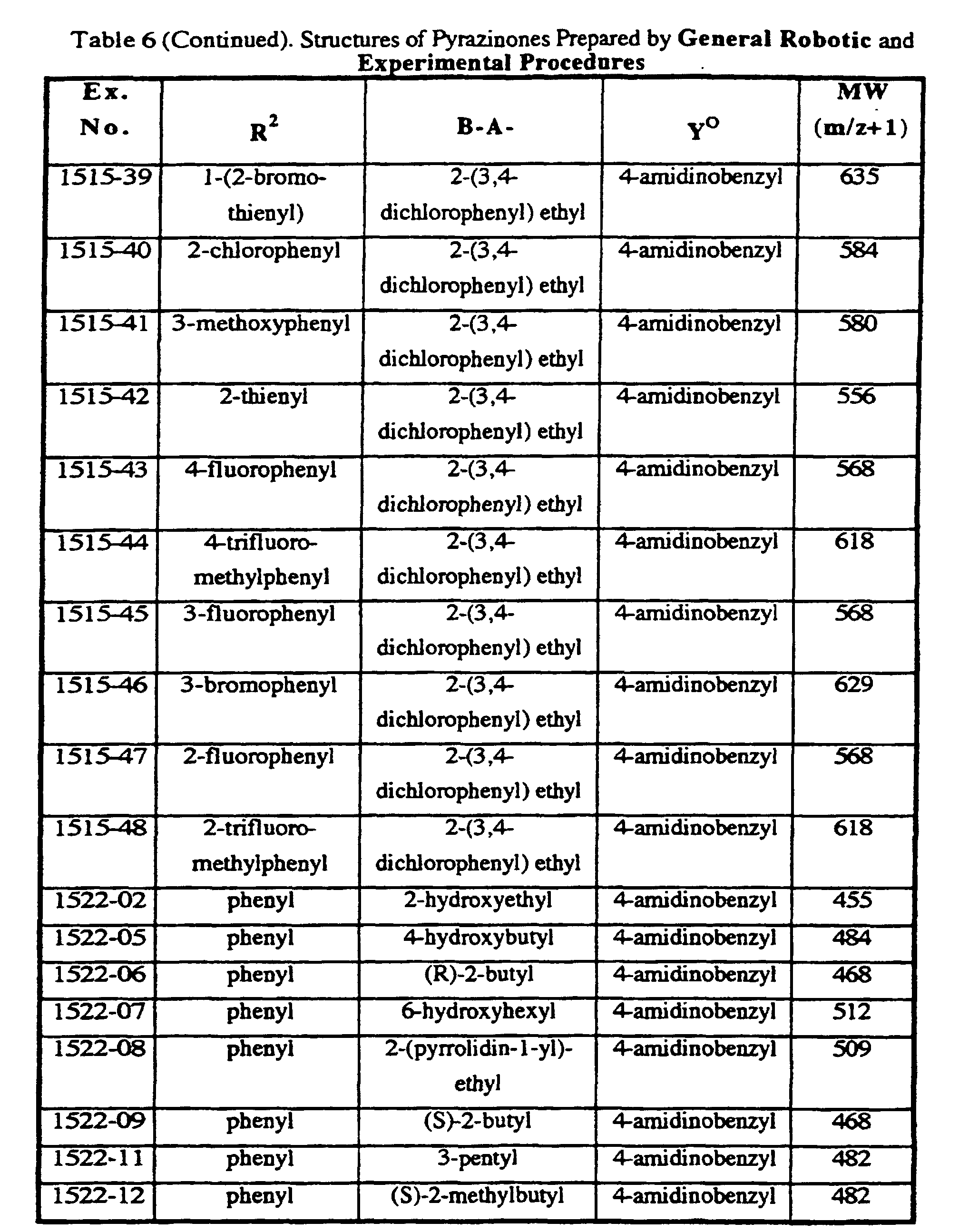

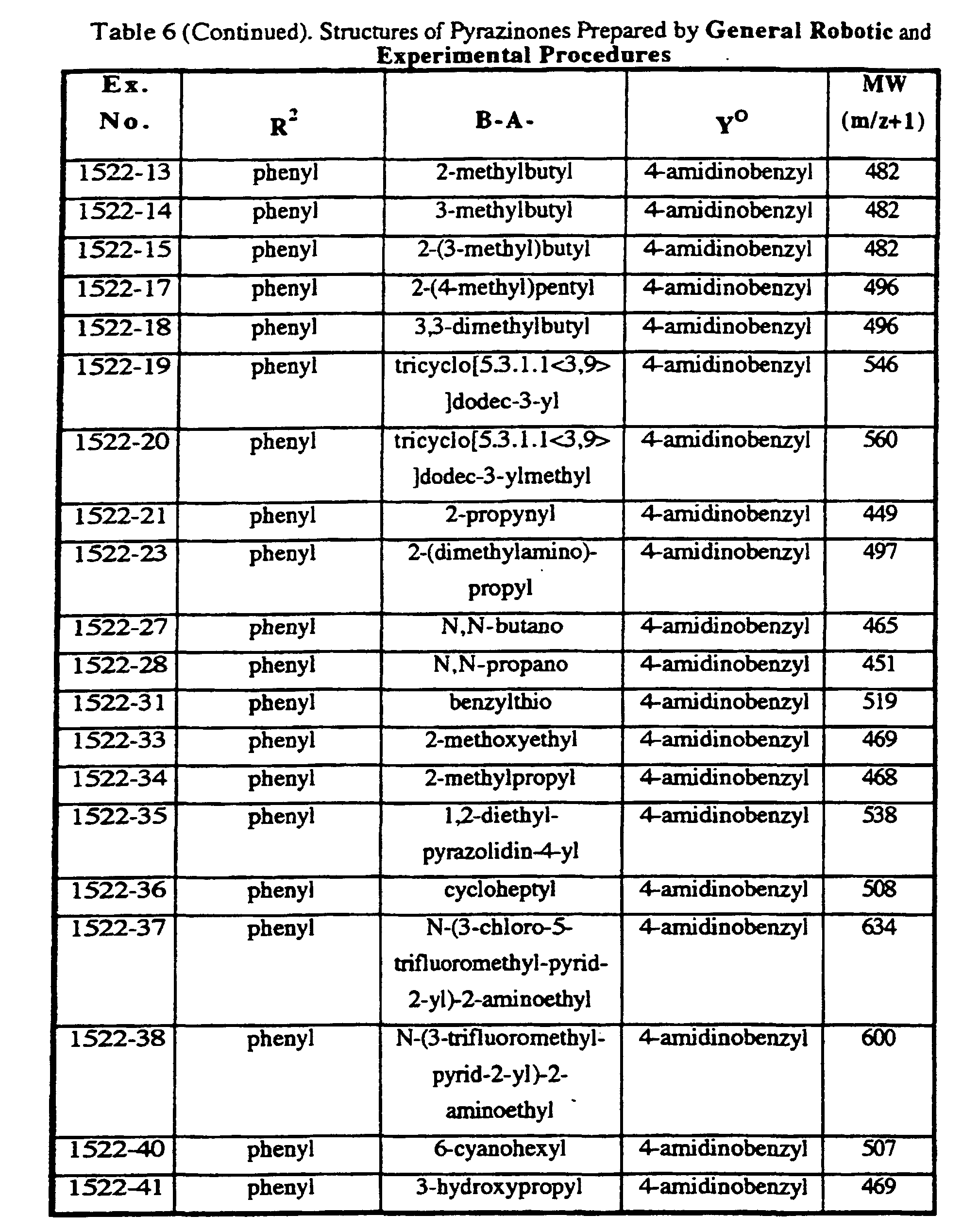

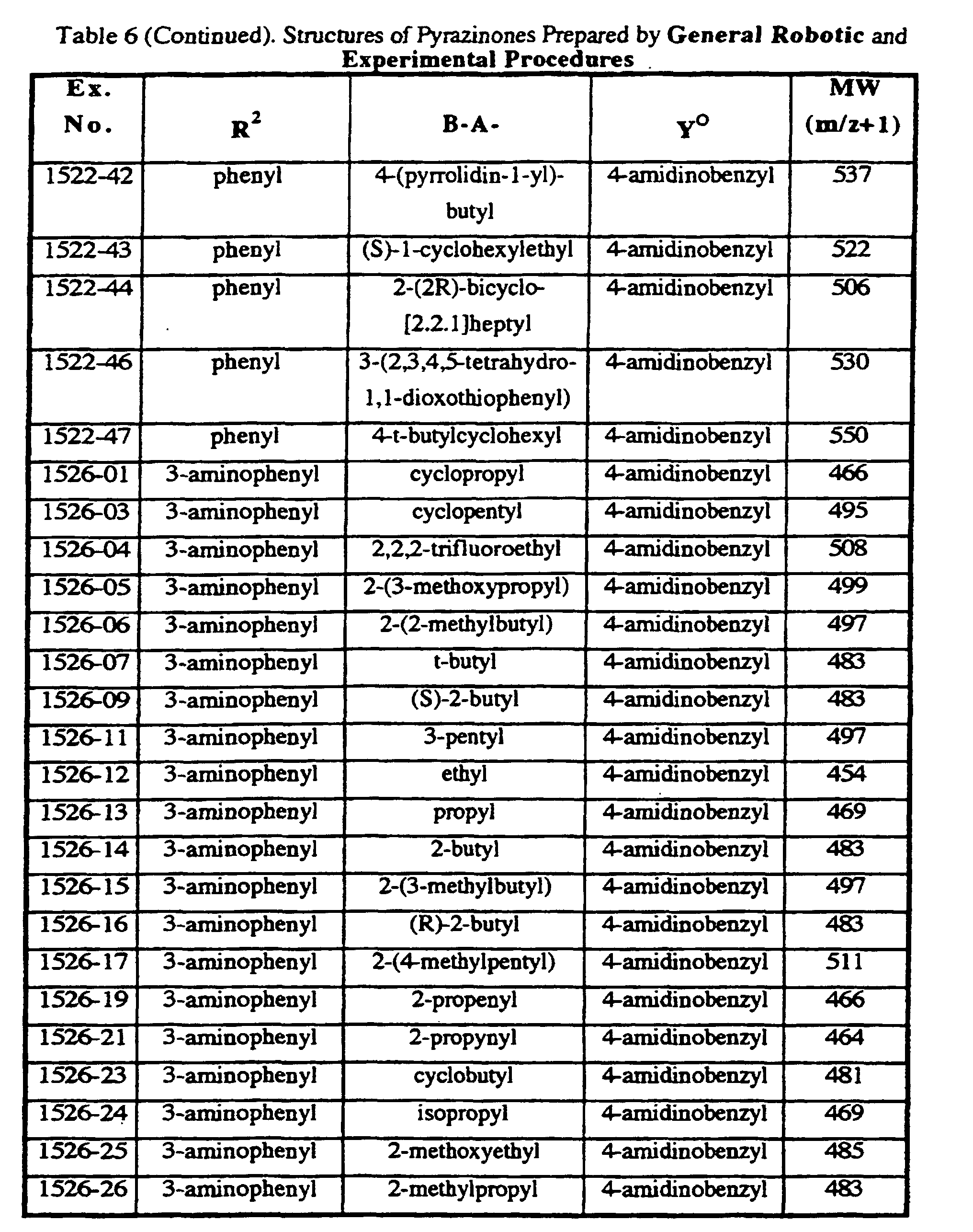

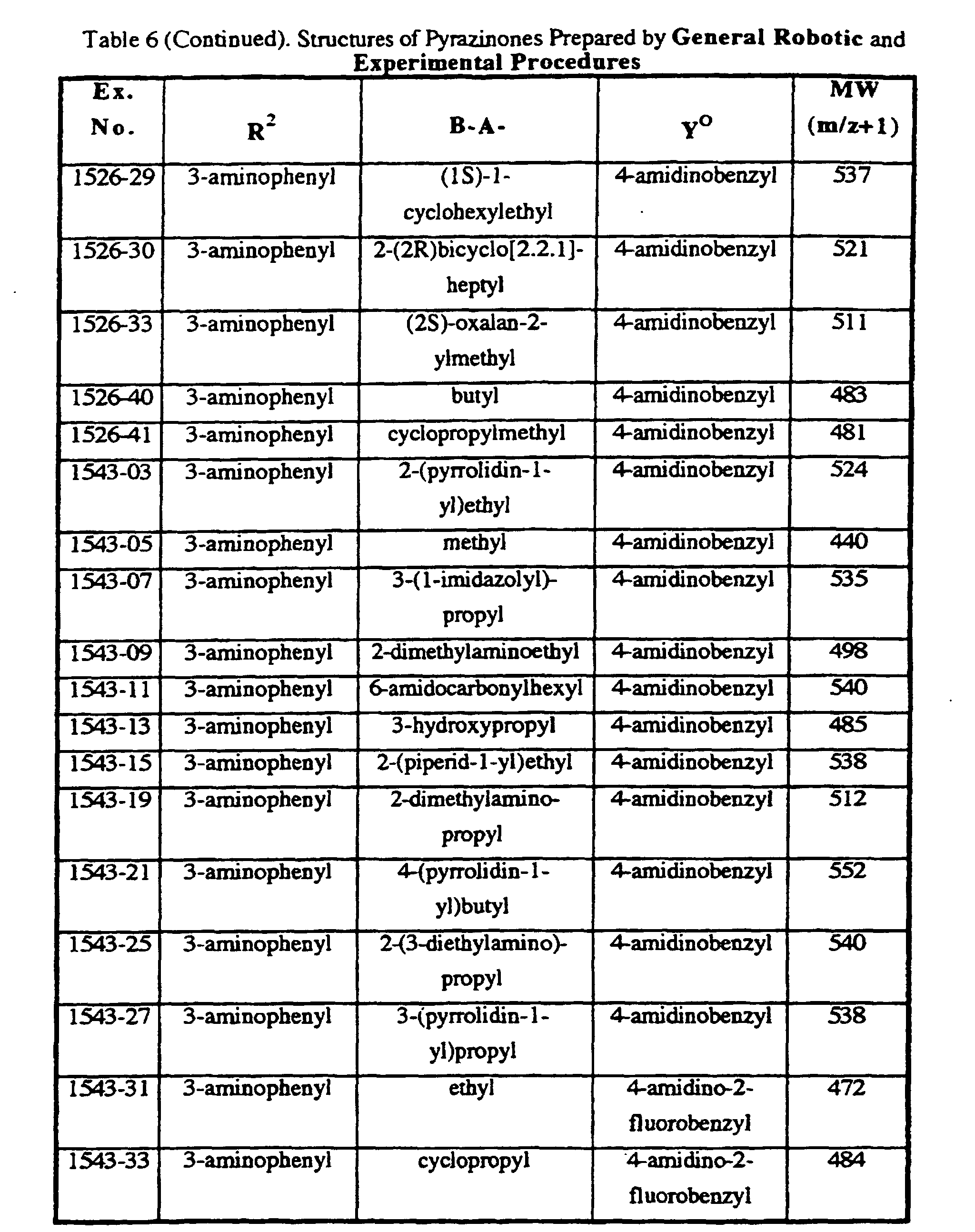

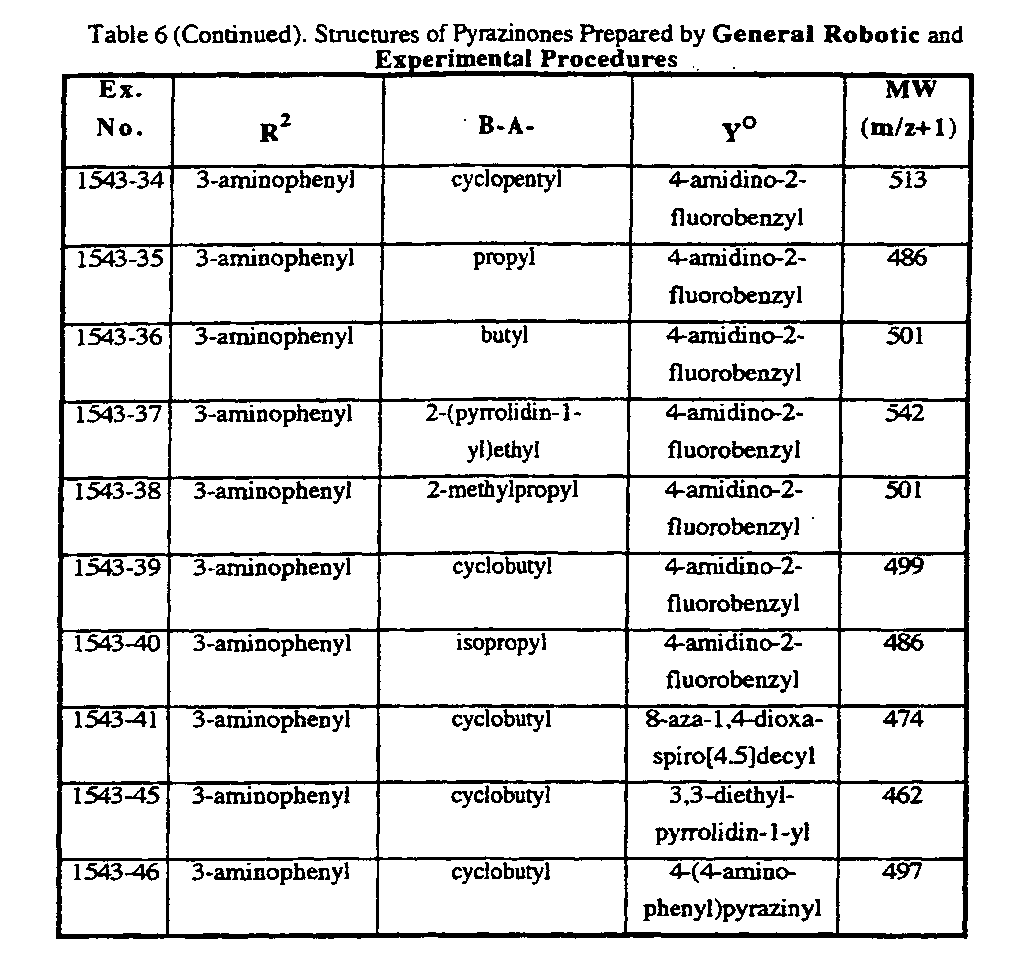

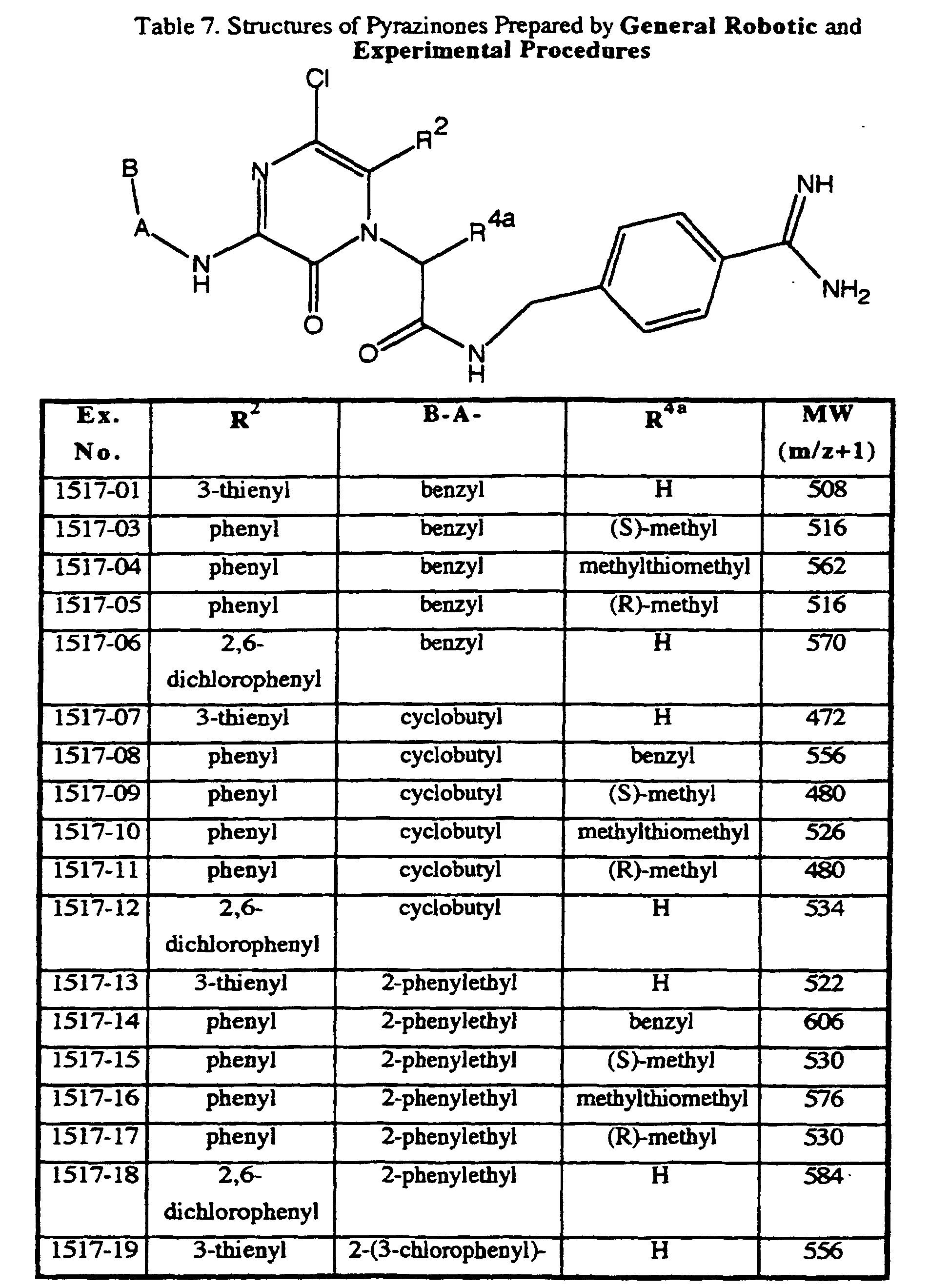

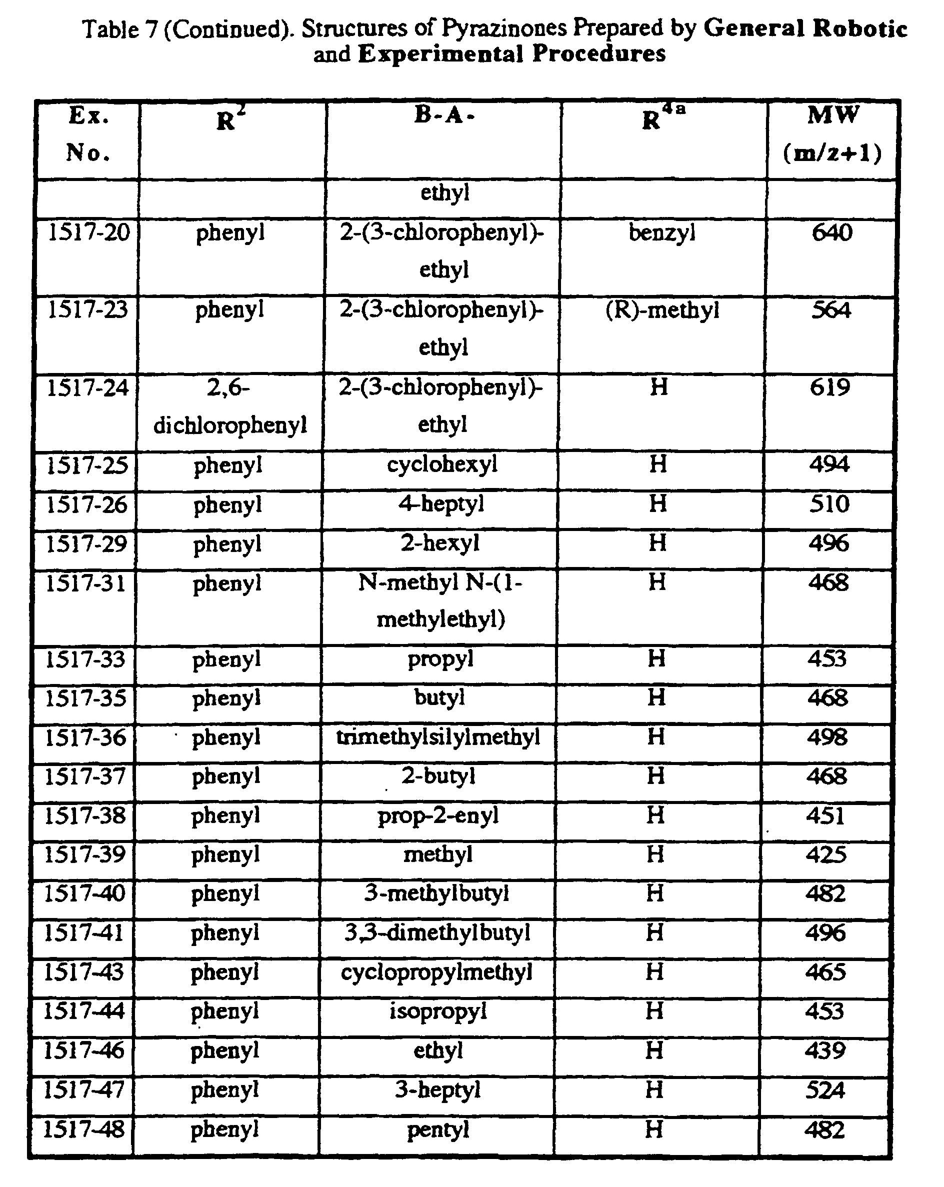

Formula (I) compounds of this invention possessing hydroxyl, thiol, and amine functional groups can be converted to a wide variety derivatives. Alternatively, derivatized Formula (I) compounds can be obtained by first derivatizing one or more intermediates in the processes of preparation before further transforming the derivatized intermediate to comounds of Formula (I). A hydroxyl group in the form of an alcohol or phenol can be readily converted to esters of carboxylic, sulfonic, carbamic, phosphonic, and phosphoric acids. Acylation to form a carboxylic acid ester is readily effected using a suitable acylating reagent such as an aliphatic acid anhydride or acid chloride. The corresponding aryl and heteroaryl acid anhydrides and acid chlorides can also be used. Such reactions are generally carried out using an amine catalyst such as pyridine in an inert solvent. Similarly, carbamic acid esters (urethanes) can be obtained by reacting a hydroxyl group with isocyanates and carbamoyl chlorides. Sulfonate, phosphonate, and phosphate esters can be prepared using the corresponding acid chloride and similar reagents. Compounds of Formula (I) that have at least one thiol group present can be converted to the corresponding thioesters derivatives analogous to those of alcohols and phenols using the same reagents and comparable reaction conditions. Compounds of Formula (I) that have at least one primary or secondary amine group present can be converted to the corresponding amide derivatives. Amides of carboxylic acids can be prepared using the appropriate acid chloride or anhydrides with reaction conditions analogous to those used with alcohols and phenols. Ureas of the corresponding primary or secondary amine can be prepared using isocyanates directly and carbamoyl chlorides in the presence of an acid scavenger such as triethylamine or pyridine. Sulfonamides can be prepared from the corresponding sulfonyl chloride in the presence of aqueous sodium hydroxide or a tertiary amine. Suitable procedures and methods for preparing these derivatives can be found in House's Modern Synthetic Reactions, W. A. Benjamin, Inc., Shriner, Fuson, and Curtin in The Systematic Identification of Organic Compounds, 5th Edition, John Wiley & Sons, and Fieser and Fieser in Reagents for Organic Synthesis, Volume 1, John Wiley & Sons. Reagents of a wide variety that can be used to derivatize hydroxyl, thiol, and amines of compounds of Formula (I) are available from commercial sources or the references cited above, which are incorporated herein by reference.

Formula (I) compounds of this invention possessing hydroxyl, thiol, and amine functional groups can be alkylated to a wide variety of derivatives. Alternatively, alkylated Formula (I) compounds can be obtained by first alkylating one or more intermediates in the processes of preparation before further transforming the alkylated intermediate to comounds of Formula (I). A hydroxyl group of compounds of Formula (I) can be readily converted to ethers. Alkylation to form an ether is readily effected using a suitable alkylating reagent such as an alkyl bromide, alkyl iodide or alkyl sulfonate. The corresponding aralkyl, heteroaralkyl, alkoxyalkyl, aralkyloxyalkyl, and heteroaralkyloxyalkyl bromides, iodides, and sulfonates can also be used. Such reactions are generally carried out using an alkoxide forming reagent such as sodium hydride, potassium t-butoxide, sodium amide, lithium amide, and n-butyl lithium using an inert polar solvent such as DMF, DMSO, THF, and similar, comparable solvents. amine catalyst such as pyridine in an inert solvent. Compounds of Formula (I) that have at least one thiol group present can be converted to the corresponding thioether derivatives analogous to those of alcohols and phenols using the same reagents and comparable reaction conditions. Compounds of Formula (I) that have at least one primary, secondary or tertiary amine group present can be converted to the corresponding secondary, tertiary or quaternary ammonium derivative. Quaternary ammonium derivatives can be prepared using the appropriate bromides, iodides, and sulfonates analogous to those used with alcohols and phenols. Conditions involve reaction of the amine by warming it with the alkylating reagent with a stoichiometric amount of the amine (i.e., one equivalent with a tertiary amine, two with a secondary, and three with a primary). With primary and secondary amines, two and one equivalents, respectively, of an acid scavenger are used concurrently. Secondary or tertiary amines can be prepared from the corresponding primary or secondary amine. A primary amine can be dialkylated by reductive amination using an aldehyde, such as formaldehyde, and sodium cyanoborohydride in the presence of glacial acetic acid. A primary amine can be monoalkylated by first mono-protecting the amine with a ready cleaved protecting group, such as trifluoroacetyl. An alkylating agent, such as dimethylsulfate, in the presence of a non-nucleophilic base, such as Barton's base (2-*tert*-butyl-1,1,3,3-tetramethylguanidine), gives the monomethylated protected amine. Removal of the protecting group using aqueous potassium hydroxide gives the desired monoalkylated amine. Additional suitable procedures and methods for preparing these derivatives can be found in House's Modem Synthetic Reactions, W. A. Benjamin, Inc., Shriner, Fuson, and Curtin in The Systematic Identification of Organic Compounds, 5th Edition, John Wiley & Sons, and Fieser and Fieser in Reagents for Organic Synthesis published by John Wiley & Sons. Perfluoroalkyl derivatives can be prepared as described by DesMarteau in J. Chem. Soc. Chem. Commun. 2241 (1998). Reagents of a wide variety that can be used to derivatize hydroxyl, thiol, and amines of compounds of Formula (I) are available from commercial sources or the references cited above, which are incorporated herein by reference.

The examples of synthetic approaches to the preparation pyrazinones derivatized in a nucleophilic substituent such as may be present in B, R¹, R² and Y⁰ are shown in specific **Examples 100 through 104** below. The specific examples recited below should be considered a being merely illustrative of the wide variety possible and not as limiting to one of ordinary skill in the art.

### Example 100

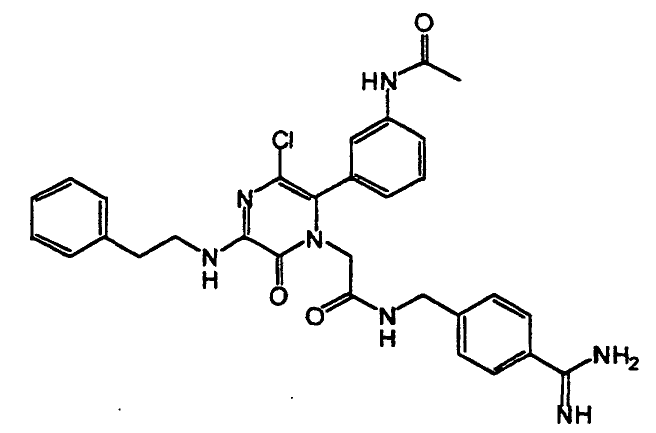

By following the method of **Example 1** and substituting 3-nitrobenzaldehyde for benzaldehyde, 1-benzyloxycarbonylmethyl-3,5-dichloro-6-(3-nitrophenyl)pyrazinone (**EX-100A**) was obtained.
The pyrazinone, 1-benzyloxycarbonylmethyl-3,5-dichloro-6-(3-nitrophenyl)pyrazinone (**EX-100A**), (15.01 g, 34.6 mmol) was taken up in 325 mL of 50% EtOH (w/w) and heated to 75 °C. EtOAc was added until the solution was homogeneous ( about 80 mL). Iron powder (9.4 g, 168 mmol) was added, followed by 0.57 mL of 12 M HCl (6.8 mmol) in about 0.6 mL of 50% EtOH. The reaction was monitored by TLC (80% EtOAc/hexanes) and was complete within 40 minutes. The reaction mixture was cooled to room temperature, and the iron was removed by filtration through Celite. The yellow solution was diluted with 600 mL of EtOAc and 300 mL of water. Saturated NaCl was added to help separate the layers. The organic phase was washed with saturated NaHCO₃ (2x250 mL), saturated NaCl (1x250 mL), dried over MgSO₄, filtered, and the solvents were removed under reduced pressure. The residue was taken up in 20-25 mL of 3.4 M HCl in EtOAc. Additional EtOAc (about 25 mL) was added, and the mixture was heated to dissolve all of the compound. The volatile components were removed under reduced pressure. The residue (crusty solid) was taken up in EtOAc and slowly dripped into hexanes. The pale yellow solid that precipitated was filtered and dried under vacuum at room temperature to yield 12.19 g (80% yield) of 1-benzyloxycarbonylmethyl-3,5-dichloro-6-(3-aminophenyl)pyrazinone hydrochloride (**EX-100B**) as a pale yellow solid: ¹H NMR (300 MHz, CD₃OD) d 4.61 (AB q, 2 H, J=17 Hz), 5.20 (AB q, 2H, J=12 Hz), 7.31-7.51 (m, 7H), 7.63-7.67 (m, 2H); HPLC purity (retention time): 91% (3.0 min); LRMS *m*/*z* 404 (M⁺ + H).

The pyrazinone, 1-benzyloxycarbonylmethyl-3,5-dichloro-6-(3-aminophenyl)pyrazinone hydrochloride (**EX-100B**), (78.2 mg, 0.18 mmol) was taken up in 5 mL of dichloromethane. Pyridine (32 mL, 0.40 mmol) was added, followed by acetyl chloride (26 mL, 036 mmol) in I mL of dichloromethane. The reaction was stirred at ambient temperature until the reaction was complete by TLC and LC/MS after 24 hours. The reaction solution was then washed with saturated NaHCO₃ (4 x 5 mL), saturated NaCl (1 x 5 mL), dried over MgSO₄ and concentrated to give 68.9 mg (86% yield) of the product 1-benzyloxycarbonylmethyl-3,5-dichloro-6-(3-acetamidophenyl)pyrazinone (**EX-100C**): ¹H NMR (300 MHz, CDCl₃) d 2.21 (s, 3H), 4.55 (AB q, 2H, J = 16.6 Hz), 5.17 (s, 2H), 6.96 (d, 1H, J = 7.7 Hz), 7.26-7.40 (m, 5H), 7.57 (s, 1H), 7.74-7.79 (m, 1H), 8.19-8.24 (br m, 1H), 8.65 (br s, 1H).

Following the necessary final steps of the procedure of **Example 1, EX-100C** was converted to the product: HPLC parity (retention time): 100% (2.9 min); LRMS *m*/*z* 572.5 (M⁺ + H).

### Example 101

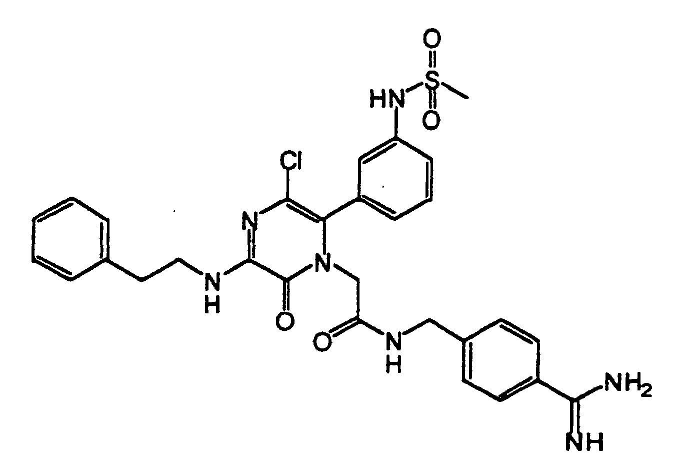

By following the method of **Example 100** and substituting methanesulfonyl chloride for acetyl chloride the product was prepared: HPLC purity (retention time): 100% (2.9 min); LRMS *m*/*z* 608.2 (M⁺ + H).

### Example 102

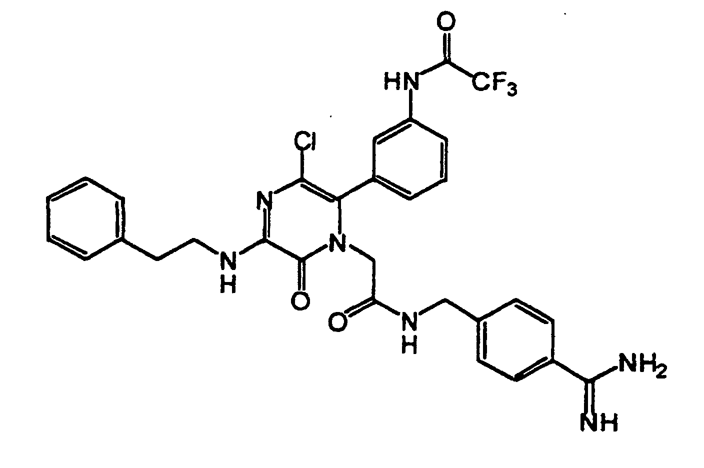

By following the method of **Example 100** and substituting trifluoroacetic anhydride for acetyl chloride, the product was prepared: HPLC purity (retention time): 100% (3.3 min); LRMS *m*/*z* 6263 (M⁺ + H).

### Example 103

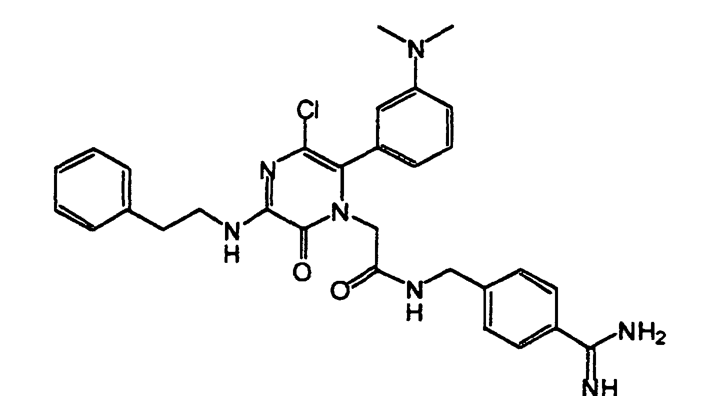

By following the method of **Example 1** and substituting 3-nitrobenzaldehyde for benzaldehyde, 1-benzyloxycarbonylmethyl-3,5-dichloro-6-(3-aminophenyl)pyrazinone was obtained.

The 1-benzyloxycarbonylmethyl-3,5-dichloro-6-(3-aminophenyl)-pyrazinone (210.6 mg, 0.48 mmol) was taken up in 9 mL of acetonitrile. Polyamine resin (1.05 g, 4.9 mmol) was added, along with about 10 mL of dichloromethane. After agitating about 10 mins the resin was filtered, rinsed with acetonitrile, and the solvents concentrated to about 10 mL. Formaldehyde (37%) (0.4 mL, 4.9 mmol) was added, followed by NaCNBH₃ (1.0 M in THF, 1.5 mL, 1.5 mmol) and the dropwise addition of two 50 mL portions of glacial acetic acid (17.4 M, 1.74 mmol). The reaction was monitored by LC/MS. A third 50 mL portion of glacial acetic acid was added after 3.5 h to force the reaction to completion. The solution was diluted with about 40 mL of diethyl ether and washed with 1.2 M NaOH (3 x 5 mL), saturated NaCl (1 x 5 mL), dried over MgSO₄, and the solvents were removed under reduced pressure to give 0.17 g (82% yield) of 1-benzyloxycarbonylmethyl-3,5-dichloro-6-(3-[N,N-dimethylamino]phenyl)pyrazinone (**EX-103A**): ¹H NMR (300 MHz, CDCl₃): d 2.96 (s, 6H), 4.59 (s, 2H), 5.19 (s, 2H), 6.55 (m, 2H), 6.82 (d, 1H), 7.25-7.40 (m, 6H).

Following, the necessary final steps of the procedure of **Example 1, EX-103A** was converted to the product: HPLC purity (retention time): 94% (2.6 min); LRMS *m*/*z* 558.4 (M⁺ + H).

### Example 104

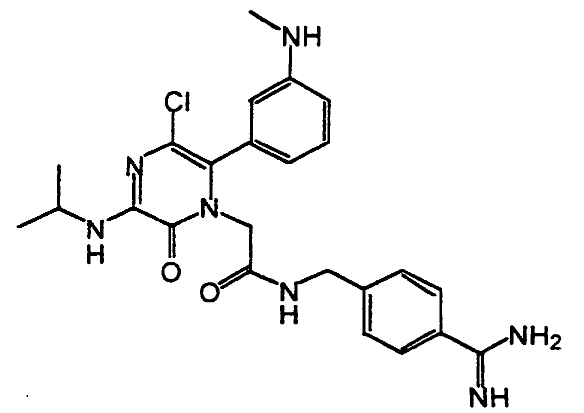

By following the method of **Example 100** and replacing phenethylamine with isopropylamine, 1-benzyloxycarbonylmethyl-3-isopropylamino-5-chloro-6-(3-aminophenyl)pyrazinone was obtained.

The 1-benzyloxycarbonylmethyl-3-isopropylamino-5-chloro-6-(3-aminophenyl)pyrazinone (1.01 g, 2.4 mmol) was dissolved in 25 mL of THF. Pyridine (0.37 mL, 4.6 mmol) was added, followed by pentafluoropyridine trifluoroacetate (0.79 mL, 4.6 mmol). After 2 h, polyamine resin (3.1 g, 8.7 mmol) and 25 mL of dichloromethane was added, and the mixture was vigorously stirred for 1-2 h. The resin was filtered, rinsed with dichloromethane (3 x 5 mL), and the volatiles were removed under reduced presssure to give the desired product **EX-104A** in quantitative yield: ¹H NMR (300 MHz, CDCl₃) d 1.30 (d, 3H, J = 1.4 Hz), 1.32 (d, 3H, J = 1.4 Hz), 4.24 (m, 1H), 4.47 (AB q, 2H, J = 16.9 Hz), 5.15 (s, 2H), 6.22 (d, 1H, J = 8.2 Hz), 7.12 (d, 1H, J = 7.7 Hz), 7.25-7.42 (m, 5H), 7.54 (s, 1H), 7.73-7.81 (m, 1H), 8.62 (d, 1H, J = 4.2 Hz), 9.10 (br s, 1H).

The 1-benzyloxycarbonylmethyl-3-isopropylamino-5-chloro-6-(3-[N-trifluoroacetamido]phenyl)pyrazinone (**EX-104A**) (0.63 g, 1.2 mmol) was dissolved in 20 mL of dichloromethane. Barton's base (2-*tert*-butyl- 1,1,3,3-tetramethylguanidine) (0-5 mL, 25 mmol) and dimethylsulfate (0.66 mL, 7 mmol) wee added, and the reaction was stirred at ambient temperature overnight. The reaction was monitored by LC/MS, and after completion the solution was washed with aqueous NH₄OH (2 x 10 mL) and 5% HCl (1 x 10 mL). The combined aqueous washes were extracted with dichloromethane (1 x 10 mL). The combined organic phases were washed with saturated NaCl (1 x 10 mL), dried over MaSO₄, filtered, and the volatiles were removed under reduced pressure to give 0.51 g (80% yield) of the desired product **(EX-104B):** HPLC purity (retention time): 97% (4.4 min); LRMS *m*/*z* 537.5 (M⁺ + H).

Following the necessary final steps of the procedure of **Example 1, EX- 104B** was converted to the product: ¹H NMR (300 MHz, CD₃OD) d 131 (s, 3H), 1.33 (s, 3H), 2.94 (s, 3H), 4.22 (m, 1H), 4.40-4.52 (m, 4H), 7.01-7.05 (m, 2H), 7.17-7.19 (m, 1H), 7.42-7.45 (m, 1H), 7.49 (d, 2H, J = 8.3 Hz) 7.80 (d, 2H, J = 83 Hz); HPLC purity (retention time): 100% (2.1 min); LRMS m/z 481.6 (M⁺+ H).

Pyrazinones, wherein a B-A substituent is introduced by reaction of a 3-amino group of an intermediate pyrazinone with an electrophilic reagent, can be prepared using the general procedures and processes shown in **Scheme 9 and Scheme 10** and as illustrated below in specific **Examples 105-109**.

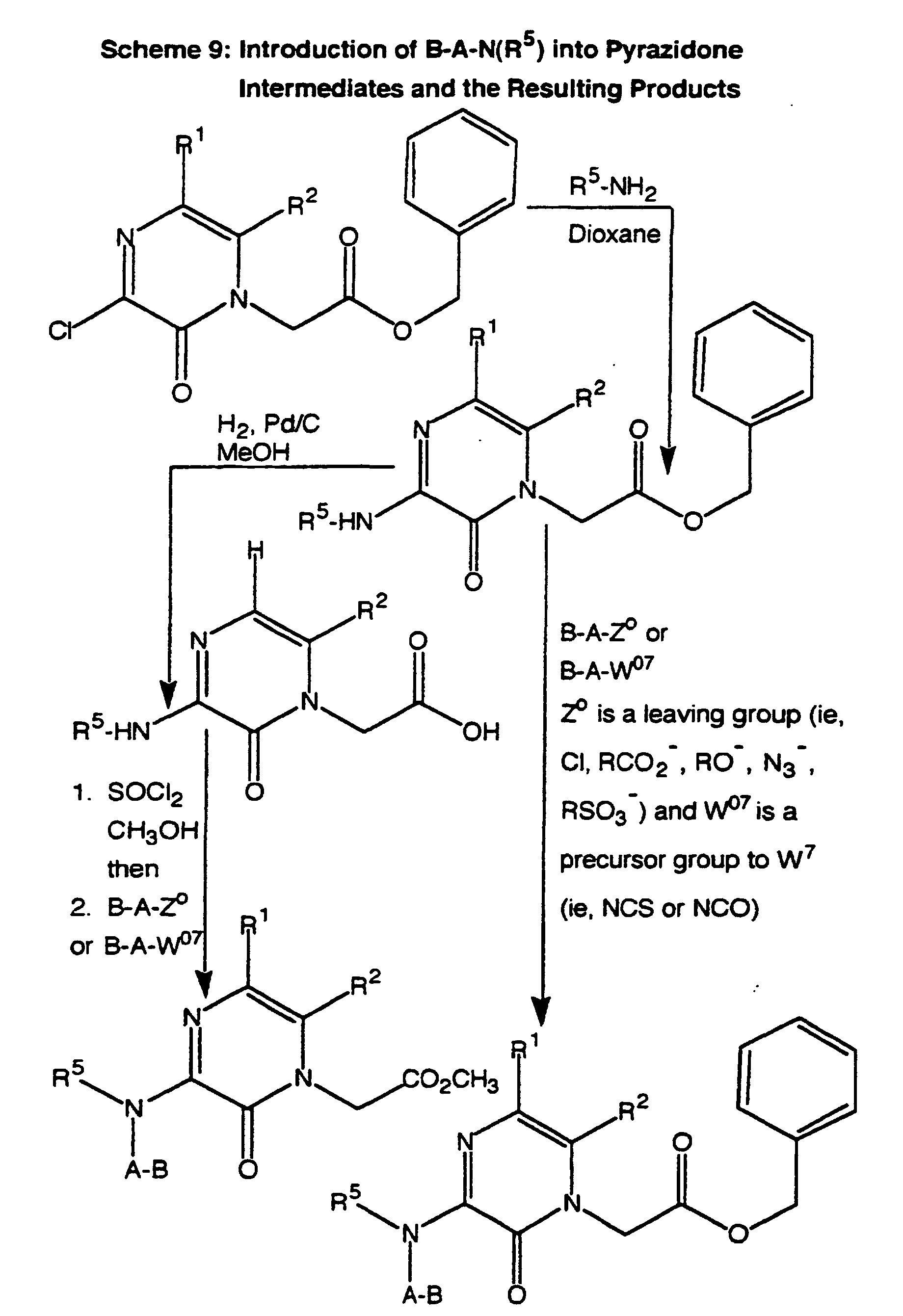

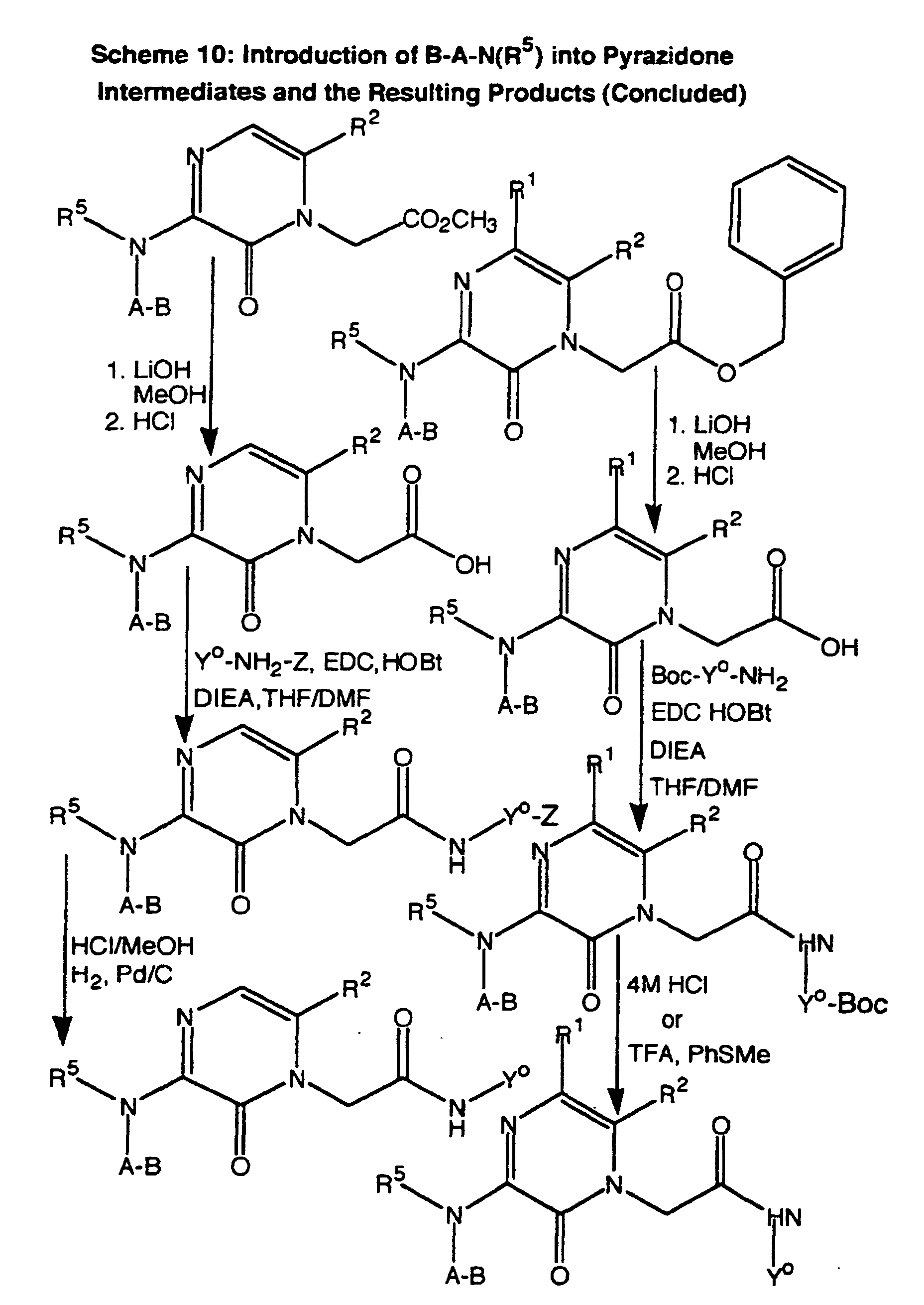

The examples of synthetic approaches to the preparation pyrazinones in which the substituents represented by B-A are introduced by reaction of a 3-amino group of the pyrazinone with an electrophilic reagent are shown in specific **Examples 105 through 109** below. The specific examples recited below should be considered a being merely illustrative of the wide variety possible and not construed as limiting to one of ordinary skill in the art.

### Example 105

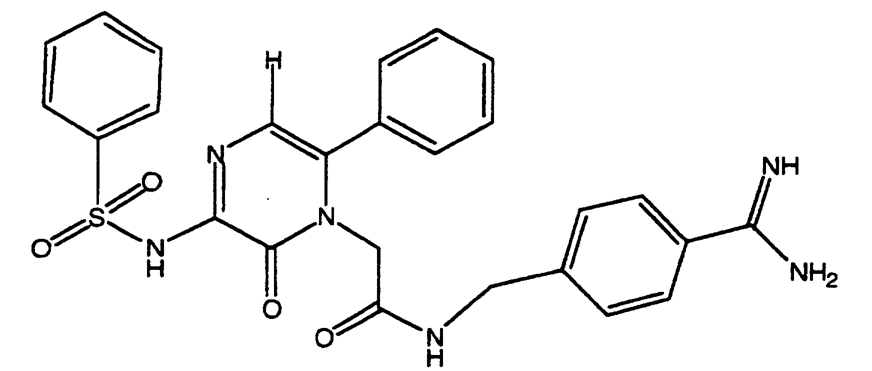

1-Benzyloxycarbonylmethyl-3,5-dichloro-6-phenylpyrazinone (**EX-1B**) (0.8 g, 2.06 mmol) was mixed with 20 ml 0.5 M ammonia in dioxane in a sealed tube. The tube was heated to 100°C for 12 hours. After removing the dioxane under reduced pressure, the residue was dissolved in ethyl acetate. The ethyl acetate solution was washed with water and brine and dried over anhydrous Na₂SO₄. After removing the solvent, the product was recrystallized in acetone to yield the pure amino pyrazinone **EX-105A** as a white crystal solid (0.76 g, 99%): HPLC-MS (0 to 95% AcCN / 6 min @ 1.0 mL / Min @ 254 nm @ 50 °C): retention time 3.75 min, M+H⁺ = 370.0 for formula C₁₉H₁₇ClN₃O₃. ¹H NMR (400 MHz, CDCl₃): d 4.43 (s, 2H), 5.13 (s, 2H), 5.78 (b, 2H), 7.21-7.27 (m, 5H), 7.35-739 (m, 5H).

**EX-105A** (4.7 g, 12.73 mmol) was mixed with 1.34 g 10% Pd/C in 100 ml methanol. The mixture was stirred under hydrogen atmosphere that was introduced via a balloon for 48 hours. After filtration and removing the solvent, a white crystal solid was obtained as the carboxylic acid product **EX-105B** (3.0 g, 97%): HPLC-MS (0 to 95% AcCN / 6 min @ 1.0 mL / Min @ 254 nm @ 50 °C): retention time 1.45 min, M+H⁺ = 246.0 for formula C₁₂H₁₂N₃O₃.

**EX-105B** (3.0 g, 12.2 mmol) in 100 ml methanol was cooled down to-50 °C. SOCl₂ (1.4 ml, 19.1 mmol) was added to the solution. After stirring at room temperature for four hours, the mix was heated to reflux for three hours. After removing the solvent, the residue was subjected to a silica gel plug using ethyl acetate to elute. The pure product was obtained by recrystallization in methanol as a white crystal solid **EX-105C** (2.22 g, 68%): HPLC-MS (0 to 95% AcCN / 6 min @ 1.0 mL / Min @ 254 nm @ 50 °C): retention time 1.94 min, M+H⁺ = 260.0 for formula C₁₃H₁₄N₃O₃.

**EX-105C** (0.258 g, 1 mmol) was mixed with benzenesulfonyl chloride (0353g, 2 mmol ) in 3 ml pyridine. The reaction mixture was heated at 90 °C for 2 hours. After removing the pyridine, the crude product was obtained by an aqueous work-up procedure. The crude product **EX-105D** was dissolved in 10 ml methanol and treated with 10 ml 1M LiOH solution for 15 minutes. After the solution was acidified with 2 N HCl to a pH of about 2 and the methanol removed under reduced pressure, a yellow precipitate was obtained via filtration and washing with water. The pure sulfonamide **EX-105E** is a yellow crystalline solid (0.267 g, 70%): HPLC-MS (0 to 95% AcCN / 6 min @ 1.0 mL / Min @ 254 nm @ 50 °C): retention time 2.88 min, M+H⁺ = 386.0 for formula C₁₈H₁₆N₃O₅S. ¹H NMR (400 MHz, methanol-d₄): d 4.44 (s, 2H), 6.77 (s, 1H), 7.32 (dd, J =8.0, 1.6 Hz, 2H), 7.42-7.48 (m, 3H), 7.54 (t, J = 8.0 Hz, 2H), 7.60-7.64 (m, 1H), 8.09 (d, J = 8.0, 2H). ¹³C NMR (101 MHz, methanol-d₄): d 48.4, 129.2, 129.9, 130.0, 130.6, 131.0, 132.6, 134.4, 141.4, 146.1, 157.0, 159.0, 160.0, 170.3.

**EX-105E** (0.106 g, 0.275 mmol) was mixed with EDC (0.055 g, 0.289 mmol) and HOBt (0.044 g, 0.289 mmol) in 2 ml DMF. The mixture was stirred for 10 minutes. To this mixture was then added the protected amidine, 4-(N-benzyloxycarbonylamidino)benzylamine hydrogen chloride salt (0.289 mmol), and DIEA (0.144 ml, 0.825 mmol) in I ml DMF. The reaction solution was stirred for 2 hours at room temperature. The DMF was removed under reduced pressure. The remaining residue was triturated in 1 N HCl and washed with water to yield the product **EX-105F** as an off-white amorphous solid (0.152 g, 85%): HPLC-MS (0 to 95% AcCN / 6 min @ 1.0 mL / Min @ 254 nm @ 50 °C): retention time 3.14 min, M+H⁺ = 6513 for formula C₃₄H₃₀N₅O₇S. ¹H NMR (400 MHz, methanol-d₄): d 4.42 (s, 2H), 4.51 (s, 2H), 5.40 (s, 2H), 6.76 (s, 1H), 7.35-7.62 (m, 15H), 7.75 (d, J =8.0 Hz, 2H), 8.08 (d, J = 8.0 Hz, 2H). ¹³C NMR (101 MHz, methanol-d₄): d 43.6, 49.7, 70.7, 111.6, 118.2, 119.7, 127.3, 128.7. 129.0, 129.1, 129.8, 129.9, 130.0, 130.8, 131.0, 132.6, 134.4, 135.8, 136.2, 141.5, 146.3, 147.6, 153.0, 154.5, 167.9, 169.0.

**EX-105F** (0.148 g, 0.228 mmol), *p*-toluenesulfonic acid mono hydrate (0.045 g, 0.24 mmol) and 10% Pd on activated carbon (0.012 g, 0.007 mmol) were mixed with 5 ml methanol. The mixture was stirred for 2 hours under an atmosphere of hydrogen that was introduced through a rubber balloon. After filtering off the catalyst and removing the methanol, the remaining residue was triturated in a solvent of 2:1 ether to methanol to yield a white amorphous solid as the product (0.105 g, 95%) as the mono-salt of *p*-toluenesulfonic acid: HPLC-MS (0 to 95% AcCN / 6 min @ 1.0 mL / Min @ 254 nm @ 50 °C): retention time 2.64 min, M+H⁺ = 517.5 for formula C₂₆H₂₅N₆O₄S. ¹H NMR (400 MHz, methanol-d₄): d 2.35 (s, 3H), 4.40 (s, 2H), 4.52 (s, 2H), 6.77 (s, 1H), 7.21 (d, J = 7.6 Hz, 2H), 734 (d, J = 7.2 Hz, 2H), 7.41-7.51 (m, 3H), 7.55 (t, J = 7.6 Hz, 2H), 7.64 (t, J = 7.2 Hz, 1H), 7.79 (d, J = 8.0 Hz, 2H), 7.74 (d, J = 8.0 Hz, 2H). ¹³C NMR (101 MHz, methanol-d₄): d 21.2, 43.5, 70.7, 119.5, 126.8, 128.0, 128.7, 128.9, 129.0, 129.7, 129.8, 129.9, 130.6, 130.9, 132.4, 134.3, 136.0, 1413, 141.6, 146.2, 146.4, 152.9, 167.9, 168.7.

### Example 106

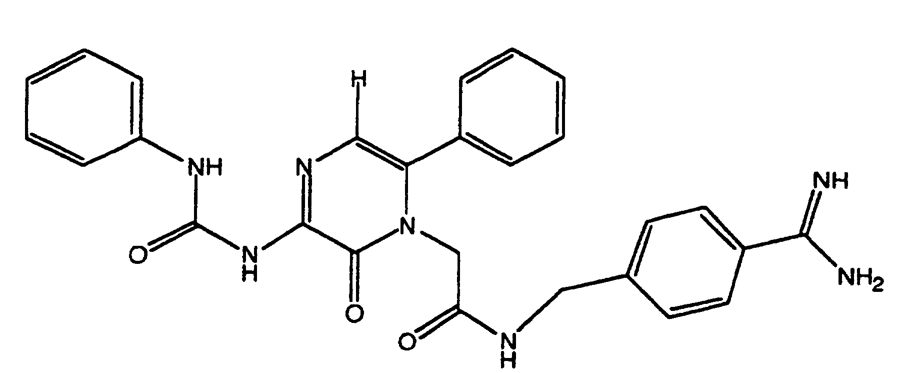

Following the method of **Example 105**, **EX-105C** (0.0932 g, 0.36 mmol) was treated with phenyl isocyanate (0.128 g, 1.08 mmol) and 0.2 ml pyridine in 2 ml acetonitrile at 80 °C for 3 hours instead of benzenesulfonyl chloride. After the reaction mixture was kept in a freezer for two days, a nice crystal solid formed as the pure pyrazinone urea **EX-106A** (0.129 g, 95%): HPLC-MS (0 to 95% AcCN / 6 min @ 1.0 mL / Min @ 254 nm @ 50 °C): retention time 3.73 min, M+H⁺ = 3793 for formula C₂₀H₁₉N₄O₄.
¹H NMR (400 MHz, CDCl₃): d 3.75 (s, 3H), 4.55 (s, 2H), 6.97 (s, 1H), 7.10 (t, J = 7.6 Hz, 1H), 732-7.38 (m, 4H), 7.46-7.52 (m, 3H), 7.58 (d, J = 8.0 Hz, 2H), 8.28 (s, 1H), 11.1 (s, 1H). ¹³C NMR (101 MHz, CDCl₃): d 47.4, 52.8, 119.6, 120.2, 123.9, 128.9, 129.1, 129.4, 130.1, 130.9, 133.8, 137.8, 145.7, 150.8, 150.9, 167.3.

Saponification of compound **EX-106A** manner similar to the procedure as described in the synthesis of **EX-105D** yielded compound **EX-106B**. HPLC-MS (0 to 95% AcCN / 6 min @ 1.0 mL / Min @ 254 nm @ 50 °C): retention time 3.34 min, M+H⁺ = 365.1 for formula C₁₉H₁₆N₄O₄. Compound **EX-106B** was coupled with 4-(N-benzyloxycarbonylamidino)benzylamine hydrogen chloride salt using EDC, HOBt and DIEA as described before to yield the protected product **EX-106C** as an off-white solid: HPLC-MS (0 to 95% AcCN / 6 min @ 1.0 mL / Min @ 254 nm @ 50 °C): retention time 3.58 min, M+H⁺ = 630.0 for formula C₃₅H₃₂N₇O₅. ¹H NMR (400 MHz, methanol-d₄): d 4.49 (s, 2H), 4.61 (s, 2H), 5.40 (s, 2H), 7.06 (s, 1H), 7.11 (t, J = 7.2 Hz, 1H), 7.32-7.56 (m, 16H), 7.76 (d, J = 8 Hz, 2H).

**EX-106C** was converted to the HCl salt of the product by hydrogenation in methanol in the presence of HCl with Pd/C as the catalyst. The product was an off-white amorphous solid: HPLC-MS (0 to 95% AcCN / 6 min @ 1.0 mL / Min @ 254 nm @ 50 °C): retention time 3.01 min, M+H⁺ = 496.4 for formula C₂₇H₂₆N₇O₃. ¹H NMR (400 MHz, methanol-d₄): d 4.47 (s, 2H), 4.65 (s, 2H), 6.97 (s, 1H), 7.15 (t, J = 7.2 Hz, 1H), 7.27-7.54 (m, 9H), 7.57 (d, J = 7.2 Hz, 2H) 7.78 (d, J = 8.0 Hz, 2H), 8.76 (s, 1H), 9.26 (s, 1H). HRMS m/z MH⁺ 496.2036, calcd for C₂₇H₂₆N₇O₃ 496.2097.

### Example 107

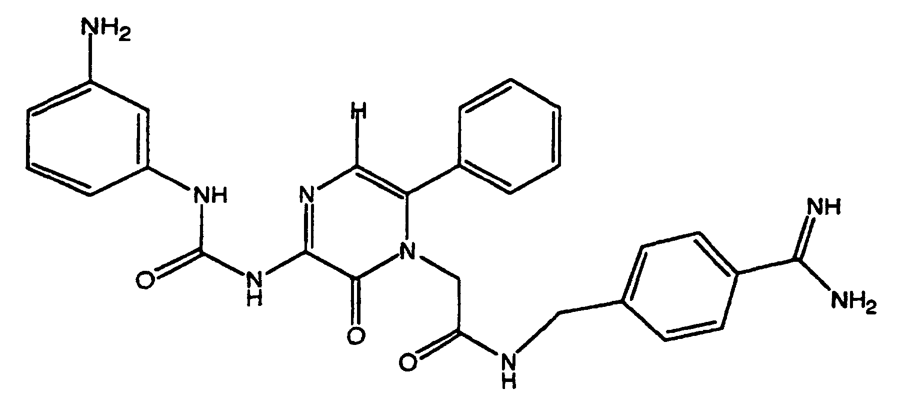

Using the procedure of **Example 106** and substituting 3-(benzyloxycarbonylamido)phenyl isocyanate for phenyl isocyanate, the product was obtained as the HCl salt: HPLC-MS (0 to 95% AcCN / 6 min @ 1.0 mL / Min @ 254 nm @ 50 °C): retention time 1.84 min, M+H⁺ = 511.6 for formula C₂₇H₂₇N₈O₃. ¹H NMR (400 MHz, methanol-d₄): d 4.46 (s, 2H), 4.65 (s, 2H), 6.97 (s, 1H), 7.17 (d, J = 6.8 Hz, 1H), 7.45-7.60 (m, 8H), 7.78 (m, 3H) 7.94 (s, 1H), 8.77 (s, 1H), 9.26 (s, 1H). HRMS m/z MH⁺ 511.2251, calcd for C₂₇H₂₇N₈O₃ 511.2206.

### Example 108

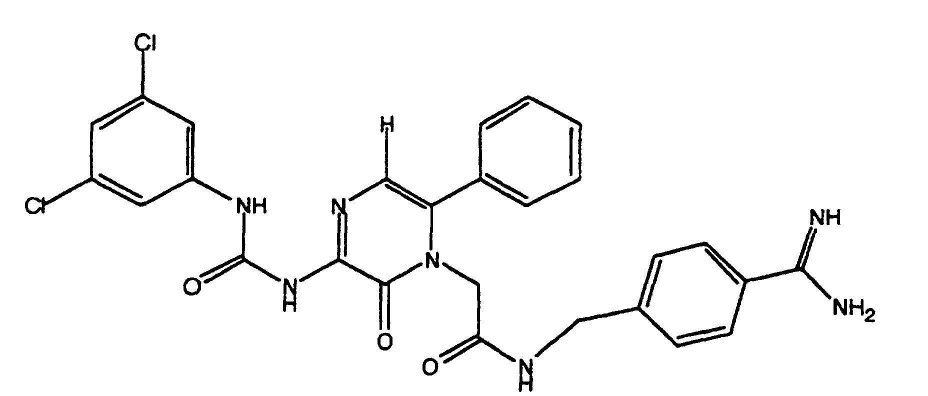

Using the procedure of **Example 106** and substituting 3,5-dichlorophenyl isocyanate for phenyl isocyanate, the product was obtained as the HCl salt: HPLC-MS (0 to 95% AcCN / 6 min @ 1.0 mL / Min @ 254 nm @ 50 °C): retention time 3.41 min, M+H⁺ = 564.4 for formula C₂₇H₂₄Cl₂N₇O₃. ¹H NMR (400 MHz, methanol-d₄): d 4.47 (s, 2H), 4.63 (s, 2H), 7.04 (s, 1H), 7.18 (t, J = 1.6 Hz, 1H), 7.45-7.56 (m, 7H), 7.64 (d, J = 2 Hz, 2H) 7.78 (d. J = 8.0 Hz, 2H), 8.77 (s, 1H), 9.26 (s, 1H). HRMS m/z MH⁺ 564.1351, calcd for C₂₇H₂₄Cl₂N₇O₃ 564.1318.

### Example 109

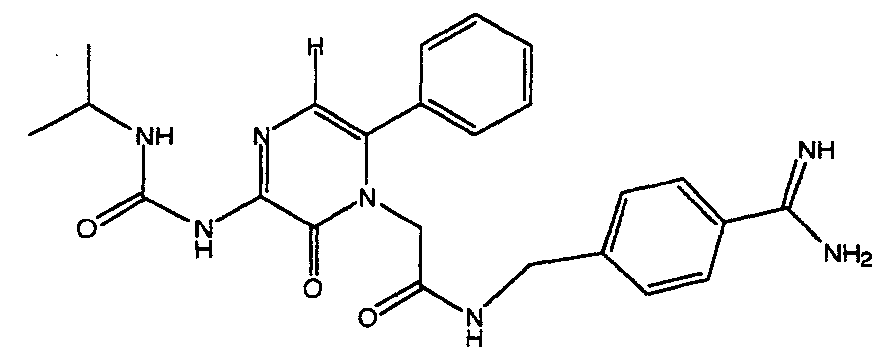

Using the procedure of **Example 106** and substituting isopropyl isocyanate for phenyl isocyanate, the product was obtained as the HCl salt: HPLC-MS (0 to 95% AcCN / 6 min @ 1.0 mL / Min @ 254 nm @ 50 °C): retention time 2.43 min, M+H⁺ = 462.4 for formula C₂₄H₂₈N₇O₃. ¹H NMR (400 MHz, methanol-d₄): d 1.25 (d, J = 6.4 Hz, 6H),3.99(m, 1H), 4.45 (s, 2H), 4.64 (s, 2H), 6.82 (s, 1H), 7.43-7.53 (m, 5H), 7.60 (m, 1H) 7.67 (t, J = 6.4 Hz, 1H), 7.78 (d, J = 8.0 Hz, 2H), 8.77 (s, 1H), 9.26 (s, 1H). HRMS m/z MH⁺ 462.2230, calcd for C₂₄H₂₈N₇O₃ 462.2254.

### Assays for Biological Activity

### TF-VIIa Assay

In this assay 100 nM recombinant soluble tissue factor and 2nM recombinant human factor VIIa are added to a 96-well assay plate containing 0.4 mM of the substrate, N-Methylsulfonyl-D-phe-gly-arg-p-nitroaniline and either inhibitor or buffer (5 mM CaCl₂,50 mM Tris-HCl, pH 8.0, 100 mM NaCl, 0.1% BSA). The reaction, in a final volume of 100 ul is measured immediately at 405 nm to determine background absorbance. The plate is incubated at room temperature for 60 min, at which time the rate of hydrolysis of the substrate is measured by monitoring the reaction at 405 nm for the release of p-nitroaniline. Percent inhibition of TF-VIIa activity is calculated from OD₄₀₅ₙₘ value from the experimental and control sample.

### Xa Assay

Human factor Xa (0.3 nM) and 0.15 mM N-α-Benzyloxycarbonyl-D-arginyl-L-glycyl-L-arginine-p-nitroaniline-dihydrochloride (S-2765) are added to a 96-well assay plate containing either inhibitor or buffer (50 mM Tris-HCl, pH 8.0, 100 mM NaCl, 0.1% BSA). The reaction, in a final volume of 100 ul is measured immediately at 405 nm to determine background absorbance. The plate is incubated at room temperature for 60 min, at which time the rate of hydrolysis of the substrate is measured by monitoring the reaction at 405 nm for the release of p-nitroaniline. Percent inhibition of Xa activity is calculated from OD₄₀₅ₙₘ value from the experimental and control sample.

### Thrombin Assay

Human thrombin (0.28 nM)and 0.06 mM H-D-Phenylalanyl-L-pipecolyl-L-arginine-p-nitroaniline dihydrochloride are added to a 96-well assay plate containing either inhibitor or buffer (50 mM Tris-HCl, pH 8.0, 100 mM NaCl, 0.1% BSA). The reaction, in a final volume of 100 ul is measured immediately at 405 nm to determine background absorbance. The plate is incubated at room temperature for 60 min, at which time the rate of hydrolysis of the substrate is measured by monitoring the reaction at 405 nm for the release of p-nitroaniline. Percent inhibition of thrombin activity is calculated from OD₄₀₅ₙₘ value from the experimental and control sample.

### Trypsin Assay

Trypsin (5 ug/ml; type IX from porcine pancreas) and 0.375 mM N-α-Benzoyl-L-arginine-p-nitroanilide (L-BAPNA) are added to a 96-well assay plate containing either inhibitor or buffer (50 mM Tris-HCl, pH 8.0, 100 mM NaCl, 0.1% BSA). The reactions, in a final volume of 100 ul are measured immediately at 405 nm to determine background absorbance. The plate is incubated at room temperature for 60 min, at which time the rate of hydrolysis of the substrate is measured by monitoring the reaction at 405 nm for the release of p-nitroaniline. Percent inhibition of trypsin activity is calculated from OD₄₀₅ₙₘ value from the experimental and control sample.

Recombinant soluble TF, consisting of amino acids 1-219 of the mature protein sequence was expressed in E. coli and purified using a Mono Q Sepharose FPLC. Recombinant human VIIa was purchased from American Diagnostica, Greenwich CT and chromogenic substrate N-Methylsulfonyl-D-phe-gly-arg-p-nitroaniline was prepared by American Peptide Company, Inc., Sunnyvale, CA. Factor Xa was obtained from Enzyme Research Laboratories, South Bend IN, thrombin from Calbiochem, La Jolla, CA, and trypsin and L-BAPNA from Sigma, St. Louis MO. The chromogenic substrates S-2765 and S-2238 were purchased from Chromogenix, Sweden.

Using bioassay procedures described herein, the biological activity of the compounds of **Examples 1** through **Example 109** and **Tables 1** through **Table 7** are summarized in **Table 8** and **Table 9**.

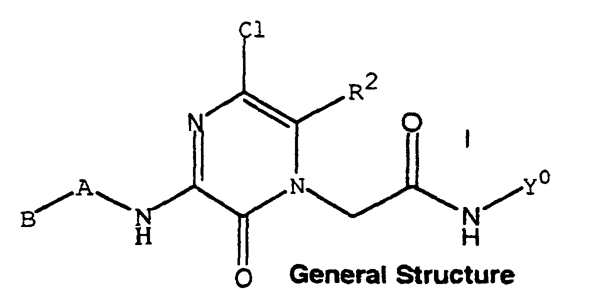

**Table 8.**

| Inhibitory Activity of Pyrazinones toward TF-VIIA, Thrombin II, Factor Xa, and Trypsin II. | | | | |
|---|---|---|---|---|
| **Ex.** **No.** | **IC50 or %** **Inhibition** **TF-VIIa** **(30** υ**M)** | **IC50 or %** **Inhibition** **Thrombin** **II(30** υ**M)** | **IC50 or %** **Inhibition** **Factor Xa** **(30** υ**M)** | **IC50 or %** **Inhibition** **Trpysin II** **(30** υ**M)** |
| 1 | 1 | 1 | >100 | 0.2 |
| 2 | 5 | 0.4 | >100 | 1 |
| 3 | 0.2 | <0.04 | 1 | 0.4 |
| 4 | 0.3 | 0.1 | 3 | 0.5 |
| 5 | 7 | 1 | 4 | 1 |
| 6 | 0.1 | <0.04 | 1 | 0.4 |
| 7 | 0.2 | 0.1 | 3 | 0.4 |
| 8 | 3 | 0.2 | 1 | 0.4 |
| 9 | >30 | 30 | >0 | >30 |
| 10 | >30 | >0 | >0 | >30 |
| 11 | 91 | <0.1 | >100 | 1 |
| 12 | >100 | 7 | >100 | 1 |
| 13 | >100 | 48 | >100 | 14 |
| 14 | >100 | 29 | >100 | 0.3 |
| 15 | >100 | >100 | >100 | 1 |
| 16 | >100 | 13 | >100 | 1 |
| 17 | 0.71 | 1532 | 41% | 0.24 |
| 18 | 47% | 20% | 0 | 1.13 |
| 19 | 0.8 | 0.22 | 22%@30 | 0.27 |
| 20 | 41%@30 | 4 | 6%@30 | 7 |
| 21 | 8%@30 | 26.8 | 21%@30 | 2 |
| 22 | 0%@30 | 47%@30 | 13%@30 | 1.7 |
| 23 | 1.7 | 17 | 38%@30 | 0.32 |
| 24 | 0%@30 | 46%@30 | 22%@30 | 13 |
| 25 | 7% | 3% | 4% | 9% |
| 26 | 1% | 4% | 5% | 3% |
| 27 | 12.5 | >30 | >30 | 16 |
| 28 | 3 | >30 | >30 | 0.23 |
| 29 | pending | pending | pending | pending |
| 30 | pending | pending | pending | pending |
| 31 | 22% | 4% | 4% | 20% |
| 32 | 12 | 0 | 0 | 0 |
| 33 | 4% | 30 | 0 | 3.6 |
| 34 | 20% | 0.59 | 0 | 1.3 |
| 35 | 29% | 5% | 10% | 2.88 |
| 36 | 5% | 31% | 0 | 30% |
| 37 | 1.8 | 1.9 | 0 | 0.3 |
| 38 | 0.9 | 0.58 | 22% | 0.4 |
| 39 | 1.7 | 0.26 | 0 | 0.28 |
| 40 | 0.06 | 0 | 0 | 0.1 |
| 41 | 0.02 | 8 | 0 | 0.1 |
| 42 | 0.5 | 17% | 0 | 0.53 |
| 43 | 0.25 | 43% | 0 | 0.15 |
| 44 | 2.1 | 40% | 4% | 0.54 |
| 45 | 0.89 | 13 | 9% | 0.25 |
| 46 | 0.98 | 18.7 | 10% | 0.23 |
| 47 | 0.66 | 1.14 | 10.6 | 0.20 |
| 48 | 0.57 | 6.4 | 20% | 0.38 |
| 49 | 0.57 | 6.4 | 28.9 | 0.19 |
| 50 | 0.50 | 14.2 | 39% | 0.24 |
| 51 | 0.65 | 2.14 | 9.68 | 0.17 |
| 52 | 0.59 | 7.0 | 24.0 | 0.15 |
| 53 | 0.30 | 11.1 | 7% | 0.15 |
| 54 | 11.3 | 0% | 0% | 0% |
| 55 | 0.15 | 0.12 | 0% | 0.18 |
| 56 | 16.7 | 3.13 | 0% | 0.738 |
| 57 | 1.4 | 0.15 | 0% | 0.22 |
| 58 | 0% | 1% | 1% | 4% |
| 59 | 0% | 0% | 2% | 7% |
| 60 | 0.901 | <0.04 | 5.65 | 0.192 |
| 61 | 1% | 0% | 0% | 10% |
| 62 | 5.6 | 4% | 10% | 2.2 |
| 63 | 28% | 6% | 0 | 7 |
| 64 | 0.78 | 2 | 19% | 0.07 |
| 65 | 0.36 | 2.8 | 12% | 0.3 |
| 66 | 0.34 | 40% | 0 | 0.28 |
| 67 | 0.20 | 4.25 | 0 | 0.14 |
| 68 | 32% | 0% | 6% | 4.8 |
| 69 | 0.06 | 2.6 | 2.6 | 0.07 |
| 73 | 0.03 | 4.4 | 0 | 0.1 |
| 74 | 3.25 | 1.78 | 13% | 0.15 |
| 75 | 0.21 | 2 | 9% | 0.04 |
| 76 | 0.12 | 0 | 0 | 0.28 |
| 77 | 2.52 | 0.98 | >30 | 1.13 |
| 78 | 0.43 | 03 | 16%@30 | 0.23 |
| 79 | 4.14 | 0.3 | 30%@30 | 0.15 |
| 80 | 1.29 | 4.55 | 29%@30 | 0.16 |
| 81 | 1 | 0.89 | 33%@30 | 0.22 |
| 82 | 20 | 4.67 | 32%@30 | 0.17 |
| 83 | 0.75 | 0.84 | >30 | 0.36 |
| 84 | 3.03 | 0.5 | >30 | 0.2 |
| 85 | 0.88 | 0.92 | 42%@30 | 0.19 |
| 86 | 0.07 | 0.9 | 26%@30 | 78%@1 |
| 87 | 0.07 | 1.5 | >30 | 0.26 |
| 88 | 0.1 | 15 | >30 | 0.27 |
| 89 | 5%@30 | 3%@30 | 5%@30 | 9%@30 |
| 90 | 0.02 | 8 | 16%@30 | 0.1 |
| 91 | 0.07 | 0.4 | 7 | 0.5 |
| 92 | 0.053 | 17.7 | 18%@30 | 0.25 |
| 93 | 0.07 | 15.4 | >30 | 0.16 |
| 94 | 0.04 | 48%@30 | >30 | 0.24 |
| 95 | 0.6 | 0.58 | >30 | 0.21 |
| 96 | 0.26 | 6.39 | >30 | 0.12 |
| 97 | 0.04 | 29%@30 | >30 | 0.24 |
| 98 | 0.09 | 20%@30 | 10%@30 | 0.07 |
| 99 | 6%@30 | 0%@30 | 8%@30 | 7%@30 |
| 100 | 1.6 | 0.1 | >30 | 62%@1 |
| 101 | 0.6 | 0.1 | >30 | 83%@1 |
| 102 | 1.2 | 1 | 43%@30 | 0.39 |
| 103 | 03 | 13 | 11%@30 | 0.45 |
| 104 | 1.2 | 19 | >30 | 0.7 |
| 105 | 0 | 17% | 5% | 0.49 |
| 106 | 1.11 | 28.8 | 30% | 0.25 |
| 107 | 0.57 | 48% | 35% | 0.24 |
| 108 | 4.13 | 16% | 20% | 0.5 |
| 109 | 6.17 | 46% | 10% | 0.22 |
| 1471-1 | 8.17 | 2.57 | 10% | 0.79 |
| 1471-2 | 3.62 | 86%@.04 | 10% | 0 |
| 1471-3 | 13.59 | 6.31 | 0 | 1.29 |
| 1471-4 | 29.4 | 183 | 0 | 3.1 |
| 1471-5 | 8.69 | 0.09 | 12% | 1.79 |
| 1471-6 | 2.85 | 63%@.04 | 14% | 0.68 |
| 1471-7 | 4.28 | 0.06 | 4% | 0.87 |
| 1471-8 | 3.86 | 70%@.04 | 27% | 0.65 |
| 1471-9 | 0.77 | 7.63 | 29% | 0.24 |
| 1471-10 | 0.6 | 69%@.04 | 45% | 0.18 |
| 1471-11 | 1.69 | 12.03 | 9% | 0.93 |
| 1471-12 | 7.97 | 37%@30 | 9% | 0.93 |
| 1471-13 | 1.47 | 0.33 | 0% | 0.53 |
| 1471-14 | 0.18 | 62%@.04 | 23 | 0.05 |
| 1471-15 | 0.63 | 0.16 | 10% | 0.24 |
| 1471-16 | 0.82 | 0.1 | 23% | 0.28 |
| 1471-17 | 11.01 | 3.76 | 16 | 0.24 |
| 1471-18 | 11.21 | 0.15 | 26% | 0.8 |
| 1471-19 | 33% | 16.86 | 26% | 1.6 |
| 1471-20 | 42% | 9.46 | 34% | 0.57 |
| 1471-21 | 8.18 | 0.08 | 30 | 0.24 |
| 1471-22 | 14.56 | 0.32 | 28% | 0.5 |
| 1471-23 | 8.88 | 0.2 | 36% | 0.28 |
| 1471-24 | 10.05 | 0.05 | 23 | 0.27 |
| 1471-25 | 2.45 | 24.47 | 31% | 0.36 |
| 1471-27 | 4.19 | 40% | 16% | 0.59 |
| 1471-28 | 7.22 | 26.99 | 8% | 0.39 |
| 1471-29 | 2.18 | 0.67 | 21% | 0.47 |
| 1471-30 | 0.83 | 0.13 | 41% | 0.08 |
| 1471-31 | 1.98 | 0.72 | 17% | 0.47 |
| 1471-32 | 1.19 | 0.17 | 25% | 0.23 |
| 1471-33 | 31% | 34% | 22% | 0.36 |
| 1471-34 | 17.47 | 0.74 | 24% | 0.25 |
| 1471-35 | 37% | 40% | 22% | 0.27 |
| 1471-36 | 11% | 15% | 6% | 0.74 |
| 1471-37 | 39% | 6.02 | 7% | 0.43 |
| 1471-38 | 2438 | 3.92 | 18% | 0.34 |
| 1471-39 | 23.45 | 4.06 | 11% | 0.29 |
| 1471-40 | 40% | 4.03 | 11% | 0.47 |
| 1471-41 | 18.33 | 25.8 | 34% | 0.33 |
| 1471-42 | 10.45 | 0.55 | 15% | 0.45 |
| 1471-43 | 25.26 | 34% | 18% | 0.44 |
| 1471-44 | 34% | 25% | 13% | 0.53 |
| 1471-45 | 13.22 | 1.19 | 24% | 0.27 |
| 1471-46 | 13.02 | 1.76 | 21% | 0.37 |
| 1471-47 | 12.59 | 2.24 | 22% | 0.41 |
| 1471-48 | 15.7 | 0.92 | 17% | 0.42 |
| 1471-57 | 4.73 | 0.9 | 11% | 0.67 |
| 1471-58 | 12.44 | 76%@.04 | 0% | 4.03 |
| 1471-59 | 7.52 | 1.95 | 0% | 1.03 |
| 1471-60 | 13.01 | 2.97 | 0% | 1.52 |
| 1471-61 | 5 | 0.04 | 0% | 1.2 |
| 1471-62 | 4% | 24% | 0% | >30 |
| 1471-63 | 3.45 | 56%@.04 | 2% | 0.84 |
| 1471-64 | 3.02 | 78%@.04 | 2% | 0.61 |
| 1471-67 | 12% | 27% | 16% | 0.82 |
| 1471-70 | 10% | 33% | 12% | 1.01 |
| 1471-71 | 6% | 24% | 0% | 2.58 |
| 1471-72 | 15% | 12.2 | 22% | 0.72 |
| 1507-01 | 6.3 | 18.9 | 16% | 0.9 |
| 1507-02 | 9.4 | 36% | 9% | 1.1 |
| 1507-03 | 3.6 | 21 | 21% | 0.8 |
| 1507-04 | 29.5 | 9.5% | >30 | 9%@1 |
| 1507-05 | 4.7 | 22 | 10% | |
| 1507-06 | 20 | 0.5 | >30 | 8%@1 |
| 1507-07 | 25% | 27 | >30 | 7%@1 |
| 1507-08 | 7% | 10% | 1% | |
| 1507-09 | 22.6 | 0.97 | 13% | 0.7 |
| 1507-10 | 24% | 39.5% | >30 | 7%@ 1 |
| 1507-11 | 3 | <0.04 | >30 | 27%@1 |
| 1507-12 | 6.7 | 0.6 | >30 | 12%@1 |
| 1507-13 | 26.7 | 6% | >30 | 8%@1 |
| 1507-15 | 12 | 25% | >30 | 11%@1 |
| 1507-16 | 5.8 | 21.3 | 18% | 1 |
| 1507-17 | 27.4 | 0.9 | 16% | 2.8 |
| 1507-18 | 10.6 | 10.6 | >30 | 6%@1 |
| 1507-19 | 12.9 | 45% | 4% | |
| 1507-20 | 20.9 | 10.5% | >30 | 11%@1 |
| 1507-21 | 0.4 | 4.6 | 23% | 0.36 |
| 1507-22 | 45% | 1.5% | >30 | 8%@1 |
| 1507-23 | 28.9 | 11 % | >30 | 11%@1 |
| 1507-24 | 0% | 7.5% | 15% | 25%@30 |
| 1507-25 | 1.6 | 22.4 | >30 | 60%@1 |
| 1507-26 | 20.5 | 11.5% | >30 | 11%@1 |
| 1507-28 | 38% | 1.5 | >30 | 1%@1 |
| 1507-29 | 5.8 | 27% | 4% | |
| 1507-30 | 13.4 | 18.5 | >30 | 0%@1 |
| 1507-31 | 3.7 | 21.5% | >30 | 2%@1 |
| 1507-32 | 21.3 | 16.5% | >30 | 0%@1 |
| 1507-33 | 28.4 | 8.5% | >30 | 2%@1 |
| 1507-34 | 8.5 | 20% | >30 | 0%@1 |
| 1507-35 | 19.9 | 15.9 | 28% | 0.49 |
| 1507-36 | 13.8 | 18% | 0% | |
| 1507-38 | 16 | 34.5% | >30 | 0%@1 |
| 1507-40 | 5.7 | 30 | 11% | |
| 1507-41 | 48% | 10.5% | 17% | 13.9 |
| 1507-42 | 11.6 | 23.5% | >30 | 0%@1 |
| 1507-43 | 4.1 | 7.1 | 21% | 0.97 |
| 1507-44 | 6 | 0.5 | 9% | |
| 1507-45 | 7.3 | 1 | >30 | 0%@1 |
| 1507-46 | 4.3 | 36% | 8% | |
| 1507-47 | 17.6 | 37% | 19% | 1.6 |
| 1507-48 | 183 | 14% | >30 | 11%@1 |
| 1512-01 | 18 | 18% | 4% | |
| 1512-02 | 19.2 | 19% | 3% | |
| 1512-04 | 10.8 | 17% | 8% | |
| 1512-05 | 16.7 | 1.7 | 10% | |
| 1512-06 | 12.6 | 23.6 | 4% | |
| 1512-07 | 3.6 | 30 | 4% | |
| 1512-11 | 30 | 22% | 5% | |
| 1512-12 | 11.3 | 10.5 | 5% | |
| 1512-14 | 3.2 | 27.9 | 7% | |
| 1512-15 | 20.9 | 23% | 6% | |
| 1512-16 | 6.5 | 34% | 10% | |
| 1512-17 | 39% | 6% | 4% | |
| 1512-19 | 14.3 | 17% | 3% | |
| 1512-20 | 11.2 | 9% | 8% | |
| 1512-23 | 14.6 | 34% | 6% | |
| 1512-26 | 26.9 | 7% | 5% | |
| 1512-27 | 16.5 | 13% | 3% | |
| 1512-29 | 47% | 8% | 4% | |
| 1512-31 | 43% | 9% | 14% | |
| 1512-32 | 21.9 | 10% | 5% | |
| 1512-35 | 22.5 | 23% | 4% | |
| 1512-36 | 3.8 | 48% | 9% | |
| 1512-39 | 6 | 47% | 4% | |
| 1512-40 | 40% | 9% | 3% | |
| 1512-41 | 30 | 4.4 | 4% | |
| 1512-42 | 25 | 15% | 1% | |
| 1512-43 | 11 | 27% | 9% | |
| 1512-44 | 43% | 20% | 6% | |
| 1512-45 | 27% | 16% | 11% | |
| 1512-46 | 11.7 | 27.2 | 10% | |
| 1512-47 | 3.5 | 0.3 | 13% | |
| 1515-01 | 7 | 25% | >30 | 2.3 |
| 1515-02 | 3.8 | 26% | >30 | 1.5 |
| 1515-03 | 1.9 | 40% | >30 | 2.9 |
| 1515-04 | 5.2 | 43% | >30 | 2.8 |
| 1515-05 | 4 | 9.8 | >30 | 0.9 |
| 1515-06 | 1.7 | 6.2 | >30 | 1.1 |
| 1515-07 | 14.2 | 3.8 | >30 | 1.2 |
| 1515-08 | 11 % | 9% | >30 | 2 |
| 1515-09 | 4.1 | 44% | >30 | 1.1 |
| 1515-10 | 5.7 | 38% | >30 | 1.1 |
| 1515-11 | 4.3 | 44% | >30 | 1.9 |
| 1515-12 | 6.3 | 9% | >30 | 8.2 |
| 1515-13 | 1.4 | 37% | >30 | 13 |
| 1515-14 | 2.9 | 7% | >30 | 4 |
| 1515-15 | 2.6 | 1% | >30 | 11.7 |
| 1515-16 | 8.8 | 3% | >30 | 17.3 |
| 1515-17 | 4.4 | 43% | >30 | 3.2 |
| 1515-18 | 0.6 | 4.4 | >30 | 1.1 |
| 1515-19 | 24.2 | 9.4 | >30 | 8.4 |
| 1515-20 | 32% | 36% | >30 | 1.2 |
| 1515-22 | 4.2 | 25% | >30 | 2.7 |
| 1515-23 | 6.2 | 18% | >30 | 8.5 |
| 1515-24 | 1.6 | 22% | >30 | 6.7 |
| 1515-25 | 2.9 | 3.5 | >30 | 1.4 |
| 1515-26 | 1.6 | 3.3 | >30 | 1.3 |
| 1515-27 | 1.1 | 2 | >30 | 2 |
| 1515-28 | 3 | 2.1 1 | >30 | 2.4 |
| 1515-29 | 5.2 | 1 | >30 | 2.2 |
| 1515-30 | 1.2 | 0.4 | >30 | 1.2 |
| 1515-31 | 8.1 | 0.1 1 | >30 | 1.2 |
| 1515-32 | 25% | 6.3 | >30 | 1.4 |
| 1515-33 | 2.3 | 1.1 | >30 | 1 |
| 1515-34 | 4.4 | 2.4 | >30 | 1.3 |
| 1515-35 | 1.8 | 1.2 | >30 | 1.1 |
| 1515-36 | 2.7 | 8.6 | >30 | 5.4 |
| 1515-37 | 3.6 | 4 | >30 | 1.6 |
| 1515-38 | 1.8 | 2.5 | >30 | 1.4 |
| 1515-39 | 1.6 | 1.7 | >30 | 2.3 |
| 1515-40 | 4.4 | 1.7 | >30 | 2.4 |
| 1515-41 | 2.6 | 0.4 | >30 | 1 |
| 1515-42 | 1.8 | 0.5 | >30 | 1.4 |
| 1515-43 | 6.8 | 0.1 | >30 | 1.1 |
| 1515-44 | 30% | 7.7 | >30 | 1.4 |
| 1515-45 | 2.9 | 1 | >30 | 1.2 |
| 1515-46 | 4.8 | 2 | >30 | 13 |
| 1515-47 | 3.1 | 1.6 | >30 | 1.8 |
| 1515-48 | 4.1 | 6.2 | >30 | 4.1 |
| 1517-01 | 1.2 | 1.8 | 0 | 0.38 |
| 1517-03 | 9 | 36% | 0 | 2 |
| 1517-04 | 5.7 | 14.5 | 0 | 4.5 |
| 1517-05 | 4.6 | 29.8 | 0 | 1.2 |
| 1517-06 | 2.5 | 11.9 | 0 | 1.6 |
| 1517-07 | 0.4 | 1.1 | 0 | 0.32 |
| 1517-08 | 43% | 30% | 0 | 3.8 |
| 1517-09 | 2.6 | 30% | 0 | 1.5 |
| 1517-10 | 2 | 38% | 0 | 2.1 |
| 1517-11 | 3 | 22% | 0 | 1.9 |
| 1517-12 | 0.8 | 18.1 | 0 | 2.1 |
| 1517-13 | 0.6 | 0.1 | 0 | 0.28 |
| 1517-14 | 30% | 20% | 0 | 8.4 |
| 1517-15 | 3.1 | 5.3 | 0 | 1.5 |
| 1517-16 | 3.4 | 26.1 | 0 | 5.1 |
| 1517-17 | 1.8 | 4.1 | 0 | 0.85 |
| 1517-18 | 1.3 | 1 | 0 | 0.98 |
| 1517-19 | 0.9 | 0.1 | 0 | 0.4 |
| 1517-20 | 22% | 25% | 0 | 7.3 |
| 1517-23 | 2.3 | 4.3 | 0 | 1.4 |
| 1517-24 | 1.8 | 1 | 0 | 0.84 |
| 1517-25 | 1.4 | 16.5 | 0 | 0.72 |
| 1517-26 | 1.8 | 2.1 | 0 | 0.32 |
| 1517-29 | 0.9 | 1.2 | 0 | 03 |
| 1517-31 | 9.1 | 19.9 | 0 | 0.69 |
| 1517-33 | 0.4 | 5.2 | 0 | 0.21 |
| 1517-35 | 33% | 9% | 0 | 23.6 |
| 1517-36 | 3 | 28.1 | 0 | 0.61 |
| 1517-37 | 0.4 | 3.7 | 0 | 0.28 |
| 1517-38 | 0.7 | 8.7 | 0 | 0.33 |
| 1517-39 | 0.7 | 20.4 | 0 | 0.43 |
| 1517-40 | 1.2 | 6.4 | 0 | 0.37 |
| 1517-41 | 2.2 | 16.6 | 0 | 0.59 |
| 1517-43 | 25% | 4% | 0 | 43% |
| 1517-44 | 0.2 | 4 | 0 | 0.22 |
| 1517-46 | 0.4 | 11.3 | 0 | 0.36 |
| 1517-47 | 9.8 | 5.2 | 0 | 0.65 |
| 1517-48 | 1.2 | 2.4 | 0 | 0.29 |
| 1522-02 | 0.5 | 20 | >30 | 0.2 |
| 1522-05 | 0.6 | 10 | >30 | 0.2 |
| 1522-06 | 5.5 | 20 | >30 | 03 |
| 1522-07 | 0.47 | 1 | >30 | 0.15 |
| 1522-08 | 0.27 | 1 | >30 | 0.1 |
| 1522-09 | 0.2 | 3 | >30 | 0.2 |
| 1522-11 | 0.43 | 1 | >30 | 0.2 |
| 1522-12 | 0.81 1 | 8 | >30 | 0.3 |
| 1522-13 | 0.75 | 8 | >30 | 0.2 |
| 1522-14 | 0.84 | 8 | >30 | 0.2 |
| 1522-15 | 0.54 | 4 | >30 | 0.2 |
| 1522-17 | 0.62 | 1 | >30 | 0.35 |
| 1522-18 | 1.7 | 20 | >30 | 0.2 |
| 1522-19 | 13 | >30 | >30 | 0.3 |
| 1522-20 | 5 | >30 | >30 | 0.2 |
| 1522-21 | 0.72 | 3 | >30 | 0.2 |
| 1522-23 | 2.2 | 20 | >30 | 0.3 |
| 1522-27 | 6.3 | 20 | >30 | 0.35 |
| 1522-28 | >30 | >30 | >30 | 2 |
| 1522-31 | 6.4 | 8 | >30 | 0.35 |
| 1522-33 | 0.88 | 10 | >30 | 0.2 |
| 1522-34 | 0.5 | 8 | >30 | 0.2 |
| 1522-35 | 4 | 10 | >30 | 0.35 |
| 1522-36 | 1 | 10 | >30 | 0.3 |
| 1522-37 | 2 | 0.08 | >30 | 0.3 |
| 1522-38 | 1 | 0.1 | >30 | 0.3 |
| 1522-40 | 0.5 | 0.8 | >30 | 0.2 |
| 1522-41 | 0.5 | 10 | >30 | 0.2 |
| 1522-42 | 1 | 2 | >30 | 0.15 |
| 1522-43 | 0.8 | 20 | >30 | 0.3 |
| 1522-44 | 0.8 | 10 | >30 | 0.2 |
| 1522-46 | 1 | 10 | >30 | 0.2 |
| 1522-47 | 3 | 15 | >30 | 2 |
| 1526-01 | 0.02 | 13.13 | >30 | 0.15 |
| 1526-03 | 0.06 | 173 | >30 | 0.15 |
| 1526-04 | 0.03 | 18.08 | >30 | 0.16 |
| 1526-05 | 0.1 | 15 | >30 | 0.19 |
| 1526-06 | 0.17 | 12.04 | >30 | 0.21 |
| 1526-07 | 0.08 | 20.91 | >30 | 0.19 |
| 1526-09 | 0.03 | 11.23 | >30 | 0.14 |
| 1526-11 | 0.05 | 3.21 | >30 | 0.15 |
| 1526-12 | 0.04 | 28.41 | >30 | 0.2 |
| 1526-13 | 0.06 | 22.42 | >30 | 0.2 |
| 1526-14 | 0.04 | 7.63 | >30 | 0.14 |
| 1526-15 | 0.06 | 6.88 | >30 | 0.15 |
| 1526-16 | 0.04 | 5.6 | >30 | 0.13 |
| 1526-17 | 0.08 | 2.21 | >30 | 0.17 |
| 1526-19 | 0.04 | 16.97 | >30 | 0.15 |
| 1526-21 | 0.05 | 20.03 | >30 | 0.18 |
| 1526-23 | 0.03 | 9.45 | >30 | 0.15 |
| 1526-24 | 0.04 | 11.06 | >30 | 0.17 |
| 1526-25 | 0.14 | 40% | >30 | 0.19 |
| 1526-26 | 0.06 | 14.27 | >30 | 0.16 |
| 1526-29 | 0.12 | 50% | >30 | 0.22 |
| 1526-30 | 0.08 | 21.42 | >30 | 0.2 |
| 1526-33 | 0.26 | 29% | >30 | 0.24 |
| 1526-40 | 0.1 | 17.85 | >30 | 0.22 |
| 1526-41 | 0.07 | 24.04 | >30 | 0.16 |
| 1543-03 | 0.64 | 26% | 34% | 0.06 |
| 1543-05 | 0.06 | 27% | 14% | 0.07 |
| 1543-07 | 0.21 | 24.56 | 19% | 0.09 |
| 1543-09 | 0.69 | 14% | 12% | 0.18 |
| 1543-11 | 0.07 | 16.56 | 24% | 0.04 |
| 1543-13 | 0.09 | 41% | 12% | 0.06 |
| 1543-15 | 0.95 | 45% | 41% | 0.14 |
| 1543-19 | 0.22 | 37% | 11% | 0.08 |
| 1543-21 | 0.29 | 17.12 | 27% | 0.06 |
| 1543-25 | 0.28 | 8.12 | 26% | 0.05 |
| 1543-27 | 0.38 | 24.6 | 27% | 0.06 |
| 1543-31 | 0.04 | 24.26 | 8% | 0.06 |
| 1543-33 | 0.03 | 19.34 | 14% | 0.05 |
| 1543-34 | 0.08 | 17.32 | 20% | 0.06 |
| 1543-35 | 0.07 | 23.61 | 8% | 0.07 |
| 1543-36 | 0.08 | 12.57 | 15% | 0.05 |
| 1543-37 | 1.23 | 16% | 16% | 0.12 |
| 1543-38 | 0.09 | 18.26 | 14% | 0.06 |
| 1543-39 | 0.04 | 12.77 | 14% | 0.05 |
| 1543-40 | 0.04 | 11.45 | 10% | 0.04 |
| 1543-41 | 3% | 0% | 9% | 6% |
| 1543-45 | 1% | 0% | 5% | 8% |
| 1543-46 | 22% | 0% | 11 % | 18% |

**Table 9.**

| Inhibitory Activity of Pyrazinones toward Factor Xa, TF-VIIA, Thrombin II, and Trypsin II. | | | | |
|---|---|---|---|---|
| **Example** **Number** | **%** **Inhibition** **TF-VIIa** **(100 υM)** | **%** **Inhibition** **Thrombin** **II (100 υM)** | **%** **Inhibition** **Factor Xa** **(100 υM)** | **%** **Inhibition** **Trpysin II** **(100 υM)** |
| E-0001 | 0 | 0 | 1 | 0 |
| E-0002 | 0 | 0 | 0 | 0 |
| E-0003 | 0 | 10 | 0 | 0 |
| E-0004 | 0 | 6 | 0 | 0 |
| E-0005 | 0 | 0 | 0 | 0 |
| E-0006 | 2 | 0 | 0 | 1 |
| E-0007 | 0 | 0 | 0 | 0 |
| E-0008 | 0 | -0.2 | 0 | 0 |
| E-0009 | 0 | 8 | 1 | 1 |
| E-0010 | 0 | 5 | 0 | 0 |
| E-0011 | 0 | 0 | 0 | 0 |
| E-0012 | 0 | 0 | 0 | 0 |
| E-0013 | 0 | 2 | 0 | 1 |
| E-0014 | 0 | 6 | 0 | 0 |
| E-0015 | 0 | 3 | 0 | 3 |
| E-0016 | 0 | 10 | 0 | 4 |
| E-0017 | 0 | 10 | 0 | 1 |
| E-0018 | 1 | 10 | 0 | 4 |
| E-0019 | 0 | 9 | 0 | 2 |
| E-0020 | 0 | 13 | 1 | 4 |
| E-0021 | 0 | 9 | 1 | 5 |
| E-0022 | 0 | 12 | 0 | 2 |
| E-0023 | 0 | 5 | 0 | 1 |
| E-0024 | 0 | 0 | 0 | 1 |
| E-0025 | 0 | 13 | 0 | 3 |
| E-0026 | 0 | 13 | 0 | 3 |
| E-0027 | 0 | 10 | 0 | 2 |
| E-0028 | 0 | 8 | 0 | 3 |
| E-0029 | 0 | 8 | 0 | 2 |
| E-0030 | 0 | 8 | 0 | 4 |
| E-0031 | 0 | 4 | 0 | 1 |
| E-0032 | 0 | 6 | 0 | 3 |
| E-0033 | 0 | 6 | 0 | 5 |
| E-0034 | 0 | 7 | 6 | 3 |
| E-0035 | 4 | 12 | 0 | 2 |
| E-0036 | 0 | 1 | 0 | 0 |
| E-0037 | 0 | 5 | 0 | 2 |
| E-0038 | 0 | 9 | 0 | 3 |
| E-0039 | 0 | 9 | 1 | 2 |
| E-0040 | 0 | 7 | 0 | 4 |
| E-0041 | 0 | 8 | 0 | 2 |
| E-0042 | 0 | 8 | 0 | 5 |
| E-0043 | 0 | 12 | 0 | 3 |
| E-0044 | 0 | 9 | 0 | 3 |
| E-0045 | 0 | 7 | 0 | 4 |
| E-0046 | 0 | 7 | 0 | 4 |
| E-0047 | 0 | 9 | 0 | 2 |
| E-0048 | 0 | 1 | 0 | 0 |
| E-0049 | 2 | 0 | 0 | 0 |
| E-0050 | 0 | 0 | 0 | 0 |
| E-0051 | 0 | 0 | 0 | 0 |
| E-0052 | 0 | 0 | 0 | 0 |
| E-0053 | 0 | 0 | 0 | 0 |
| E-0054 | 0 | 0 | 0 | 0 I |
| E-0055 | 0 | 0 | 0 | 0 |
| E-0056 | 0 | 0 | 0 | 0 |
| E-0057 | 0 | 0 | 6 | 0 |
| E-0058 | 0 | 0 | 0 | 0 |
| E-0059 | 0 | 0 | 0 | 0 |
| E-0060 | 0 | 0 | 0 | 0 |
| E-0061 | 0 | 0 | 0 | 0 |
| E-0062 | 0 | 0 | 0 | 0 |
| E-0063 | 0 | 0 | 0 | 0 |
| E-0064 | 0 | 0 | 0 | 0 |
| E-0065 | 0 | 0 | 0 | 0 |
| E-0066 | 0 | -0.2 | 0 | 0 |
| E-0067 | 0 | 1 | 0 | 0 |
| E-0068 | 0 | 3 | 0 | 0 |
| E-0069 | 0 | 0 | 0 | 0 |
| E-0070 | 0 | 0 | 0 | 0 |
| E-0071 | 0 | 0 | 0 | 0 |
| E-0072 | 0 | 0 | 0 | 0 |
| E-0073 | 0 | 0 | 2 | 6 |
| E-0074 | 0 | 0 | 0 | 8 |
| E-0075 | 0 | 0 | 0 | 7 |
| E-0076 | 0 | 0 | 1 | 10 |
| E-0077 | 0 | 0 | 3 | 7 |
| E-0078 | 0 | 3 | 1 | 10 |
| E-0079 | 0 | 0 | 4 | 11 |
| E-0080 | 1 | 0 | 4 | 5 |
| E-0081 | 0 | 24 | 5 | 8 |
| E-0082 | 4 | 0 | 4 | 3 |
| E-0083 | 2 | 53 | 3 | 6 |
| E-0084 | 0 | 0 | 0 | 8 |
| E-0085 | 0 | 5 | 0 | 9 |
| E-0086 | 0 | 0 | 4 | 11 |
| E-0087 | 0 | 0 | 0 | 10 |
| E-0088 | 0 | 0 | 3 | 9 |
| E-0089 | 3 | 0 | 4 | 8 |
| E-0090 | 0 | 0 | 2 | 11 |
| E-0091 | 1 | 0 | 4 | 8 |
| E-0092 | 1 | 0 | 3 | 9 |
| E-0093 | 0 | 14 | 0 | 10 |
| E-0094 | 2 | 0 | 2 | 7 |
| E-0095 | 2 | 0 | 4 | 9 |
| E-0096 | 0 | 5 | 0 | 10 |
| E-0097 | 0 | 0 | 3 | 11 |
| E-0098 | 0 | 0 | 2 | 8 |
| E-0099 | 0 | 0 | 1 | 10 |
| E-0100 | 7 | 0 | 6 | 12 |
| E-0101 | 11 | 2 | 10 | 11 |
| E-0102 | 0.4 | 0 | 3 | 13 |
| E-0103 | 2 | 0 | 3 | 11 |
| E-0104 | 3 | 0 | 5 | 9 |
| E-0105 | 0 | 0 | 3 | 12 |
| E-0106 | 5 | 0 | 3 | 9 |
| E-0107 | 4 | 0 | 6 | 12 |
| E-0108 | 0 | 0 | 4 | 12 |
| E-0109 | 2 | 0 | 3 | 12 |
| E-0110 | 0 | 0 | 3 | 14 |
| E-0111 | 4 | 0 | 1 | 14 |
| E-0112 | 11 | 0 | 1 | 13 |
| E-0113 | 14 | 0 | 3 | 11 |
| E-0114 | 10 | 0 | 3 | 14 |
| E-0115 | is | 0 | 3 | 11 |
| E-0116 | 13 | 0 | 4 | 10 |
| E-0117 | 9 | 0 | 1 | 9 |
| E-0118 | 12 | 0 | 3 | 9 |
| E-0119 | 13 | 0 | 5 | 10 |
| E-0120 | 8 | 0 | 1 | 9 |
| E-0121 | 0 | 8 | 0.1 | 0 |
| E-0122 | 0 | 8 | 0.1 | 0 |
| E-0123 | 0 | 6 | 0.1 | 1 |
| E-0124 | 0 | 6 | 0.1 | 0 |
| E-0125 | 0 | 4 | 0.1 | 0 |
| - E-0126 | 0 | 4 | 0.1 | 0 |
| E-0127 | 0 | 5 | 0.1 | 0 |
| E-0128 | 0 | 7 | 0.1 | 0 |
| E-0129 | 0 | 5 | 0.1 | 0 |
| E-0130 | 0 | 2 | 0.1 | 0 |
| E-0131 | 0 | 0 | 0.1 | 1 |
| E-0132 | 0 | 0 | 0.1 | 0 |
| E-0133 | 0 | 5 | 0.1 | 0 |
| E-0134 | 0 | 5 | 0.1 | 1 |
| E-0135 | 0 | 3 | 0.1 | 2 |
| E-0136 | 0 | 3 | 0.1 | 1 |
| E-0137 | 2 | 3 | 0.1 | 0 |
| E-0138 | 1 | 4 | 0.1 | 3 |
| E-0139 | 0 | 4 | 0.1 | 3 |
| E-0140 | 0 | 4 | 0.1 | 2 |
| E-0141 | 0 | 4 | 0.1 | 2 |
| E-0142 | 1 | 5 | 0.1 | 3 |
| E-0143 | 1 | 2 | 0.1 | 1 |
| E-0144 | 0 | 0 | 0.1 | 1 |
| E-0145 | 0 | 5 | 0.1 | 0 |
| E-0146 | 0 | 8 | 0.1 | 0 |
| E-0147 | 0 | 3 | 0.1 | 1 |
| E-0148 | 0 | 5 | 0.1 | 3 |
| E-0149 | 0 | 4 | 0.1 | 0 |
| E-0150 | 0 | 6 | 0.1 | 2 |
| E-0151 | 0 | 6 | 0.1 | 3 |
| E-0152 | 0 | 6 | 0.1 | 4 |
| E-0153 | 0 | 3 | 0.1 | 1 |
| E-0154 | 0 | 5 | 0.1 | 3 |
| E-0155 | 2 | 6 | 0.1 | 4 |
| E-0156 | 0 | -0.4 | 0.1 | 1 |
| E-0157 | 0 | 5 | 0.1 | 0 |
| E-0158 | 0 | 3 | 0.1 | 6 |
| E-0159 | 0 | 4 | 0.1 | 1 |
| E-0160 | 0 | 6 | 0.1 | 2 |
| E-0161 | 0 | 6 | 0.1 | 1 |
| E-0162 | 0 | 7 | 0.1 | 4 |
| E-0163 | 0 | 5 | 0.1 | 0 |
| E-0164 | 0 | 5 | 0.1 | 0 |
| E-0165 | 0 | 7 | 0.1 | 0 |
| E-0166 | 0 | 6 | 0.1 | 1 |
| E-0167 | 0 | 4 | 0.1 | 1 |
| E-0168 | 7 | 5 | 0.1 | 0 |
| E-0169 | 0 | 2 | 0.1 | 1 |
| E-0170 | 0 | 7 | 0.1 | 0 |
| E-0171 | 0 | 9 | 0.1 | 2 |
| E-0172 | 0 | 6 | 0.1 | 0 |
| E-0173 | 0 | 5 | 0.1 | 1 |
| E-0174 | 0 | 5 | 0.1 | 1 |
| E-0175 | 0 | 6 | 0.1 | 2 |
| E-0176 | 0 | 7 | 0.1 | 0 |
| E-0177 | 0 | 6 | 0.1 | 0 |
| E-0178 | 0 | 3 | 0.1 | 2 |
| E-0179 | 0 | 10 | 0.1 | 0 |
| E-0180 | 0 | 3 | 0.1 | 0 |
| E-0181 | 0 | 5 | 0.1 | 3 |
| E-0182 | 0 | 5 | 0.1 | 0 |
| E-0183 | 0 | 5 | 0.1 | 2 |
| E-0184 | 0 | 2 | 0.1 | 0 |
| E-0185 | 0 | 3 | 0.1 | 2 |
| E-0186 | 0 | 5 | 0.1 | 0 |
| E-0187 | 0 | 8 | 0.1 | 1 |
| E-0188 | 0 | 2 | 0.1 | 8 |
| E-0189 | 0 | 6 | 0.1 | 1 |
| E-0190 | 0 | 4 | 0.1 | 3 |
| E-0191 | 0 | 6 | 0.1 | 0 |
| E-0192 | 0 | 0 | 0.1 | 0 |
| E-0193 | 0 | 5 | 0.1 | 0 |
| E-0194 | 0 | 14 | 0.2 | 0 |
| E-0195 | 0 | 1 | 0.1 | 0 |
| E-0196 | 0 | 1 | 0.1 | 0 |
| E-0197 | 0 | 3 | 0.1 | 1 |
| E-0198 | 0 | 0 | 0.1 | 3 |
| E-0199 | 0 | 1 | 0.1 | 2 |
| E-0200 | 0 | 33 | 0.2 | 2 |
| E-0201 | 0 | 1 | 0.1 | 1 |
| E-0202 | 0 | 1 | 0.1 | 1 |
| E-0203 | 0 | 5 | 0.1 | 2 |
| E-0204 | 0 | 1 | 0.1 | 1 |
| E-0205 | 0 | 1 | 0.1 | 2 |
| E-0206 | 0 | 2 | 0.1 | 1 |
| E-0207 | 0 | 2 | 0.1 | 1 |
| E-0208 | 0 | 4 | 0.1 | 1 |
| E-0209 | 1 | 3 | 0.1 | 3 |
| E-0210 | 0 | 6 | 0.1 | 2 |

## Claims

1. A compound having the Formula: 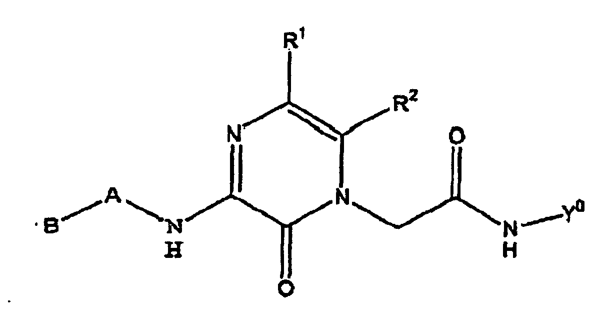 or a pharmaceutically acceptable salt thereof, wherein;
B is selected from the group consisting of:
(i) aryl and heteroaryl wherein a carbon adjacent to the carbon at the point of attachment is optionally substituted by R³², the other carbon adjacent to the carbon at the point of attachment is optionally substituted by R³⁶, a carbon adjacent to R³² and two atoms from the carbon at the point of attachment is optionally substituted by R³³, a carbon adjacent to R³⁶ and two atoms from the carbon at the point of attachment is optionally substituted by R³⁵, and any carbon adjacent to both R³³ and R³⁵ is optionally substituted by R³⁴;
(ii) hydrido, C2-C8 alkyl, C3-C8 alkenyl, C3-C8 alkynyl, and C2-C8 haloalkyl, wherein each member of group B is optionally substituted at any carbon up to and including 6 atoms from the point of attachment of B to A with one or more of the group consisting of R³², R³³, R³⁴, R³⁵, and R³⁶; and
(iii) C3-C7 cycloalkyl and C4-C6 saturated heterocyclyl, wherein each ring carbon is optionally substituted with R³³, a ring carbon other than the ring carbon at the point of attachment of B to A is optionally substituted with oxo provided that no more than one ring carbon is substituted by oxo at the same time, ring carbons and a nitrogen adjacent to the carbon atom at the point of attachment are optionally substituted with R⁹ or R¹³, a ring carbon or nitrogen adjacent to the R⁹ position and two atoms from the point of attachment is optionally substituted with R¹⁰, a ring carbon or nitrogen adjacent to the R¹³ position and two atoms from the point of attachment is optionally substituted with R¹², a ring carbon or nitrogen three atoms from the point of attachment and adjacent to the R¹⁰ position is optionally substituted with R¹¹, a ring carbon or nitrogen three atoms from the point of attachment and adjacent to the R¹² position is optionally substituted with R³³, and a ring carbon or nitrogen four atoms from the point of attachment and adjacent to the R¹¹ and R³³ positions is optionally substituted with R³⁴;
R³², R³³, R³⁴, R³⁵, and R³⁶ are independently selected from the group consisting of hydrido, acetamido, haloacetamido, amidino, guanidino, alkoxy, hydroxy, amino, alkoxyamino, lower alkylamino, alkylthio, amidosulfonyl, monoalkyl amidosulfonyl, dialkyl amidosulfonyl, alkyl, halo, haloalkyl, haloalkoxy, hydroxyalkyl, carboalkoxy, carboxy, carboxamido, and cyano;
A is selected from the group consisting of single covalent bond and (CH(R¹⁵))ₚₐ-(W⁷)ᵣᵣ wherein rr is an integer selected from 0 through 1, pa is an integer selected from 0 through 3, and W⁷ is N(R⁷);
R⁷ is selected from the group consisting of hydrido and alkyl;
R¹⁵ is selected from the group consisting of hydrido, halo, alkyl, and haloalkyl;
R¹ is selected from the group consisting of hydrido, cyano, haloalkyl, and halo;
R² is Z⁰-Q;
Z⁰ is a covalent single bond;
Q is phenyl wherein a carbon two atoms from the carbon at the point of attachment is optionally substituted by amino;
R⁹, R¹¹, and R¹³ are independently selected from the group consisting of hydrido, hydroxy, amino, amidino, guanidino, lower alkylamino, alkylthio, alkoxy, alkylsulfinyl, alkylsulfonyl, amidosulfonyl, monoalkylamidosulfonyl, alkyl, halo, haloalkyl, haloalkoxy, hydroxyalkyl, carboxy, carboxamido, and cyano;
R¹⁰ and R¹² are independently selected from the group consisting of hydrido, acetamido, haloacetamido, amidino, guanidino, alkyl, alkoxy, alkoxyamino, aminoalkyl, hydroxy, amino, lower alkylamino, alkylsulfonamido, amidosulfonyl, monoalkyl amidosulfonyl, dialkyl amidosulfonyl, hydroxyalkyl, aminoalkyl, halo, haloalkyl, carboalkoxy, carboxy, carboxyamido, carboxyalkyl, and cyano;
Y⁰ is formula (IV): 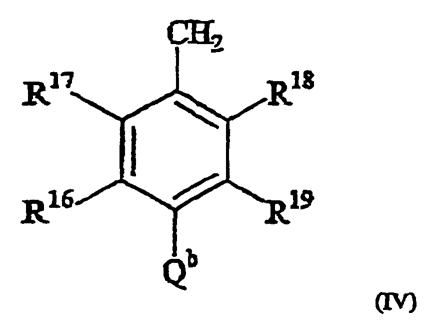 wherein
R¹⁶, R¹⁷, R¹⁸, and R¹⁹ are independently selected from the group consisting of hydrido, amidino, guanidino, carboxy, haloalkylthio, alkoxy, hydroxy, amino, lower alkylamino, alkylthio, alkylsulfinyl, alkylsulfonyl, alkanoyl, haloalkanoyl, alkyl, halo, haloalkyl, haloalkoxy, hydroxyalkyl, aminoalkyl, and cyano;
Q^{b} is C(NR²⁵)NR²³R²⁴;
R²³, R²⁴, and R²⁵ are hydrido;
and wherein
the term "alkyl", either alone or within other terms, unless indicated otherwise, means an acyclic alkyl radical containing from one to ten carbon atoms;
the term "alkoxy" means a linear or branched oxy-containing radical having an alkyl portion of one to ten carbon atoms;
the term "aryl" alone or in combination, means a carbocyclic aromatic system containing one, two or three rings wherein such rings may be attached together in a pendant manner or may be fused;
the term "heterocyclyl" means saturated and partially saturated heteroatom-containing ring-shaped radicals having from 4 through 15 ring members selected from carbon, nitrogen, sulfur and oxygen, wherein at least one ring atom is a heteroatom;
the term "heteroaryl" means fully unsaturated heteroatom-containing ring-shaped aromatic radicals having from 4 through 15 ring members selected from carbon, nitrogen, sulfur and oxygen, wherein at least one ring atom is a heteroatom.

2. The compound as recited in Claim 1 or a pharmaceutically acceptable salt thereof, wherein;
B is selected from the group consisting of phenyl, 2-thienyl, 3-thienyl, 2-furyl, 3-furyl, 2-pyrrolyl, 3-pyrrolyl, 2-imidazolyl, 4-imidazolyl, 3-pyrazolyl, 4-pyrazolyl, 2-thiazolyl, 3-isoxazolyl, and 5-isoxazolyl, wherein a carbon adjacent to the carbon at the point of attachment is optionally substituted by R³², the other carbon adjacent to the carbon at the point of attachment is optionally substituted by R³⁶, a carbon adjacent to R³² and two atoms from the carbon at the point of attachment is optionally substituted by R³³, a carbon adjacent to R³⁶ and two atoms from the carbon at the point of attachment is optionally substituted by R³⁵, and any carbon adjacent to both R³³ and R³⁵ is optionally substituted by R³⁴;
R³², R³³, R³⁴, R³⁵, and R³⁶ are independently selected from the group consisting of hydrido, amidino, guanidino, methyl, ethyl, methoxy, ethoxy, hydroxy, amino, N-methyl amino, dimethyl amino, methylthio, ethylthio, trifluoromethyl, pentafluoroethyl, 2,2,2-trifluoroethyl, fluoro, chloro, bromo, amidosulfonyl, N-methylamidosulfonyl, hydroxymethyl, amidocarbonyl, carboxy, and cyano;
A is selected from the group consisting of single covalent bond, NH, N(CH₃), CH₂, CH₃CH, and CH₂CH₂;
R¹ is selected from the group consisting of hydrido, trifluoromethyl, pentafluoroethyl, fluoro, and chloro;
R⁹, R¹¹, and R¹³ are independently selected from the group consisting of hydrido, methyl, ethyl, methoxy, ethoxy, hydroxy, amino, N-methylamino, N,N-dimethylamino, methylthio, trifluoromethyl, pentafluoroethyl, 2,2,2-trifluoroethyl, fluoro, chloro, bromo,
amidosulibnyl, N-methylamidosulfonyl, N,N-dimethylamidosulfonyl, hydroxymethyl, 1-hydroxyethyl, amidocarbonyl, N-methylamidocarbonyl, carboxy, and cyano;
R¹⁰ and R¹² are independently selected from the group consisting of hydrido, amidino, amidocarbonyl, N-methylamidocarbonyl, guanidino, methyl, ethyl, methoxy, ethoxy, hydroxy, hydroxymethyl, 1-hydroxyethyl, 2-hydroxyethyl, carboxy, carboxymethyl, amino, acetamido, trifluoromethyl, pentafluoroethyl, 2,2,2-trifluoroethyl, trifluoroacetamido, aminomethyl, N-methylamino, dimethylamino, amidosulfonyl, N-methylamidosulfonyl N,N-dimethylamidosulfonyl, methoxycarbonyl, fluoro, chloro, bromo, and cyano;
Y⁰ is 1-Q^{b}-4-Q^{s}-2-R¹⁶-3-R¹⁷-5-R¹⁸-6-R¹⁹benzene;
R¹⁶, R¹⁷, R¹⁸, and R¹⁹ are independently selected from the group consisting of hydrido, methyl, ethyl, amidino, guanidino, methoxy, hydroxy, amino, aminomethyl, 1-aminoethyl, 2-aminoethyl, N-methylamimo, dimethylamino, methylthio, ethylthio, trifluoromethylthio, methylsulfinyl, methylsulfonyl, trifluoromethyl, pentafluoroethyl, 2,2,2-trifluoroethyl, trifluoromethoxy, fluoro, chloro, amidosulfonyl, N-methylamidosulfonyl, hydroxymethyl, carboxy, and cyano.

3. The compound as recited in Claim 1 or a pharmaceutically acceptable salt thereof, wherein;
B is selected from the group consisting of 2-aminophenyl, 3-aminophenyl, 3-amidinophenyl, 4-amidinophenyl, 3-carboxyphenyl, 3-carboxy-5-hydroxyphenyl, 3-chlorophenyl, 4-chlorophenyl, 3,4-dichlorophenyl, 2-fluorophenyl, 3-fluorophenyl, 3,4-difluorophenyl, 3-hydroxyphenyl, 4-hydroxyphenyl, 3-methoxyaminophenyl, 3-methoxyphenyl, 4-methoxyphenyl, 3-methylphenyl, 4-methylphenyl, phenyl, 3-trifluoromethylphenyl, 2-imidazoyl, 2-pyridyl, 3-pyridyl, 5-chloro-3-trifluoromethyl-2-pyridyl, 4-pyridyl, 2-thienyl, 3-thienyl, and 3-trifluoromethyl-2-pyridyl;
A is selected from the group consisting of CH₂, CH₃CH, CF₃CH, NHC(O), CH₂CH₂,and CH₂CH₂CH₂;
R¹ is selected from the group consisting of hydrido, trifluoromethyl, pentafluoroethyl, fluoro, and chloro;
Y⁰ is 1-Q^{b}-4-Q^{s}-2-R¹⁶-3-R¹⁷-5-R¹⁸-6-R¹⁹benzene;
R¹⁶ and R¹⁹ are independently selected from the group consisting of:
(i) hydrido, amidino, amino, aminomethyl, methoxy, methylamino, hydroxy, hydroxymethyl fluoro, chloro, and cyano; and
(ii) Q^{b} with the proviso that no more than one of R¹⁶ and R¹⁹ is Q^{b} at the same time;
R¹⁷ and R¹⁸ are independently selected from the group consisting of hydrido, fluoro, chloro, hydroxy, hydroxymethyl, amino, carboxy, and cyano.

4. The compound as recited in Claim 3 or a pharmaceutically acceptable salt thereof, wherein;
B is selected from the group consisting of 3-aminophenyl, 3-amidinophenyl, 4-amidinophenyl, 3-chlorophenyl, 4-chlorophenyl, 3,4-dichlorophenyl, 2-fluorophenyl, 4-methylphenyl, phenyl, 2-imidazoyl, 3-pyridyl, 4-pyridyl, and 3-trifluoromethyl-2-pyridyl;
A is selected from the group consisting of CH₂, NHC(O), CH₂CH₂,and CH₂CH₂CH₂;
R¹ is selected from the group consisting of hydrido and chloro;
Y⁰ is selected from the group consisting of 4-amidinobenzyl, 2-fluoro-4-amidinobenzyl, and 3-fluoro-4-amdinobenzyl.

5. A compound as recited in Claim 1 or a pharmaceutically acceptable salt thereof, wherein:
R² is 3-aminophenyl, B is 3-chlorophenyl, A is CH₂CH₂, Y⁰ is 4-amidinobenzyl, and R¹ is chloro;
R² is 3-aminophenyl, B is phenyl, A is CH₂, Y⁰ is 4-amidinobenzyl, and R¹ is chloro;
R² is 3-aminophenyl, B is 2-imidazoyl, A is CH₂CH₂CH₂, Y⁰ is 4-amidinobenzyl, and R¹ is chloro.

6. The compound as recited in Claim 1 or a pharmaceutically acceptable salt thereof, wherein;
B is selected from the group consisting of hydrido, ethyl, 2-propenyl, 2-propynyl, propyl, isopropyl, butyl, 2-butenyl, 2-butynyl, sec-butyl, *tert*-butyl, isobutyl, 2-methylpropenyl, 1-pentyl, 2-pentenyl, 3-pentenyl, 2-pentynyl, 3-pentynyl, 2-pentyl, 3-pentyl, 2-methylbutyl, 2-methyl-2-butenyl, 3-methylbutyl, 3-methyl-2-butenyl, 1-hexyl, 2-hexenyl, 3-hexenyl, 4-hexenyl, 2-hexynyl, 3-hexynyl, 4-hexynyl, 2-hexyl, 1-methyl-2-pentenyl, 1-methyl-3-pentenyl, 1-methyl-2-pentynyl, 1-methyl-3-pentynyl, 3-hexyl, 1-ethyl-2-butenyl, 1-heptyl, 2-heptenyl, 3-heptenyl, 4-heptenyl, 5-heptenyl, 2-heptynyl, 3-heptynyl, 4-heptynyl, 5-heptynyl, 2-heptyl, 1-methyl-2-hexenyl, 1-methyl-3-hexenyl, 1-methyl-4-hexenyl, 1-methyl-2-hexynyl, 1-methyl-3-hexynyl, 1-methyl-4-hexynyl, 3-heptyl, 1-ethyl-2-pentenyl, 1-ethyl-3-pentenyl, 1-ethyl-2-pentynyl, 1-ethyl-3-pentynyl, 2,2,2-trifluoroethyl, 2,2-difluoropropyl, 4-trifluoromethyl-5,5,5-trifluoropentyl, 4-trifluoromethylpentyl, 5,5,6,6,6-pentafluorobexyl, and 3,3,3-trifluoropropyl, wherein each member of group B is optionally substituted at any carbon up to and including 5 atoms from the point of attachment of B to A with one or more of the group consisting of R³², R³³, R³⁴, R³⁵, and R³⁶;
R³², R³³, R³⁴, R³⁵, and R³⁶ are independently selected from the group consisting of hydrido, amidino, guanidino, methyl, ethyl, methoxy, ethoxy, hydroxy, amino, N-methylamino, dimethylamino, methylthio, ethylthio, trifluoromethyl, pentafluoroethyl, 2,2,2-trifluoroethyl, fluoro, chloro, bromo, amidosulfonyl, N-methylamidosulfonyl, hydroxymethyl, amidocarbonyl, carboxy, and cyano;
A is selected from the group consisting of:
(i) a single covalent bond, NH, N(CH₃), CH₂, CH₃CH, and CH₂CH₂; and
(ii) CH₂N(CH₃), CH₂N(CH₂CH₃), CH₂CH₂N(CH₃), and CH₂CH₂N(CH₂CH₃) with the proviso that B is hydrido;
R¹ is selected from the group consisting of hydrido, trifluoromethyl, pentafluoroethyl, fluoro, and chloro;
R⁹, R¹¹, and R¹³ are independently selected from the group consisting of hydrido, methyl, ethyl, methoxy, ethoxy, hydroxy, amino, N-methylamino, N,N-dimethylamino, methylthio, trifluoromethyl, pentafluoroethyl, 2,2,2-trifluoroethyl, fluoro, chloro, bromo, amidosulfonyl, N-methylamidosulfonyl, N,N-dimethylamidosulfonyl, hydroxymethyl, 1-hydroxyethyl, amidocarbonyl, N-methylamidocarbonyl, carboxy, and cyano;
R¹⁰ and R¹² are independently selected from the group consisting of hydrido, amidino, amidocarbonyl, N-methylamidocarbonyl, guanidino, methyl, ethyl, methoxy, ethoxy, hydroxy, hydroxymethyl, 1-hydroxyethyl, 2-hydroxyethyl, carboxy, carboxymethyl, amino, acetamido, trifluoromethyl, pentafluoroethyl, 2,2,2-trifluoroethyl, trifluoroacetamido, aminomethyl, N-methylamino, dimethylamino, amidosulfonyl, N-methylamidosulfonyl, N,N-dimethylamidosulfonyl, methoxycarbonyl, fluoro, chloro, bromo, and cyano;
Y⁰ is 1-Q^{b}-4-Q^{s}-2-R¹⁶-3-R¹⁷-5-R¹⁸-6-R¹⁹benzene;
R¹⁶, R¹⁷, R¹⁸, and R¹⁹ are independently selected from the group consisting of hydrido, methyl, ethyl, amidino, guanidino, methoxy, hydroxy, amino, aminomethyl, 1-aminoethyl, 2-aminoethyl, N-methylamino, dimethylamino, methylthio, ethylthio, trifluoromethylthio, methylsulfinyl, methylsulfonyl, trifluoromethyl, pentafluoroethyl, 2,2,2-trifluoroethyl, trifluoromethoxy, fluoro, chloro, amidosulfonyl, N-methylarnidosulfonyl, hydroxymethyl, carboxy, and cyano.

7. The compound as recited in Claim 6 or a pharmaceutically acceptable salt thereof, wherein;
B is selected from the group consisting of hydrido, ethyl, 2-propenyl, 2-propynyl, propyl, isopropyl, butyl, 2-butyl, (R)-2-butyl,(S)-2-butyl, *tert*-butyl, isobutyl, 1-pentyl, 3-pentyl, 2-methylbutyl, 2,2,2-trifluoroethyl, 6-amidocarbonylhexyl, 4-methyl-2-pentyl, 3-hydroxypropyl, 3-methoxy-2-propyl, 2-methoxyethyl, 2-methyl-2-butyl, 3-methyl-2-butyl, 2-dimethylaminopropyl, 2-cyanoethyl, 6-hydroxyhexyl, 2-hydroxyethyl, 2-amidinoethyl, 2-guanidinoethyl, 3-guanidinopropyl, 4-guanidinobutyl, 3-hydroxypropyl, 4-hydroxybutyl, 6-cyanohexyl, 2-dimethylaminoethyl, 3-methylbutyl, 2-methylbutyl, (S)-2-methylbutyl, 3-aminopropyl, 2-hexyl, and 4-aminobutyl;
A is selected from the group consisting of single covalent bond, CH₂, CH₃CH, and CH₂CH₂;
R¹ is selected from the group consisting of hydrido, trifluoromethyl, fluoro, and chloro;
Y⁰ is 1-Q^{b}-4-Q^{s}-2-R¹⁶-3-R¹⁷-5-R¹⁸-6-R¹⁹benzene;
R¹⁶ and R¹⁹ are independently selected from the group consisting of:
(i) hydrido, amidino, amino, aminomethyl, methoxy, methylamino, hydroxy, hydroxymethyl, fluoro, chloro, and cyano; and
(ii) Q^{b} with the proviso that no more than one of R¹⁶ and R¹⁹ is Q^{b} at the same time;
R¹⁷ and R¹⁸ are independently selected from the group consisting of hydrido, fluoro, chloro, hydroxy, hydroxymethyl, amino, carboxy, and cyano.

8. The compound as recited in Claim 7 or a pharmaceutically acceptable salt thereof, wherein;
B is selected from the group consisting of hydrido, ethyl, 2-propenyl, 2-propynyl, propyl, isopropyl, butyl, 2-butyl, (R)-2-butyl,(S)-2-butyl, *tert*-butyl, isobutyl, 1-pentyl, 3-pentyl, 2-methylbutyl, 2,2,2-trifluoroethyl, 6-amidocarbonylhexyl, 4-methyl-2-pentyl, 3-hydroxypropyl, 3-methoxy-2-propyl, 2-methoxyethyl, 2-methyl-2-butyl, 3-methyl-2-butyl, 2-dimethylaminopropyl, 2-cyanoethyl, 6-hydroxyhexyl, 2-hydroxyethyl, 2-amidinoethyl, 2-guanidinoethyl, 3-guanidinopropyl, 4-guanidinobutyl, 3-hydroxypropyl, 4-hydroxybutyl, 6-cyanohexyl, 2-dimethylaminoethyl, 3-methylbutyl, 2-methylbutyl, (S)-2-methylbutyl, 3-aminopropyl, 2-hexyl, and 4-aminobutyl;
A is selected from the group consisting of single covalent bond, CH₂, CH₃CH, and CH₂CH₂;
R¹ is selected from the group consisting of hydrido and chloro;
Y⁰ is selected from the group consisting of 4-amidinobenzyl, 2-fluoro-4-amidinobenzyl, and 3-fluoro-4-amidinobenzyl.

9. A compound as recited in Claim 1 or a pharmaceutically acceptable salt thereof, wherein:
R² is 3-aminophenyl, B is 2,2,2-trifluoroethyl, A is single bond, Y⁰ is 4-amidinobenzyl, and R¹ is chloro;
R² is 3-aminophenyl, B is (S)-2-butyl, A is single bond, Y⁰ is 4-amidinobenzyl, and R¹ is chloro;
R² is 3-aminophenyl, B is ethyl, A is single bond, Y⁰ is 4-amidinobenzyl, and R¹ is chloro;
R² is 3-aminophenyl, B is ethyl, A is single bond, Y⁰ is 4-amidino-2-fluorobenzyl, and R¹ is chloro;
R² is 3-aminophenyl, B is 2-propenyl, A is single bond, Y⁰ is 4-amidinobenzyl, and R¹ is chloro;
R² is 3-aminophenyl, B is isopropyl, A is single bond, Y⁰ is 4-amidino-2-fluorobenzyl, and R¹ is chloro;
R² is 3-aminophenyl, B is isopropyl, A is single bond, Y⁰ is 4-amidinobenzyl, and R¹ is chloro;
R² is 3-aminophenyl, B is 2-butyl, A is single bond, Y⁰ is 4-amidinobenzyl, and R¹ is chloro;
R² is 3-aminophenyl, B is (R)-2-butyl, A is single bond, Y⁰ is 4-amidinobenzyl, and R¹ is chloro;
R² is 3-aminophenyl, B is 2-propynyl, A is single bond, Y⁰ is 4-amidinobenzyl, and R¹ is chloro;
R² is 3-aminophenyl, B is 3-pentyl, A is single bond, Y⁰ is 4-amidinobenzyl, and R¹ is hydrido;
R² is 3-aminophenyl, B is hydrido, A is CH₂, Y⁰ is 4-amidinobenzyl, and R¹ is chloro;
R² is 3-aminophenyl, B is ethyl, A is CH₂, Y⁰ is 4-amidinobenzyl, and R¹ is chloro;
R² is 3-aminophenyl, B is 2-methylpropyl, A is single bond, Y⁰ is 4-amidinobenzyl, and R¹ is chloro;
R² is 3-aminophenyl, B is 2-propyl, A is CH₃CH, Y⁰ is 4-amidinobenzyl, and R¹ is chloro;
R² is 3-aminophenyl, B is propyl, A is single bond, Y⁰ is 4-amidino-2-fluorobenzyl, and R¹ is chloro;
R² is 3-aminophenyl, B is 6-amidocarbonylhexyl, A is single bond, Y⁰ is 4-amidinobenzyl, and R¹ is chloro;
R² is 3-aminophenyl, B is tert-butyl, A is single bond, Y⁰ is 4-amidinobenzyl, and R¹ is hydrido;
R² is 3-aminophenyl, B is tert-butyl, A is single bond, Y⁰ is 4-amidinobenzyl, and R¹ is chloro;
R² is 3-aminophenyl, B is 3-hydroxypropyl, A is single bond, Y⁰ is 4-amidinobenzyl, and R¹ is chloro;
R² is 3-aminophenyl, B is 2-methylpropyl, A is single bond, Y⁰ is 4-amidino-2-fluorobenzyl, and R¹ is chloro;
R² is 3-aminophenyl, B is butyl, A is single bond, Y⁰ is 4-amidinobenzyl, and R¹ is chloro;
R² is 3-aminophenyl, B is 3-methoxy-2-propyl, A is single bond, Y⁰ is 4-amidinobenzyl, and R¹ is chloro;
R² is 3-aminophenyl, B is 3-methoxy-2-propyl, A is single bond, Y⁰ is 4-amidinobenzyl, and R¹ is chloro;
R² is 3-aminophenyl, B is 2-methoxy-2-ethyl, A is single bond, Y⁰ is 4-amidinobenzyl, and R¹ is chloro;
R² is 3-aminophenyl, B is 2-propyl, A is single bond, Y⁰ is 4-amidino-3-fluorobenzyl, and R¹ is hydrido;
R² is 3-aminophenyl, B is 2-propyl, A is single bond, Y⁰ is 4-amidino-3-fluorobenzyl, and R¹ is chloro.

10. The compound as recited in Claim I or a pharmaceutically acceptable salt thereof, wherein;
B is selected from the group consisting of cyclopropyl, cyclobutyl, cyclopentyl, cyclohexyl, cycloheptyl, oxalan-2-yl, 2-(2R)-bicyclo[2.2.1]-heptyl, 1,1-dioxothiolan-3-yl, oxetan-3-yl, azetidin-1-yl, azetidin-2-yl, azetidin-3-yl, bicyclo[3.1.0]hexan-6-yl, 2-morpholinyl, 3-morpholinyl, 4-morpholinyl, 1-piperazinyl, 2-piperazinyl, 1-piperidinyl, 2-piperidinyl, 3-piperidinyl, 4-piperidinyl, 1-pyrrolidinyl, 2-pyrrolidinyl, 3-pyrrolidinyl, 2-dioxanyl, 2-tetrahydrofuranyl, 3-tetrahydrofuranyl, 2-tetrahydropyranyl, 3-tetrahydropyranyl, 4-tetrahydropyranyl, 2-tetrahydrothienyl, and 3-tetrahydrothienyl, wherein each ring carbon is optionally substituted with R³³, ring carbons and a nitrogen adjacent to the carbon atom at the point of attachment are optionally substituted with R⁹ or R¹³, a ring carbon or nitrogen adjacent to the R⁹ position and two atoms from the point of attachment are optionally substituted with R¹⁰, and a ring carbon or nitrogen atom adjacent to the R¹³ position and two atoms from the point of attachment is optionally substituted with R¹²;
R⁹, R¹¹, and R¹³ are independently selected from the group consisting of hydrido, methyl, ethyl, methoxy, ethoxy, hydroxy, amino, N-methylamino, N,N-dimethylamino, methylthio, trifluoromethyl, pentafluoroethyl, 2,2,2-trifluoroethyl, fluoro, chloro, bromo, amidosulfonyl, N-methylamidosulfonyl, N,N-dimethylamidosulfonyl, hydroxymethyl, 1-hydroxyethyl, amidocarbonyl, N-methylamidocarbonyl, carboxy, and cyano;
R¹⁰ and R¹² are independently selected from the group consisting of hydrido, amidino, amidocarbonyl, N-methylamidocarbonyl, guanidino, methyl, ethyl, methoxy, ethoxy, hydroxy, hydroxymethyl, 1-hydroxyethyl, 2-hydroxyethyl, carboxy, carboxymethyl, amino, acetamido, trifluoromethyl, pentafluoroethyl, 2,2,2-trifluoroethyl, trifluoroacetamido, aminomethyl, N-methylamino, dimethylamino, amidosulfonyl, N-methylamidosulfonyl, N,N-dimethylamidosulfonyl, methoxycarbonyl, fluoro, chloro, bromo, and cyano;
R³³ is selected from the group consisting of hydrido, amidino, guanidino, methyl, ethyl, methoxy, ethoxy, hydroxy, carboxy, amino, N-methylamino, dimethylamino, methylthio, ethylthio, trifluoromethyl, pentafluoroethyl, 2,2,2-trifluoroethyl, fluoro, chloro, bromo, amidosulfonyl, N-methylamidosulfonyl, hydroxymethyl, amidocarbonyl, and cyano;
A is selected from the group consisting of single covalent bond, NH, N(CH₃), CH₂, CH₃CH, CH₂CH₂, and CH₂CH₂CH₂;
R¹ is selected from the group consisting of hydrido, trifluoromethyl, pentafluoroethyl, fluoro, and chloro;
Y⁰ is 1-Q^{b}-4-Q^{s}-2-R¹⁶-3-R¹⁷-5-R¹⁸-6-R¹⁹benzene;
R¹⁶, R¹⁷, R¹⁸, and R¹⁹ are independently selected from the group consisting of hydrido, methyl, ethyl, amidino, guanidino, methoxy, hydroxy, amino, aminomethyl, 1-aminoethyl, 2-aminoethyl, N-methylamino, dimethylamino, methylthio, ethylthio, trifluoromethylthio, methylsulfinyl, methylsulfonyl, trifluoromethyl, pentafluoroethyl, 2,2,2-trifluoroethyl, trifluoromethoxy, fluoro, chloro, amidosulfonyl, N-methylamidosulfonyl, hydroxymethyl, carboxy, and cyano.

11. The compound as recited in Claim 10 or a pharmaceutically acceptable salt thereof, wherein;
B is selected from the group consisting of cyclopropyl, cyclobutyl, cyclopentyl, cyclohexyl, oxalan-2-yl, 2-(2R)-bicyclo[2.2.1]-heptyl, 1,1-dioxothiolan-3-yl, oxetan-3-yl, azetidin-1-yl, azetidin-2-yl, azetidin-3-yl, 1-pyrrolidinyl and 1-piperidinyl;
A is selected from the group consisting of a single covalent bond, CH₂, NHC(O), CH₂CH₂ and CH₂CH₂CH₂;
R¹ is selected from the group consisting of hydrido, trifluoromethyl, fluoro, and chloro;
Y⁰ is 1-Q^{b}-4-Q^{s}-2-R¹⁶-3-R¹⁷-5-R¹⁸-6-R¹⁹benzene;
R¹⁶ and R¹⁹ are independently selected from the group consisting of:
(i) hydrido, amidino, amino, aminomethyl, methoxy, methylamino, hydroxy, hydroxymethyl, fluoro, chloro, and cyano;
(ii) Q^{b} with the proviso that no more than one of R¹⁶ is Q^{b} at the same time;
R¹⁷ and R¹⁸ are independently selected from the group consisting of hydrido, fluoro, chloro, hydroxy, hydroxymethyl, amino, carboxy, and cyano.

12. The compound as recited in Claim 11 or a pharmaceutically acceptable salt thereof, wherein;
B is selected from the group consisting of cyclopropyl, cyclobutyl, cyclopentyl, cyclohexyl, oxalan-2-yl, 2-(2R)-bicyclo[2.2.1]-heptyl, 1,1-dioxothiolan-3-yl, oxetan-3-yl, azetidin-1-yl, azetidin-2-yl, azetidin-3-yl, andl-piperidinyl;
A is selected from the group consisting of a single covalent bond, CH2, CH₂CH₂ and CH₂CH₂CH₂;
R¹ is selected from the group consisting of hydride and chloro;
Y⁰ is selected from the group consisting of 4-amidinobenzyl, 2-fluoro-4-amidinobenzyl, and 3-fluoro-4-amdinobenzyl.

13. A compound as recited in Claim 1 or a pharmaceutically acceptable salt thereof, wherein:
R² is 3-aminophenyl, B is cyclopropyl, A is single bond, Y⁰ is 4-amidinobenzyl, and R¹ is chloro;
R² is 3-aminophenyl, B is cyclobutyl, A is single bond, Y⁰ is 4-amidino-2-fluorobenzyl, and R¹ is chloro;
R² is 3-aminophenyl, B is cyclobutyl, A is single bond, Y⁰ is 4-amidinobenzyl, and R¹ is chloro;
R² is 3-aminophenyl, B is cyclopropyl, A is single bond, Y⁰ is 4-amidino-2-fluorobenzyl, and R¹ is chloro;
R² is 3-aminophenyl, B is cyclobutyl, A is single bond, Y⁰ is 4-amidinobenzyl, and R¹ is hydrido;
R² is 3-aminophenyl, B is cyclobutyl, A is single bond, Y⁰ is 4-amidino-3-fluorobenzyl, and R¹ is chloro;
R² is 3-aminophenyl, B is cyclopentyl, A is single bond, Y⁰ is 4-amidinobenzyl, and R¹ is chloro;
R² is 3-aminophenyl, B is cyclopropyl, A is CH₂, Y⁰ is 4-amidinobenzyl, and R¹ is chloro;
R² is 3-aminophenyl, B is 2-(2R)-bicyclo[2.2.1]-heptyl, A is single bond, Y⁰ is 4-amidinobenzyl, and R¹ is chloro;
R² is 3-aminophenyl, B is cyclopentyl, A is single bond, Y⁰ is 4-amidino-2-fluorobenzyl, and R¹ is chloro;
R² is 3-aminophenyl, B is cyclohexyl, A is CH₂CH₂, Y⁰ is 4-amidinobenzyl, and R¹ is hydrido;
R² is 3-aminophenyl, B is oxalan-2-yl, A is CH₂, Y⁰ is 4-amidinobenzyl, and R¹ is chloro;
R² is 3-aminophenyl, B is 1-piperidinyl, A is CH₂CH₂, Y⁰ is 4-amidinobenzyl, and R¹ is chloro;
R² is 3-aminophenyl, B is 1,1-dioxothiolan-3-yl, A is single bond, Y⁰ is 4-amidinobenzyl, and R¹ is chloro;
R² is 3-aminophenyl, B is 1-pyrrolidinyl, A is CH₂CH₂CH₂, Y⁰ is 4-amidinobenzyl, and R¹ is chloro.

14. A composition for inhibiting thrombotic conditions in blood comprising a compound of any one of Claims 5, 9 or 13 and a pharmaceutically acceptable carrier.

15. A composition for inhibiting thrombotic conditions in blood comprising a compound of any one of Claims 1 through 4, Claims 6 through 8, or Claims 10 through 12, and a pharmaceutically acceptable carrier.

16. The use of a compound of any one of Claims 1 through 13 or a pharmaceutically acceptable salt thereof for the manufacture of a medicament for inhibiting thrombotic conditions in blood.

17. The use of a compound of any one of Claims 1 through 13 or a pharmaceutically acceptable salt thereof for the manufacture of a medicament for inhibiting formation of blood platelet aggregates in blood.

18. The use of a compound of any one of Claims 1 through 13 or a pharmaceutically acceptable salt thereof for the manufacture of a medicament for inhibiting thrombus formation in blood.

19. The use of a compound of any one of Claims 1 through 13 or a pharmaceutically acceptable salt thereof for the manufacture of a medicament for treating or preventing venous thromboembolism and pulmonary embolism in a mammal.

20. The use of a compound of any one of Claims 1 through 13 or a pharmaceutically acceptable salt thereof for the manufacture of a medicament for treating or preventing deep vein thrombosis in a mammal.

21. The use of a compound of any one of Claims 1 through 13 or a pharmaceutically acceptable salt thereof for the manufacture of a medicament for treating or preventing cardiogenic thromboembolism in a mammal.

22. The use of a compound of any one of Claims 1 through 13 or a pharmaceutically acceptable salt thereof for the manufacture of a medicament for treating or preventing thromboembolic stroke in humans or other mammals.

23. The use of a compound of any one of Claims 1 through 13 or a pharmaceutically acceptable salt thereof for the manufacture of a medicament for treating or preventing thrombosis associated with cancer and cancer chemotherapy in humans and other mammals.

24. The use of a compound of any one of Claims 1 through 13 or a pharmaceutically acceptable salt thereof for the manufacture of a medicament for treating or preventing unstable angina in humans and other mammals.

25. The use of a compound of any one of Claims 1 through 13 or a pharmaceutically acceptable salt thereof for the manufacture of a medicament for inhibiting thrombus formation in blood.

26. The use of a compound of any one of Claims 1 through 13 or a pharmaceutically acceptable salt thereof, in the manufacture of medicament for inhibiting thrombus formation, treating thrombus formation, or preventing thrombus formation in a mammal.

## Patentansprüche

1. Verbindung der Formel: 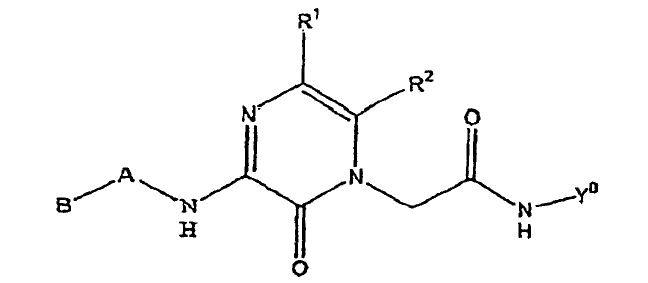 oder ein pharmazeutisch annehmbares Salz davon, worin:
B ausgewählt ist aus der Gruppe, bestehend aus:
(i) Aryl und Heteroaryl, wobei ein dem Kohlenstoff an der Bindungsstelle benachbarter Kohlenstoff gegebenenfalls mit R³² subsituiert ist, der andere dem Kohlenstoff an der Bindungsstelle benachbarte Kohlenstoff gegebenenfalls mit R³⁶ substituiert ist, ein zu R³² benachbarter und vom Kohlenstoff der Bindungsstelle zwei Atome entfernter Kohlenstoff gegebenenfalls mit R³³ substituiert ist, ein zu R³⁶ benachbarter und vom Kohlenstoffatom an der Verbindungsstelle zwei Atome entfernter Kohlenstoff gegebenenfalls mit R³⁵ substituiert ist und ein beliebiger, sowohl zu R³³ als auch zu R³⁵ benachbarter Kohlenstoff gegebenenfalls mit R³⁴ substituiert ist;
(ii) Hydrido, C2-C8-Alkyl, C3-C8-Alkenyl, C3-C8-Alkinyl und C2-C8-Halogenalkyl, wobei jedes Glied aus der Gruppe B gegebenenfalls an einem beliebigen, bis zu und einschließlich 6 Atome von der Bindungsstelle von B an A entfernten Kohlenstoffatom mit einem oder mehreren aus der Gruppe, bestehend aus R³², R³³, R³⁴, R³⁵ und R³⁶, substituiert ist; und
(iii) C3-C7-Cycloalkyl und gesättigtes C4-C6-Heterocyclyl, wobei jeder Ringkohlenstoff gegebenenfalls mit R³³ substituiert ist, ein vom Ringkohlenstoff an der Bindungsstelle von B an A verschiedener Ringkohlenstoff gegebenenfalls mit Oxo substituiert ist, unter der Maßgabe, dass nicht mehr als ein Ringkohlenstoff gleichzeitig mit Oxo substituiert ist, zum Kohlenstoff an der Bindungsstelle benachbarte Ringkohlenstoffe und ein zum Kohlenstoffatom an der Bindungsstelle benachbarter Stickstoff gegebenenfalls mit R⁹ oder R¹³ substituiert sind, ein zur R⁹-Position benachbarter und von der Bindungsstelle zwei Atome entfernter Ringkohlenstoff oder Stickstoff gegebenenfalls mit R¹⁰ substituiert ist, ein zur R¹³-Position benachbarter und zwei Atome von der Bindungsstelle entfernter Ringkohlenstoff oder Stickstoff gegebenenfalls mit R¹² substituiert ist, ein drei Atome von der Bindungsstelle und zur R¹⁰-Position benachbarter Ringkohlenstoff oder Stickstoff gegebenenfalls mit R¹¹ substituiert ist, ein drei Atome von der Bindungsstelle entfernter und zur R¹²-Position benachbarter Ringkohlenstoff oder Stickstoff gegebenenfalls mit R³³ substituiert ist und ein vier Atome von der Bindungsstelle entfernter und zu den R¹¹- und R³³-Positionen benachbarter Ringkohlenstoff oder Stickstoff gegebenenfalls mit R³⁴ substituiert ist;
R³², R³³, R³⁴, R³⁵ und R³⁶ unabhängig ausgewählt sind aus der Gruppe, bestehend aus Hydrido, Acetamido, Halogenacetamido, Amidino, Guanidino, Alkoxy, Hydroxy, Amino, Alkoxyamino, Niederalkylamino, Alkylthio, Amidosulfonyl, Monoalkylamidosulfonyl, Dialkylamidosulfonyl, Alkyl, Halogen, Halogenalkyl, Halogenalkoxy, Hydroxyalkyl, Carboalkoxy, Carboxy, Carboxamido und Cyano;
A ausgewählt ist aus der Gruppe, bestehend aus einer kovalenten Einfachbindung und (CH(R¹⁵))ₚₐ-(W⁷)ᵣᵣ, worin rr eine ganze Zahl ist, ausgewählt aus 0 bis 1, pa eine ganze Zahl ist, ausgewählt aus 0 bis 3, und W⁷ N(R⁷) ist;
R⁷ ausgewählt ist aus der Gruppe, bestehend aus Hydrido und Alkyl;
R¹⁵ ausgewählt ist aus der Gruppe, bestehend aus Hydrido, Halogen, Alkyl und Halogenalkyl;
R¹ ausgewählt ist aus der Gruppe, bestehend aus Hydrido, Cyano, Halogenalkyl und Halogen;
R² Z⁰-Q ist;
Z⁰ eine kovalente Einfachbindung ist;
Q Phenyl ist, worin ein zwei Atome vom Kohlenstoff an der Bindungsstelle entfernter Kohlenstoff gegebenenfalls mit Amino substituiert ist;
R⁹, R¹¹ und R¹³ unabhängig ausgewählt sind aus der Gruppe, bestehend aus Hydrido, Hydroxy, Amino, Amidino, Guanidino, Niederalkylamino, Alkylthio, Alkoxy, Alkylsulfinyl, Alkylsulfonyl, Amidosulfonyl, Monoalkylamidosulfonyl, Alkyl, Halogen, Halogenalkyl, Halogenalkoxy, Hydroxyalkyl, Carboxy, Carboxamido und Cyano;
R¹⁰ und R¹² unabhängig ausgewählt sind aus der Gruppe, bestehend aus Hydrido, Acetamido, Halogenacetamido, Amidino, Guanidino, Alkyl, Alkoxy, Alkoxyamino, Aminoalkyl, Hydroxy, Amino, Niederalkylamino, Alkylsulfonamido, Amidosulfonyl, Monoalkylamidosulfonyl, Dialkylamidosulfonyl, Hydroxyalkyl, Aminoalkyl, Halogen, Halogenalkyl, Carboalkoxy, Carboxy, Carboxamido, Carboxyalkyl und Cyano;
Y⁰ Formel (IV) ist: 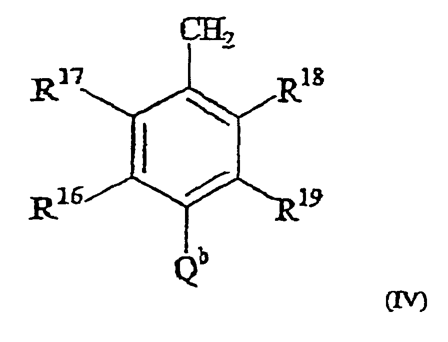
worin
R¹⁶, R¹⁷, R¹⁸ und R¹⁹ unabhängig ausgewählt sind aus der Gruppe, bestehend aus Hydrido, Amidino, Guanidino, Carboxy, Halogenalkylthio, Alkoxy, Hydroxy, Amino, Niederalkylamino, Alkylthio, Alkylsulfinyl, Alkylsulfonyl, Alkanoyl, Halogenalkanoyl, Alkyl, Halogen, Halogenalkyl, Halogenalkoxy, Hydroxyalkyl, Aminoalkyl und Cyano;
Q^{b} C(NR²⁵)NR²³R²⁴ ist;
R²³, R²⁴ und R²⁵ Hydrido sind;
und wobei
der Begriff "Alkyl", entweder allein oder in anderen Begriffen, soweit nichts anderes angegeben ist, ein acyclisches Alkylradikal bedeutet, das ein bis zehn Kohlenstoffatome enthält;
der Begriff "Alkoxy" ein lineares oder verzweigtes Oxy-enthaltendes Radikal mit einem Alkylanteil mit eins bis zehn Kohlenstoffatomen bedeutet;
der Begriff "Aryl" allein oder in Kombination ein carbocyclisches aromatisches System bedeutet, das einen, zwei oder drei Ringe enthält, wobei derartige Ringe miteinander in einer zusammenhängenden Weise verbunden sein können oder kondensiert sein können;
der Begriff "Heterocyclyl" gesättigte und teilweise gesättigte, Heteroatom-enthaltende, ringförmige Radikale mit 4 bis 15 Ringgliedern, ausgewählt aus Kohlenstoff, Stickstoff, Schwefel und Sauerstoff, wobei mindestens ein Ringatom ein Heteroatom ist, bedeutet; und
der Begriff "Heteroaryl" vollständig ungesättigte, Heteroatom-enthaltende, ringförmige, aromatische Radikale mit 4 bis 15 Ringgliedern, ausgewählt aus Kohlenstoff, Stickstoff, Schwefel und Sauerstoff, wobei mindestens ein Ringatom ein Heteroatom ist, bedeutet.

2. Verbindung nach Anspruch 1 oder ein pharmazeutisch annehmbares Salz davon, wobei:
B ausgewählt ist aus der Gruppe, bestehend aus Phenyl, 2-Thienyl, 3-Thienyl, 2-Furyl, 3-Furyl, 2-Pyrrolyl, 3-Pyrrolyl, 2-Imidazolyl, 4-Imidazolyl, 3-Pyrazolyl, 4-Pyrazolyl, 2-Thiazolyl, 3-Isoxazolyl und 5-Isoxazolyl, wobei ein dem Kohlenstoff an der Bindungsstelle benachbarter Kohlenstoff gegebenenfalls mit R³² substituiert ist, der andere dem Kohlenstoff an der Bindungsstelle benachbarte Kohlenstoff gegebenenfalls mit R³⁶ substituiert ist, ein zu R³² benachbarter und zwei Atome vom Kohlenstoff an der Bindungsstelle entfernter Kohlenstoff gegebenenfalls mit R³³ substituiert ist, ein zu R³⁶ benachbarter und zwei Atome vom Kohlenstoff an der Bindungsstelle entfernter Kohlenstoff gegebenenfalls mit R³⁵ substituiert ist und ein beliebiger, sowohl zu R³³ als auch zu R³⁵ benachbarter Kohlenstoff gegebenenfalls mit R³⁴ substituiert ist;
R³², R³³, R³⁴, R³⁵ und R³⁶ unabhängig ausgewählt sind aus der Gruppe, bestehend aus Hydrido, Amidino, Guanidino, Methyl, Ethyl, Methoxy, Ethoxy, Hydroxy, Amino, N-Methylamino, Dimethylamino, Methylthio, Ethylthio, Trifluormethyl, Pentafluorethyl, 2,2,2-Trifluorethyl, Fluor, Chlor, Brom, Amidosulfonyl, N-Methylamidosulfonyl, Hydroxymethyl, Amidocarbonyl, Carboxy und Cyano;
A ausgewählt ist aus der Gruppe, bestehend aus einer kovalenten Einfachbindung, NH, N(CH₃), CH₂, CH₃CH und CH₂CH₂;
R¹ ausgewählt ist aus der Gruppe, bestehend aus Hydrido, Trifluormethyl, Pentafluorethyl, Fluor und Chlor;
R⁹, R¹¹ und R¹³ unabhängig ausgewählt sind aus der Gruppe, bestehend aus Hydrido, Methyl, Ethyl, Methoxy, Ethoxy, Hydroxy, Amino, N-Methylamino, N,N-Dimethylamino, Methylthio, Trifluormethyl, Pentafluorethyl, 2,2,2-Trifluorethyl, Fluor, Chlor, Brom, Amidosulfonyl, N-Methylamidosulfonyl, N,N-Dimethylamidosulfonyl, Hydroxymethyl, 1-Hydroxyethyl, Amidocarbonyl, N-Methylamidocarbonyl, Carboxy und Cyano;
R¹⁰ und R¹² unabhängig ausgewählt sind aus der Gruppe, bestehend aus Hydrido, Amidino, Amidocarbonyl, N-Methylamidocarbonyl, Guanidino, Methyl, Ethyl, Methoxy, Ethoxy, Hydroxy, Hydroxymethyl, 1-Hydroxyethyl, 2-Hydroxyethyl, Carboxy, Carboxymethyl, Amino, Acetamido, Trifluormethyl, Pentafluorethyl, 2,2,2-Trifluorethyl, Trifluoracetamido, Aminomethyl, N-Methylamino, Dimethylamino, Amidosulfonyl, N-Methylamidosulfonyl, N,N-Dimethylamidosulfonyl, Methoxycarbonyl, Fluor, Chlor, Brom und Cyano;
Y⁰ 1-Q^{b}-4-Q^{s}-2-R¹⁶-3-R¹⁷-5-R¹⁸-6-R¹⁹-Benzol ist;
R¹⁶, R¹⁷, R¹⁸ und R¹⁹ unabhängig ausgewählt sind aus der Gruppe, bestehend aus Hydrido, Methyl, Ethyl, Amidino, Guanidino, Methoxy, Hydroxy, Amino, Aminomethyl, 1-Aminoethyl, 2-Aminoethyl, N-Methylamino, Dimethylamino, Methylthio, Ethylthio, Trifluormethylthio, Methylsulfinyl, Methylsulfonyl, Trifluormethyl, Pentafluorethyl, 2,2,2-Trifluorethyl, Trifluormethoxy, Fluor, Chlor, Amidosulfonyl, N-Methylamidosulfonyl, Hydroxymethyl, Carboxy und Cyano.

3. Verbindung nach Anspruch 1 oder ein pharmazeutisch annehmbares Salz davon, wobei:
B ausgewählt ist aus der Gruppe, bestehend aus 2-Aminophenyl, 3-Aminophenyl, 3-Amidinophenyl, 4-Amidinophenyl, 3-Carboxyphenyl, 3-Carboxy-5-hydroxyphenyl, 3-Chlorphenyl, 4-Chlorphenyl, 3,4-Dichlorphenyl, 2-Fluorphenyl, 3-Fluorphenyl, 3,4-Difluorphenyl, 3-Hydroxyphenyl, 4-Hydroxyphenyl, 3-Methoxyaminophenyl, 3-Methoxyphenyl, 4-Methoxyphenyl, 3-Methylphenyl, 4-Methylphenyl, Phenyl, 3-Trifluormethylphenyl, 2-Imidazoyl, 2-Pyridyl, 3-Pyridyl, 5-Chlor-3-trifluormethyl-2-pyridyl, 4-Pyridyl, 2-Thienyl, 3-Thienyl und 3-Trifluormethyl-2-pyridyl;
A ausgewählt ist aus der Gruppe, bestehend aus CH₂, CH₃CH, CF₃CH, NHC(O), CH₂CH₂ und CH₂CH₂CH₂;
R¹ ausgewählt ist aus der Gruppe, bestehend aus Hydrido, Trifluormethyl, Pentafluorethyl, Fluor und Chlor;
Y⁰ 1-Q^{b}-4-Q^{s}-2-R¹⁶-3-R¹⁷-5-R¹⁸-6-R¹⁹-Benzol ist;
R¹⁶ und R¹⁹ unabhängig ausgewählt sind aus der Gruppe, bestehend aus:
(i) Hydrido, Amidino, Amino, Aminomethyl, Methoxy, Methylamino, Hydroxy, Hydroxymethyl, Fluor, Chlor und Cyano; und
(ii) Q^{b} unter der Maßgabe, dass nicht mehr als eines aus R¹⁶ und R¹⁹ gleichzeitig Q^{b} ist; und
R¹⁷ und R¹⁸ unabhängig ausgewählt sind aus der Gruppe, bestehend aus Hydrido, Fluor, Chlor, Hydroxy, Hydroxymethyl, Amino, Carboxy und Cyano.

4. Verbindung nach Anspruch 3 oder ein pharmazeutisch annehmbares Salz davon, wobei:
B ausgewählt ist aus der Gruppe, bestehend aus 3-Aminophenyl, 3-Amidinophenyl, 4-Amidinophenyl, 3-Chlorphenyl, 4-Chlorphenyl, 3,4-Dichlorphenyl, 2-Fluorphenyl, 4-Methylphenyl, Phenyl, 2-Imidazoyl, 3-Pyridyl, 4-Pyridyl und 3-Trifluormethyl-2-pyridyl;
A ausgewählt ist aus der Gruppe, bestehend aus CH₂, NHC(O), CH₂CH₂ und CH₂CH₂CH₂;
R¹ ausgewählt ist aus der Gruppe, bestehend aus Hydrido und Chlor; und
Y⁰ ausgewählt ist aus der Gruppe, bestehend aus 4-Amidinobenzyl, 2-Fluor-4-amidinobenzyl und 3-Fluor-4-amidinobenzyl.

5. Verbindung nach Anspruch 1 oder ein pharmazeutisch annehmbares Salz davon, wobei:
R² 3-Aminophenyl ist, B 3-Chlorphenyl ist, A CH₂CH₂ ist, Y⁰ 4-Amidinobenzyl ist und R¹ Chlor ist;
R² 3-Aminophenyl ist, B Phenyl ist, A CH₂ ist, Y⁰ 4-Amidinobenzyl ist und R¹ Chlor ist; und
R² 3-Aminophenyl ist, B 2-Imidazoyl ist, A CH₂CH₂CH₂ ist, Y⁰ 4-Amidinobenzyl ist und R¹ Chlor ist.

6. Verbindung nach Anspruch 1 oder ein pharmazeutisch annehmbares Salz davon, wobei:
B ausgewählt ist aus der Gruppe, bestehend aus Hydrido, Ethyl, 2-Propenyl, 2-Propinyl, Propyl, Isopropyl, Butyl, 2-Butenyl, 2-Butinyl, sec-Butyl, *tert*-Butyl, Isobutyl, 2-Methylpropenyl, 1-Pentyl, 2-Pentenyl, 3-Pentenyl, 2-Pentinyl, 3-Pentinyl, 2-Pentyl, 3-Pentyl, 2-Methylbutyl, 2-Methyl-2-butenyl, 3-Methylbutyl, 3-Methyl-2-butenyl, 1-Hexyl, 2-Hexenyl, 3-Hexenyl, 4-Hexenyl, 2-Hexinyl, 3-Hexinyl, 4-Hexinyl, 2-Hexyl, 1-Methyl-2-pentenyl, 1-Methyl-3-pentenyl, 1-Methyl-2-pentinyl, 1-Methyl-3-pentinyl, 3-Hexyl, 1-Ethyl-2-butenyl, 1-Heptyl, 2-Heptenyl, 3-Heptenyl, 4-Heptenyl, 5-Heptenyl, 2-Heptinyl, 3-Heptinyl, 4-Heptinyl, 5-Heptinyl, 2-Heptyl, 1-Methyl-2-hexenyl, 1-Methyl-3-hexenyl, 1-Methyl-4-hexenyl, 1-Methyl-2-hexinyl, 1-Methyl-3-hexinyl, 1-Methyl-4-hexinyl, 3-Heptyl, 1-Ethyl-2-pentenyl, 1-Ethyl-3-pentenyl, 1-Ethyl-2-pentinyl, 1-Ethyl-3-pentinyl, 2,2,2-Trifluorethyl, 2,2-Difluorpropyl, 4-Trifluormethyl-5,5,5-trifluorpentyl, 4-Trifluormethylpentyl, 5,5,6,6,6-Pentafluorhexyl und 3,3,3-Trifluorpropyl, wobei jedes Glied der Gruppe B gegebenenfalls an einem beliebigen, bis zu und einschließlich 5 Atome von der Bindungsstelle von B an A entfernten Kohlenstoff mit einer oder mehreren aus der Gruppe, bestehend aus R³², R³³, R³⁴, R³⁵ und R³⁶, substituiert ist;
R³², R³³, R³⁴, R³⁵ und R³⁶ unabhängig ausgewählt sind aus der Gruppe, bestehend aus Hydrido, Amidino, Guanidino, Methyl, Ethyl, Methoxy, Ethoxy, Hydroxy, Amino, N-Methylamino, Dimethylamino, Methylthio, Ethylthio, Trifluormethyl, Pentafluorethyl, 2,2,2-Trifluorethyl, Fluor, Chlor, Brom, Amidosulfonyl, N-Methylamidosulfonyl, Hydroxymethyl, Amidocarbonyl, Carboxy und Cyano;
A ausgewählt ist aus der Gruppe, bestehend aus:
(i) einer kovalenten Einfachbindung, NH, N(CH₃), CH₂, CH₃CH und CH₂CH₂; und
(ii) CH₂N(CH₃), CH₂N(CH₂CH₃), CH₂CH₂N(CH₃) und CH₂CH₂N(CH₂CH₃) unter der Maßgabe, dass B Hydrido ist;
R¹ ausgewählt ist aus der Gruppe, bestehend aus Hydrido, Trifluormethyl, Pentafluorethyl, Fluor und Chlor;
R⁹, R¹¹ und R¹³ unabhängig ausgewählt sind aus der Gruppe, bestehend aus Hydrido, Methyl, Ethyl, Methoxy, Ethoxy, Hydroxy, Amino, N-Methylamino, N,N-Dimethylamino, Methylthio, Trifluormethyl, Pentafluorethyl, 2,2,2-Trifluorethyl, Fluor, Chlor, Brom, Amidosulfonyl, N-Methylamidosulfonyl, N,N-Dimethylamidosulfonyl, Hydroxymethyl, 1-Hydroxyethyl, Amidocarbonyl, N-Methylamidocarbonyl, Carboxy und Cyano;
R¹⁰ und R¹² unabhängig ausgewählt sind aus der Gruppe, bestehend aus Hydrido, Amidino, Amidocarbonyl, N-Methylamidocarbonyl, Guanidino, Methyl, Ethyl, Methoxy, Ethoxy, Hydroxy, Hydroxymethyl, 1-Hydroxyethyl, 2-Hydroxyethyl, Carboxy, Carboxymethyl, Amino, Acetamino, Trifluormethyl, Pentafluorethyl, 2,2,2-Trifluorethyl, Trifluoracetamido, Aminomethyl, N-Methylamino, Dimethylamino, Amidosulfonyl, N-Methylamidosulfonyl, N,N-Dimethylamidosulfonyl, Methoxycarbonyl, Fluor, Chlor, Brom und Cyano;
Y⁰ 1-Q^{b}-4-Q^{s}-2-R¹⁶-3-R¹⁷-5-R¹⁸-6-R¹⁹-Benzol ist;
R¹⁶, R¹⁷, R¹⁸und R¹⁹ unabhängig ausgewählt sind aus der Gruppe, bestehend aus Hydrido, Methyl, Ethyl, Amidino, Guanidino, Methoxy, Hydroxy, Amino, Aminomethyl, 1-Aminoethyl, 2-Aminoethyl, N-Methylamino, Dimethylamino, Methylthio, Ethylthio, Trifluormethylthio, Methylsulfinyl, Methylsulfonyl, Trifluormethyl, Pentafluorethyl, 2,2,2-Trifluorethyl, Trifluormethoxy, Fluor, Chlor, Amidosulfonyl, N-Methylamidosulfonyl, Hydroxymethyl, Carboxy und Cyano.

7. Verbindung nach Anspruch 6 oder ein pharmazeutisch annehmbares Salz davon, wobei,
B ausgewählt ist aus der Gruppe, bestehend aus Hydrido, Ethyl, 2-Propenyl, 2-Propinyl, Propyl, Isopropyl, Butyl, 2-Butyl, (R)-2-Butyl, (S)-2-Butyl, *tert*-Butyl, Isobutyl, 1-Pentyl, 3-Pentyl, 2-Methylbutyl, 2,2,2-Trifluorethyl, 6-Amidocarbonylhexyl, 4-Methyl-2-pentyl, 3-Hydroxypropyl, 3-Methoxy-2-propyl, 2-Methoxyethyl, 2-Methyl-2-butyl, 3-Methyl-2-butyl, 2-Dimethylaminopropyl, 2-Cyanoethyl, 6-Hydroxyhexyl, 2-Hydroxyethyl, 2-Amidinoethyl, 2-Guanidinoethyl, 3-Guanidinopropyl, 4-Guanidinobutyl, 3-Hydroxypropyl, 4-Hydroxybutyl, 6-Cyanohexyl, 2-Dimethylaminoethyl, 3-Methylbutyl, 2-Methylbutyl, (S)-2-Methylbutyl, 3-Aminopropyl, 2-Hexyl und 4-Aminobutyl;
A ausgewählt ist aus der Gruppe, bestehend aus einer kovalenten Einfachbindung, CH₂, CH₃CH und CH₂CH₂;
R¹ ausgewählt ist aus der Gruppe, bestehend aus Hydrido, Trifluormethyl, Fluor und Chlor;
Y⁰ 1-Q^{b}-4-Q^{s}-2-R¹⁶-3-R¹⁷-5-R¹⁸-6-R¹⁹-Benzol ist;
R¹⁶ und R¹⁹ unabhängig ausgewählt sind aus der Gruppe, bestehend aus:
(i) Hydrido, Amidino, Amino, Aminomethyl, Methoxy, Methylamino, Hydroxy, Hydroxymethyl, Fluor, Chlor und Cyano; und
(ii) Q^{b} unter der Maßgabe, dass nicht mehr als eines aus R¹⁶ und R¹⁹ gleichzeitig Q^{b} ist; und
R¹⁷ und R¹⁸ unabhängig ausgewählt sind aus der Gruppe, bestehend aus Hydrido, Fluor, Chlor, Hydroxy, Hydroxymethyl, Amino, Carboxy und Cyano.

8. Verbindung nach Anspruch 7 oder ein pharmazeutisch annehmbares Salz davon, wobei:
B ausgewählt ist aus der Gruppe, bestehend aus Hydrido, Ethyl, 2-Propenyl, 2-Propinyl, Propyl, Isopropyl, Butyl, 2-Butyl, (R)-2-Butyl, (S)-2-Butyl, *tert*-Butyl, Isobutyl, 1-Pentyl, 3-Pentyl, 2-Methylbutyl, 2,2,2-Trifluorethyl, 6-Amidocarbonylhexyl, 4-Methyl-2-pentyl, 3-Hydroxypropyl, 3-Methoxy-2-propyl, 2-Methoxyethyl, 2-Methyl-2-butyl, 3-Methyl-2-butyl, 2-Dimethylaminopropyl, 2-Cyanoethyl, 6-Hydroxyhexyl, 2-Hydroxyethyl, 2-Amidinoethyl, 2-Guanidinoethyl, 3-Guanidinopropyl, 4-Guanidinobutyl, 3-Hydroxypropyl, 4-Hydroxybutyl, 6-Cyanohexyl, 2-Dimethylaminoethyl, 3-Methylbutyl, 2-Methylbutyl, (S)-2-Methylbutyl, 3-Aminopropyl, 2-Hexyl und 4-Aminobutyl;
A ausgewählt ist aus der Gruppe, bestehend aus einer kovalenten Einfachbindung, CH₂, CH₃CH und CH₂CH₂;
R¹ ausgewählt ist aus der Gruppe, bestehend aus Hydrido und Chlor; und
Y⁰ ausgewählt ist aus der Gruppe, bestehend aus 4-Amidinobenzyl, 2-Fluor-4-Amidinobenzyl und 3-Fluor-4-Amidinobenzyl.

9. Verbindung nach Anspruch 1 oder ein pharmazeutisch annehmbares Salz davon, wobei:
R² 3-Aminophenyl ist, B 2,2,2-Trifluorethyl ist, A eine Einfachbindung ist, Y⁰ 4-Amidinobenzyl ist und R¹ Chlor ist;
R² 3-Aminophenyl ist, B (S)-2-Butyl ist, A eine Einfachbindung ist, Y⁰ 4-Amidinobenzyl ist und R¹ Chlor ist;
R² 3-Aminophenyl ist, B Ethyl ist, A eine Einfachbindung ist, Y⁰ 4-Amidinobenzyl ist und R¹ Chlor ist;
R² 3-Aminophenyl ist, B Ethyl ist, A eine Einfachbindung ist, Y⁰ 4-Amidino-2-fluorbenzyl ist und R¹ Chlor ist;
R² 3-Aminophenyl ist, B 2-Propenyl ist, A eine Einfachbindung ist, Y⁰ 4-Amidinobenzyl ist und R¹ Chlor ist;
R² 3-Aminophenyl ist, B Isopropyl ist, A eine Einfachbindung ist, Y⁰ 4-Amidino-2-fluorbenzyl ist und R¹ Chlor ist;
R² 3-Aminophenyl ist, B Isopropyl ist, A eine Einfachbindung ist, Y⁰ 4-Amidinobenzyl ist und R¹ Chlor ist;
R² 3-Aminophenyl ist, B 2-Butyl ist, A eine Einfachbindung ist, Y⁰ 4-Amidinobenzyl ist und R¹ Chlor ist;
R² 3-Aminophenyl ist, B (R)-2-Butyl ist, A eine Einfachbindung ist, Y⁰ 4-Amidinobenzyl ist und R¹ Chlor ist;
R² 3-Aminophenyl ist, B 2-Propinyl ist, A eine Einfachbindung ist, Y⁰ 4-Amidinobenzyl ist und R¹ Chlor ist;
R² 3-Aminophenyl ist, B 3-Pentyl ist, A eine Einfachbindung ist, Y⁰ 4-Amidinobenzyl ist und R¹ Hydrido ist;
R² 3-Aminophenyl ist, B Hydrido ist, A CH₂ ist, Y⁰ 4-Amidinobenzyl ist und R¹ Chlor ist;
R² 3-Aminophenyl ist, B Ethyl ist, A CH₂ ist, Y⁰ 4-Amidinobenzyl ist und R¹ Chlor ist;
R² 3-Aminophenyl ist, B 2-Methylpropyl ist, A eine Einfachbindung ist, Y⁰ 4-Amidinobenzyl ist und R¹ Chlor ist;
R² 3-Aminophenyl ist, B 2-Propyl ist, A CH₃CH ist, Y⁰ 4-Amidinobenzyl ist und R¹ Chlor ist;
R² 3-Aminophenyl ist, B Propyl ist, A eine Einfachbindung ist, Y⁰ 4-Amidino-2-fluorbenzyl ist und R¹ Chlor ist;
R² 3-Aminophenyl ist, B 6-Amidocarbonylhexyl ist, A eine Einfachbindung ist, Y⁰ 4-Amidinobenzyl ist und R¹ Chlor ist;
R² 3-Aminophenyl ist, B tert-Butyl ist, A eine Einfachbindung ist, Y⁰ 4-Amidinobenzyl ist und R¹ Hydrido ist;
R² 3-Aminophenyl ist, B tert-Butyl ist, A eine Einfachbindung ist, Y⁰ 4-Amidinobenzyl ist und R¹ Chlor ist;
R² 3-Aminophenyl ist, B 3-Hydroxypropyl ist, A eine Einfachbindung ist, Y⁰ 4-Amidinobenzyl ist und R¹ Chlor ist;
R² 3-Aminophenyl ist, B 2-Methylpropyl ist, A eine Einfachbindung ist, Y⁰ 4-Amidino-2-fluorbenzyl ist und R¹ Chlor ist;
R² 3-Aminophenyl ist, B Butyl ist, A eine Einfachbindung ist, Y⁰ 4-Amidinobenzyl ist und R¹ Chlor ist;
R² 3-Aminophenyl ist, B 3-Methoxy-2-propyl ist, A eine Einfachbindung ist, Y⁰ 4-Amidinobenzyl ist und R¹ Chlor ist;
R² 3-Aminophenyl ist, B 3-Methoxy-2-propyl ist, A eine Einfachbindung ist, Y⁰ 4-Amidinobenzyl ist und R¹ Chlor ist;
R² 3-Aminophenyl ist, B 2-Methoxy-2-ethyl ist, A eine Einfachbindung ist, Y⁰ 4-Amidinobenzyl ist und R¹ Chlor ist;
R² 3-Aminophenyl ist, B 2-Propyl ist, A eine Einfachbindung ist, Y⁰ 4-Amidino-3-fluorbenzyl ist und R¹ Hydrido ist; und
R² 3-Aminophenyl ist, B 2-Propyl ist, A eine Einfachbindung ist, Y⁰ 4-Amidino-3-fluorbenzyl ist und R¹ Chlor ist.

10. Verbindung nach Anspruch 1 oder ein pharmazeutisch annehmbares Salz davon, wobei:
B ausgewählt ist aus der Gruppe, bestehend aus Cyclopropyl, Cyclobutyl, Cyclopentyl, Cyclohexyl, Cycloheptyl, Oxalan-2-yl, 2-(2R)-bicyclo[2.2.1]heptyl, 1,1-Dioxothiolan-3-yl, Oxetan-3-yl, Azetidin-1-yl, Azetidin-2-yl, Azetidin-3-yl, Bicyclo[3.1.0.]hexan-6-yl, 2-Morpholinyl, 3-Morpholinyl, 4-Morpholinyl, 1-Piperazinyl, 2-Piperazinyl, 1-Piperidinyl, 2-Piperidinyl, 3-Piperidinyl, 4-Piperidinyl, 1-Pyrrolidinyl, 2-Pyrrolidinyl, 3-Pyrrolidinyl, 2-Dioxanyl, 2-Tetrahydrofuranyl, 3-Tetrahydrofuranyl, 2-Tetrahydropyranyl, 3-Tetrahydropyranyl, 4-Tetrahydropyranyl, 2-Tetrahydrothienyl und 3-Tetrahydrothienyl, wobei jeder Ringkohlenstoff gegebenenfalls mit R³³ substituiert ist, dem Kohlenstoffatom an der Bindungsstelle benachbarte Ringkohlenstoffe und ein dem Kohlenstoffatom an der Bindungsstelle benachbarter Stickstoff gegebenenfalls mit R⁹ oder R¹³ substituiert sind, ein der R⁹-Position benachbarter und zwei Atome von der Bindungsstelle entfernter Ringkohlenstoff oder Stickstoff gegebenenfalls mit R¹⁰ substituiert ist und ein der R¹³-Position benachbarter und zwei Atome von der Bindungsstelle entfernter Ringkohlenstoff oder Stickstoff gegebenenfalls mit R¹² substituiert ist;
R⁹, R¹¹ und R¹³ unabhängig ausgewählt sind aus der Gruppe, bestehend aus Hydrido, Methyl, Ethyl, Methoxy, Ethoxy, Hydroxy, Amino, N-Methylamino, N,N-Dimethylamino, Methylthio, Trifluormethyl, Pentafluorethyl, 2,2,2-Trifluorethyl, Fluor, Chlor, Brom, Amidosulfonyl, N-Methylamidosulfonyl, N,N-Dimethylamidosulfonyl, Hydroxymethyl, 1-Hydroxyethyl, Amidocarbonyl, N-Methylamidocarbonyl, Carboxy und Cyano;
R¹⁰ und R¹² unabhängig ausgewählt sind aus der Gruppe, bestehend aus Hydrido, Amidino, Amidocarbonyl, N-Methylamidocarbonyl, Guanidino, Methyl, Ethyl, Methoxy, Ethoxy, Hydroxy, Hydroxymethyl, 1-Hydroxyethyl, 2-Hydroxyethyl, Carboxy, Carboxymethyl, Amino, Acetamido, Trifluormethyl, Pentafluorethyl, 2,2,2-Trifluorethyl, Trifluoracetamido, Aminomethyl, N-Methylamino, Dimethylamino, Amidosulfonyl, N-Methylamidosulfonyl, N,N-Dimethylamidosulfonyl, Methoxycarbonyl, Fluor, Chlor, Brom und Cyano;
R³³ ausgewählt ist aus der Gruppe, bestehend aus Hydrido, Amidino, Guanidino, Methyl, Ethyl, Methoxy, Ethoxy, Hydroxy, Carboxy, Amino, N-Methylamino, Dimethylamino, Methylthio, Ethylthio, Trifluormethyl, Pentafluorethyl, 2,2,2-Trifluorethyl, Fluor, Chlor, Brom, Amidosulfonyl, N-Methylamidosulfonyl, Hydroxymethyl, Amidocarbonyl und Cyano;
A ausgewählt ist aus der Gruppe, bestehend aus einer kovalenten Einfachbindung, NH, N(CH₃), CH₂, CH₃CH, CH₂CH₂ und CH₂CH₂CH₂;
R¹ ausgewählt ist aus der Gruppe, bestehend aus Hydrido, Trifluormethyl, Pentafluorethyl, Fluor und Chlor;
Y⁰ 1-Q^{b}-4-Q^{s}-2-R¹⁶-3-R¹⁷-5-R¹⁸-6-R¹⁹-Benzol ist; und
R¹⁶, R¹⁷, R¹⁸ und R¹⁹ unabhängig ausgewählt sind aus der Gruppe, bestehend aus Hydrido, Methyl, Ethyl, Amidino, Guanidino, Methoxy, Hydroxy, Amino, Aminomethyl, 1-Aminoethyl, 2-Aminoethyl, N-Methylamino, Dimethylamino, Methylthio, Ethylthio, Trifluormethylthio, Methylsulfinyl, Methylsulfonyl, Trifluormethyl, Pentafluorethyl, 2,2,2-Trifluorethyl, Trifluormethoxy, Fluor, Chlor, Amidosulfonyl, N-Methylamidosulfonyl, Hydroxymethyl, Carboxy und Cyano.

11. Verbindung nach Anspruch 10 oder ein pharmazeutisch annehmbares Salz davon, wobei:
B ausgewählt ist aus der Gruppe, bestehend aus Cyclopropyl, Cyclobutyl, Cyclopentyl, Cyclohexyl, Oxalan-2-yl, 2-(2R)-Bicyclo[2.2.1]heptyl, 1,1-Dioxothiolan-3-yl, Oxetan-3-yl, Azetidin-1-yl, Azetidin-2-yl, Azetidin-3-yl, 1-Pyrrolidinyl und 1-Piperidinyl;
A ausgewählt ist aus der Gruppe, bestehend aus einer kovalenten Einfachbindung, CH₂, NHC(O), CH₂CH₂ und CH₂CH₂CH₂;
R¹ ausgewählt ist aus der Gruppe, bestehend aus Hydrido, Trifluormethyl, Fluor und Chlor;
Y⁰ 1-Q^{b}-4-Q^{s}-2-R¹⁶-3-R¹⁷-5-R¹⁸-6-R¹⁹-Benzol ist;
R¹⁶ und R¹⁹ unabhängig ausgewählt sind aus Gruppe, bestehend aus:
(i) Hydrido, Amidino, Amino, Aminomethyl, Methoxy, Methylamino, Hydroxy, Hydroxymethyl, Fluor, Chlor und Cyano; und
(ii) Q^{b} unter der Maßgabe, dass nicht mehr als eines aus R¹⁶ gleichzeitig Q^{b} ist; und
R¹⁷ und R¹⁸ unabhängig ausgewählt sind aus der Gruppe, bestehend aus Hydrido, Fluor, Chlor, Hydroxy, Hydroxymethyl, Amino, Carboxy und Cyano.

12. Verbindung nach Anspruch 11 oder ein pharmazeutisch annehmbares Salz davon, wobei:
B ausgewählt ist aus der Gruppe, bestehend aus Cyclopropyl, Cyclobutyl, Cyclopentyl, Cyclohexyl, Oxalan-2-yl, 2-(2R)-Bicyclo[2.2.1]heptyl, 1,1-Dioxothiolan-3-yl, Oxetan-3-yl, Azetidin-1-yl, Azetidin-2-yl, Azetidin-3-yl und 1-Piperidinyl;
A ausgewählt ist aus der Gruppe, bestehend aus einer kovalenten Einfachbindung, CH₂, CH₂CH₂ und CH₂CH₂CH₂;
R¹ ausgewählt ist aus der Gruppe, bestehend aus Hydrido und Chlor; und
Y⁰ ausgewählt ist aus der Gruppe, bestehend aus 4-Amidinobenzyl, 2-Fluor-4-amidinobenzyl und 3-Fluor-4-amidinobenzyl.

13. Verbindung nach Anspruch 1 oder ein pharmazeutisch annehmbares Salz davon, wobei:
R² 3-Aminophenyl ist, B Cyclopropyl ist, A eine Einfachbindung ist, Y⁰ 4-Amidinobenzyl ist und R¹ Chlor ist;
R² 3-Aminophenyl ist, B Cyclobutyl ist, A eine Einfachbindung ist, Y⁰ 4-Amidino-2-fluorbenzyl ist und R¹ Chlor ist;
R² 3-Aminophenyl ist, B Cyclobutyl ist, A eine Einfachbindung ist, Y⁰ 4-Amidinobenzyl ist und R¹ Chlor ist;
R² 3-Aminophenyl ist, B Cyclopropyl ist, A eine Einfachbindung ist, Y⁰ 4-Amidino-2-fluorbenzyl ist und R¹ Chlor ist;
R² 3-Aminophenyl ist, B Cyclobutyl ist, A eine Einfachbindung ist, Y⁰ 4-Amidinobenzyl ist und R¹ Hydrido ist;
R² 3-Aminophenyl ist, B Cyclobutyl ist, A eine Einfachbindung ist, Y⁰ 4-Amidino-3-fluorbenzyl ist und R¹ Chlor ist;
R² 3-Aminophenyl ist, B Cyclopentyl ist, A eine Einfachbindung ist, Y⁰ 4-Amidinobenzyl ist und R¹ Chlor ist;
R² 3-Aminophenyl ist, B Cyclopropyl ist, A CH₂ ist, Y⁰ 4-Amidinobenzyl ist und R¹ Chlor ist;
R² 3-Aminophenyl ist, B 2-(2R)-Bicyclo[2.2.1]heptyl ist, A eine Einfachbindung ist, Y⁰ 4-Amidinobenzyl ist und R¹ Chlor ist;
R² 3-Aminophenyl ist, B Cyclopentyl ist, A eine Einfachbindung ist, Y⁰ 4-Amidino-2-fluorbenzyl ist und R¹ Chlor ist;
R² 3-Aminophenyl ist, B Cyclohexyl ist, A CH₂CH₂ ist, Y⁰ 4-Amidinobenzyl ist und R¹ Hydrido ist;
R² 3-Aminophenyl ist, B Oxalan-2-yl ist, A CH₂ ist, Y⁰ 4-Amidinobenzyl ist und R¹ Chlor ist;
R² 3-Aminophenyl ist, B 1-Piperidinyl ist, A CH₂CH₂ ist, Y⁰ 4-Amidinobenzyl ist und R¹ Chlor ist;
R² 3-Aminophenyl ist, B 1,1-Dioxothiolan-3-yl ist, A eine Einfachbindung ist, Y⁰ 4-Amidinobenzyl ist und R¹ Chlor ist; und
R² 3-Aminophenyl ist, B 1-Pyrrolidinyl ist, A CH₂CH₂CH₂ ist, Y⁰ 4-Amidinobenzyl ist und R¹ Chlor ist.

14. Zusammensetzung zur Inhibierung thrombotischer Zustände im Blut, umfassend eine Verbindung nach einem der Ansprüche 5, 9 oder 13 und einen pharmazeutisch annehmbaren Träger.

15. Zusammensetzung zur Inhibierung thrombotischer Zustände im Blut, umfassend eine Verbindung nach einem der Ansprüche 1 bis 4, 6 bis 8 oder 10 bis 12 und einen pharmazeutisch annehmbaren Träger.

16. Verwendung einer Verbindung nach einem der Ansprüche 1 bis 13 oder eines pharmazeutisch annehmbaren Salzes davon für die Herstellung eines Medikaments zur Inhibierung thrombotischer Zustände im Blut.

17. Verwendung einer Verbindung nach einem der Ansprüche 1 bis 13 oder eines pharmazeutisch annehmbaren Salzes davon zur Herstellung eines Medikaments zur Inhibierung der Bildung von Blutplättchenaggregaten im Blut.

18. Verwendung einer Verbindung nach einem der Ansprüche 1 bis 13 oder eines pharmazeutisch annehmbaren Salzes davon zur Herstellung eines Medikaments zur Inhibierung der Thrombusbildung im Blut.

19. Verwendung einer Verbindung nach einem der Ansprüche 1 bis 13 oder eines pharmazeutisch annehmbaren Salzes davon zur Herstellung eines Medikaments zur Behandlung oder Prävention venöser Thromboembolie und Lungenembolie bei einem Säuger.

20. Verwendung einer Verbindung nach einem der Ansprüche 1 bis 13 oder eines pharmazeutisch annehmbaren Salzes davon zur Herstellung eines Medikaments zur Behandlung oder Prävention von tiefer Beinvenenthrombose bei einem Säuger.

21. Verwendung einer Verbindung nach einem der Ansprüche 1 bis 13 oder eines pharmazeutisch annehmbaren Salzes davon zur Herstellung eines Medikaments zur Behandlung oder Prävention kardiogener Thromboembolie bei einem Säuger.

22. Verwendung einer Verbindung nach einem der Ansprüche 1 bis 13 oder eines pharmazeutisch annehmbaren Salzes davon zur Herstellung eines Medikaments zur Behandlung oder Prävention von thromboembolischem Schlaganfall bei Menschen oder anderen Säugern.

23. Verwendung einer Verbindung nach einem der Ansprüche 1 bis 13 oder eines pharmazeutisch annehmbaren Salzes davon zur Herstellung eines Medikaments zur Behandlung oder Prävention von Thrombose in Verbindung mit Krebs und Krebschemotherapie bei Menschen und anderen Säugern.

24. Verwendung einer Verbindung nach einem der Ansprüche 1 bis 13 oder eines pharmazeutisch annehmbaren Salzes davon zur Herstellung eines Medikaments zur Behandlung oder Prävention instabiler Angina bei Menschen und anderen Säugern.

25. Verwendung einer Verbindung nach einem der Ansprüche 1 bis 13 oder eines pharmazeutisch annehmbaren Salzes davon zur Herstellung eines Medikaments zur Inhibierung der Thrombusbildung im Blut.

26. Verwendung einer Verbindung nach einem der Ansprüche 1 bis 13 oder eines pharmazeutisch annehmbaren Salzes davon zur Herstellung eines Medikaments zur inhibierung der Thrombusbildung, Behandlung der Thrombusbildung oder Prävention von Thrombusbildung bei einem Säuger.

## Revendications

1. Composé ayant la formule : 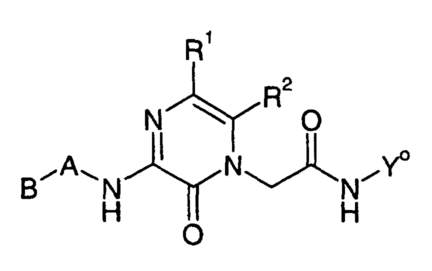 ou un sel pharmaceutiquement acceptable de celui-ci, dans lequel ;
B est sélectionné parmi le groupe constitué de
(i) un aryle et un hétéroaryle où un carbone adjacent au carbone au point d'attache est facultativement substitué par R³², l'autre carbone adjacent au carbone au point d'attache est facultativement substitué par R³⁶, un carbone adjacent à R³² et à deux atomes à partir du carbone au point d'attache est facultativement substitué par R³³, un carbone adjacent à R³⁶ et à deux atomes à partir du carbone au point d'attache est facultativement substitué par R³⁵, et tout carbone adjacent à la fois à R³³ et à R³⁵ est facultativement substitué par R³⁴ ;
(ii) un hydrido, un alkyle en C₂ à C₈, un alcényle en C₃ à C₈, un alcynyle en C₃ à C₈, et un haloalkyle en C₂ à C₈, où chaque membre du groupe B est facultativement substitué à tout carbone jusqu'à et y compris 6 atomes du point d'attache de B à A avec l'un ou plusieurs du groupe constitué de R³², R³³, R³⁴, R³⁵ et R³⁶ ; et
(iii) un cycloalkyle en C₃ à C₇ et un hétérocyclyle en C₄ à C₆ saturé, où chaque carbone cyclique est facultativement substitué par R³³, un carbone cyclique autre que le carbone cyclique au point d'attache de B à A est facultativement substitué par un oxo à condition que pas plus d'un carbone cyclique soit substitué par un oxo au même moment, les carbones cycliques et un azote adjacent à l'atome de carbone au point d'attache sont facultativement substitués par R⁹ ou R¹³, un carbone cyclique ou un azote adjacent à la position R⁹ et à deux atomes à partir du point d'attache est facultativement substitué par R¹⁰, un carbone cyclique ou un azote adjacent à la position de R¹³ et à deux atomes à partir du point d'attache est facultativement substitué par R¹², un carbone cyclique ou trois atomes d'azote à partir du point d'attache et adjacents à la position de R¹⁰ sont facultativement substitués par R¹¹, un carbone cyclique ou trois atomes d'azote à partir du point d'attache et adjacents à la position de R¹² sont facultativement substitués par R³³, et un carbone cyclique ou quatre atomes d'azote à partir du point d'attache et adjacents aux positions de R¹¹ et R³³ sont facultativement substitués par R³⁴ ;
R³², R³³ R³⁴, R³⁵ et R³⁶ sont indépendamment sélectionnés parmi le groupe constitué d'un hydrido, d'un acétamido, d'un haloacétamido, d'un amidino, d'un guanidino, d'un alcoxy, d'un hydroxy, d'un amino, d'un alcoxy-amino, d'un alkylamino inférieur, d'un alkylthio, d'un amidosulfonyle, d'un monoalkyl amidosulfonyle, d'un dialkyl amidosulfonyle, d'un alkyle, d'un halo, d'un haloalkyle, d'un haloalcoxy, d'un hydroxyalkyle, d'un carboalcoxy, d'un carboxy, d'un carboxyamido, et d'un cyano;
A est sélectionné parmi le groupe constitué d'une liaison covalente simple et (CH(R¹⁵))ₚₐ₋(W⁷)ᵣᵣ où rr est un nombre entier sélectionné de 0 à 1, pa est un nombre entier sélectionné de 0 à 3, et W⁷ est N(R⁷) ;
R⁷ est sélectionné parmi le groupe constitué d'un hydrido et d'un alkyle ;
R¹⁵ est sélectionné parmi le groupe constitué d'un hydrido, d'un halo, d'un alkyle et d'un haloalkyle ;
R¹ est sélectionné parmi le groupe constitué d'un hydrido, d'un cyano, d'un haloalkyle et d'un halo ;
R² est Z⁰-Q ;
Z⁰ est une liaison covalente simple ;
Q est un phényle dans lequel deux atomes de carbone à partir du carbone au point d'attache sont facultativement substitués par un amino ;
R⁹, R¹¹, et R¹³ sont indépendamment sélectionnés parmi le groupe constitué d'un hydrido, d'un hydroxy, d'un amino, d'un amidino, d'un guanidino, d'un alkylamino inférieur, d'un alkylthio, d'un alcoxy, d'un alkylsulfinyle, d'un alkylsulfonyle, d'un amidosulfonyle, d'un monoalkylamidosulfonyle, d'un alkyle, d'un halo, d'un haloalkyle, d'un haloalcoxy, d'un hydroxyalkyle, d'un carboxy, d'un carboxamido, et d'un cyano;
R¹⁰ et R¹² sont indépendamment sélectionnés parmi le groupe constitué d'un hydrido, d'un acétamido, d'un haloacétamido, d'un amidino, d'un guanidino, d'un alkyle, d'un alcoxy, d'un alcoxyamino, d'un aminoalkyle, d'un hydroxy, d'un amino, d'un alkylamino inférieur, d'un alkylsulfonamido, d'un amidosulfonyle, d'un monoalkyl amidosulfonyle, d'un dialkyl amidosulfonyle, d'un hydroxyalkyle, d'un aminoalkyle, d'un halo, d'un haloalkyle, d'un carboalcoxy, d'un carboxy, d'un carboxyamido, d'un carboxyalkyle, et d'un cyano;
Y° est de formule (IV) : 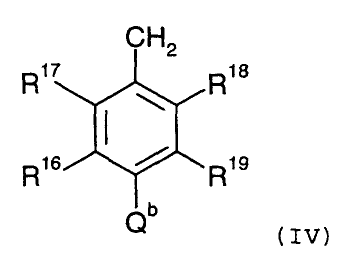 dans lequel
R¹⁶, R¹⁷, R¹⁸ et R¹⁹ sont indépendamment sélectionnés parmi le groupe constitué d'un hydrido, d'un amidino, d'un guanidino, d'un carboxy, d'un haloalkylthio, d'un alcoxy, d'un hydroxy, d'un amino, d'un alkylamino inférieur, d'un alkylthio, d'un alkylsulfinyle, d'un alkylsulfonyle, d'un alcanoyle, d'un haloalcanoyle, d'un alkyle, d'un halo, d'un haloalkyle, d'un haloalcoxy, d'un hydroxyalkyle, d'un aminoalkyle, et d'un cyano ;
Q^{b} est C(NR²⁵)NR²³R²⁴ ;
R²³, R²⁴ et R²⁵ sont un hydrido ;
et dans lequel
Le terme "alkyle" soit seul soit dans d'autres termes, sauf mention contraire, signifie un radical alkyle acyclique contenant de 1 à 10 atomes de carbone ;
Le terme "alcoxy" signifie un radical contenant un oxy linéaire ou ramifié ayant une portion alkyle de 1 à 10 atomes de carbone ;
Le terme "aryle" seul ou en combinaison, signifie un système aromatique carbocyclique contenant 1, 2 ou 3 cycles dans lequel de tels cycles peuvent être rattachés ensemble de manière pendante ou peuvent être condensés ;
Le terme "hétérocyclyle" signifie des radicaux de forme cyclique contenant des hétéroatomes saturés et partiellement saturés ayant de 4 à 15 éléments cycliques sélectionnés parmi le carbone, l'azote, le soufre et l'oxygène, où au moins un atome cyclique est un hétéroatome ;
Le terme "hétéroaryle" signifie des radicaux aromatiques de forme cyclique contenant des hétéroatomes complètement non saturés ayant de 4 à 15 éléments cycliques sélectionnés parmi le carbone, l'azote, le soufre et l'oxygène, où au moins un atome cyclique est un hétéroatome.

2. Composé selon la revendication 1 ou un sel pharmaceutiquement acceptable de celui-ci, dans lequel ;
B est sélectionné parmi le groupe constitué d'un phényle, d'un 2-thiényle, d'un 3-thiényle, d'un 2-furyle, d'un 3-furyle, d'un 2-pyrrolyle, d'un 3-pyrrolyle, d'un 2-imidazolyle, d'un 4-imidazolyle, d'un 3-pyrazolyle, d'un 4-pyrazolyle, d'un 2-thiazolyle, d'un 3-isoxazolyle, et d'un 5-isoxazolyle, dans lequel un carbone adjacent au carbone au point d'attache est facultativement substitué par R³², l'autre carbone adjacent au carbone au point d'attache est facultativement substitué par R³⁶, un carbone adjacent à R³² et à deux atomes à partir de carbone au point d'attache est facultativement substitué par R³³, un carbone adjacent à R³⁶ et à deux atomes à partir du carbone au point d'attache est facultativement substitué par R³⁵, et tout carbone adjacent à la fois à R³³ et R³⁵ est facultativement substitué par R³⁴ ;
R³², R³³, R³⁴, R³⁵, et R³⁶ sont indépendamment sélectionnés parmi le groupe constitué d'un hydrido, d'un amidino, d'un guanidino, d'un méthyle, d'un éthyle, d'un méthoxy, d'un éthoxy, d'un hydroxy, d'un amino, d'un N-méthylamino, d'un diméthylamino, d'un méthylthio, d'un éthylthio, d'un trifluorométhyle, d'un pentafluoroéthyle, d'un 2,2,2-trifluoroéthyle, d'un fluoro, d'un chloro, d'un bromo, d'un amidosulfonyle, d'un N-méthylamidosulfonyle, d'un hydroxyméthyle, d'un amidocarbonyle, d'un carboxy, et d'un cyano ;
A est sélectionné parmi le groupe constitué d'une liaison covalente simple, NH, N(CH₃), CH₂, CH₃CH, et CH₂CH₂ ;
R¹ est sélectionné parmi le groupe constitué d'un hydrido, d'un trifluorométhyle, d'un pentafluoroéthyle, d'un fluoro et d'un chloro ;
R⁹, R¹¹, et R¹³ sont indépendamment sélectionnés parmi le groupe constitué d'un hydrido, d'un méthyle, d'un éthyle, d'un méthoxy, d'un éthoxy, d'un hydroxy, d'un amino, d'un N-méthylamino, d'un N,N-diméthylamino, d'un méthylthio, d'un trifluorométhyle, d'un pentafluoroéthyle, d'un 2,2,2-trifluoroéthyle, d'un fluoro, d'un chloro, d'un bromo, d'un amidosulfonyle, d'un N-méthylamidosulfonyle, d'un N,N-diméthylamidosulfonyle, d'un hydroxyméthyle, d'un 1-hydroxyéthyle, d'un amidocarbonyle, d'un N-méthylamidocarbonyle, d'un carboxy, et d'un cyano ;
R¹⁰ et R¹² sont indépendamment sélectionnés parmi le groupe constitué d'un hydrido, d'un amidino, d'un amidocarbonyle, d'un N-méthylamidocarbonyle, d'un guanidino, d'un méthyle, d'un éthyle, d'un méthoxy, d'un éthoxy, d'un hydroxy, d'un hydroxyméthyle, d'un 1-hydroxyéthyle, d'un 2-hydroxyéthyle, d'un carboxy, d'un carboxyméthyle, d'un amino, d'un acétamido, d'un trifluorométhyle, d'un pentafluorométhyle, d'un 2,2,2-trifluoroéthyle, d'un trifluoroacétamido, d'un aminométhyle, d'un N-méthylamino, d'un diméthylamino, d'un amidosulfonyle, d'un N-méthylamidosulfonyle, d'un N,N-diméthylamidosulfonyle, d'un méthoxycarbonyle, d'un fluoro, d'un chloro, d'un bromo, et d'un cyano ;
Y° est un 1-Q^{b}-4-Q^{s}-2-R¹⁶-3-R¹⁷-5-R¹⁸-6-R¹⁹benzène ;
R¹⁶, R¹⁷, R¹⁸, et R¹⁹ sont indépendamment sélectionnés parmi le groupe constitué d'un hydrido, d'un méthyle, d'un éthyle, d'un amidino, d'un guanidino, d'un méthoxy, d'un hydroxy, d'un amino, d'un aminométhyle, d'un 1-aminoéthyle, d'un 2-aminoéthyle, d'un N-méthylamino, d'un diméthylamino, d'un méthylthio, d'un éthylthio, d'un trifluorométhylthio, d'un méthylsulfinyle, d'un méthylsulfonyle, d'un trifluorométhyle, d'un pentafluoroéthyle, d'un 2,2,2-trifluoroéthyle, d'un trifluorométhoxy, d'un fluoro, d'un chloro, d'un amidosulfonyle, d'un N-méthylamidosulfonyle, d'un hydroxyméthyle, d'un carboxy, et d'un cyano.

3. Composé selon la revendication 1 ou un sel pharmaceutiquement acceptable de celui-ci, dans lequel ;
B est sélectionné parmi le groupe constitué de 2-aminophényle, 3-aminophényle, 3-amidinophényle, 4-amidinophényle, 3-carboxyphényle, 3-carboxy-5 hydroxyphényle, 3-chlorophényle, 4-chlorophényle, 3,4-dichlorophényle, 2-fluorophényle, 3-fluorophényle, 3,4-difluorophényle, 3-hydroxyphényle, 4-hydroxyphényle, 3-méthoxyaminophényle, 3-méthoxyphényle, 4-méthoxyphényle, 3-méthylphényle, 4-méthylphényle, phényle, 3-trifluorométhylphényle, 2-imidazoyle, 2 pyridyle, 3 pyridyle, 5-chloro-3-trifluorométhyl-2-pyridyle, 4-pyridyle, 2-thiényle, 3-thiényle, et 3 trifluorométhyl-2-pyridyle ;
A est sélectionné parmi le groupe constitué de CH₂, CH₃CH, CF₃CH, NHC(O), CH₂CH₂, et CH₂CH₂CH₂ ;
R¹ est sélectionné parmi le groupe constitué d'un hydrido, d'un trifluorométhyle, d'un pentafluoroéthyle, d'un fluoro et d'un chloro ;
Y° est un 1-Q^{b}-4-Q^{s}-2-R¹⁶-3-R¹⁷-5-R¹⁸-6-R¹⁹benzène ;
R¹⁶ et R¹⁹ sont indépendamment sélectionnés parmi le groupe constitué de :
(i) un hydrido, un amidino, un amino, un aminométhyle, un méthoxy, un méthylamino, un hydroxy, un hydroxyméthyle, un fluoro, un chloro, et un cyano ; et
(ii) Q^{b} à condition que pas plus de un de R¹⁶ et R¹⁹ soit Q^{b} au même moment ;
R¹⁷ et R¹⁸ sont indépendamment sélectionnés parmi le groupe constitué d'un hydrido, d'un fluoro, d'un chloro, d'un hydroxy, d'un hydroxyméthyle, d'un amino, d'un carboxy, et d'un cyano.

4. Composé selon la revendication 3 ou un sel pharmaceutiquement acceptable de celui-ci, dans lequel :
B est sélectionné parmi le groupe constitué de 3-aminophényle, 3-amidinophényle, 4-amidinophényle, 3-chlorophényle, 4-chlorophényle, 3,4-dichlorophényle, 2-fluorophényle, 4-méthylphényle, phényle, 2-imidazoyle, 3-pyridyle, 4-pyridyle, et 3-trifluorométhyl-2-pyridyle ;
A est sélectionné parmi le groupe constitué de CH₂, NHC(O), CH₂CH₂, et CH₂CH₂CH₂ ;
R¹ est sélectionné parmi le groupe constitué d'un hydrido et d'un chloro ;
Y° est sélectionné parmi le groupe constitué d'un 4-amidinobenzyle, 2-fluoro-4-amidinobenzyle, et 3-fluoro-4-amidinobenzyle ;

5. Composé selon la revendication 1 ou un sel pharmaceutiquement acceptable de celui-ci, dans lequel :
R² est un 3-aminophényle, B est un 3-chlorophényle, A est CH₂CH₂, Y° est un 4-amidinobenzyle et R¹ est un chloro ;
R² est un 3-aminophényle, B est un phényle, A est CH₂, Y° est un 4-amidinobenzyle et R¹ est un chloro ;
R² est un 3-aminophényle, B est un 2-imidazoyle, A est CH₂CH₂CH₂, Y° est un 4-amidinobenzyle et R¹ est un chloro.

6. Composé selon la revendication 1 ou un sel pharmaceutiquement acceptable de celui-ci, dans lequel ;
B est sélectionné parmi le groupe constitué d'un hydrido, d'un éthyle, d'un 2-propényle, d'un 2-propynyle, d'un propyle, d'un isopropyle, d'un butyle, d'un 2-butényle, d'un 2-butynyle, d'un sec-butyle, d'un tert-butyle, d'un isobutyle, d'un 2-méthylpropényle, d'un 1-pentyle, d'un 2-pentényle, d'un 3-pentényle, d'un 2-pentynyle, d'un 3-pentynyle, d'un 2-pentyle, d'un 3-pentyle, d'un 2-méthylbutyle, d'un 2-méthyl-2-butényle, d'un 3-méthylbutyle, d'un 3-méthyl-2-butényle, d'un 1-hexyle, d'un 2-hexényle, d'un 3-hexényle, d'un 4-hexényle, d'un 2-hexynyle, d'un 3-hexynyle, d'un 4-hexynyle, d'un 2-hexyle, d'un 1-méthyl-2-pentényle, d'un 1-méthyl-3-pentényle, d'un 1-méthyl-2-pentynyle, d'un 1-méthyl-3-pentynyle, d'un 3-hexyle, d'un 1-éthyl-2-butényle, d'un 1-heptyle, d'un 2-heptényle, d'un 3-heptényle, d'un 4-heptényle, d'un 5-heptényle, d'un 2-heptynyle, d'un 3-heptynyle, d'un 4-heptynyle, d'un 5-heptynyle, d'un 2-heptyle, d'un 1-méthyl-2-hexényle, d'un 1-méthyl-3-hexényle, d'un 1-méthyl-4-hexényle, d'un 1-méthyl-2-hexynyle, d'un 1-méthyl-3-hexynyle, d'un 1-méthyl-4-hexynyle, d'un 3-heptyle, d'un 1-éthyl-2-pentényle, d'un 1-éthyl-3-pentényle, d'un 1-éthyl-2-pentynyle, d'un 1-éthyl-3-pentynyle, d'un 2,2,2-trifluoroéthyle, d'un 2,2-difluoropropyle, d'un 4-trifluorométhyl-5,5,5-trifluoropentyle, d'un 4-trifluorométhylpentyle, d'un 5,5,6,6,6-pentafluorohexyle, et d'un 3,3,3-trifluoropropyle, dans lequel chaque élément du groupe B est facultativement substitué à tout carbone jusqu'à et y compris 5 atomes à partir du point d'attache de B à A avec l'un ou plusieurs du groupe constitué de R³², R³³, R³⁴, R³⁵ et R³⁶ ;
R³², R³³, R³⁴, R³⁵ et R³⁶ sont indépendamment sélectionnés parmi le groupe constitué d'un hydrido, d'un amidino, d'un guanidino, d'un méthyle, d'un éthyle, d'un méthoxy, d'un éthoxy, d'un hydroxy, d'un amino, d'un N-méthylamino, d'un diméthylamino, d'un méthylthio, d'un éthylthio, d'un trifluorométhyle, d'un pentafluoroéthyle, d'un 2,2,2-trifluoroéthyle, d'un fluoro, d'un chloro, d'un bromo, d'un amidosulfonyle, d'un N-méthylamidosulfonyle, d'un hydroxyméthyle, d'un amidocarbonyle, d'un carboxy, et d'un cyano ;
A est sélectionné parmi le groupe constitué :
(i) d'une liaison covalente simple, NH, (N(CH₃), CH₂, CH₃CH, et CH₂CH₂ ; et
(ii) CH₂N(CH₃), CH₂N(CH₂CH₃), CH₂CH₂N(CH₃), et CH₂CH₂N(CH₂CH₃) à condition que B soit un hydrido ;
R¹ est sélectionné parmi le groupe constitué d'un hydrido, d'un trifluorométhyle, d'un pentafluoroéthyle, d'un fluoro et d'un chloro ;
R⁹, R¹¹, et R¹³ sont indépendamment sélectionnés parmi le groupe constitué d'un hydrido, d'un méthyle, d'un éthyle, d'un méthoxy, d'un éthoxy, d'un hydroxy, d'un amino, d'un N-méthylamino, d'un N,N-diméthylamino, d'un méthylthio, d'un trifluorométhyle, d'un pentafluoroéthyle, d'un 2,2,2-trifluoroéthyle, d'un fluoro, d'un chloro, d'un bromo, d'un amidosulfonyle, d'un N-méthylamidosulfonyle, d'un N,N-diméthylamidosulfonyle, d'un hydroxyméthyle, d'un 1-hydroxyéthyle, d'un amidocarbonyle, d'un N-méthylamidocarbonyle, d'un carboxy, et d'un cyano ;
R¹⁰ et R¹² sont indépendamment sélectionnés parmi le groupe constitué d'un hydrido, d'un amidino, d'un amidocarbonyle, d'un N-méthylamidocarbonyle, d'un guanidino, d'un méthyle, d'un éthyle, d'un méthoxy, d'un éthoxy, d'un hydroxy, d'un hydroxyméthyle, d'un 1-hydroxyéthyle, d'un 2-hydroxyéthyle, d'un carboxy, d'un carboxyméthyle, d'un amino, d'un acétamido, d'un trifluorométhyle, d'un pentafluoroéthyle, d'un 2,2,2-trifluoroéthyle, d'un trifluoroacétamido, d'un aminométhyle, d'un N-méthylamino, d'un diméthylamino, d'un amidosulfonyle, d'un N-méthylamidosulfonyle, d'un N,N-diméthylamidosulfonyle, d'un méthoxycarbonyle, d'un fluoro, d'un chloro, d'un bromo, et d'un cyano ;
Y^{o} est un 1-Q^{b}-4-Q^{s}-2-R¹⁶-3-R¹⁷-5-R¹⁸-6-R¹⁹benzène;
R¹⁶, R¹⁷, R¹⁸, et R¹⁹ sont indépendamment sélectionnés parmi le groupe constitué d'un hydrido, d'un méthyle, d'un éthyle, d'un amidino, d'un guanidino, d'un méthoxy, d'un hydroxy, d'un amino, d'un aminométhyle, d'un 1-aminoéthyle, d'un 2-aminoéthyle, d'un N-méthylamino, d'un diméthylamino, d'un méthylthio, d'un éthylthio, d'un trifluorométhylthio, d'un méthylsulfinyle, d'un méthylsulfonyle, d'un trifluorométhyle, d'un pentafluoroéthyle, d'un 2,2,2-trifluoroéthyle, d'un trifluorométhoxy, d'un fluoro, d'un chloro, d'un amidosulfonyle, d'un N-méthylamidosulfonyle, d'un hydroxyméthyle, d'un carboxy, et d'un cyano

7. Composé selon la revendication 6 ou un sel pharmaceutiquement acceptable de celui-ci, dans lequel :
B est sélectionné parmi le groupe constitué d'un hydrido, d'un éthyle, d'un 2-propényle, d'un 2-propynyle, d'un propyle, d'un isopropyle, d'un butyle, d'un 2-butyle, d'un (R)-2-butyle,(S)-2-butyle, d'un tert-butyle, d'un isobutyle, d'un 1-pentyle, d'un 3-pentyle, d'un 2-méthylbutyle, d'un 2,2,2-trifluoroéthyle, d'un 6-amidocarbonylhexyle, d'un 4-méthyl-2-pentyle, d'un 3-hydroxypropyle, d'un 3-méthoxy-2-propyle, d'un 2-méthoxyéthyle, d'un 2-méthyl-2-butyle, d'un 3-méthyl-2-butyle, d'un 2-diméthylaminopropyle, d'un 2-cyanoéthyle, d'un 6-hydroxyhexyle, d'un 2-hydroxyéthyle, d'un 2-amidinoéthyle, d'un 2-guanidinoéthyle, d'un 3-guanidinopropyle, d'un 4-guanidinobutyle, d'un 3-hydroxypropyle, d'un 4-hydroxybutyle, d'un 6-cyanohexyle, d'un 2-diméthylaminoéthyle, d'un 3-méthylbutyle, d'un 2-méthylbutyle, d'un (S)-2-méthylbutyle, d'un 3-aminopropyle, d'un 2-hexyle, et d'un 4-aminobutyle ;
A est sélectionné parmi le groupe constitué d'une liaison covalente simple, CH₂, CH₃CH et CH₂CH₂ ;
R¹ est sélectionné parmi le groupe constitué d'un hydrido, d'un trifluorométhyle, d'un fluoro et d'un chloro ;
Y° est un 1-Q^{b}-4-Q^{s}-2-R¹⁶-3-R¹⁷-5-R¹⁸-6-R¹⁹benzène ;
R¹⁶ et R¹⁹ sont indépendamment sélectionnés parmi le groupe constitué de :
(i) d'un hydrido, d'un amidino, d'un amino, d'un aminométhyle, d'un méthoxy, d'un méthylamino, d'un hydroxy, d'un hydroxyméthyle, d'un fluoro, d'un chloro, et d'un cyano ; et
(ii) Q^{b} à condition que pas plus de un de R¹⁶ et R¹⁹ soit Q^{b} au même moment ;
R¹⁷ et R¹⁸ sont indépendamment sélectionnés parmi le groupe constitué d'un hydrido, d'un fluoro, d'un chloro, d'un hydroxy, d'un hydroxyméthyle, d'un amino, d'un carboxy, et d'un cyano.

8. Composé selon la revendication 7 ou un sel pharmaceutiquement acceptable de celui-ci, dans lequel :
B est sélectionné parmi le groupe constitué d'un hydrido, d'un éthyle, d'un 2-propényle, d'un 2-propynyle, d'un propyle, d'un isopropyle, d'un butyle, d'un 2-butyle, d'un (R)-2-butyle,(S)-2-butyle, d'un tert-butyle, d'un isobutyle, d'un 1-pentyle, d'un 3-pentyle, d'un 2-méthylbutyle, d'un 2,2,2-trifluoroéthyle, d'un 6-amidocarbonylhexyle, d'un 4-méthyl-2-pentyle, d'un 3-hydroxypropyle, d'un 3-méthoxy-2-propyle, d'un 2-méthoxyéthyle, d'un 2-méthyl-2-butyle, d'un 3-méthyl-2-butyle, d'un 2-diméthylaminopropyle, d'un 2-cyanoéthyle, d'un 6-hydroxyhexyle, d'un 2-hydroxyéthyle, d'un 2-anidinoéthyle, d'un 2-guanidinoéthyle, d'un 3-guanidinopropyle, d'un 4-guanidinobutyle, d'un 3-hydroxypropyle, d'un 4-hydroxybutyle, d'un 6-cyanohexyle, d'un 2-diméthylaminoéthyle, d'un 3-méthylbutyle, d'un 2-méthylbutyle, d'un (S)-2-méthylbutyle, d'un 3-aminopropyle, d'un 2-hexyle, et d'un 4-aminobutyle ;
A est sélectionné parmi le groupe constitué d'une liaison covalente simple, CH₂, CH₃CH et CH₂CH₂ ;
R¹ est sélectionné parmi le groupe constitué d'un hydrido, et d'un chloro ;
Y° est sélectionné parmi le groupe constitué d'un 4-amidinobenzyle, d'un 2-fluoro-4-amidinobenzyle et d'un 3-fluoro-4-amidinobenzyle.

9. Composé selon la revendication 1 ou un sel pharmaceutiquement acceptable de celui-ci, dans lequel :
R² est un 3-aminophényle, B est un 2,2,2-trifluoroéthyle, A est une liaison simple, Y° est 4-amidinobenzyle et R¹ est un chloro ;
R² est un 3-aminophényle, B est un (S)-2-butyle, A est une liaison simple, Y° est un 4-amidinobenzyle, et R¹ est un chloro ;
R² est un 3-aminophényle, B est un éthyle, est une liaison simple, Y° est un 4-amidinobenzyle, et R¹ est un chloro ;
R² est un 3-aminophényle, B est un éthyle, A est une liaison simple, Y° est un 4-amidino-2-fluorobenzyle, et R¹ est un chloro ;
R² est un 3-aminophényle, B est un 2-propényle, A est une liaison simple, y° est un 4-amidinobenzyle et R¹ est un chloro ;
R² est un 3-aminophényle, B est un isopropyle, A est une liaison simple, Y° est un 4-amidino-2-fluorobenzyle et R¹ est un chloro ;
R² est un 3-aminophényle, B est un isopropyle, A est une liaison simple, Y° est un 4-amidinobenzyle et R¹ est un chloro ;
R² est un 3-aminophényle, B est un 2-butyle, A est une liaison simple, Y° est un 4-amidinobenzyle et R¹ est un chloro ;
R² est un 3-aminophényle, B est un (R)-2-butyle, A est une liaison simple, Y° est un 4-amidinobenzyle et R¹ est un chloro ;
R² est un 3-aminophényle, B est un 2-propynyle, A est une liaison simple, Y° est un 4-amidinobenzyle et R¹ est un chloro ;
R² est un 3-aminophényle, B est un 3-pentyle, A est une liaison simple, Y° est un 4-amidinobenzyle et R¹ est un hydrido ;
R² est un 3-aminophényle, B est un hydrido, A est CH₂, Y° est un 4-amidinobenzyle et R¹ est un chloro ;
R² est un 3-aminophényle, B est un éthyle, A est CH₂, Y° est un 4-amidinobenzyle et R¹ est un chloro ;
R² est un 3-aminophényle, B est un 2-méthylpropyle, A est une liaison simple, Y° est un 4-amidinobenzyle et R¹ est un chloro ;
R² est un 3-aminophényle, B est un 2-propyle, A est CH₃CH, Y° est un 4-amidinobenzyle et R¹ est un chloro ;
R² est un 3-aminophényle, B est un propyle, A est une liaison simple, Y° est un 4-amidino-2-fluorobenzyle et R¹ est un chloro ;
R² est un 3-aminophényle, B est un 6-amidocarbonylhexyle, A est une liaison simple, Y° est un 4-amidinobenzyle et R¹ est un chloro ;
R² est un 3-aminophényle, B est un tert-butyle, A est une liaison simple, Y° est un 4-amidinobenzyle et R¹ est un hydrido ;
R² est un 3-aminophényle, B est tert-butyle, A est une liaison simple, Y° est un 4-amidinobenzyle et R¹ est un chloro ;
R² est un 3-aminophényle, B est un 3-hydroxypropyle, A est une liaison simple, Y° est un 4-amidinobenzyle et R¹ est un chloro ;
R² est un 3-aminophényle, B est un 2-méthylpropyle, A est une liaison simple, Y° est un 4-amidino-2-fluorobenzyle et R¹ est un chloro ;
R² est un 3-aminophényle, B est un butyle, A est une liaison simple, Y° est un 4-amidinobenzyle et R¹ est un chloro ;
R² est un 3-aminophényle, B est un 3-méthoxy-2-propyle, A est une liaison simple, Y° est un 4-amidinobenzyle et R¹ est un chloro ;
R² est un 3-aminophényle, B est un 3-méthoxy-2-propyle, A est une liaison simple, Y° est un 4-amidinobenzyle et R¹ est un chloro ;
R² est un 3-aminophényle, B est un 2-méthoxy-2-éthyle, A est une liaison simple, Y° est un 4-amidinobenzyle et R¹ est un chloro ;
R² est un 3-aminophényle, B est un 2-propyle, A est une liaison simple, Y° est un 4-amidino-3-fluorobenzyle et R¹ est un hydrido ;
R² est un 3-aminophényle, B est un 2-propyle, A est une liaison simple, Y° est un 4-amidino-3-fluorobenzyle et R¹ est un chloro.

10. Composé selon la revendication 1 ou un sel pharmaceutiquement acceptable de celui-ci, dans lequel ;
B est sélectionné parmi le groupe constitué d'un cyclopropyle, d'un cyclobutyle, d'un cyclopentyle, d'un cyclohexyle, d'un cycloheptyle, d'un oxalan-2-yle, d'un 2-(2R)-bicyclo[2,2-1] heptyle, d'un 1,1-dioxothiolan-3-yle, d'un oxétan-3-yle, d'un azétidin-1-yle, d'un azétidin-2-yle, d'un azétidin-3-yle, d'un bicyclo[3,1,0]hexan-6-yle, d'un 2-morpholinyle, d'un 3-morpholinyle, d'un 4-morpholinyle, d'un 1-pipérazinyle, d'un 2-pipérazinyle, d'un 1-pipéridinyle, d'un 2-pipéridinyle, d'un 3-pipéridinyle, d'un 4-pipéridinyle, d'un 1-pyrrolidinyle, d'un 2-pyrrolidinyle, d'un 3-pyrrolidinyle, d'un 2-dioxanyle, d'un 2-tétrahydrofuranyle, d'un 3-tétrahydrofuranyle, d'un 2-tétrahydropyranyle, d'un 3-tétrahydropyranyle, d'un 4-tétrahydropyranyle, d'un 2-tétrahydrothiényle, et d'un 3-tétrahydrothiényle, dans lequel chaque carbone cyclique est facultativement substitué par R³³, des carbones cycliques et un azote adjacent à l'atome de carbone au point d'attache sont facultativement substitués par R⁹ ou R¹³, un carbone cyclique ou un azote adjacent à la position de R⁹ et à deux atomes partir du point d'attache est facultativement substitué par R¹⁰, et un carbone cyclique ou un atome d'azote adjacent à la position de R¹³ et à deux atomes à partir du point d'attache est facultativement substitué par R¹² ;
R⁹, R¹¹, et R¹³ sont indépendamment sélectionnés parmi le groupe constitué d'un hydrido, d'un méthyle, d'un éthyle, d'un méthoxy, d'un éthoxy, d'un hydroxy, d'un amino, d'un N-méthylamino, d'un N,N-diméthylamino, d'un méthylthio, d'un trifluorométhyle, d'un pentafluoroéthyle, d'un 2,2,2-trifluoroéthyle, d'un fluoro, d'un chloro, d'un bromo, d'un amidosulfonyle, d'un N-méthylamidosulfonyle, d'un N,N-diméthylamidosulfonyle, d'un hydroxyméthyle, d'un 1-hydroxyéthyle, d'un amidocarbonyle, d'un N-méthylamidocarbonyle, d'un carboxy, et d'un cyano;
R¹⁰ et R¹² sont indépendamment sélectionnés parmi le groupe constitué d'un hydrido, d'un amidino, d'un amidocarbonyle, d'un N-méthylamidocarbonyle, d'un guanidino, d'un méthyle, d'un éthyle, d'un méthoxy, d'un éthoxy, d'un hydroxy, d'un hydroxyméthyle, d'un 1-hydroxyéthyle, d'un 2-hydroxyéthyle, d'un carboxy, d'un carboxyméthyle, d'un amino, d'un acétamido, d'un trifluorométhyle, d'un pentafluoroêthyle, d'un 2,2,2-trifluoroéthyle, d'un trifluoroacétamido, d'un aminométhyle, d'un N-méthylamino, d'un diméthylamino, d'un amidosulfonyle, d'un N-méthylamidosulfonyle, d'un N,N-diméthylamidosulfonyle, d'un méthoxycarbonyle, d'un fluoro, d'un chloro, d'un bromo, et d'un cyano;
R³³ est sélectionné parmi le groupe constitué d'un hydrido, d'un amidino, d'un guanidino, d'un méthyle, d'un éthyle, d'un méthoxy, d'un éthoxy, d'un hydroxy, d'un carboxy, d'un amino, d'un N-méthylamino, d'un diméthylamino, d'un méthylthio, d'un éthylthio, d'un trifluorométhyle, d'un pentafluoroéthyle, d'un 2,2,2-trifluoroéthyle, d'un fluoro, d'un chloro, d'un bromo, d'un amidosulfonyle, d'un N-méthylamidosulfonyle, d'un hydroxyméthyle, d'un amidocarbonyle, et d'un cyano;
A est sélectionné parmi le groupe constitué d'une liaison covalente simple, NH, N(CH₃), CH₂, CH₃CH, CH₂CH₂, et CH₂CH₂CH₂ ;
R¹ est sélectionné parmi le groupe constitué d'un hydrido, d'un trifluorométhyle, d'un pentafluoroéthyle, d'un fluoro et d'un chloro ;
Y^{o} est un 1-Q^{b}-4-Q^{s}-2-R¹⁶-3-R¹⁷-5-R¹⁸-6-R¹⁹benzène;
R¹⁶, R¹⁷, R¹⁸, et R¹⁹ sont indépendamment sélectionnés parmi le groupe constitué d'un hydrido, d'un méthyle, d'un éthyle, d'un amidino, d'un guanidino, d'un méthoxy, d'un hydroxy, d'un amino, d'un aminométhyle, d'un 1-aminoéthyle, d'un 2-aminoéthyle, d'un N-méthylamino, d'un diméthylamino, d'un méthylthio, d'un éthylthio, d'un trifluorométhylthio, d'un méthylsulfinyle, d'un méthylsulfonyle, d'un trifluorométhyle, d'un pentafluoroéthyle, d'un 2,2,2-trifluoroéthyle, d'un trifluorométhoxy, d'un fluoro, d'un chloro, d'un amidosulfonyle, d'un N-méthylamidosulfonyle, d'un hydroxyméthyle, d'un carboxy, et d'un cyano.

11. Composé selon la revendication 10 ou un sel pharmaceutiquement acceptable de celui-ci, dans lequel ;
B est sélectionné parmi le groupe constitué d'un cyclopropyle, d'un cyclobutyle, d'un cyclopentyle, d'un cyclohexyle, d'un oxalan-2-yle, d'un 2-(2R)-bicyclo[2,2,1]-heptyle, d'un 1,1-dioxothiolan-3-yle, d'un oxétan-3-yle, d'un azétidin-1-yle, d'un azétidin-2-yle, d'un azétidin-3-yle, d'un 1-pyrrolidinyl et d'un 1-pipéridinyle ;
A est sélectionné parmi le groupe constitué d'une liaison covalente simple, CH₂, NHC(O), CH₂CH₂ et CH₂CH₂CH₂ ;
R¹ est sélectionné parmi le groupe constitué d'un hydrido, d'un trifluorométhyle, d'un pentafluoroéthyle, d'un fluoro et d'un chloro ;
Y^{o} est un 1-Q^{b}-4-Q^{s}-2-R¹⁶-3-R¹⁷-5-R¹⁸-6-R¹⁹benzène ;
R¹⁶ et R¹⁹ sont indépendamment sélectionnés parmi le groupe constitué de
(i) un hydrido, un amidino, un amino, un aminométhyle, un méthoxy, un méthylamino, un hydroxy, un hydroxyméthyle, un fluoro, un chloro, et un cyano;
(ii) Q^{b} à condition que pas plus de un de R¹⁶ soit Q^{b} au même moment ;
R¹⁷ et R¹⁸ sont indépendamment sélectionnés parmi le groupe constitué d'un hydrido, d'un fluoro, d'un chloro, d'un hydroxy, d'un hydroxyméthyle, d'un amino, d'un carboxy, et d'un cyano.

12. Composé selon la revendication 11 ou un sel pharmaceutiquement acceptable de celui-ci, dans lequel ;
B est sélectionné parmi le groupe constitué d'un cyclopropyle, d'un cyclobutyle, d'un cyclopentyle, d'un cyclohexyle, d'un oxalan-2-yle, d'un 2-(2R)-bicyclo[2,2,1]-heptyle, d'un 1,1-dioxothiolan-3-yle, d'un oxétan-3-yle, d'un azétidin-1-yle, d'un azétidin-2-yle, d'un azétidin-3-yle, et d'un 1-pipéridinyle ;
A est sélectionné parmi le groupe constitué d'une liaison covalente simple de CH₂, CH₂CH₂ et CH₂CH₂CH₂ ;
R¹ est sélectionné parmi le groupe constitué d'un hydrido, et d'un chloro ;
Y° est sélectionné parmi le groupe constitué d'un 4-amidinobenzyle, d'un 2-fluoro-4-amidinobenzyle et d'un 3-fluoro-4-amidinobenzyle.

13. Composé selon la revendication 1 ou un sel pharmaceutiquement acceptable de celui-ci, dans lequel :
R² est un 3-aminophényle, B est un cyclopropyle, A est une liaison simple, Y^{o} est un 4-amidinobenzyle et R¹ est un chloro ;
R² est un 3-aminophényle, B est un cyclobutyle, A est une liaison simple, Y^{o} est un 4-amidino-2-fluorobenzyle et R¹ est un chloro ;
R² est un 3-aminophényle, B est un cyclobutyle, A est une liaison simple, Y^{o} est un 4-amidinobenzyle et R¹ est un chloro ;
R² est un 3-aminophényle, B est un cyclopropyle, A est une liaison simple, Y^{o} est un 4-amidino-2-fluorobenzyle et R¹ est un chloro ;
R² est un 3-aminophényle, B est un cyclobutyle, A est une liaison simple, Y^{o} est un 4-amidinobenzyle et R¹ est un hydrido ;
R² est un 3-aminophényle, B est un cyclobutyle, A est une liaison simple, Y^{o} est un 4-amidino-3-fluorobenzyle et R¹ est un chloro ;
R² est un 3-aminophényle, B est un cyclopentyle, A est une liaison simple, Y^{o} est un 4-amidinobenzyle et R¹ est un chloro ;
R² est un 3-aminophényle, B est un cyclopropyle, A est CH₂, Y^{o} est un 4-amidinobenzyle et R¹ est un chloro ;
R² est un 3-aminophényle, B est un 2(2R)-bicyclo[2,2,1]-heptyle, A est une liaison simple, Y° est un 4-amidinobenzyle et R¹ est un chloro ;
R² est un 3-aminophényle, B est un cyclopentyle, A est une liaison simple, Y^{o} est un 4-amidino-2-fluorobenzyle et R¹ est un chloro ;
R² est un 3-aminophényle, B est un cyclohexyle, A est CH₂CH₂, Y^{o} est un 4-amidinobenzyle et R¹ est un hydrido ;
R² est un 3-aminophényle, B est un oxalan-2-yle, A est CH₂, Y^{o} est un 4-amidinobenzyle et R¹ est un chloro ;
R² est un 3-aminophényle, B est un 1-pipéridinyle, A est CH₂CH₂, Y^{o} est un 4-amidinobenzyle et R¹ est un chloro ;
R² est un 3-aminophényle, B est un 1,1-dioxothiolan-3-yle, A est une liaison simple, Y^{o} est un 4-amidinobenzyle et R¹ est un chloro ;
R² est un 3-aminophényle, B est un 1-pyrrolidinyle, A est CH₂CH₂CH₂, Y^{o} est un 4-amidinobenzyle et R¹ est un chloro ;

14. Composition pour inhiber les manifestations thrombotiques dans le sang comprenant un composé selon l'une quelconque des revendications 5, 9 ou 13 et un support pharmaceutiquement acceptable.

15. Composition pour inhiber les manifestations thrombotiques dans le sang comprenant un composé selon l'une quelconque des revendications 1 à 4, des revendications 6 à 8, ou des revendications 10 à 12, et un support pharmaceutiquement acceptable.

16. Utilisation d'un composé selon l'une quelconque des revendications 1 à 13 ou d'un sel pharmaceutiquement acceptable de celui-ci pour la fabrication d'un médicament pour inhiber les troubles thrombotiques dans le sang.

17. Utilisation d'un composé selon l'une quelconque des revendications 1 à 13 ou d'un sel pharmaceutiquement acceptable de celui-ci pour la fabrication d'un médicament pour inhiber la formation d'agrégats de plaquettes sanguines dans le sang.

18. Utilisation d'un composé selon l'une quelconque des revendications 1 à 13 ou d'un sel pharmaceutiquement acceptable de celui-ci pour la fabrication d'un médicament pour inhiber la formation de thrombus dans le sang.

19. Utilisation d'un composé selon l'une quelconque des revendications 1 à 13 ou d'un sel pharmaceutiquement acceptable de celui-ci pour la fabrication d'un médicament pour traiter ou empêcher la thrombo-embolie veineuse et l'embolie pulmonaire chez un mammifère.

20. Utilisation d'un composé selon l'une quelconque des revendications 1 à 13 ou d'un sel pharmaceutiquement acceptable de celui-ci pour la fabrication d'un médicament pour traiter ou empêcher la thrombose veineuse profonde chez un mammifère.

21. Utilisation d'un composé selon l'une quelconque des revendications 1 à 13 ou d'un sel pharmaceutiquement acceptable de celui-ci pour la fabrication d'un médicament pour traiter ou empêcher la thrombo-embolie cardiogénique chez un mammifère.

22. Utilisation d'un composé selon l'une quelconque des revendications 1 à 13 ou d'un sel pharmaceutiquement acceptable de celui-ci pour la fabrication d'un médicament pour traiter ou empêcher l'accident thrombo-embolique chez les humains ou d'autres mammifères.

23. Utilisation d'un composé selon l'une quelconque des revendications 1 à 13 ou d'un sel pharmaceutiquement acceptable de celui-ci pour la fabrication d'un médicament pour traiter ou empêcher la thrombose associée à un cancer et à la chimiothérapie cancéreuse chez les humains et d'autres mammifères.

24. Utilisation d'un composé selon l'une quelconque des revendications 1 à 13 ou d'un sel pharmaceutiquement acceptable de celui-ci pour la fabrication d'un médicament pour traiter ou empêcher l'angor instable chez les humains et d'autres mammifères.

25. Utilisation d'un composé selon l'une quelconque des revendications 1 à 13 ou d'un sel pharmaceutiquement acceptable de celui-ci pour la fabrication d'un médicament pour inhiber la formation de thrombus dans le sang.

26. Utilisation d'un composé selon l'une quelconque des revendications 1 à 13 ou d'un sel pharmaceutiquement acceptable de celui-ci, dans la fabrication d'un médicament pour inhiber la formation de thrombus, traiter la formation de thrombus, ou empêcher la formation de thrombus chez un mammifère.
